(19) Europäisches Patentamt
European Patent Office
Office européen des brevets

(11) EP 3 882 349 A1

(12) **EUROPEAN PATENT APPLICATION**
published in accordance with Art. 153(4) EPC

(43) Date of publication:
**22.09.2021 Bulletin 2021/38**

(21) Application number: **19884507.5**

(22) Date of filing: **13.11.2019**

(51) Int Cl.:
***C12N 15/13*** *(2006.01)* ***A61K 39/395*** *(2006.01)*
***A61K 47/68*** *(2017.01)* ***A61P 35/00*** *(2006.01)*
***C07K 16/28*** *(2006.01)* ***C12N 1/15*** *(2006.01)*
***C12N 1/19*** *(2006.01)* ***C12N 1/21*** *(2006.01)*
***C12N 5/10*** *(2006.01)* ***C12N 15/63*** *(2006.01)*
***C12P 21/08*** *(2006.01)*

(86) International application number:
**PCT/JP2019/044588**

(87) International publication number:
**WO 2020/100954 (22.05.2020 Gazette 2020/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.11.2018 JP 2018214110**
**25.03.2019 JP 2019057327**

(71) Applicant: **Daiichi Sankyo Company, Limited**
**Tokyo 103-8426 (JP)**

(72) Inventors:
- **SAITO Atsuko**
**Tokyo 103-8426 (JP)**
- **HARADA Naoya**
**Tokyo 103-8426 (JP)**
- **YONEDA Kozo**
**Tokyo 103-8426 (JP)**
- **HAYAKAWA Ichiro**
**Tokyo 103-8426 (JP)**
- **MEGURO Masaki**
**Tokyo 103-8426 (JP)**
- **DOI Fuminao**
**Tokyo 103-8426 (JP)**

(74) Representative: **Fairbairn, Angus Chisholm**
**Marks & Clerk LLP**
**15 Fetter Lane**
**London EC4A 1BW (GB)**

(54) **(ANTI-CDH6 ANTIBODY)-(PYRROLOBENZODIAZEPINE DERIVATIVE) CONJUGATE**

(57) An object of the present invention is to provide an antibody specifically binding to CDH6 and having a high internalization activity, an antibody-drug conjugate comprising the antibody and a high antitumor activity, a pharmaceutical product having the antibody-drug conjugate and having a therapeutic effect on a tumor, a method for treating a tumor using the antibody, the antibody-drug conjugate or the pharmaceutical product, and the like.

According to the present invention, it is possible to provide an anti-CDH6 antibody prepared having internalization activity, an anti-CDH6 antibody-drug conjugate prepared by connecting the anti-CDH6 antibody and a novel PBD derivative and exhibiting high anti-tumor activity, a pharmaceutical product and a therapeutic method for treating tumors using these.

EP 3 882 349 A1

## Description

Technical Field

The present invention relates to an antibody-drug conjugate useful as an antitumor drug, obtained by connecting an anti-CDH6 antibody, which binds to CDH6 and has an internalization effect, and a pyrrolobenzodiazepine derivative via a linker structure moiety.

Background Art

Cadherins are glycoproteins present on the surface of cell membranes and function as cell-cell adhesion molecules through the calcium ion-dependent binding of their N-terminal extracellular domains, or as signal molecules responsible for cell-cell interaction. Classic cadherins are in the cadherin superfamily and are single-pass transmembrane proteins composed of five extracellular domains (EC domains), one transmembrane region, and an intracellular domain. The classic cadherins are classified into the type I family typified by E-cadherin and N-cadherin, and the type II family according to the homologies of their amino acid sequences.
Cadherin-6 (CDH6) is a single-pass transmembrane protein composed of 790 amino acids, which is classified into the type II cadherin family, and this protein has N-terminal extracellular and C-terminal intracellular domains. The human CDH6 gene was cloned for the first time in 1995 (Non Patent Literature 1), and its sequence can be referred to under, for example, accession Nos. NM_004932 and NP_004923 (NCBI).
CDH6 is specifically expressed in the brain and the kidney during development and has been reported to play an important role in the circuit formation of the central nervous system (Non Patent Literature 2 and 3) and nephron development in the kidney (Non Patent Literature 4 and 5). The expression of CDH6 in the normal tissues of adult humans is localized to the tubules of the kidney, bile duct epithelial cells, and the like.
Meanwhile, it is known that CDH6 is specifically overexpressed at tumor sites in some types of human adult cancers. The correlation of CDH6 expression with poor prognosis and its applicability as a tumor marker has been reported with respect to human renal cell carcinoma, particularly, renal clear cell carcinoma (Non Patent Literature 6 and 7). The high expression of CDH6 has also been reported with respect to human ovarian cancer (Non Patent Literature 8). It has also been reported that CDH6 is involved in the epithelial-mesenchymal transition of human thyroid cancer (Non Patent Literature 9). Furthermore, it has been reported that CDH6 is also expressed in human bile duct cancer and human small-cell lung cancer (Non Patent Literature 12 and 13).
Cancers rank high in causes of death. Although the number of cancer patients is expected to increase with aging of the population, treatment needs have not yet been sufficiently satisfied. The problems of conventional chemotherapeutics are that: due to their low selectivity, these chemotherapeutics are toxic not only to tumor cells but also to normal cells and thereby have adverse reactions; and the chemotherapeutics cannot be administered in sufficient amounts and thus cannot produce their effects to a sufficient degree. Hence, in recent years, more highly selective molecular target drugs or antibody drugs have been developed, which target molecules that exhibit mutations or a high expression characteristic in cancer cells, or specific molecules involved in malignant transformation of cells.
Antibodies are highly stable in blood, and specifically bind to their target antigens. For these reasons, a reduction in adverse reactions is expected, and a large number of antibody drugs have been developed for molecules highly expressed on the surface of cancer cells. One of the techniques that relies on the antigen-specific binding ability of antibodies is the use of an antibody-drug conjugate (ADC). An ADC is a conjugate in which an antibody that binds to an antigen expressed on the surface of cancer cells and can internalize the antigen into the cell through such binding is conjugated to a drug having cytotoxic activity. An ADC can efficiently deliver the drug to cancer cells, and can thereby be expected to kill the cancer cells by accumulating the drug in the cancer cells (Non Patent Literature 10 and Patent Literature 1 and 2). With regard to ADCs, Adcetris(TM) (brentuximab vedotin) comprising an anti-CD30 monoclonal antibody conjugated to monomethyl auristatin E has, for example, been approved as a therapeutic drug for Hodgkin's lymphoma and anaplastic large cell lymphoma. Also, Kadcyla(TM) (trastuzumab emtansine) comprising an anti-HER2 monoclonal antibody conjugated to emtansine is used in the treatment of HER2-positive progressive or recurrent breast cancer.
The features of a target antigen suitable for an ADC as an antitumor drug are that: the antigen is specifically highly expressed on the surface of cancer cells but has low expression or is not expressed in normal cells; the antigen can be internalized into cells; the antigen is not secreted from the cell surface; etc. Furthermore, the important features of an antibody suitable for an ADC are that the antibody has high internalization ability in addition to specifically binding to the target antigen. The internalization ability of the antibody depends on the properties of both the target antigen and the antibody. It is difficult to predict an antigen-binding site suitable for internalization from the molecular structure of a target or to predict an antibody having high internalization ability from binding strength, physical properties, and the like of the antibody. Hence, an important challenge in developing an ADC having high efficacy is obtaining an antibody

having high internalization ability against the target antigen (Non Patent Literature 11).

An ADC comprising DM4 conjugated to an anti-CDH6 antibody specifically binding to EC domain 5 (EC5) of CDH6 is known as an ADC targeting CDH6 (Patent Literature 3).

Useful examples of drugs to be conjugated for ADCs are pyrrolobenzodiazepines (PBDs). PBDs exhibit cytotoxicity, for example, by binding to the PuGPu sequence in the DNA minor groove. Anthramycin, a naturally-occurring PBD, was first discovered in 1965, and since this discovery various naturally-occurring PBDs and PBD analogs thereof have been discovered (Non Patent Literatures 14 to 17).

The general structural formula of PBDs is represented by the following formula:

[Formula 1]

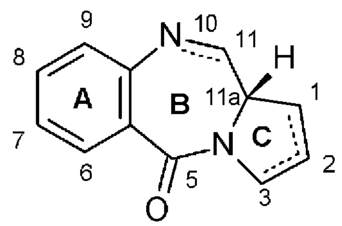

Known are PBDs different in the number of, types of, and sites of substituents in the A and C ring parts, and those different in degree of unsaturation in the B and C ring parts.

PBDs are known to exhibit dramatically enhanced cytotoxicity through the formation of a dimer structure (Non Patent Literatures 18, 19), and various ADCs with a dimer PBD have been reported (Patent Literatures 4 to 16). However, a PBD having a spiro ring at its C2-position and an ADC form thereof have not been known.

Citation List

Patent Literatures

Patent Literature 1: WO2014/057687
Patent Literature 2: US2016/0297890
Patent Literature 3: WO2016/024195
Patent Literature 4: WO2013/173496
Patent Literature 5: WO2014/130879
Patent Literature 6: WO2017/004330
Patent Literature 7: WO2017/004025
Patent Literature 8: WO2017/020972
Patent Literature 9: WO2016/036804
Patent Literature 10: WO2015/095124
Patent Literature 11: WO2015/052322
Patent Literature 12: WO2015/052534
Patent Literature 13: WO2016/115191
Patent Literature 14: WO2015/052321
Patent Literature 15: WO2015/031693
Patent Literature 16: WO2011/130613


Non Patent Literatures

Non Patent Literature 1: Shimoyama Y, et al., Cancer Research, 2206-2211, 55, May 15, 1995
Non Patent Literature 2: Inoue T, et al., Developmental Biology, 183-194, 1997
Non Patent Literature 3: Osterhout J A, et al., Neuron, 632-639, 71, Aug 25, 2011
Non Patent Literature 4: Cho E A, et al., Development, 803-812, 125, 1998
Non Patent Literature 5: Mah S P, et al., Developmental Biology, 38-53, 223, 2000

Non Patent Literature 6: Paul R, et al., Cancer Research, 2741-2748, July 1, 57, 1997
Non Patent Literature 7: Shimazui T, et al., Cancer, 963-968, 101(5), Sep.1, 2004
Non Patent Literature 8: Koebel M, et al., PLoS Medicine, 1749-1760, 5(12), e232, Dec. 2008
Non Patent Literature 9: Gugnoni M, et al., Oncogene, 667-677, 36, 2017
Non Patent Literature 10: Polakis P., Pharmacological Reviews, 3-19, 68, 2016
Non Patent Literature 11: Peters C, et al., Bioscience Reports, 1-20, 35, 2015
Non Patent Literature 12: Goeppert B, et al., Epigenetics, 780-790, 11(11), 2016
Non Patent Literature 13: Yokoi S, et al., American Journal of Pathology, 207-216, 161, 1, 2002
Non Patent Literature 14: Julia Mantaj, et al., Angewandte Chemie Internationl Edition 2016, 55, 2-29
Non Patent Literature 15: Dyeison Antonow.et al., Chemical Reviews 2010, 111, 2815-2864
Non Patent Literature 16: In Antibiotics III. Springer Verlag, New York, pp.3-11
Non Patent Literature 17: Accounts of Chemical Research 1986, 19, 230
Non Patent Literature 18: Journal of the American Chemical Society 1992, 114, 4939
Non Patent Literature 19: Journal of Organic Chemistry 1996, 61, 8141

## Summary of Invention

### Technical Problem

It is an object of the present invention to provide an antibody specifically binding to CDH6 and having high internalization activity, an antibody-drug conjugate comprising the antibody and having high antitumor activity, a pharmaceutical product comprising the antibody-drug conjugate and having therapeutic effects on a tumor, a method for treating a tumor using the antibody, the antibody-drug conjugate or the pharmaceutical product, and the like.

### Solution to Problem

The present inventors have conducted intensive studies directed towards achieving the above-described object, and found that, surprisingly, an antibody specifically binding to extracellular domain 3 (in the present description, also referred to as EC3) of CDH6 has exceedingly high internalization activity against cells expressing CDH6 and is useful as an antibody for ADCs. The present inventors also succeeded in obtaining an anti-CDH6 antibody-drug conjugate by connecting the anti-CDH6 antibody to a novel PBD derivative, and found that the anti-CDH6 antibody-drug conjugate has strong antitumor activity.

The present invention relates to the following.

[1] An antibody-drug conjugate represented by the following formula (X):

[Formula 2]

$$Ab\left[(N297\ glycan)\left[L-D\right]_{m^2}\right]_2 \qquad (X)$$

wherein $m^2$ represents an integer of 1 or 2,
Ab represents an IgG antibody specifically binding to an amino acid sequence comprising the amino acid sequence shown in SEQ ID NO: 4 and having internalization ability that permits cellular uptake, or a functional fragment thereof,
L represents a linker linking a glycan bonding to N297 of Ab (N297 glycan) and D,
N297 glycan may be a remodeled glycan, and
D is any one of the following formulas:

[Formula 3]

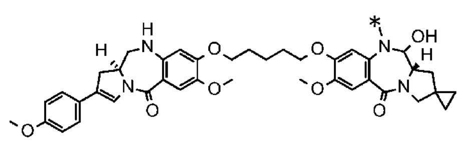
,
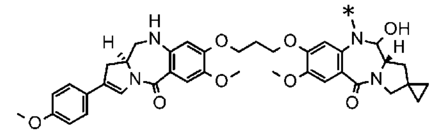
,
(XI)
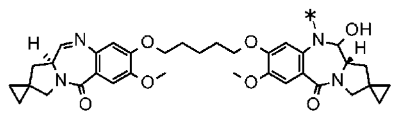
or
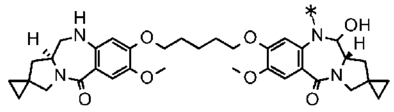

wherein the asterisk (*) represents bonding to L.

[2] The antibody-drug conjugate according to [1], wherein the antibody or a functional fragment thereof exhibits competitive inhibitory activity, for binding to the amino acid sequence comprising the amino acid sequence shown in SEQ ID NO: 4, against at least any one antibody selected from the group consisting of the following antibodies (1) to (8):

(1) an antibody having a light chain consisting of the amino acid sequence at positions 21 to 233 in SEQ ID NO: 23 and a heavy chain consisting of the amino acid sequence at positions 20 to 471 in SEQ ID NO: 26,
(2) an antibody having a light chain consisting of the amino acid sequence at positions 21 to 233 in SEQ ID NO: 31 and a heavy chain consisting of the amino acid sequence at positions 20 to 471 in SEQ ID NO: 39,
(3) an antibody having a light chain consisting of the amino acid sequence at positions 21 to 233 in SEQ ID NO: 31 and a heavy chain consisting of the amino acid sequence at positions 20 to 471 in SEQ ID NO: 43,
(4) an antibody having a light chain consisting of the amino acid sequence at positions 21 to 233 in SEQ ID NO: 35 and a heavy chain consisting of the amino acid sequence at positions 20 to 471 in SEQ ID NO: 43,
(5) an antibody having a light chain consisting of the amino acid sequence at positions 21 to 233 in SEQ ID NO: 31 and a heavy chain consisting of the amino acid sequence at positions 20 to 471 in SEQ ID NO: 47,
(6) an antibody having a light chain consisting of the amino acid sequence at positions 21 to 233 in SEQ ID NO: 31 and a heavy chain consisting of the amino acid sequence at positions 20 to 471 in SEQ ID NO: 65,
(7) an antibody having a light chain consisting of the amino acid sequence at positions 21 to 233 in SEQ ID NO: 31 and a heavy chain consisting of the amino acid sequence at positions 20 to 471 in SEQ ID NO: 67, and
(8) an antibody having a light chain consisting of the amino acid sequence at positions 21 to 233 in SEQ ID NO: 31 and a heavy chain consisting of the amino acid sequence at positions 20 to 471 in SEQ ID NO: 69.

[3] The antibody-drug conjugate according to [1] or [2], wherein the antibody or a functional fragment thereof comprises CDRL1, CDRL2 and CDRL3; and CDRH1, CDRH2 and CDRH3 selected from the group consisting of the following (1) to (4):

(1) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and CDRH1 consisting of the amino acid sequence shown in SEQ ID NO: 17, CDRH2 consisting of the amino acid sequence shown in SEQ ID NO: 18 and CDRH3 consisting of the amino acid sequence shown in SEQ ID NO: 19,
(2) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and CDRH1 consisting of the amino acid sequence shown in SEQ ID NO: 17, CDRH2 consisting of the amino acid sequence shown in SEQ ID NO: 30 and CDRH3 consisting of the amino acid sequence shown in SEQ ID NO: 19,
(3) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and CDRH1 consisting of the amino acid sequence shown in SEQ ID NO: 57, CDRH2 consisting of the amino acid sequence shown in SEQ ID NO: 58 and CDRH3 consisting of the amino acid sequence shown in SEQ ID NO: 59, and

(4) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14 and CDRH1 consisting of the amino acid sequence shown in SEQ ID NO: 62, CDRH2 consisting of the amino acid sequence shown in SEQ ID NO: 63 and CDRH3 consisting of the amino acid sequence shown in SEQ ID NO: 64.

[4] The antibody-drug conjugate according to any one of [1] to [3], wherein the antibody or a functional fragment thereof comprises any one light chain variable region selected from the group consisting of the following variable regions (1) to (4):

(1) a light chain variable region consisting of the amino acid sequence shown in SEQ ID NO: 33,
(2) a light chain variable region consisting of the amino acid sequence shown in SEQ ID NO: 37,
(3) an amino acid sequence having a sequence identity of at least 95% to the sequence of a framework region except CDR sequences in the amino acid sequence of (1) or (2) and
(4) an amino acid sequence having a deletion, substitution or addition of one or several amino acids in the sequence of a framework region except CDR sequences in the amino acid sequences of (1) to (3); and

any one heavy chain variable region selected from the group consisting of the following variable regions (5) to (11) :

(5) a heavy chain variable region consisting of the amino acid sequence shown in SEQ ID NO: 41,
(6) a heavy chain variable region consisting of the amino acid sequence shown in SEQ ID NO: 45,
(7) a heavy chain variable region consisting of the amino acid sequence shown in SEQ ID NO: 49,
(8) a heavy chain variable region consisting of the amino acid sequence shown in SEQ ID NO: 55,
(9) a heavy chain variable region consisting of the amino acid sequence shown in SEQ ID NO: 60,
(10) an amino acid sequence having a sequence identity of at least 95% to the sequence of a framework region other than at each CDR sequence in the amino acid sequences of (5) to (9), and
(11) an amino acid sequence having a deletion, substitution or addition of one or several amino acids in the sequence of a framework region other than at each CDR sequence in the amino acid sequences of (5) to (10).

[5] The antibody-drug conjugate according to any one of [1] to [4], wherein the antibody or a functional fragment thereof comprises any one of the following (1) to (6):

(1) a light chain variable region consisting of the amino acid sequence shown in SEQ ID NO: 33 and a heavy chain variable region consisting of the amino acid sequence shown in SEQ ID NO: 41,
(2) a light chain variable region consisting of the amino acid sequence shown in SEQ ID NO: 33 and a heavy chain variable region consisting of the amino acid sequence shown in SEQ ID NO: 45,
(3) a light chain variable region consisting of the amino acid sequence shown in SEQ ID NO: 37 and a heavy chain variable region consisting of the amino acid sequence shown in SEQ ID NO: 45,
(4) a light chain variable region consisting of the amino acid sequence shown in SEQ ID NO: 33 and a heavy chain variable region consisting of the amino acid sequence shown in SEQ ID NO: 49,
(5) a light chain variable region consisting of the amino acid sequence shown in SEQ ID NO: 33 and a heavy chain variable region consisting of the amino acid sequence shown in SEQ ID NO: 55, and
(6) a light chain variable region consisting of the amino acid sequence shown in SEQ ID NO: 33 and a heavy chain variable region consisting of the amino acid sequence shown in SEQ ID NO: 60.

[6] The antibody-drug conjugate according to any one of [1] to [5], wherein the antibody or a functional fragment thereof has any one of the following (1) to (7):

(1) a light chain consisting of the amino acid sequence at positions 21 to 233 in SEQ ID NO: 31 and a heavy chain consisting of the amino acid sequence at positions 20 to 471 in SEQ ID NO: 39,
(2) a light chain consisting of the amino acid sequence at positions 21 to 233 in SEQ ID NO: 31 and a heavy chain consisting of the amino acid sequence at positions 20 to 471 in SEQ ID NO: 43,
(3) a light chain consisting of the amino acid sequence at positions 21 to 233 in SEQ ID NO: 35 and a heavy chain consisting of the amino acid sequence at positions 20 to 471 in SEQ ID NO: 43,
(4) a light chain consisting of the amino acid sequence at positions 21 to 233 in SEQ ID NO: 31 and a heavy chain consisting of the amino acid sequence at positions 20 to 471 in SEQ ID NO: 47,
(5) a light chain consisting of the amino acid sequence at positions 21 to 233 in SEQ ID NO: 31 and a heavy chain consisting of the amino acid sequence at positions 20 to 471 in SEQ ID NO: 65,

(6) a light chain consisting of the amino acid sequence at positions 21 to 233 in SEQ ID NO: 31 and a heavy chain consisting of the amino acid sequence at positions 20 to 471 in SEQ ID NO: 67, and
(7) a light chain consisting of the amino acid sequence at positions 21 to 233 in SEQ ID NO: 31 and a heavy chain consisting of the amino acid sequence at positions 20 to 471 in SEQ ID NO: 69.

[7] The antibody-drug conjugate according to [6], wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 39.
[8] The antibody-drug conjugate according to [6], wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 43.
[9] The antibody-drug conjugate according to [6], wherein the antibody or a functional fragment thereof has a light chain consisting of the amino acid sequence at positions 21 to 233 in SEQ ID NO: 35 and a heavy chain consisting of the amino acid sequence at positions 20 to 471 in SEQ ID NO: 43.
[10] The antibody-drug conjugate according to [6], wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 47.
[11] The antibody-drug conjugate according to [6], wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 65
[12] The antibody-drug conjugate according to [6], wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 67.
[13] The antibody-drug conjugate according to [6], wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 69.
[14] The antibody-drug conjugate according to any one of [1] to [13], wherein

L is represented by -Lb-La-Lp-NH-B-$CH_2$-O(C=O)-*, wherein the asterisk * represents bonding to the nitrogen atom at the N10'-position of D,
B represents a 1,4-phenyl group, a 2,5-pyridyl group, a 3,6-pyridyl group, a 2,5-pyrimidyl group or a 2,5-thienyl group,
Lp represents any one selected from the group consisting of:
-GGVA-, -GG-(D-)VA-, -VA-, -GGFG-, -GGPI-, -GGVCit-, - GGVK- and -GGPL-,
La represents any one selected from the group consisting of:

- C(=O)-$CH_2CH_2$-C(=O)-,
- C(=O)-$(CH_2CH_2)_2$-C(=O)-,
- C(=O)-$CH_2CH_2$-C(=O)-NH-$(CH_2CH_2)_2$-C(=O)-,
- C(=O)-$CH_2CH_2$-C(=O)-NH-$(CH_2CH_2O)_2$-$CH_2$-C(=O)-,
- C(=O)-$CH_2CH_2$-NH-C(=O)-$(CH_2CH_2O)_4$-$CH_2CH_2$-C(=O)-,
- $CH_2$-OC(=O)- and
- OC(=O)-,

and
Lb represents the following formula:

[Formula 4]

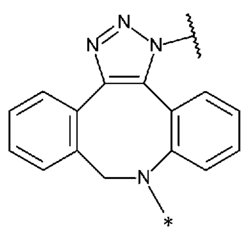

or

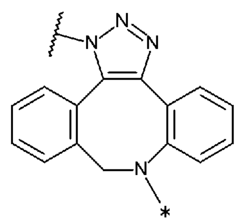

[Formula 5]

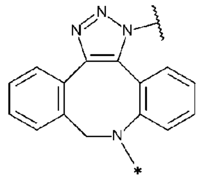

or

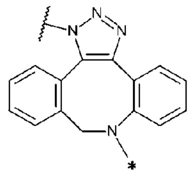

or

[Formula 6]

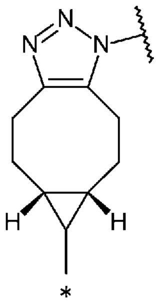

or

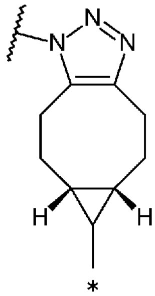

wherein, in each structural formula of Lb shown above, each asterisk * represents bonding to La and each wavy line represents bonding to the glycan presented by Ab or a remodeled glycan.

[15] The antibody-drug conjugate according to any one of [1] to [14], wherein

L represents any one selected from the group consisting of the following structural formulas:

- $Z^1$-C(=O)-$CH_2CH_2$-C(=O)-GGVA-NH-B-$CH_2$-OC(=O)-,
- $Z^1$-C(=O)-$CH_2CH_2$-C(=O)-GG-(D-)VA-NH-B-$CH_2$-OC(=O)-,
- $Z^1$-C(=O)-$CH_2CH_2$-C(=O)-VA-NH-B-$CH_2$-OC(=O)-,
- $Z^1$-C(=O)-$(CH_2CH_2)_2$-C(=O)-VA-NH-B-$CH_2$-OC(=O)-,
- $Z^1$-C(=O)-$CH_2CH_2$-C(=O)-GGPI-NH-B-$CH_2$-OC(=O)-,
- $Z^1$-C(=O)-$CH_2CH_2$-C(=O)-GGFG-NH-B-$CH_2$-OC(=O)-,
- $Z^1$-C(=O)-$CH_2CH_2$-C(=O)-GGVCit-NH-B-$CH_2$-OC(=O)-,
- $Z^1$-C(=O)-$CH_2CH_2$-C(=O)-GGVK-NH-B-$CH_2$-OC(=O)-,
- $Z^1$-C(=O)-$CH_2CH_2$-C(=O)-GGPL-NH-B-$CH_2$-OC(=O)-,
- $Z^1$-C(=O)-$CH_2CH_2$-C(=O)-NH-$(CH_2CH_2)_2$-C(=O)-VA-NH-B-$CH_2$-OC(=O)-,
- $Z^1$-C(=O)-$CH_2CH_2$-C(=O)-NH-$(CH_2CH_2O)_2$-$CH_2$-C(=O)-VA-NH-B-$CH_2$-OC(=O)-,
- $Z^1$-C(=O)-$CH_2CH_2$-NH-C(=O)-$(CH_2CH_2O)_4$-$CH_2CH_2$-C(=O)-VA-NH-B-$CH_2$-OC(=O)-,
- $Z^2$-OC(=O)-GGVA-NH-B-$CH_2$-OC(=O) - and
- $Z^3$-$CH_2$-OC(=O)-GGVA-NH-B-$CH_2$-OC(=O) -

wherein $Z^1$ represents the following structural formula:

[Formula 7]

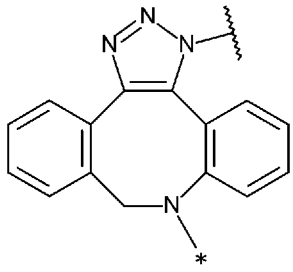

or

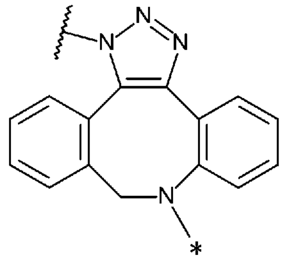

$Z^2$ represents the following structural formula:

[Formula 8]

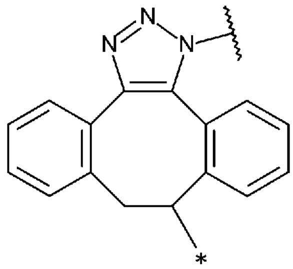

or

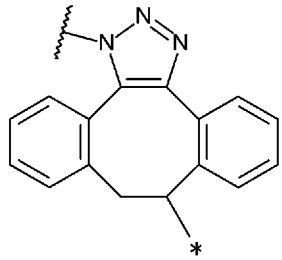

$Z^3$ represents the following structural formula:

[Formula 9]

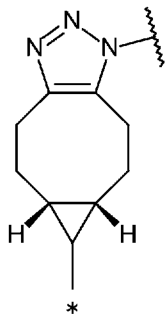

or

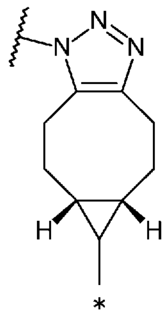

wherein in each structural formula for $Z^1$, $Z^2$ and $Z^3$, each asterisk * represents bonding to the C(=O), O or $CH_2$ neighboring $Z^1$, $Z^2$ or $Z^3$; each wavy line represents bonding to the glycan presented by Ab or a remodeled glycan, and
B represents a 1,4-phenyl group.

[16] The antibody-drug conjugate according to any one of [1] to [15], wherein

L represents any one selected from the group consisting of:

- $Z^1$-C(=O)-$CH_2CH_2$-C(=O)-GGVA-NH-B-$CH_2$-OC(=O)-,
- $Z^1$-C(=O)-$CH_2CH_2$-C(=O)-VA-NH-B-$CH_2$-OC(=O)-,
- $Z^1$-C(=O)-$(CH_2CH_2)_2$-C(=O)-VA-NH-B-$CH_2$-OC(=O)-,
- $Z^1$-C(=O)-$CH_2CH_2$-C(=O)-GGVCit-NH-B-$CH_2$-OC(=O)-,
- $Z^1$-C(=O)-$CH_2CH_2$-C(=O)-NH-$(CH_2CH_2)_2$-C(=O)-VA-NH-B-$CH_2$-OC(=O)-,
- $Z^1$-C(=O)-$CH_2CH_2$-C(=O)-NH-$(CH_2CH_2O)_2$-$CH_2$-C(=O)-VA-NH-B-$CH_2$-OC(=O)- and

- $Z^1$-C(=O)-$CH_2CH_2$-NH-C(=O)-$(CH_2CH_2O)_4$-$CH_2CH_2$-C(=O)-VA-NH-B-$CH_2$-OC(=O)-,

wherein B represents a 1,4-phenyl group, and $Z^1$ represents the following structural formula:

[Formula 10]

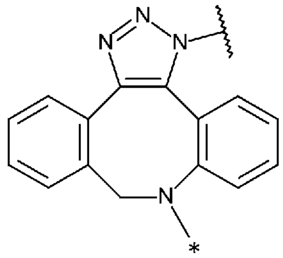

or

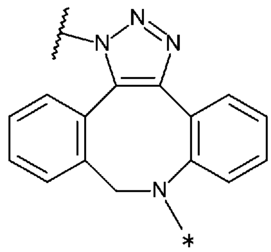

wherein, in the structural formula for $Z^1$, each asterisk * represents bonding to the C(=O) neighboring $Z^1$, and each wavy line represents bonding to the glycan bonding to N297 of Ab (N297 glycan) or bonding to a remodeled glycan.

[17] The antibody-drug conjugate according to any one of [1] to [16], wherein the antibody is IgG1, IgG2 or IgG4.
[18] The antibody-drug conjugate according to any one of [1] to [17], wherein the N297 glycan is a remodeled glycan.
[19] The antibody-drug conjugate according to any one of [1] to [18], wherein the N297 glycan is N297-(Fuc)MSG1, N297-(Fuc)MSG2, or a mixture thereof, or N297-(Fuc)SG, with N297-(Fuc)MSG1, N297-(Fuc)MSG2, and N297-(Fuc)SG having structures represented by the following formulas:

[Formula 11]

Fucα1
|
6
Galβ1-4GlcNAcβ1-2Manα1— 6
Manβ1-4GlcNAcβ1-4GlcNAcβ1-⊰-
*—L(PEG)-NeuAcα2-6Galβ1-4GlcNAcβ1-2Manα1— 3

[N297-(Fuc)MSG1]

[Formula 12]

Fucα1
|
6
* - L(PEG)-NeuAcα2-6Galβ1-4GlcNAcβ1-2Manα1— 6
Manβ1-4GlcNAcβ1-4GlcNAcβ1-⊰-
Galβ1-4GlcNAcβ1-2Manα1— 3

[N297-(Fuc)MSG2]

[Formula 13]

Fucα1
|
6
*- L(PEG)-NeuAcα2-6Galβ1-4GlcNAcβ1-2Manα1— 6
Manβ1-4GlcNAcβ1-4GlcNAcβ1-⊰-
*- L(PEG)-NeuAcα2-6Galβ1-4GlcNAcβ1-2Manα1— 3

[N297-(Fuc)SG]

wherein the wavy line represents bonding to Asn297 of the antibody,
*-L(PEG)- in the N297 glycan represents *-$(CH_2CH_2$-O)$n^5$-$CH_2CH_2$-NH-, wherein $n^5$ represents an integer of 2

to 5, the amino group at the right end is bound via an amide bond to the carboxylic acid at the 2-position of the sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the individual structural formulas.

[20] The antibody-drug conjugate according to [19], wherein $n^5$ represents 3.
[21] An antibody-drug conjugate represented by the following formula (XII):

[Formula 14]

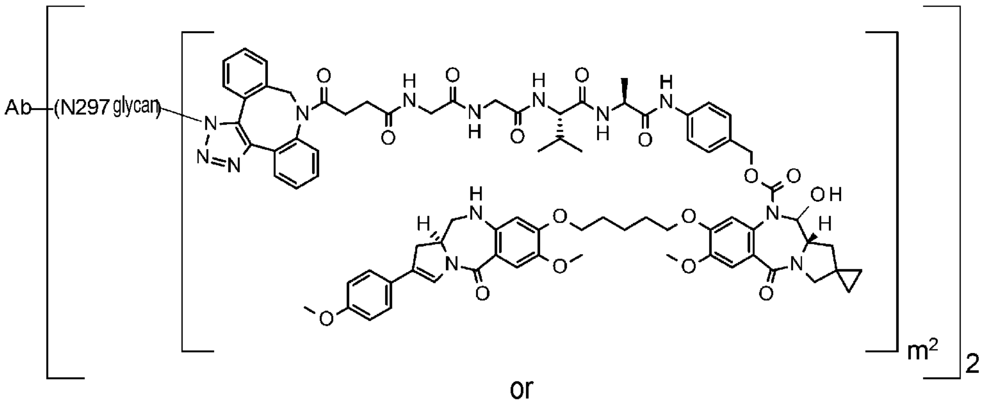

or

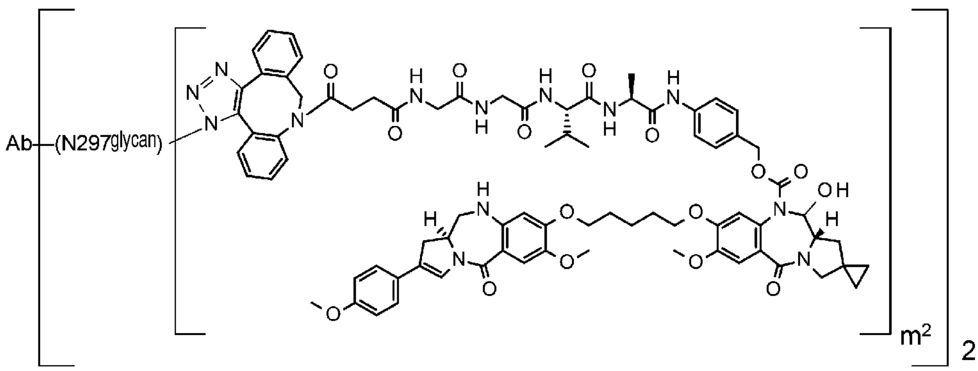

(XII)

wherein, in each structural formula shown above,

$m^2$ represents an integer of 1 or 2,
Ab represents an IgG antibody specifically binding to an amino acid sequence comprising the amino acid sequence shown in SEQ ID NO: 4 and having internalization ability that permits cellular uptake, or a functional fragment thereof,
the N297 glycan of Ab represents any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 15]

Fucα1
|
6
Galβ1-4GlcNAcβ1-2Manα1— 6
Manβ1-4GlcNAcβ1-4GlcNAcβ1—
* —L(PEG)-NeuAcα2-6Galβ1-4GlcNAcβ1-2Manα1— 3

[N297-(Fuc)MSG1]

[Formula 16]

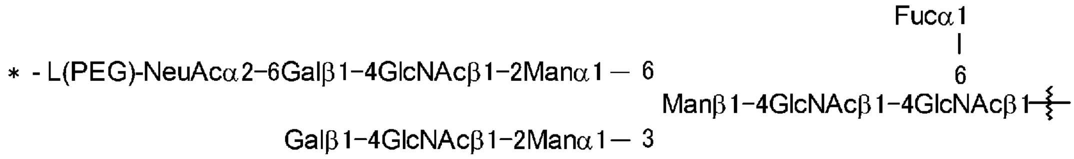

[N297-(Fuc)MSG2]

[Formula 17]

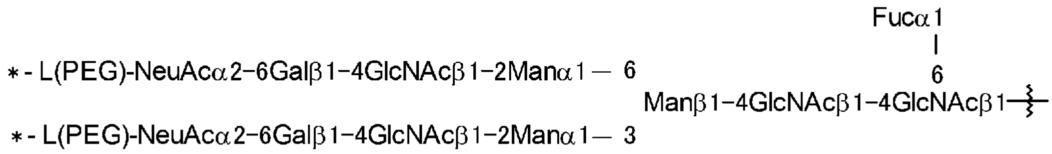

[N297-(Fuc)SG]

wherein each wavy line represents bonding to Asn297 (N297) of the antibody,

*-L(PEG)- in the N297 glycan represents *-$(CH_2CH_2\text{-}O)_3\text{-}CH_2CH_2$-NH-, wherein the amino group at the right end is bound via an amide bond to the carboxylic acid at the 2-position of the sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the corresponding structural formula.

[22] The antibody-drug conjugate according to [21], wherein the compound represented by formula (XII) is represented by the following formula (XII'):

[Formula 18]

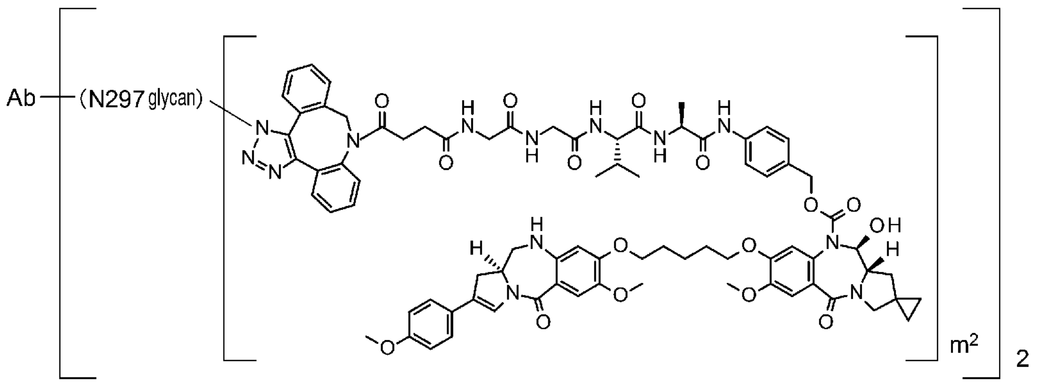

or

(XII')

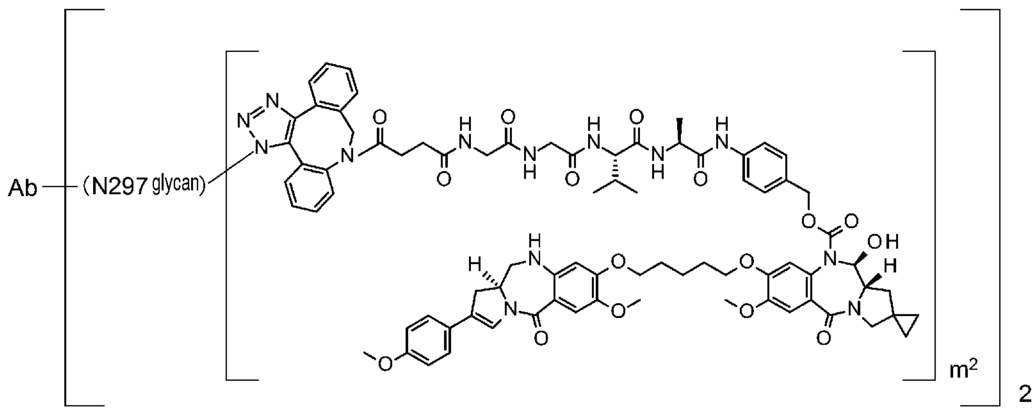

[23] An antibody-drug conjugate represented by the following formula (XIII):

[Formula 19]

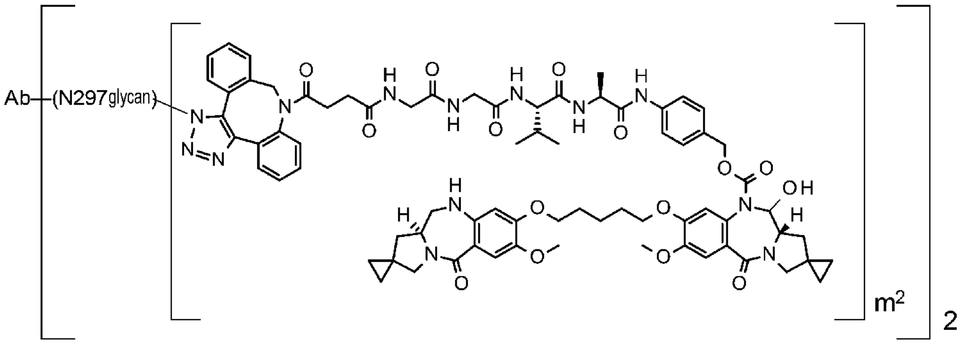

or

(XIII)

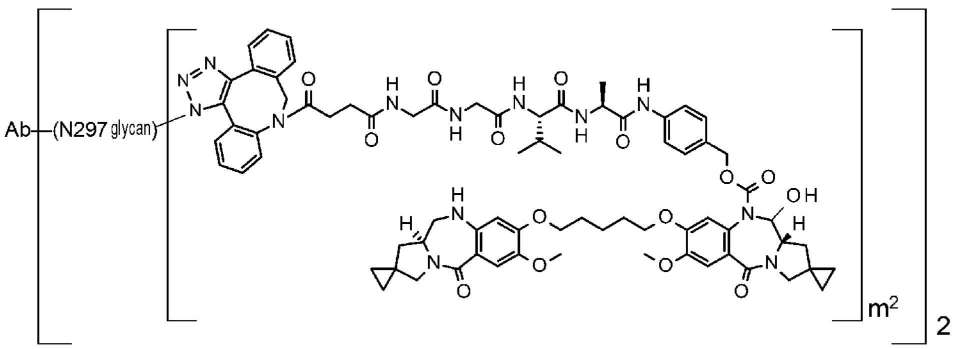

wherein, in each structural formula shown above,

$m^2$ represents an integer of 1 or 2,

Ab represents an IgG antibody specifically binding to an amino acid sequence comprising the amino acid sequence shown in SEQ ID NO: 4 and having internalization ability that permits cellular uptake, or a functional fragment thereof, and

the N297 glycan of Ab represents any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 20]

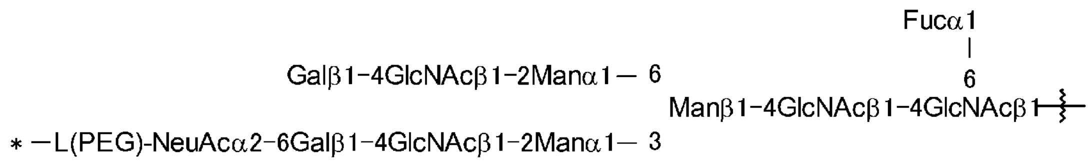

[N297-(Fuc)MSG1]

[Formula 21]

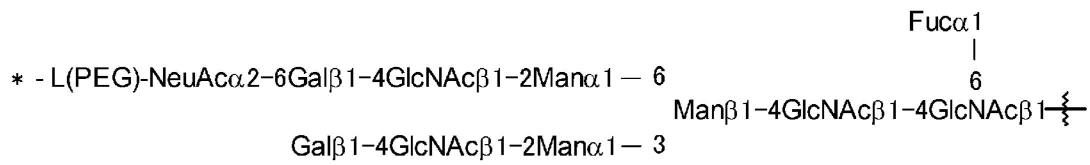

[N297-(Fuc)MSG2]

[Formula 22]

Fucα1
|
6

∗- L(PEG)-NeuAcα2−6Galβ1−4GlcNAcβ1−2Manα1— 6

Manβ1−4GlcNAcβ1−4GlcNAcβ1—

∗- L(PEG)-NeuAcα2−6Galβ1−4GlcNAcβ1−2Manα1— 3

[N297-(Fuc)SG]

wherein each wavy line represents bonding to Asn297 (N297) of the antibody, *-L(PEG)- in the N297 glycan represents *-$(CH_2CH_2\text{-}O)_3\text{-}CH_2CH_2\text{-}NH$-, wherein the amino group at the right end is bound via an amide bond to the carboxylic acid at the 2-position of the sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the individual structural formulas.

[24] The antibody-drug conjugate according to [23], wherein the compound represented by formula (XIII) is represented by the following formula (XIII'):

[Formula 23]

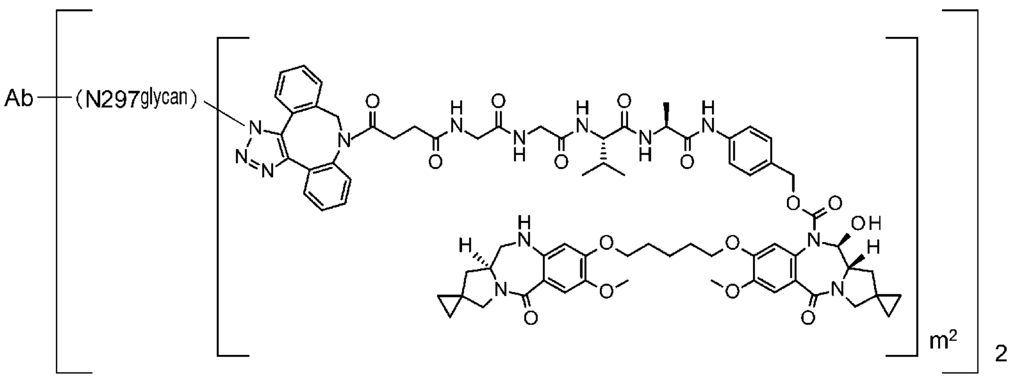

or

(XIII')

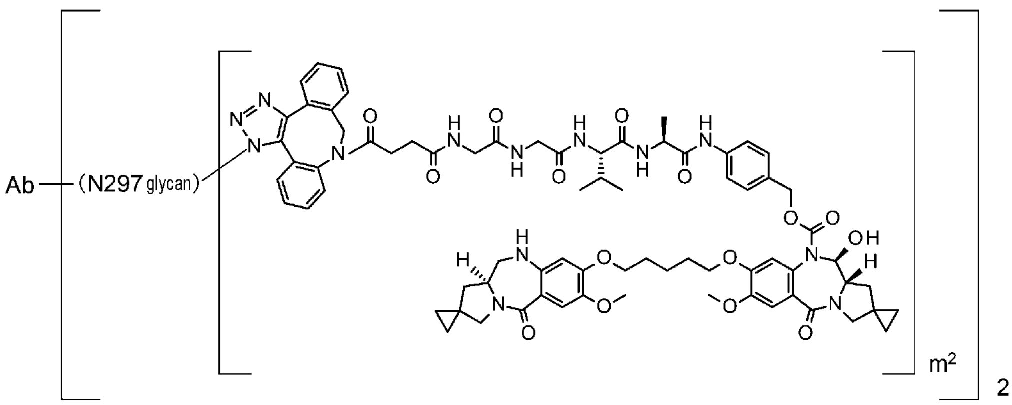

[25] An antibody-drug conjugate represented by the following formula (XIV):

[Formula 24]

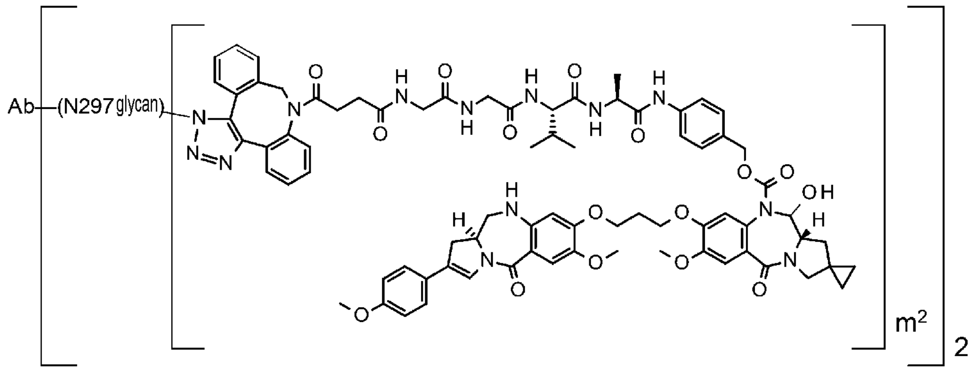

or

(XIV)

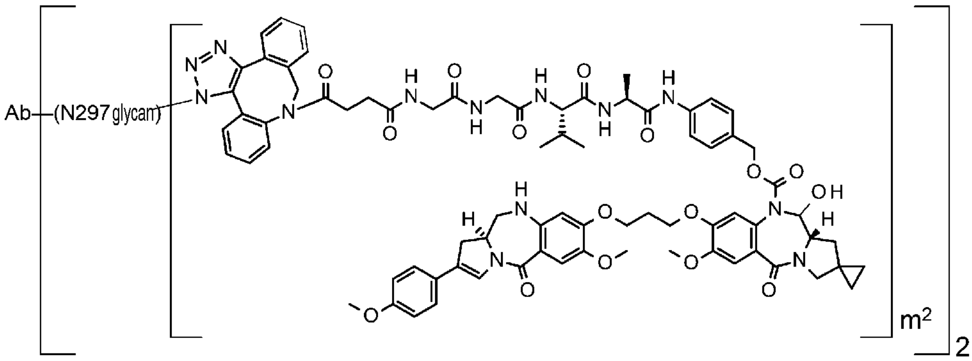

wherein, in each structural formula shown above,

$m^2$ represents an integer of 1 or 2,
Ab represents an IgG antibody specifically binding to an amino acid sequence comprising the amino acid sequence shown in SEQ ID NO: 4 and having internalization ability that permits cellular uptake, or a functional fragment thereof, and
the N297 glycan of Ab represents any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 25]

Fucα1
|
6
Galβ1−4GlcNAcβ1−2Manα1— 6
Manβ1−4GlcNAcβ1−4GlcNAcβ1—
* —L(PEG)-NeuAcα2−6Galβ1−4GlcNAcβ1−2Manα1— 3

[N297-(Fuc)MSG1]

[Formula 26]

Fucα1
|
6
* - L(PEG)-NeuAcα2−6Galβ1−4GlcNAcβ1−2Manα1 — 6
Manβ1−4GlcNAcβ1−4GlcNAcβ1—
Galβ1−4GlcNAcβ1−2Manα1— 3

[N297-(Fuc)MSG2]

[Formula 27]

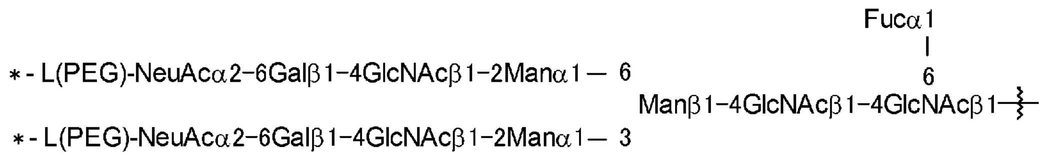

[N297-(Fuc)SG]

wherein each wavy line represents bonding to Asn297 (N297) of the antibody,
*-L(PEG)- in the N297 glycan represents *-$(CH_2CH_2-O)_3-CH_2CH_2-NH-$, wherein the amino group at the right end is bound via an amide bond to the carboxylic acid at the 2-position of the sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the individual structural formulas.

[26] The antibody-drug conjugate according to [25], wherein the compound represented by formula (XIV) is represented by the following formula (XIV'):

[Formula 28]

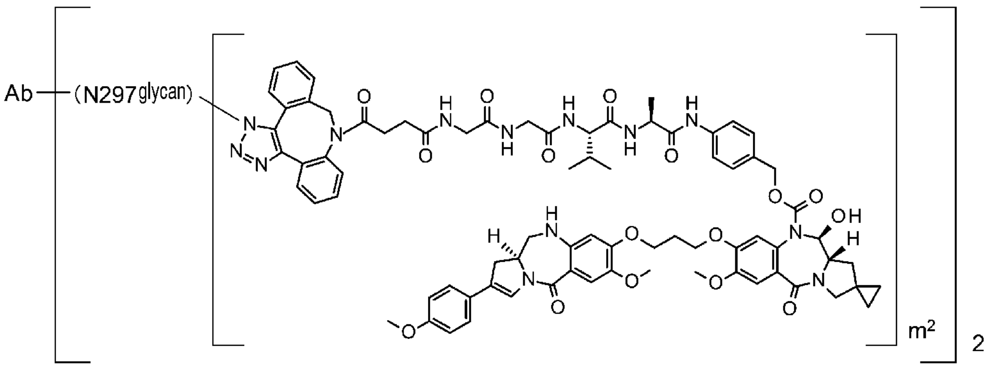

or

(XIV')

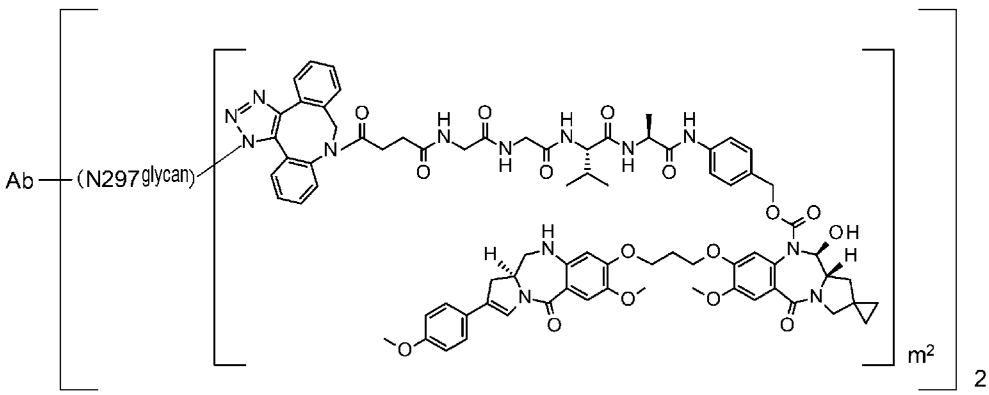

[27] An antibody-drug conjugate represented by the following formula (XV):

[Formula 29]

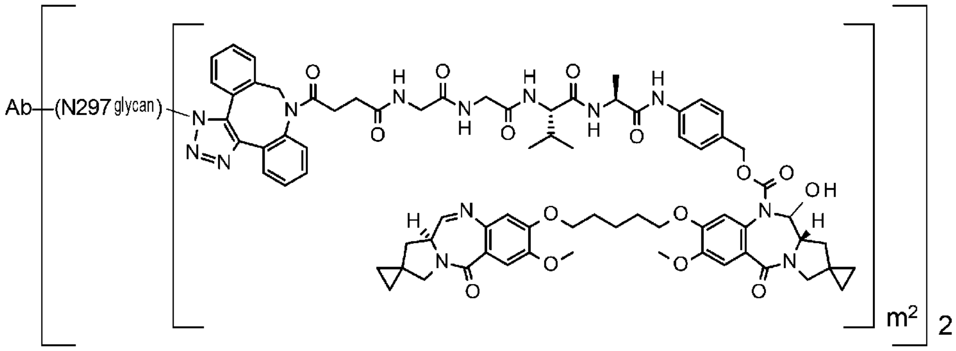

or

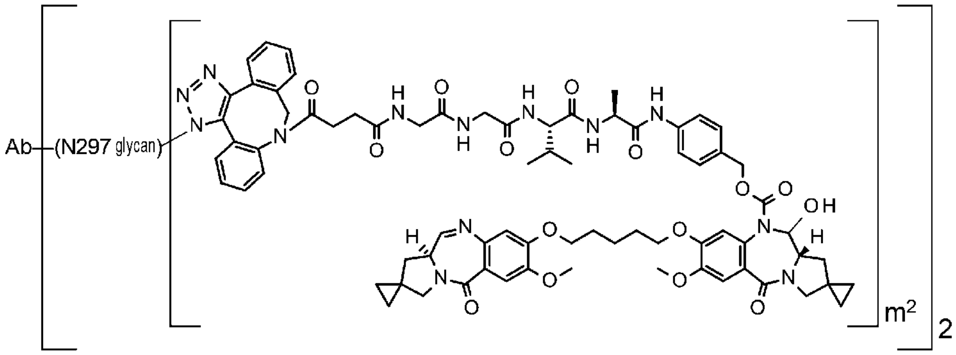

(XV)

wherein, in each structural formula shown above,

$m^2$ represents an integer of 1 or 2,
Ab represents an IgG antibody specifically binding to an amino acid sequence comprising the amino acid sequence shown in SEQ ID NO: 4 and having internalization ability that permits cellular uptake, or a functional fragment thereof, and
the N297 glycan of Ab represents any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 30]

Fucα1
|
6
Galβ1-4GlcNAcβ1-2Manα1— 6
Manβ1-4GlcNAcβ1-4GlcNAcβ1—
∗ —L(PEG)-NeuAcα2-6Galβ1-4GlcNAcβ1-2Manα1— 3

[N297-(Fuc)MSG1]

[Formula 31]

Fucα1
|
6
∗ - L(PEG)-NeuAcα2-6Galβ1-4GlcNAcβ1-2Manα1— 6
Manβ1-4GlcNAcβ1-4GlcNAcβ1—
Galβ1-4GlcNAcβ1-2Manα1— 3

[N297-(Fuc)MSG2]

[Formula 32]

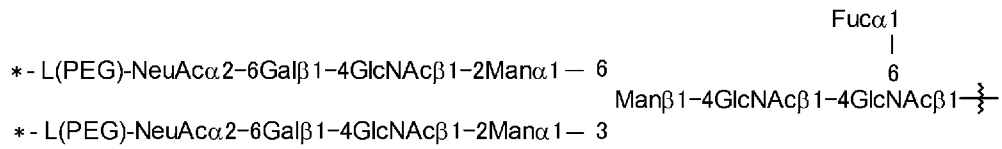

[N297-(Fuc)SG]

wherein each wavy line represents bonding to Asn297 (N297) of the antibody,
*-L(PEG)- in the N297 glycan represents *-$(CH_2CH_2\text{-}O)_3\text{-}CH_2CH_2\text{-}NH$-, wherein the amino group at the right end is bound via an amide bond to the carboxylic acid at the 2-position of the sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the individual structural formulas.

[28] The antibody-drug conjugate according to [27], wherein the compound represented by formula (XV) is represented by the following formula (XV'):

[Formula 33]

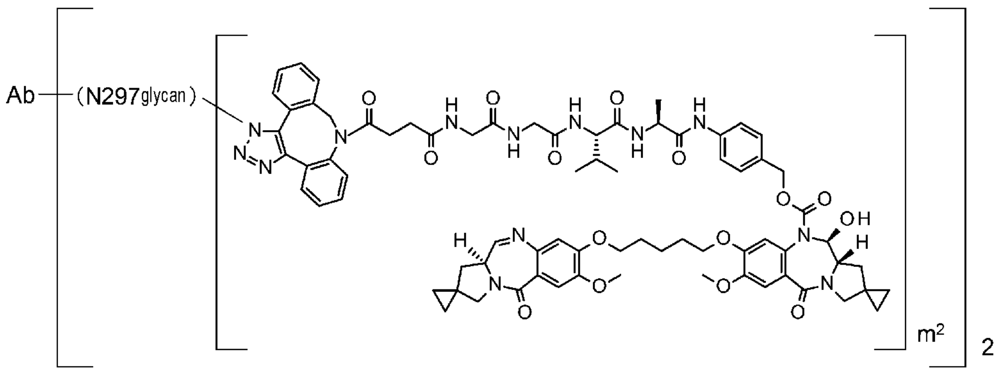

or (XV')

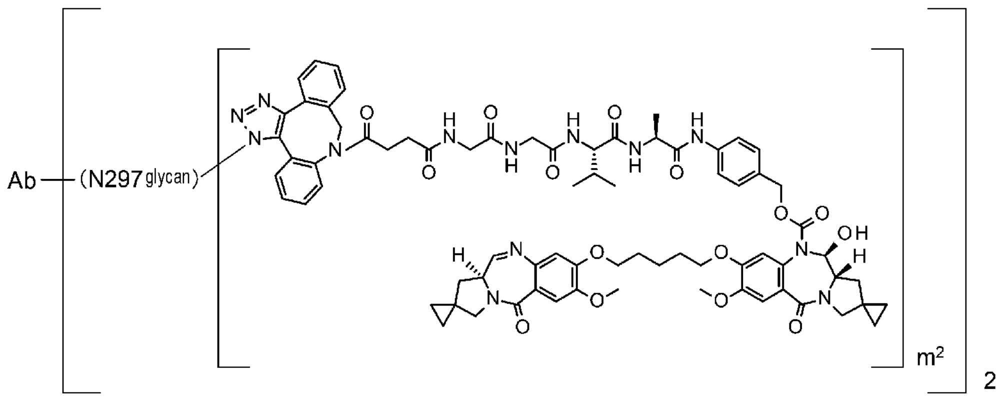

[29] The antibody-drug conjugate according to any one of [21] to [28], wherein the antibody or a functional fragment thereof comprises CDRL1, CDRL2 and CDRL3 and CDRH1, CDRH2 and CDRH3 selected from the group consisting of the following (1) to (4):

(1) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and CDRH1 consisting of the amino acid sequence shown in SEQ ID NO: 17, CDRH2 consisting of

the amino acid sequence shown in SEQ ID NO: 18 and CDRH3 consisting of the amino acid sequence shown in SEQ ID NO: 19,

(2) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and CDRH1 consisting of the amino acid sequence shown in SEQ ID NO: 17, CDRH2 consisting of the amino acid sequence shown in SEQ ID NO: 30 and CDRH3 consisting of the amino acid sequence shown in SEQ ID NO: 19,

(3) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and CDRH1 consisting of the amino acid sequence shown in SEQ ID NO: 57, CDRH2 consisting of the amino acid sequence shown in SEQ ID NO: 58 and CDRH3 consisting of the amino acid sequence shown in SEQ ID NO: 59, and

(4) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14 and CDRH1 consisting of the amino acid sequence shown in SEQ ID NO: 62, CDRH2 consisting of the amino acid sequence shown in SEQ ID NO: 63 and CDRH3 consisting of the amino acid sequence shown in SEQ ID NO: 64.

[30] The antibody-drug conjugate according to any one of [21] to [29], wherein the antibody or a functional fragment thereof comprises any one of the following (1) to (6):

(1) a light chain variable region consisting of the amino acid sequence shown in SEQ ID NO: 33 and a heavy chain variable region consisting of the amino acid sequence shown in SEQ ID NO: 41,

(2) a light chain variable region consisting of the amino acid sequence shown in SEQ ID NO: 33 and a heavy chain variable region consisting of the amino acid sequence shown in SEQ ID NO: 45,

(3) a light chain variable region consisting of the amino acid sequence shown in SEQ ID NO: 37 and a heavy chain variable region consisting of the amino acid sequence shown in SEQ ID NO: 45,

(4) a light chain variable region consisting of the amino acid sequence shown in SEQ ID NO: 33 and a heavy chain variable region consisting of the amino acid sequence shown in SEQ ID NO: 49,

(5) a light chain variable region consisting of the amino acid sequence shown in SEQ ID NO: 33 and a heavy chain variable region consisting of the amino acid sequence shown in SEQ ID NO: 55, and

(6) a light chain variable region consisting of the amino acid sequence shown in SEQ ID NO: 33 and a heavy chain variable region consisting of the amino acid sequence shown in SEQ ID NO: 60.

[31] The antibody-drug conjugate according to any one of [21] to [30], wherein the antibody or a functional fragment thereof comprises any one of the following (1) to (7) :

(1) a light chain consisting of the amino acid sequence at positions 21 to 233 in SEQ ID NO: 31 and a heavy chain consisting of the amino acid sequence at positions 20 to 471 in SEQ ID NO: 39,

(2) a light chain consisting of the amino acid sequence at positions 21 to 233 in SEQ ID NO: 31 and a heavy chain consisting of the amino acid sequence at positions 20 to 471 in SEQ ID NO: 43,

(3) a light chain consisting of the amino acid sequence at positions 21 to 233 in SEQ ID NO: 35 and a heavy chain consisting of the amino acid sequence at positions 20 to 471 in SEQ ID NO: 43,

(4) a light chain consisting of the amino acid sequence at positions 21 to 233 in SEQ ID NO: 31 and a heavy chain consisting of the amino acid sequence at positions 20 to 471 in SEQ ID NO: 47,

(5) a light chain consisting of the amino acid sequence at positions 21 to 233 in SEQ ID NO: 31 and a heavy chain consisting of the amino acid sequence at positions 20 to 471 in SEQ ID NO: 65,

(6) a light chain consisting of the amino acid sequence at positions 21 to 233 in SEQ ID NO: 31 and a heavy chain consisting of the amino acid sequence at positions 20 to 471 in SEQ ID NO: 67, and

(7) a light chain consisting of the amino acid sequence at positions 21 to 233 in SEQ ID NO: 31 and a heavy chain consisting of the amino acid sequence at positions 20 to 471 in SEQ ID NO: 69.

[32] The antibody-drug conjugate according to [31], wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 39.

[33] The antibody-drug conjugate according to [31], wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 43.

[34] The antibody-drug conjugate according to [31], wherein the antibody or a functional fragment thereof comprises a light chain consisting of the amino acid sequence at positions 21 to 233 in SEQ ID NO: 35 and a heavy chain consisting of the amino acid sequence at positions 20 to 471 in SEQ ID NO: 43.
[35] The antibody-drug conjugate according to [31], wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 47.
[36] The antibody-drug conjugate according to [31], wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 65.
[37] The antibody-drug conjugate according to [31], wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 67.
[38] The antibody-drug conjugate according to [31], wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 69.
[39] The antibody-drug conjugate according to any one of [1] to [38], wherein the average number of conjugated drug molecules per antibody molecule in the antibody-drug conjugate is 1 to 3.
[40] The antibody-drug conjugate according to any one of [1] to [38], wherein the average number of conjugated drug molecules per antibody molecule in the antibody-drug conjugate is 3 to 5.
[41] The antibody-drug conjugate according to any one of [1] to [40], wherein the antibody contains a heavy chain having one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, and a deletion of one or two amino acids from the carboxyl terminus.
[42] The antibody-drug conjugate according to any one of [1] to [41], wherein the lysine residue at the carboxyl terminus of the heavy chain is deleted.
[43] A method for producing a glycan-remodeled antibody, the method comprising the steps of:

i) producing an IgG antibody specifically binding to an amino acid sequence comprising the amino acid sequence shown in SEQ ID NO: 4 and having internalization ability that permits cellular uptake or a functional fragment thereof,
ii) treating the antibody obtained in step i) with hydrolase to produce a (Fuc$\alpha$1,6)GlcNAc-antibody; and
iii)-1 reacting the (Fuc$\alpha$1,6)GlcNAc-antibody and a glycan donor molecule in the presence of a transglycosidase, the glycan donor molecule obtained by introducing a PEG linker having an azide group to the carbonyl group of carboxylic acid at the 2-position of a sialic acid in MSG (9) or SG (10) and oxazolinating the reducing terminal, or
iii)-2 reacting the (Fuc$\alpha$1,6)GlcNAc-antibody and a glycan donor molecule in the presence of a transglycosidase, the glycan donor molecule obtained by introducing a PEG linker having an azide group to the carbonyl group of carboxylic acid at the 2-position of a sialic acid in (MSG-)Asn or (SG-)Asn with an $\alpha$-amino group optionally protected and to the carbonyl group of carboxylic acid in the Asn, causing action of hydrolase, and then oxazolinating the reducing terminal.

[44] A method for producing an antibody-drug conjugate according to any one of [1] to [42], comprising the step of reacting the glycan-remodeled antibody obtained by the method according to [43] and a drug linker.
[45] The antibody-drug conjugate according to any one of [1] to [42], produced by the method according to [44] .
[46] An antibody or a functional fragment thereof, comprising a light chain variable region, which contains CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14, and a heavy chain variable region, which contains CDRH1 consisting of the amino acid sequence shown in SEQ ID NO: 57, CDRH2 consisting of the amino acid sequence shown in SEQ ID NO: 58 and CDRH3 consisting of the amino acid sequence shown in SEQ ID NO: 59.
[47] An antibody or a functional fragment thereof, comprising a light chain variable region, which contains CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and a heavy chain variable region, which contains CDRH1 consisting of the amino acid sequence shown in SEQ ID NO: 62, CDRH2 consisting of the amino acid sequence shown in SEQ ID NO: 63 and CDRH3 consisting of the amino acid sequence shown in SEQ ID NO: 64.
[48] The antibody or a functional fragment thereof according to [46], comprising a light chain variable region consisting of the amino acid sequence shown in SEQ ID NO: 33 and a heavy chain variable region consisting of the amino

acid sequence shown in SEQ ID NO: 55.

[49] The antibody or a functional fragment thereof according to [47], comprising a light chain variable region consisting of the amino acid sequence shown in SEQ ID NO: 33 and a heavy chain variable region consisting of the amino acid sequence shown in SEQ ID NO: 60.

[50] The antibody or a functional fragment thereof according to [46] or [48], having a light chain consisting of the amino acid sequence at positions 21 to 233 in SEQ ID NO: 31 and a heavy chain consisting of the amino acid sequence at positions 20 to 471 in SEQ ID NO: 67.

[51] The antibody or a functional fragment thereof according to [47] or [49], having a light chain consisting of the amino acid sequence at positions 21 to 233 in SEQ ID NO: 31 and a heavy chain consisting of the amino acid sequence at positions 20 to 471 in SEQ ID NO: 69.

[52] An antibody or a functional fragment thereof having a light chain consisting of the amino acid sequence at positions 21 to 233 in SEQ ID NO: 31 and a heavy chain consisting of the amino acid sequence at positions 20 to 471 in SEQ ID NO: 65.

[53] A polynucleotide encoding the antibody according to any one of [46] to [52].

[54] An expression vector containing the polynucleotide according to [53].

[55] A host cell transformed with the expression vector according to [54].

[56] A method for producing the antibody or a functional fragment thereof according to [46] to [52], comprising a step of culturing the host cell according to [55] and collecting the targeted antibody from the culture obtained from the step of culturing.

[57] An antibody or a functional fragment thereof, obtained by the method according to [56].

[58] A pharmaceutical composition containing the antibody-drug conjugate according to any one of [1] to [42] and [45] or the antibody or a functional fragment thereof according to any one of [46] to [52] and [57].

[59] The pharmaceutical composition according to [58], which is an antitumor drug.

[60] The pharmaceutical composition according to [59], wherein the tumor is a tumor expressing CDH6.

[61] The pharmaceutical composition according to [60], wherein the tumor is renal cell carcinoma, renal clear cell carcinoma, papillary renal cell carcinoma, ovarian cancer, ovarian serous adenocarcinoma, thyroid cancer, bile duct cancer, lung cancer, small-cell lung cancer, non-small cell lung cancer, glioblastoma, mesothelioma, uterine cancer, pancreatic cancer, Wilms' tumor or neuroblastoma.

[62] A method for treating a tumor, which comprises administering the antibody-drug conjugate according to any one of [1] to [42] and [45], the antibody or a functional fragment thereof according to any one of [46] to [52] and [57] or the pharmaceutical composition according to any one of [58] to [61] to an individual.

[63] The treatment method according to [62], wherein the tumor is a tumor expressing CDH6.

[64] The treatment method according to [62] or [63], wherein the tumor is renal cell carcinoma, renal clear cell carcinoma, papillary renal cell carcinoma, ovarian cancer, ovarian serous adenocarcinoma, thyroid cancer, bile duct cancer, lung cancer, small-cell lung cancer, non-small cell lung cancer, glioblastoma, mesothelioma, uterine cancer, pancreatic cancer, Wilms' tumor or neuroblastoma.

[65] The treatment method according to any one of [62] to [64], which comprises administering the antibody-drug conjugate according to any one of [1] to [42] and [45]; the antibody or a functional fragment thereof according to any one of [46] to [52] and [57]; or the pharmaceutical composition according to any one of [58] to [61] and at least one antitumor drug to an individual, simultaneously, separately or continuously.

## Advantageous Effects of Invention

Features of the anti-CDH6 antibody of the present invention are to recognize EC domain 3 (EC3) of CDH6 specifically and to have high internalization activity. An anti-CDH6 antibody-drug conjugate, which is prepared by connecting the anti-CDH6 antibody of the present invention and a novel PBD derivative of the present invention via a linker, can be expected to achieve excellent antitumor effects and safety by administration to patients having cancer cells expressing CDH6. Specifically, the anti-CDH6 antibody-drug conjugate of the present invention is useful as an antitumor agent.

## Brief Description of Drawings

[Figure 1] Figure 1 shows flow cytometry results of examining the binding of each of rat anti-CDH6 monoclonal antibody rG019 and a negative control antibody rat IgG2b to control cells (shaded) or hCDH6 transfected 293T cells (open solid line). The abscissa depicts FITC fluorescence intensity indicating the amount of the antibody bound, and the ordinate depicts cell count.

[Figure 2-1] The figure (1) shows the binding activity of rat anti-CDH6 monoclonal antibody rG019 or negative control antibody Rat IgG2b against control cells (shaded) or full-length hCDH6-transfected 293 cells (open solid line). The figure (2) shows the binding activity of rat anti-CDH6 monoclonal antibody rG019 or negative control antibody Rat IgG2b against control cells (shaded) or EC1-deleted hCDH6-transfected 293 cells (open solid line). The figure (3) shows the binding activity of rat anti-CDH6 monoclonal antibody rG019 or negative control antibody Rat IgG2b against control cells (shaded) or EC2-deleted hCDH6-transfected 293 cells (open solid line). In the figures (1) to (3), the abscissa depicts FITC fluorescence intensity indicating the amount of antibody bound, and the ordinate depicts cell count.

[Figure 2-2] The figure (4) shows the binding activity of rat anti-CDH6 monoclonal antibody rG019 or negative control antibody Rat IgG2b against control cells (shaded) or EC3-deleted hCDH6-transfected 293 cells (open solid line). The figure (5) shows the binding activity of rat anti-CDH6 monoclonal antibody rG019 or negative control antibody Rat IgG2b against control cells (shaded) or EC4-deleted hCDH6-transfected 293 cells (open solid line). The figure (6) shows the binding activity of rat anti-CDH6 monoclonal antibody rG019 or negative control antibody Rat IgG2b against control cells (shaded) or EC5-deleted hCDH6-transfected 293 cells (open solid line). In the figures (4) to (6), the abscissa depicts FITC fluorescence intensity indicating the amount of antibody bound, and the ordinate depicts cell count.

[Figure 3] Figure 3 shows flow cytometry results of evaluating the expression of CDH6 on the cell membrane surface of 5 types of human tumor cell lines (human ovarian tumor cell lines NIH:OVCAR-3, PA-1, and OV-90 and human renal cell tumor cell line 786-O, Caki-1). The abscissa depicts FITC fluorescence intensity indicating the amount of antibody bound, and the ordinate depicts cell count. The shaded histogram shows that the negative control mIgG1 was used in staining, and the open solid line histogram shows that the anti-human CDH6 antibody was used in staining.

[Figure 4] Figure 4 shows the binding of human chimeric anti-CDH6 antibody chG019 to human CDH6 and monkey CDH6. The abscissa depicts antibody concentration, and the ordinate depicts the amount of antibody bound based on mean fluorescence intensity.

[Figure 5-1] Figure 5-1 shows the binding activity of four humanized hG019 antibodies (H01L02, H02L02, H02L03 and H04L02), anti-CDH6 antibody NOV0712 or negative control antibody hIgG1 against control cells (shaded) or full-length hCDH6-transfected 293α cells (open solid line). The abscissa depicts APC fluorescence intensity indicating the amount of antibody bound, and the ordinate depicts cell count.

[Figure 5-2] Figure 5-2 shows the binding activity of four humanized hG019 antibodies (H01L02, H02L02, H02L03 and H04L02), anti-CDH6 antibody NOV0712 or negative control antibody hIgG1 against control cells (shaded) or EC1-deleted hCDH6-transfected 293α cells (open solid line). The abscissa depicts APC fluorescence intensity indicating the amount of antibody bound, and the ordinate depicts cell count.

[Figure 5-3] Figure 5-3 shows the binding activity of four humanized hG019 antibodies (H01L02, H02L02, H02L03 and H04L02), anti-CDH6 antibody NOV0712 or negative control antibody hIgG1 against control cells (shaded) or EC2-deleted hCDH6-transfected 293α cells (open solid line). The abscissa depicts APC fluorescence intensity indicating the amount of antibody bound, and the ordinate depicts cell count.

[Figure 5-4] Figure 5-4 shows the binding activity of four humanized hG019 antibodies (H01L02, H02L02, H02L03 and H04L02), anti-CDH6 antibody NOV0712 or negative control antibody hIgG1 against control cells (shaded) or EC3-deleted hCDH6-transfected 293α cells (open solid line). The abscissa depicts APC fluorescence intensity indicating the amount of antibody bound, and the ordinate depicts cell count.

[Figure 5-5] Figure 5-5 shows the binding activity of four humanized hG019 antibodies (H01L02, H02L02, H02L03 and H04L02), anti-CDH6 antibody NOV0712 or negative control antibody hIgG1 against control cells (shaded) or EC4-deleted hCDH6-transfected 293α cells (open solid line). The abscissa depicts APC fluorescence intensity indicating the amount of antibody bound, and the ordinate depicts cell count.

[Figure 5-6] Figure 5-6 shows the binding activity of four humanized hG019 antibodies (H01L02, H02L02, H02L03 and H04L02), anti-CDH6 antibody NOV0712 or negative control antibody hIgG1 against control cells (shaded) or EC5-deleted hCDH6-transfected 293α cells (open solid line). The abscissa depicts APC fluorescence intensity indicating the amount of antibody bound, and the ordinate depicts cell count.

[Figure 6] Figure 6 shows flow cytometry results of examining the expression of human CDH6 in a 786-O/hCDH6 stably expressing cell line (open solid line) and its parent cell line 786-O (shaded). The abscissa depicts Alexa Fluor 647 fluorescence intensity indicating the amount of antibody bound, and the ordinate depicts cell count.

[Figure 7] Figure 7 shows the binding competition assay of four unlabeled humanized hG019 antibodies (H01L02, H02L02, H02L03 and H04L02), anti-CDH6 antibody NOV0712 or negative control hIgG1 using labeled NOV0712 (upper stage) or labeled H01L02 (lower stage). The abscissa depicts the final concentration of the added unlabeled antibody, and the ordinate depicts the amount of antibody bound based on mean fluorescence intensity.

[Figure 8] Figure 8 shows the results on which the internalization activity of four humanized hG019 antibodies (H01L02, H02L02, H02L03 and H04L02), anti-CDH6 antibody NOV0712 and a negative control antibody was evaluated in NIH:OVCAR-3 cells, 786-O cells or PA-1 cells using anti-human IgG reagent Hum-ZAP conjugated with a toxin (saporin) inhibiting protein synthesis, or F(ab')2 Fragment Goat Anti-human IgG, Fc (gamma) Fragment Specific unconjugated with the toxin as a negative control. A cell survival rate (%), calculated as a relative survival rate when the number of live cells in a well supplemented with the negative control instead of Hum-ZAP was defined as 100%, is shown below each entry.

[Figure 9] Figure 9 shows the IC50 (50% inhibition concentration) (nM) of each of anti-LPS antibody-PBD (ADC11), H01L02-PBD (ADC1), H01L02A-PBD (ADC5), H11L02A-PBD (ADC7) and H31L02A-PBD (ADC6) relative to CDH6 positive human ovarian tumor cell lines NIH: OVCAR-3, OV-90, PA-1 and CDH6 positive human renal-cell carcinoma cell line 786-O.

[Figure 10] Figure 10 shows the *in vivo* anti-tumor effect of each of H01L02-PBD (ADC1), NOV0712-DM4 and anti-LPS antibody-PBD (ADC11). The evaluation was conducted using animal models in which CDH6-positive human ovarian tumor cell line OV-90 was inoculated into immunodeficient mice. The abscissa depicts the number of days, and the ordinate depicts tumor volume. The error range depicts a SE value.

[Figure 11] Figure 11 shows the *in vivo* anti-tumor effect of each of H01L02-PBD (ADC1), NOV0712-DM4 and anti-LPS antibody-PBD (ADC11). The evaluation was conducted using animal models in which CDH6-positive human renal tumor cell line Caki-1 was inoculated into immunodeficient mice. The abscissa depicts the number of days, and the ordinate depicts tumor volume. The error range depicts a SE value.

[Figure 12] Figure 12 shows the *in vivo* anti-tumor effect of each of H01L02-PBD (ADC1), H01L02A-PBD (ADC5), H31L02A-PBD (ADC6) and anti-LPS antibody-PBD (ADC11). The evaluation was conducted using animal models in which CDH6-positive human ovarian tumor cell line NIH: OVCAR-3 was inoculated into immunodeficient mice. The abscissa depicts the number of days, and the ordinate depicts tumor volume. The error range depicts a SE value.

[Figure 13] Figure 13 shows the *in vivo* anti-tumor effect of each of H01L02-PBD (ADC1), H01L02A-PBD (ADC5), H31L02A-PBD (ADC6) and H11L02A-PBD (ADC7). The evaluation was conducted using animal models in which CDH6-positive human ovarian tumor cell line OV-90 was inoculated into immunodeficient mice. The abscissa depicts the number of days, and the ordinate depicts tumor volume. The error range depicts a SE value.

[Figure 14] Figure 14 shows the *in vivo* anti-tumor effect of each of H01L02A-PBD (ADC5), H31L02A-PBD (ADC6) and anti-LPS antibody-PBD (ADC11). The evaluation was conducted using animal models in which CDH6-positive human ovarian tumor cell line PA-1 was inoculated into immunodeficient mice. The abscissa depicts the number of days, and the ordinate depicts tumor volume. The error range depicts a SE value.

[Figure 15] Figure 15 shows the *in vivo* anti-tumor effect of each of H01L02A-PBD (ADC5), H31L02A-PBD (ADC6) and anti-LPS antibody-PBD (ADC11). The evaluation was conducted using animal models in which CDH6-positive human renal tumor cell line 786-O was inoculated into immunodeficient mice. The abscissa depicts the number of days, and the ordinate depicts tumor volume. The error range depicts a SE value.

## Description of Embodiments

Hereinafter, the preferred embodiments for carrying out the present invention will be described with reference to the drawings. It is to be noted that the embodiments described below merely illustrate the representative embodiments of the present invention, and the scope of the present invention shall not be narrowly interpreted due to these examples.

In the present description, the term "cancer" is used to have the same meaning as that of the term "carcinoma" and "tumor".

In the present description, the term "gene" is used to include not only DNA but also its mRNA and cDNA, and cRNA thereof.

In the present description, the term "polynucleotide" or "nucleotide" is used to have the same meaning as that of a nucleic acid, and also includes DNA, RNA, a probe, an oligonucleotide, and a primer. In the present description, the terms "polynucleotide" and "nucleotide" can be used interchangeably with each other unless otherwise specified.

In the present description, the terms "polypeptide" and "protein" can be used interchangeably with each other.

In the present description, the term "cell" includes cells in an individual animal, and cultured cells.

In the present description, the term "CDH6" can be used to have the same meaning as that of the CDH6 protein. In the present description, human CDH6 is also referred to as "hCDH6".

In the present description, the term "cytotoxic activity" is used to mean that a pathologic change is caused to cells in any given way. The term not only means a direct trauma, but also means all types of structural or functional damage caused to cells, such as DNA cleavage, formation of a base dimer, chromosomal cleavage, damage to cell mitotic apparatus, and a reduction in the activities of various types of enzymes.

In the present description, the phrase "exerting toxicity in cells" is used to mean that toxicity is exhibited in cells in any given way. The term not only means a direct trauma, but also means all types of structural, functional, or metabolic influences caused to cells, such as DNA cleavage, formation of a base dimer, chromosomal cleavage, damage to cell mitotic apparatus, a reduction in the activities of various types of enzymes, and suppression of effects of cell growth factors.

In the present invention, the term "functional fragment of an antibody", also called "antigen-binding fragment of an antibody", is used to mean a partial fragment of the antibody having binding activity against an antigen, and includes Fab, F(ab')2, Fv, scFv, a diabody, a linear antibody and a multispecific antibody formed from antibody fragments, and the like. Fab', which is a monovalent fragment of antibody variable regions obtained by treating F(ab')2 under reducing conditions, is also included in the antigen-binding fragment of an antibody. However, the antigen-binding fragment of an antibody is not limited to these molecules, as long as the antigen-binding fragment has antigen-binding ability. These antigen-binding fragments include not only those obtained by treating a full-length molecule of an antibody protein with an appropriate enzyme, but proteins produced in appropriate host cells using a genetically engineered antibody gene.

The functional fragment of the present invention includes a functional fragment that has well conserved asparagine (Asn297 or N297) to be modified with an **N-**linked glycan in the IgG heavy chain Fc region and amino acids around Asn297, while retaining binding activity to an antigen.

In the present description, the term "epitope" is used to mean the specific partial peptide or partial three-dimensional structure of CDH6, to which an anti-CDH6 antibody binds. Such an epitope, which is the above-described partial peptide of CDH6, can be determined by a method well known to a person skilled in the art, such as an immunoassay. First, various partial structures of an antigen are produced. As regards production of such partial structures, a known oligonucleotide synthesis technique can be applied. For example, a series of polypeptides, in which CDH6 has been successively truncated at an appropriate length from the C-terminus or N-terminus thereof, are produced by a genetic recombination technique well known to a person skilled in the art. Thereafter, the reactivity of an antibody to such polypeptides is studied, and recognition sites are roughly determined. Thereafter, further shorter peptides are synthesized, and the reactivity thereof to these peptides can then be studied, so as to determine an epitope. When an antibody binding to a membrane protein having a plurality of extracellular domains is directed to a three-dimensional structure composed of a plurality of domains as an epitope, the domain to which the antibody binds can be determined by modifying the amino acid sequence of a specific extracellular domain, and thereby modifying the three-dimensional structure. The epitope, which is a partial three-dimensional structure of an antigen that binds to a specific antibody, can also be determined by specifying the amino acid residues of an antigen adjacent to the antibody by X-ray structural analysis.

In the present description, the phrase "antibodies binding to the same epitope" is used to mean antibodies that bind to a common epitope. If a second antibody binds to a partial peptide or a partial three-dimensional structure to which a first antibody binds, it can be determined that the first antibody and the second antibody bind to the same epitope. Alternatively, by confirming that a second antibody competes with a first antibody for the binding of the first antibody to an antigen (i.e., a second antibody interferes with the binding of a first antibody to an antigen), it can be determined that the first antibody and the second antibody bind to the same epitope, even if the specific sequence or structure of the epitope has not been determined. In the present description, the phrase "binding to the same epitope" refers to the case where it is determined that the first antibody and the second antibody bind to a common epitope by any one or both of these determination methods. When a first antibody and a second antibody bind to the same epitope and further, the first antibody has special effects such as antitumor activity or internalization activity, the second antibody can be expected to have the same activity as that of the first antibody.

In the present description, the term "CDR" is used to mean a complementarity determining region. It is known that the heavy chain and light chain of an antibody molecule each have three CDRs. Such a CDR is also referred to as a hypervariable region, and is located in the variable regions of the heavy chain and light chain of an antibody. These regions have a particularly highly variable primary structure and are separated into three sites on the primary structure of the polypeptide chain in each of the heavy chain and light chain. In the present description, with regard to the CDR of an antibody, the CDRs of a heavy chain are referred to as CDRH1, CDRH2 and CDRH3, respectively, from the aminoterminal side of the amino acid sequence of the heavy chain, whereas the CDRs of a light chain are referred to as CDRL1, CDRL2 and CDRL3, respectively, from the aminoterminal side of the amino acid sequence of the light chain. These sites are located close to one another on the three-dimensional structure, and determine the specificity of the antibody to an antigen to which the antibody binds.

In the present description, the phrase "hybridizing under stringent conditions" is used to mean that hybridization is carried out in the commercially available hybridization solution ExpressHyb Hybridization Solution (manufactured by Clontech Laboratories, Inc.) at 68°C, or that hybridization is carried out under conditions in which hybridization is carried out using a DNA-immobilized filter in the presence of 0.7 to 1.0 M NaCl at 68°C, and the resultant is then washed at 68°C with a 0.1- to 2-fold concentration of SSC solution (wherein 1-fold concentration of SSC consists of 150 mM NaCl and 15 mM sodium citrate) for identification, or conditions equivalent thereto.

In the present description, the term "one to several" is used to mean 1 to 10, 1 to 9, 1 to 8, 1 to 7, 1 to 6, 1 to 5, 1 to 4, 1 to 3, or 1 or 2.

1. CDH6

Cadherins are glycoproteins present on the surface of cell membranes and function as cell-cell adhesion

molecules through the calcium ion-dependent binding of their N-terminal extracellular domains, or as signal molecules responsible for cell-cell interaction. Classic cadherins are in the cadherin superfamily and are single-pass transmembrane proteins composed of five extracellular domains (EC domains), one transmembrane region, and an intracellular domain.

CDH6 (cadherin-6) is a single-pass transmembrane protein composed of 790 amino acids, which is classified into the type II cadherin family, and this protein has N-terminal extracellular and C-terminal intracellular domains. The human CDH6 gene was cloned for the first time in 1995 (Non Patent Literature 1), and its sequence can be referred to under, for example, accession Nos. NM_004932 and NP_004923 (NCBI).

The CDH6 protein used in the present invention can be directly purified from the CDH6-expressing cells of a human or a non-human mammal (e.g., a rat, a mouse or a monkey) and can then be used, or a cell membrane fraction of the aforementioned cells can be prepared and can be used as the CDH6 protein. Alternatively, CDH6 can also be obtained by synthesizing it *in vitro,* or by allowing host cells to produce CDH6 by genetic manipulation. According to such genetic manipulation, the CDH6 protein can be obtained, specifically, by incorporating CDH6 cDNA into a vector capable of expressing the CDH6 cDNA, and then synthesizing CDH6 in a solution containing enzymes, substrate and energetic materials necessary for transcription and translation, or by transforming host cells of other prokaryotes or eukaryotes, so as to allow them to express CDH6. Also, CDH6-expressing cells based on the above-described genetic manipulation, or a cell line expressing CDH6 may be used to present the CDH6 protein. Alternatively, the expression vector into which CDH6 cDNA has been incorporated can be directly administered to an animal to be immunized, and CDH6 can be expressed in the body of the animal thus immunized.

Moreover, a protein which consists of an amino acid sequence comprising a substitution, deletion and/or addition of one or several amino acids in the above-described amino acid sequence of CDH6, and has a biological activity equivalent to that of the CDH6 protein, is also included within the term "CDH6".

The human CDH6 protein has the amino acid sequence shown in SEQ ID NO: 1. The extracellular region of the human CDH6 protein is constituted of extracellular domain 1 (in the specification, also referred to as EC1) having the 54th to 159th amino acids in the amino acid sequence shown in SEQ ID NO: 1, extracellular domain 2 (in the specification, also referred to as EC2) having the 160th to 268th amino acids in the amino acid sequence shown in SEQ ID NO: 1, extracellular domain 3 (in the specification, also referred to as EC3) having the 269th to 383rd amino acids in the amino acid sequence shown in SEQ ID NO: 1, extracellular domain 4 (in the specification, also referred to as EC4) having the 384th to 486th amino acids in the amino acid sequence shown in SEQ ID NO: 1 and extracellular domain 5 (in the specification, also referred to as EC5) having the 487th to 608th amino acids in the amino acid sequence shown in SEQ ID NO: 1. The amino acid sequences of EC1 to EC5 are respectively represented by SEQ ID NOs: 2 to 6 (Table 1).

2. Production of anti-CDH6 antibody

One example of the anti-CDH6 antibody of the present invention can include an anti-CDH6 antibody which recognizes an amino acid sequence comprising the amino acid sequence shown in SEQ ID NO: 4, and has internalization activity. One example of the anti-CDH6 antibody of the present invention can include an anti-CDH6 antibody which specifically recognizes an amino acid sequence comprising the amino acid sequence shown in SEQ ID NO: 4, and has internalization activity. One example of the anti-CDH6 antibody of the present invention can include an anti-CDH6 antibody which recognizes an amino acid sequence consisting of the amino acid sequence shown in SEQ ID NO: 4, and has internalization activity. One example of the anti-CDH6 antibody of the present invention can include an anti-CDH6 antibody which specifically recognizes an amino acid sequence consisting of the amino acid sequence shown in SEQ ID NO: 4, and has internalization activity. The phrase "specifically recognize an amino acid sequence comprising the amino acid sequence shown in SEQ ID NO: 4" or "specifically recognize an EC3 domain" as applied to an antibody is used to mean that the antibody strongly recognizes or strongly binds to the EC3 domain of CDH6 compared with the other extracellular domains of CDH6.

The anti-CDH6 antibody of the present invention may be derived from any species. Preferred examples of the species can include humans, monkeys, rats, mice and rabbits. When the anti-CDH6 antibody of the present invention is derived from a species other than humans, it is preferred to chimerize or humanize the anti-CDH6 antibody by a well-known technique. The antibody of the present invention may be a polyclonal antibody or may be a monoclonal antibody, and a monoclonal antibody is preferred.

The anti-CDH6 antibody of the present invention is an antibody that can target tumor cells. Specifically, the anti-CDH6 antibody of the present invention possesses the property of being able to recognize tumor cells, the property of being able to bind to tumor cells, and/or the property of being internalized into tumor cells by cellular uptake, and the like. Accordingly, the anti-CDH6 antibody of the present invention can be conjugated to a compound having antitumor activity via a linker to prepare an antibody-drug conjugate.

The binding activity of an antibody against tumor cells can be confirmed by flow cytometry. The uptake of an antibody into tumor cells can be confirmed by (1) an assay of visualizing a cellularly taken-up antibody under a fluorescent microscope using a secondary antibody (fluorescently labeled) binding to the antibody (Cell Death and Differentiation,

2008, 15, 751-761), (2) an assay of measuring the amount of cellularly taken-up fluorescence using a secondary antibody (fluorescently labeled) binding to the antibody (Molecular Biology of the Cell Vol. 15, 5268-5282, December 2004) or (3) a Mab-ZAP assay using an immunotoxin binding to the antibody, wherein the toxin is released upon cellular uptake, so as to suppress cell growth (Bio Techniques 28: 162-165, January 2000). A recombinant conjugated protein of a catalytic region of diphtheria toxin and protein G may be used as the immunotoxin.

In the present description, the term "high internalization ability" is used to mean that the survival rate (which is indicated by a ratio relative to a cell survival rate without antibody addition defined as 100%) of CDH6-expressing cells to which the aforementioned antibody and a saporin-labeled anti-rat IgG antibody have been administered is preferably 70% or less, and more preferably 60% or less.

The antibody-drug conjugate comprises a conjugated compound exerting an antitumor effect. Therefore, it is preferred, but not essential, that the antibody itself should have an antitumor effect. For the purpose of specifically and/or selectively exerting the cytotoxicity of the antitumor compound in tumor cells, it is important and preferred that the antibody should have a property of being internalized and transferred into tumor cells.

The anti-CDH6 antibody can be obtained by immunizing an animal with a polypeptide serving as an antigen by a method usually performed in this field, and then collecting and purifying an antibody produced in the living body thereof. It is preferred to use CDH6 retaining a three-dimensional structure as an antigen. Examples of such a method can include a DNA immunization method.

The origin of the antigen is not limited to a human, and thus, an animal can also be immunized with an antigen derived from a non-human animal such as a mouse or a rat. In this case, an antibody applicable to the disease of a human can be selected by examining the cross-reactivity of the obtained antibody binding to the heterologous antigen with the human antigen.

Furthermore, antibody-producing cells that produce an antibody against the antigen can be fused with myeloma cells according to a known method (e.g., Kohler and Milstein, Nature (1975) 256, 495-497; and Kennet, R. ed., Monoclonal Antibodies, 365-367, Plenum Press, N. Y. (1980)) to establish hybridomas, so as to obtain a monoclonal antibody.

Hereinafter, the method for obtaining an antibody against CDH6 will be specifically described.

(1) Preparation of antigen

The antigen can be obtained by allowing host cells to produce a gene encoding the antigen protein according to genetic manipulation. Specifically, a vector capable of expressing the antigen gene is produced, and the vector is then introduced into host cells, so that the gene is expressed therein, and thereafter, the expressed antigen may be purified. The antibody can also be obtained by a method of immunizing an animal with the antigen-expressing cells based on the above-described genetic manipulation, or a cell line expressing the antigen.

Alternatively, the antibody can also be obtained, without the use of the antigen protein, by incorporating cDNA of the antigen protein into an expression vector, then administering the expression vector to an animal to be immunized, and expressing the antigen protein in the body of the animal thus immunized, so that an antibody against the antigen protein is produced therein.

(2) Production of anti-CDH6 monoclonal antibody

The anti-CDH6 antibody used in the present invention is not particularly limited. For example, an antibody specified by an amino acid sequence shown in the sequence listing of the present application can suitably be used. The anti-CDH6 antibody used in the present invention is desirably an antibody having the following properties:

(1) an antibody having the following properties:

  (a) specifically binding to CDH6, and
  (b) having the activity of being internalized into CDH6-expressing cells by binding to CDH6;

(2) the antibody according to the above (1) or the aforementioned antibody, wherein the CDH6 is human CDH6; or
(3) specifically recognizing EC3 of human CDH6, and having internalization activity.

The method for obtaining the antibody against CDH6 of the present invention is not particularly limited as long as an anti-CDH6 antibody can be obtained. It is preferred to use CDH6 retaining its conformation as an antigen.

One preferred example of the method for obtaining the antibody can include a DNA immunization method. The DNA immunization method is an approach which involves transfecting an animal (e.g., mouse or rat) individual with an antigen expression plasmid, and then expressing the antigen in the individual to induce immunity against the antigen. The transfection approach includes a method of directly injecting the plasmid into a muscle, a method of injecting a

transfection reagent such as a liposome or polyethylenimine into a vein, an approach using a viral vector, an approach of injecting gold particles attached to the plasmid using a gene gun, a hydrodynamic method of rapidly injecting a plasmid solution in a large amount into a vein, and the like. With regard to the transfection method of injecting the expression plasmid into a muscle, a technique called *in vivo* electroporation, which involves applying electroporation to the intramuscular injection site of the plasmid, is known as an approach for improving expression levels (Aihara H, Miyazaki J. Nat Biotechnol. 1998 Sep; 16 (9): 867-70 or Mir LM, Bureau MF, Gehl J, Rangara R, Rouy D, Caillaud JM, Delaere P, Branellec D, Schwartz B, Scherman D. Proc Natl Acad Sci U S A. 1999 Apr 13; 96 (8): 4262-7). This approach further improves the expression level by treating the muscle with hyaluronidase before the intramuscular injection of the plasmid (McMahon JM1, Signori E, Wells KE, Fazio VM, Wells DJ., Gene Ther. 2001 Aug; 8 (16): 1264-70). Furthermore, hybridoma production can be performed by a known method, and can also be performed using, for example, a Hybrimune Hybridoma Production System (Cyto Pulse Sciences, Inc.).

Specific examples of obtaining a monoclonal antibody can include the following procedures:

(a) immune response can be induced by incorporating CDH6 cDNA into an expression vector (e.g., pcDNA3.1; Thermo Fisher Scientific Inc.), and directly administering the vector to an animal (e.g., a rat or a mouse) to be immunized by a method such as electroporation or a gene gun, so as to express CDH6 in the body of the animal. The administration of the vector by electroporation or the like may be performed one or more times, preferably a plurality of times, if necessary for enhancing antibody titer;
(b) collection of tissue (e.g., a lymph node) containing antibody-producing cells from the aforementioned animal in which the immune response has been induced;
(c) preparation of myeloma cells (hereinafter, referred to as "myelomas") (e.g., mouse myeloma SP2/0-ag14 cells);
(d) cell fusion between the antibody-producing cells and the myelomas;
(e) selection of a hybridoma group producing an antibody of interest;
(f) division into single cell clones (cloning);
(g) optionally, the culture of hybridomas for the mass production of monoclonal antibodies, or the breeding of animals into which the hybridomas are inoculated; and/or
(h) study of the physiological activity (internalization activity) and binding specificity of the monoclonal antibody thus produced, or examination of the properties of the antibody as a labeling reagent.

Examples of the method for measuring the antibody titer used herein can include, but are not limited to, flow cytometry and Cell-ELISA.

Examples of the hybridoma strain thus established can include anti-CDH6 antibody-producing hybridoma rG019. It is to be noted that, in the present description, an antibody produced by the anti-CDH6 antibody-producing hybridoma rG019 is referred to as a "rG019 antibody" or simply as "rG019".

The light chain variable region of the rG019 antibody consists of the amino acid sequence shown in SEQ ID NO: 10. The amino acid sequence of the light chain variable region of the rG019 antibody is encoded by the nucleotide sequence shown in SEQ ID NO: 11. The light chain variable region of the rG019 antibody has CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14. The heavy chain variable region of the rG019 antibody consists of the amino acid sequence shown in SEQ ID NO: 15. The amino acid sequence of the heavy chain variable region of the rG019 antibody is encoded by the nucleotide sequence shown in SEQ ID NO: 16. The heavy chain variable region of the rG019 antibody has CDRH1 consisting of the amino acid sequence shown in SEQ ID NO: 17, CDRH2 consisting of the amino acid sequence shown in SEQ ID NO: 18, and CDRH3 consisting of the amino acid sequence shown in SEQ ID NO: 19.

Further, even if a monoclonal antibody was independently obtained by steps (a) to (h) in "2.Production of anti-CDH6 antibody" again, or a monoclonal antibody was separately obtained by using another method, an antibody having internalization activity equivalent to that of the rG019 antibody can be obtained. An example of such antibodies is an antibody that binds to an epitope identical to the epitope to which the rG019 antibody binds. If a newly produced monoclonal antibody binds to a partial peptide or partial three-dimensional structure to which the rG019 antibody binds, it can be determined that the monoclonal antibody binds to an epitope identical to the epitope to which the rG019 antibody binds. By confirming that the monoclonal antibody competes with the rG019 antibody for binding to CDH6 (i.e., the monoclonal antibody interferes with binding between the rG019 antibody and CDH6), it can be determined, even when the specific sequence or structure of an epitope has not been determined, that the monoclonal antibody binds to an epitope identical to the epitope to which the anti-CDH6 antibody binds. If epitope identity has been confirmed, the monoclonal antibody is strongly expected to have antigen-binding ability, biological activity, and/or internalization activity equivalent to that of the rG019 antibody.

(3) Other antibodies

The antibody of the present invention also includes genetically recombinant antibodies that have been artificially modified for the purpose of reducing heterogenetic antigenicity to humans, such as a chimeric antibody, a humanized antibody and a human antibody, as well as the above-described monoclonal antibody against CDH6. These antibodies can be produced by known methods.

Examples of the chimeric antibody can include antibodies in which a variable region and a constant region are heterologous to each other, such as a chimeric antibody formed by conjugating the variable region of a mouse- or rat-derived antibody to a human-derived constant region(see, Proc. Natl. Acad. Sci. U.S.A., 81, 6851-6855, (1984)).

Examples of the chimeric antibody derived from the rat anti-human CDH6 antibody include an antibody consisting of a light chain comprising the light chain variable region of a rat anti-human CDH6 antibody described in the present description (e.g., rG019 antibody) and a human-derived constant region, and a heavy chain comprising the heavy chain variable region of the rat anti-human CDH6 antibody and a human-derived constant region.

Other examples of the chimeric antibody derived from the rat anti-human CDH6 antibody include an antibody consisting of a light chain comprising a light chain variable region having a substitution of one to several residues, 1 to 3 residues, 1 or 2 residues, preferably 1 residue, of amino acids in the light chain variable region of a rat anti-human CDH6 antibody described in the present description (e.g., rG019 antibody) with other amino acid residues, and a heavy chain comprising a heavy chain variable region having a substitution of one to several residues, 1 to 3 residues, 1 or 2 residues, preferably 1 residue, of amino acids in the heavy chain variable region of the rat anti-human CDH6 antibody with other amino acid residues. This antibody may have any given human-derived constant region.

Other examples of the chimeric antibody derived from the rat anti-human CDH6 antibody include an antibody consisting of a light chain comprising a light chain variable region having a substitution of 1 or 2 residues, preferably 1 residue, of amino acids in any 1 to 3 CDRs in the light chain variable region of a rat anti-human CDH6 antibody described in the present description (e.g., the rG019 antibody) with other amino acid residues, and a heavy chain comprising a heavy chain variable region having a substitution of 1 or 2 residues, preferably 1 residue, of amino acids in any 1 to 3 CDRs in the heavy chain variable region of the rat anti-human CDH6 antibody with other amino acid residues. This antibody may have any given human-derived constant region.

Examples of the chimeric antibody derived from the rG019 antibody include an antibody consisting of a light chain comprising a light chain variable region consisting of the amino acid sequence shown in SEQ ID NO: 10, and a heavy chain comprising a heavy chain variable region consisting of the amino acid sequence shown in SEQ ID NO: 15. This antibody may have any given human-derived constant region.

Other examples of the chimeric antibody derived from the rG019 antibody include an antibody consisting of a light chain comprising a light chain variable region having a substitution of one to several residues, 1 to 3 residues, 1 or 2 residues, preferably 1 residue, of amino acids in the light chain variable region consisting of the amino acid sequence shown in SEQ ID NO: 10 with other amino acid residues, and a heavy chain comprising a heavy chain variable region having a substitution of one to several residues, 1 to 3 residues, 1 or 2 residues, preferably 1 residue, of amino acids in the heavy chain variable region consisting of the amino acid sequence shown in SEQ ID NO: 15 with other amino acid residues. This antibody may have any given human-derived constant region.

Other examples of the chimeric antibody derived from the rG019 antibody include an antibody consisting of a light chain comprising a light chain variable region having a substitution of 1 or 2 residues (preferably 1 residue) of amino acids in any 1 to 3 CDRs in the light chain variable region consisting of the amino acid sequence shown in SEQ ID NO: 10 with other amino acid residues, and a heavy chain comprising a heavy chain variable region having a substitution of 1 or 2 residues (preferably 1 residue) of amino acids in any 1 to 3 CDRs in the heavy chain variable region consisting of the amino acid sequence shown in SEQ ID NO: 15 with other amino acid residues. This antibody may have any given human-derived constant region.

Other examples of the chimeric antibody derived from the rG019 antibody include an antibody consisting of a light chain comprising a light chain variable region consisting of the amino acid sequence shown in SEQ ID NO: 10, and a heavy chain comprising a heavy chain variable region consisting of the amino acid sequence shown in SEQ ID NO: 28. This antibody may have any given human-derived constant region. The amino acid sequence shown in SEQ ID NO: 28 is a sequence with a cysteine residue substituted with a proline residue in CDRH2 in the amino acid sequence shown in SEQ ID NO: 15.

Specific examples of the chimeric antibody derived from the rG019 antibody include an antibody consisting of a light chain consisting of the light chain full-length amino acid sequence shown in SEQ ID NO: 23, and a heavy chain consisting of the heavy chain full-length amino acid sequence shown in SEQ ID NO: 26. In the present description, this chimeric anti-human CDH6 antibody is referred to as a "chimeric G019 antibody", a "chG019 antibody" or "chG019". The light chain full-length amino acid sequence of the chG019 antibody is encoded by the nucleotide sequence shown in SEQ ID NO: 24, and the heavy chain full-length amino acid sequence of the chG019 antibody is encoded by the nucleotide sequence shown in SEQ ID NO: 27.

The amino acid sequence of the light chain variable region of the chG019 antibody is identical to the amino acid sequence of the light chain variable region of the rG019 antibody, and consists of the amino acid sequence shown in SEQ ID NO: 10. The light chain of the chG019 antibody has CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14, which are identical to the light chain CDRL1, CDRL2 and CDRL3, respectively, of rG019. The amino acid of the light chain variable region of the chG019 antibody is encoded by the nucleotide sequence shown in SEQ ID NO: 25.

The amino acid sequence of the heavy chain variable region of the chG019 antibody consists of the amino acid sequence shown in SEQ ID NO: 28. The heavy chain of the chG019 antibody has CDRH1 consisting of the amino acid sequence shown in SEQ ID NO: 17, CDRH2 consisting of the amino acid sequence shown in SEQ ID NO: 30 and CDRH3 consisting of the amino acid sequence shown in SEQ ID NO: 19. The amino acid sequence shown in SEQ ID NO: 28 is a sequence with a cysteine residue substituted with a proline residue in CDRH2 in the amino acid sequence shown in SEQ ID NO: 15. The CDRH2 consisting of the amino acid sequence shown in SEQ ID NO: 30 is a sequence with a cysteine residue substituted with a proline residue in the rG019 CDRH2 shown in SEQ ID NO: 18. The amino acid sequence of the heavy chain variable region of the chG019 antibody is encoded by the nucleotide sequence shown in SEQ ID NO: 29.

Examples of the humanized antibody can include an antibody formed by incorporating only complementarity determining regions (CDRs) into a human-derived antibody (see Nature (1986) 321, p. 522-525), an antibody formed by incorporating the amino acid residues from some frameworks, as well as CDR sequences, into a human antibody according to a CDR grafting method (International Publication No. WO90/07861), and an antibody formed by modifying the amino acid sequences of some CDRs while maintaining antigen-binding ability.

In the present description, the humanized antibody derived from the rG019 antibody or the chG019 antibody is not limited to a specific humanized antibody as long as the humanized antibody retains all 6 CDR sequences unique to the rG019 antibody or the chG019 antibody and has internalization activity. The amino acid sequences of some CDRs of this humanized antibody may be further modified as long as the antibody has internalization activity.

Concrete examples of the humanized antibody of the chG019 antibody can include any given combination of: a light chain comprising a light chain variable region consisting of any one amino acid sequence selected from the group consisting of (1) the light chain variable region consisting of the amino acid sequence shown in SEQ ID NO: 33 or 37, (2) an amino acid sequence having an identity of at least 95% (preferably an amino acid sequence having a sequence identity of at least 95% to the sequence of a framework region other than at each CDR sequence) to the above-described amino acid sequence (1), and (3) an amino acid sequence comprising a deletion, substitution or addition of one or several amino acids in the above-described amino acid sequence (1); and a heavy chain comprising a heavy chain variable region consisting of any one amino acid sequence selected from the group consisting of (4) the heavy chain variable region consisting of the amino acid sequence shown in SEQ ID NO: 41, 45, 49, 55 or 60, (5) an amino acid sequence having an identity of at least 95% (preferably an amino acid sequence having a sequence identity of at least 95% to the sequence of a framework region other than at each CDR sequence) to the above-described amino acid sequence (4), and (6) an amino acid sequence comprising a deletion, substitution or addition of one or several amino acids in the above-described amino acid sequence (4).

Alternatively, an antibody having a humanized heavy chain or light chain and the other chain derived from a rat antibody or a chimeric antibody can also be used. Examples of such an antibody can include any given combination of: a light chain comprising a light chain variable region consisting of any one amino acid sequence selected from the group consisting of (1) the light chain variable region consisting of the amino acid sequence shown in SEQ ID NO: 33 or 37, (2) an amino acid sequence having an identity of at least 95% (preferably an amino acid sequence having a sequence identity of at least 95% to the sequence of a framework region other than at each CDR sequence) to the above-described amino acid sequence (1), and (3) an amino acid sequence comprising a deletion, substitution or addition of one or several amino acids in the above-described amino acid sequence (1); and a heavy chain comprising a heavy chain variable region consisting of any one amino acid sequence selected from the group consisting of (4) the heavy chain variable region consisting of the amino acid sequence shown in SEQ ID NO: 15 or 28, (5) an amino acid sequence having an identity of at least 95% (preferably an amino acid sequence having a sequence identity of at least 95% to the sequence of a framework region other than at each CDR sequence) to the above-described amino acid sequence (4), and (6) an amino acid sequence comprising a deletion, substitution or addition of one or several amino acids in the above-described amino acid sequence (4). Other examples of such an antibody can include any given combination of: a light chain comprising a light chain variable region consisting of any one amino acid sequence selected from the group consisting of (1) the light chain variable region consisting of the amino acid sequence shown in SEQ ID NO: 10, (2) an amino acid sequence having an identity of at least 95% (preferably an amino acid sequence having a sequence identity of at least 95% to the sequence of a framework region other than at each CDR sequence) to the above-described amino acid sequence (1), and (3) an amino acid sequence comprising a deletion, substitution or addition of one or several amino acids in the above-described amino acid sequence (1); and a heavy chain comprising a heavy chain variable

region consisting of any one amino acid sequence selected from the group consisting of (4) the heavy chain variable region consisting of the amino acid sequence shown in SEQ ID NO: 41, 45, 49, 55 or 60, (5) an amino acid sequence having an identity of at least 95% (preferably an amino acid sequence having a sequence identity of at least 95% to the sequence of a framework region other than at each CDR sequence) to the above-described amino acid sequence (4), and (6) an amino acid sequence comprising a deletion, substitution or addition of one or several amino acids in the above-described amino acid sequence (4).

The amino acid substitution in the present description is preferably a conservative amino acid substitution. The conservative amino acid substitution is a substitution occurring within an amino acid group associated with certain amino acid side chains. Preferred amino acid groups are the following: acidic group = aspartic acid and glutamic acid; basic group = lysine, arginine and histidine; non-polar group = alanine, valine, leucine, isoleucine, proline, phenylalanine, methionine and tryptophan; and uncharged polar family = glycine, asparagine, glutamine, cysteine, serine, threonine and tyrosine. Other preferred amino acid groups are the following: aliphatic hydroxy group = serine and threonine; amide-containing group = asparagine and glutamine; aliphatic group = alanine, valine, leucine and isoleucine; and aromatic group = phenylalanine, tryptophan and tyrosine. Such amino acid substitution is preferably carried out without impairing the properties of a substance having the original amino acid sequence.

Examples of the antibody having a preferred combination of the above-described light chains and heavy chains include an antibody consisting of a light chain having the light chain variable region amino acid sequence shown in SEQ ID NO: 33 (in the present description, also referred to as a hL02 light chain variable region amino acid sequence) or a light chain having the light chain variable region amino acid sequence shown in SEQ ID NO: 37 (in the present description, also referred to as a hL03 light chain variable region amino acid sequence), and a heavy chain having the heavy chain variable region amino acid sequence shown in SEQ ID NO: 41 (in the present description, also referred to as a hH01 heavy chain variable region amino acid sequence), a heavy chain having the heavy chain variable region amino acid sequence shown in SEQ ID NO: 45 (in the present description, also referred to as a hH02 heavy chain variable region amino acid sequence) or a heavy chain having the heavy chain variable region amino acid sequence shown in SEQ ID NO: 49 (in the present description, also referred to as a hH04 heavy chain variable region amino acid sequence), a heavy chain having the heavy chain variable region amino acid sequence shown in SEQ ID NO: 55 (in the present description, also referred to as a hH11 heavy chain variable region amino acid sequence) or a heavy chain having the heavy chain variable region amino acid sequence shown in SEQ ID NO: 60 (in the present description, also referred to as a hH31 heavy chain variable region amino acid sequence).

The hH11 heavy chain variable region amino acid sequence shown in SEQ ID NO: 55 has CDRH1 consisting of the amino acid sequence shown in SEQ ID NO: 57, CDRH2 consisting of the amino acid sequence shown in SEQ ID NO: 58 and CDRH3 consisting of the amino acid sequence shown in SEQ ID NO: 59. The hH31 heavy chain variable region amino acid sequence shown in SEQ ID NO: 60 has CDRH1 consisting of the amino acid sequence shown in SEQ ID NO: 62, CDRH2 consisting of the amino acid sequence shown in SEQ ID NO: 63 and CDRH3 consisting of the amino acid sequence shown in SEQ ID NO: 64.

Preferred examples of the antibody include:

an antibody consisting of a light chain having the light chain variable region amino acid sequence shown in SEQ ID NO: 33 and a heavy chain having the heavy chain variable region amino acid sequence shown in SEQ ID NO: 41;
an antibody consisting of a light chain having the light chain variable region amino acid sequence shown in SEQ ID NO: 33 and a heavy chain having the heavy chain variable region amino acid sequence shown in SEQ ID NO: 45;
an antibody consisting of a light chain having the light chain variable region amino acid sequence shown in SEQ ID NO: 33 and a heavy chain having the heavy chain variable region amino acid sequence shown in SEQ ID NO: 49;
an antibody consisting of a light chain having the light chain variable region amino acid sequence shown in SEQ ID NO: 33 and a heavy chain having the heavy chain variable region amino acid sequence shown in SEQ ID NO: 55;
an antibody consisting of a light chain having the light chain variable region amino acid sequence shown in SEQ ID NO: 33 and a heavy chain having the heavy chain variable region amino acid sequence shown in SEQ ID NO: 60;
an antibody consisting of a light chain having the light chain variable region amino acid sequence shown in SEQ ID NO: 37 and a heavy chain having the heavy chain variable region amino acid sequence shown in SEQ ID NO: 41;
an antibody consisting of a light chain having the light chain variable region amino acid sequence shown in SEQ ID NO: 37 and a heavy chain having the heavy chain variable region amino acid sequence shown in SEQ ID NO: 45;
an antibody consisting of a light chain having the light chain variable region amino acid sequence shown in SEQ ID NO: 37 and a heavy chain having the heavy chain variable region amino acid sequence shown in SEQ ID NO: 49;
an antibody consisting of a light chain having the light chain variable region amino acid sequence shown in SEQ ID NO: 37 and a heavy chain having the heavy chain variable region amino acid sequence shown in SEQ ID NO: 55; and
an antibody consisting of a light chain having the light chain variable region amino acid sequence shown in SEQ ID NO: 37 and a heavy chain having the heavy chain variable region amino acid sequence shown in SEQ ID NO: 60.

More preferred examples of the antibody include:

an antibody consisting of a light chain having the light chain variable region amino acid sequence shown in SEQ ID NO: 33 and a heavy chain having the heavy chain variable region amino acid sequence shown in SEQ ID NO: 41;
an antibody consisting of a light chain having the light chain variable region amino acid sequence shown in SEQ ID NO: 33 and a heavy chain having the heavy chain variable region amino acid sequence shown in SEQ ID NO: 45;
an antibody consisting of a light chain having the light chain variable region amino acid sequence shown in SEQ ID NO: 33 and a heavy chain having the heavy chain variable region amino acid sequence shown in SEQ ID NO: 49;
an antibody consisting of a light chain having the light chain variable region amino acid sequence shown in SEQ ID NO: 37 and a heavy chain having the heavy chain variable region amino acid sequence shown in SEQ ID NO: 45;
an antibody consisting of a light chain having the light chain variable region amino acid sequence shown in SEQ ID NO: 33 and a heavy chain having the heavy chain variable region amino acid sequence shown in SEQ ID NO: 55; and
an antibody consisting of a light chain having the light chain variable region amino acid sequence shown in SEQ ID NO: 33 and a heavy chain having the heavy chain variable region amino acid sequence shown in SEQ ID NO: 60.

Other examples of the antibody having a preferred combination of light chains and heavy chains include an antibody consisting of a light chain consisting of the amino acid sequence at positions 21 to 233 in the light chain full-length amino acid sequence shown in SEQ ID NO: 31 (in the present description, also referred to as the hL02 light chain full-length amino acid sequence) or a light chain consisting of the amino acid sequence at positions 21 to 233 in the light chain full-length amino acid sequence shown in SEQ ID NO: 35 (in the present description, also referred to as the hL03 light chain full-length amino acid sequence), and a heavy chain consisting of the amino acid sequence at positions 20 to 471 in the heavy chain full-length amino acid sequence shown in SEQ ID NO: 39 (in the present description, also referred to as the hH01 heavy chain full-length amino acid sequence), a heavy chain consisting of the amino acid sequence at positions 20 to 471 in the heavy chain full-length amino acid sequence shown in SEQ ID NO: 43 (in the present description, also referred to as the hH02 heavy chain full-length amino acid sequence), a heavy chain consisting of the amino acid sequence at positions 20 to 471 in the heavy chain full-length amino acid sequence shown in SEQ ID NO: 47 (in the present description, also referred to as the hH04 heavy chain full-length amino acid sequence), a heavy chain consisting of the amino acid sequence at positions 20 to 471 in the heavy chain full-length amino acid sequence shown in SEQ ID NO: 65 (in the present description, also referred to as the hH01A heavy chain full-length amino acid sequence), a heavy chain consisting of the amino acid sequence at positions 20 to 471 in the heavy chain full-length amino acid sequence shown in SEQ ID NO: 67 (in the present description, also referred to as the hH11A heavy chain full-length amino acid sequence) or a heavy chain consisting of the amino acid sequence at positions 20 to 471 in the heavy chain full-length amino acid sequence shown in SEQ ID NO: 69 (in the present description, also referred to as the hH31A heavy chain full-length amino acid sequence).

Examples of another preferable antibody include,

an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 39,
an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 43,
an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 47,
an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 65,
an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 67,
an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 69,
an antibody consisting of a light chain consisting of the amino acid sequence at positions 21 to 233 in the light chain full-length amino acid sequence shown in SEQ ID NO: 35 and a heavy chain consisting of the amino acid sequence at positions 20 to 471 in the heavy chain full-length amino acid sequence shown in SEQ ID NO: 39,
an antibody consisting of a light chain consisting of the amino acid sequence at positions 21 to 233 in the light chain

full-length amino acid sequence shown in SEQ ID NO: 35 and a heavy chain consisting of the amino acid sequence at positions 20 to 471 in the heavy chain full-length amino acid sequence shown in SEQ ID NO: 43,

an antibody consisting of a light chain consisting of the amino acid sequence at positions 21 to 233 in the light chain full-length amino acid sequence shown in SEQ ID NO: 35 and a heavy chain consisting of the amino acid sequence at positions 20 to 471 in the heavy chain full-length amino acid sequence shown in SEQ ID NO: 47,

an antibody consisting of a light chain consisting of the amino acid sequence at positions 21 to 233 in the light chain full-length amino acid sequence shown in SEQ ID NO: 35 and a heavy chain consisting of the amino acid sequence at positions 20 to 471 in the heavy chain full-length amino acid sequence shown in SEQ ID NO: 65,

an antibody consisting of a light chain consisting of the amino acid sequence at positions 21 to 233 in the light chain full-length amino acid sequence shown in SEQ ID NO: 35 and a heavy chain consisting of the amino acid sequence at positions 20 to 471 in the heavy chain full-length amino acid sequence shown in SEQ ID NO: 67, or

an antibody consisting of a light chain consisting of the amino acid sequence at positions 21 to 233 in the light chain full-length amino acid sequence shown in SEQ ID NO: 35 and a heavy chain consisting of the amino acid sequence at positions 20 to 471 in the heavy chain full-length amino acid sequence shown in SEQ ID NO: 69.

Examples of a more preferable antibody include

an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 39 (in the present description, also referred to as the "H01L02 antibody" or "H01L02"),

an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 43 (in the present description, also referred to as the "H02L02 antibody" or "H02L02"),

an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 47 (in the present description, also referred to as the "H04L02 antibody" or "H04L02"),

an antibody consisting of a light chain consisting of the amino acid sequence at positions 21 to 233 in the light chain full-length amino acid sequence shown in SEQ ID NO: 35 and a heavy chain consisting of the amino acid sequence at positions 20 to 471 in the heavy chain full-length amino acid sequence shown in SEQ ID NO: 43 (in the present description, also referred to as the "H02L03 antibody" or "H02L03"),

an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 65 (in the present description, also referred to as the "H01L02A antibody" or "H01L02A),

an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 67 (in the present description, also referred to as the "H11L02A antibody" or "H11L02A), and

an antibody consisting of a light chain consisting of the amino acid sequence at positions 21 to 233 in the light chain full-length amino acid sequence shown in SEQ ID NO: 31 and a heavy chain consisting of the amino acid sequence at positions 20 to 471 in the heavy chain full-length amino acid sequence shown in SEQ ID NO: 69 (in the present description, also referred to as the "H31L02A antibody" or "H31L02A).

By combining together sequences showing a high identity to the above-described heavy chain amino acid sequences and light chain amino acid sequences, it is possible to select an antibody having a biological activity equivalent to that of each of the above-described antibodies. Such an identity is an identity of generally 80% or more, preferably 90% or more, more preferably 95% or more and most preferably 99% or more. Moreover, also by combining amino acid sequences of a heavy chain and a light chain comprising a substitution, deletion or addition of one or several amino acid residues thereof with respect to the amino acid sequence of a heavy chain or a light chain, it is possible to select an antibody having a biological activity equivalent to that of each of the above-described antibodies.

The identity between two types of amino acid sequences can be determined by aligning the sequences using the default parameters of Clustal W version 2 (Larkin MA, Blackshields G, Brown NP, Chenna R, McGettigan PA, McWilliam H, Valentin F, Wallace IM, Wilm A, Lopez R, Thompson JD, Gibson TJ and Higgins DG (2007), "Clustal W and Clustal X version 2.0", Bioinformatics.23 (21): 2947-2948).

It is to be noted that, in the hL02 light chain full-length amino acid sequence shown in SEQ ID NO: 31, the

amino acid sequence consisting of the amino acid residues at positions 1 to 20 is a signal sequence, the amino acid sequence consisting of the amino acid residues at positions 21 to 128 is a variable region, and the amino acid sequence consisting of the amino acid residues at positions 129 to 233 is a constant region.

In the hL03 light chain full-length amino acid sequence shown in SEQ ID NO: 35, the amino acid sequence consisting of the amino acid residues at positions 1 to 20 is a signal sequence, the amino acid sequence consisting of the amino acid residues at positions 21 to 128 is a variable region, and the amino acid sequence consisting of the amino acid residues at positions 129 to 233 is a constant region.

In the hH01 heavy chain full-length amino acid sequence shown in SEQ ID NO: 39, the amino acid sequence consisting of the amino acid residues at positions 1 to 19 is the signal sequence, the amino acid sequence consisting of the amino acid residues at positions 20 to 141 is a variable region, and the amino acid sequence consisting of the amino acid residues at positions 142 to 471 is a constant region.

In the hH02 heavy chain full-length amino acid sequence shown in SEQ ID NO: 43, the amino acid sequence consisting of the amino acid residues at positions 1 to 19 is a signal sequence, the amino acid sequence consisting of the amino acid residues at positions 20 to 141 is a variable region, and the amino acid sequence consisting of the amino acid residues at positions 142 to 471 is a constant region.

In the hH04 heavy chain full-length amino acid sequence shown in SEQ ID NO: 47, the amino acid sequence consisting of the amino acid residues at positions 1 to 19 is a signal sequence, the amino acid sequence consisting of the amino acid residues at positions 20 to 141 is a variable region, and the amino acid sequence consisting of the amino acid residues at positions 142 to 471 is a constant region.

In the hH01A heavy chain full-length amino acid sequence shown in SEQ ID NO: 65, the amino acid sequence consisting of the amino acid residues at positions 1 to 19 is a signal sequence, the amino acid sequence consisting of the amino acid residues at positions 20 to 141 is a variable region, and the amino acid sequence consisting of the amino acid residues at positions 142 to 471 is a constant region.

In the hH11A heavy chain full-length amino acid sequence shown in SEQ ID NO: 67, the amino acid sequence consisting of the amino acid residues at positions 1 to 19 is a signal sequence, the amino acid sequence consisting of the amino acid residues at positions 20 to 141 is a variable region, and the amino acid sequence consisting of the amino acid residues at positions 142 to 471 is a constant region.

In the hH31A heavy chain full-length amino acid sequence shown in SEQ ID NO: 69, the amino acid sequence consisting of the amino acid residues at positions 1 to 19 is a signal sequence, the amino acid sequence consisting of the amino acid residues at positions 20 to 141 is a variable region, and the amino acid sequence consisting of the amino acid residues at positions 142 to 471 is a constant region.

Further example of the antibody of the present invention can include a human antibody binding to CDH6. The anti-CDH6 human antibody refers to a human antibody having only the gene sequence of an antibody derived from human chromosomes. The anti-CDH6 human antibody can be obtained by a method using a human antibody-producing mouse having a human chromosomal fragment comprising the heavy chain and light chain genes of a human antibody (see, Tomizuka, K. et al., Nature Genetics (1997) 16, p. 133-143; Kuroiwa, Y. et al., Nucl. Acids Res. (1998) 26, p. 3447-3448; Yoshida, H. et al., Animal Cell Technology: Basic and Applied Aspects vol. 10, p. 69-73 (Kitagawa, Y., Matsuda, T. and Iijima, S. eds.), Kluwer Academic Publishers, 1999; Tomizuka, K. et al., Proc. Natl. Acad. Sci. USA (2000) 97, p. 722-727; etc.).

Such a human antibody-producing mouse can be specifically produced by using a genetically modified animal, the gene loci of endogenous immunoglobulin heavy chain and light chain of which have been disrupted and instead the gene loci of the human immunoglobulin heavy chain and light chain have been then introduced using a yeast artificial chromosome (YAC) vector or the like, then producing a knock-out animal and a transgenic animal from such a genetically modified animal, and then breeding such animals with one another.

Otherwise, the anti-CDH6 human antibody can also be obtained by transforming eukaryotic cells with cDNA encoding each of the heavy chain and light chain of such a human antibody, or preferably with a vector comprising such cDNA, according to genetic recombination techniques, and then culturing the transformed cells and producing a genetically modified human monoclonal antibody, so that the antibody can be obtained from the culture supernatant.

In this context, eukaryotic cells, and preferably, mammalian cells such as CHO cells, lymphocytes or myelomas can, for example, be used as a host.

Also known is a method of obtaining a phage display-derived human antibody that has been selected from a human antibody library (see Wormstone, I. M. et al., Investigative Ophthalmology & Visual Science. (2002) 43 (7), p. 2301-2308; Carmen, S. et al., Briefings in Functional Genomics and Proteomics (2002), 1 (2), p. 189-203; Siriwardena, D. et al., Ophthalmology (2002) 109 (3), p. 427-431; etc.).

For example, a phage display method, which comprises allowing the variable regions of a human antibody to express as a single chain antibody (scFv) on the surface of phages, and then selecting a phage binding to an antigen, can be applied (Nature Biotechnology (2005), 23, (9), p. 1105-1116).

By analyzing the phage gene that has been selected because of its binding ability to the antigen, the DNA

sequence encoding the variable region of a human antibody binding to the antigen can be determined.

Once the DNA sequence of scFv binding to the antigen is determined, an expression vector having the aforementioned sequence is produced, and the produced expression vector is then introduced into an appropriate host and can be allowed to express therein, thereby obtaining a human antibody (International Publication Nos. WO92/01047, WO92/20791, WO93/06213, WO93/11236, WO93/19172, WO95/01438 and WO95/15388, Annu. Rev. Immunol (1994) 12, p. 433-455, Nature Biotechnology (2005) 23 (9), p. 1105-1116).

If a newly produced human antibody binds to a partial peptide or a partial three-dimensional structure to which any one rat anti-human CDH6 antibody, chimeric anti-human CDH6 antibody or humanized anti-human CDH6 antibody described in the present description (e.g., the rG019 antibody, the chG019 antibody, the H01L02 antibody, the H02L02 antibody, the H02L03 antibody, the H04L02 antibody, the H01L02A antibody, the H11L02A antibody or the H31L02A antibody) binds, it can be determined that the human antibody binds to the same epitope to which the rat anti-human CDH6 antibody, the chimeric anti-human CDH6 antibody or the humanized anti-human CDH6 antibody binds. Alternatively, by confirming that the human antibody competes with the rat anti-human CDH6 antibody, the chimeric anti-human CDH6 antibody or the humanized anti-human CDH6 antibody described in the present description (e.g., the rG019 antibody, the chG019 antibody, the H01L02 antibody, the H02L02 antibody, the H02L03 antibody, the H04L02 antibody, the H01L02A antibody, the H11L02A antibody or the H31L02A antibody) in the binding of the antibody to CDH6 (e.g., the human antibody interferes with the binding of the rG019 antibody, the chG019 antibody, the H01L02 antibody, the H02L02 antibody, the H02L03 antibody, the H04L02 antibody, the H01L02A antibody, the H11L02A antibody or the H31L02A antibody to CDH6, preferably EC3 of CDH6), it can be determined that the human antibody binds to the same epitope to which the rat anti-human CDH6 antibody, the chimeric anti-human CDH6 antibody or the humanized anti-human CDH6 antibody described in the present description binds, even if the specific sequence or structure of the epitope has not been determined. In the present description, when it is determined by at least one of these determination methods that the human antibody "binds to the same epitope", it is concluded that the newly prepared human antibody "binds to the same epitope" as that for the rat anti-human CDH6 antibody, the chimeric anti-human CDH6 antibody or the humanized anti-human CDH6 antibody described in the present description. When it is confirmed that the human antibody binds to the same epitope, then it is expected that the human antibody should have a biological activity equivalent to that of the rat anti-human CDH6 antibody, the chimeric anti-human CDH6 antibody or the humanized anti-human CDH6 antibody (e.g., the rG019 antibody, the chG019 antibody, the H01L02 antibody, the H02L02 antibody, the H02L03 antibody, the H04L02 antibody, the H01L02A antibody, the H11L02A antibody or the H31L02A antibody).

The chimeric antibodies, the humanized antibodies or the human antibodies obtained by the above-described methods are evaluated for their binding activity against the antigen according to a known method, etc., so that a preferred antibody can be selected.

One example of another indicator for comparison of the properties of antibodies can include the stability of an antibody. A differential scanning calorimeter (DSC) is an apparatus capable of promptly and exactly measuring a thermal denaturation midpoint (Tm) serving as a good indicator for the relative structural stability of a protein. By using DSC to measure Tm values and making a comparison regarding the obtained values, differences in thermal stability can be compared. It is known that the preservation stability of an antibody has a certain correlation with the thermal stability of the antibody (Lori Burton, et al., Pharmaceutical Development and Technology (2007) 12, p. 265-273), and thus, a preferred antibody can be selected using thermal stability as an indicator. Other examples of an indicator for selection of an antibody can include high yield in suitable host cells and low agglutination in an aqueous solution. For example, since an antibody with the highest yield does not always exhibit the highest thermal stability, it is necessary to select an antibody most suitable for administration to a human by comprehensively determining it based on the aforementioned indicators.

The antibody of the present invention also includes a modification of an antibody. A modification is used to mean an antibody of the present invention, which is chemically or biologically modified. Examples of such a chemical modification include the binding of a chemical moiety to an amino acid skeleton, and the chemical modification of an N-linked or O-linked carbohydrate chain. Examples of such a biological modification include antibodies which have undergone a posttranslational modification (e.g., N-linked or O-linked glycosylation, N-terminal or C-terminal processing, deamidation, isomerization of aspartic acid, oxidation of methionine, and conversion of N-terminal glutamine or N-terminal glutamic acid to pyroglutamic acid), and antibodies, to the N-terminus of which, a methionine residue is added as a result of having been allowed to be expressed using prokaryote host cells. In addition, such a modification is also meant to include labeled antibodies for enabling detection or isolation of the antibody of the present invention or an antigen, for example, an enzymatically labeled antibody, a fluorescently labeled antibody, and an affinity-labeled antibody. Such a modification of the antibody of the present invention is useful for the improvement of the stability and retention in blood of an antibody; a reduction in antigenicity; detection or isolation of an antibody or an antigen; etc.

Moreover, by regulating a sugar chain modification (glycosylation, de-fucosylation, etc.) that binds to the antibody of the present invention, antibody-dependent cellular cytotoxic activity can be enhanced. As techniques of regulating the sugar chain modification of an antibody, those described in International Publication Nos. WO1999/54342,

WO2000/61739, WO2002/31140 and WO2007/133855, etc. are known, though the techniques are not limited thereto. The antibody of the present invention also includes antibodies in respect of which the aforementioned sugar chain modification has been regulated.

Once an antibody gene is isolated, the gene can be introduced into an appropriate host to produce an antibody, using an appropriate combination of a host and an expression vector. A specific example of the antibody gene can be a combination of a gene encoding the heavy chain sequence of the antibody described in the present description and a gene encoding the light chain sequence of the antibody described therein. Upon transformation of host cells, such a heavy chain sequence gene and a light chain sequence gene may be inserted into a single expression vector, or these genes may instead each be inserted into different expression vectors.

When eukaryotic cells are used as hosts, animal cells, plant cells or eukaryotic microorganisms can be used. In particular, examples of the animal cells can include mammalian cells such as COS cells which are monkey cells (Gluzman, Y., Cell (1981) 23, p. 175-182, ATCC CRL-1650), mouse fibroblasts NIH3T3 (ATCC No. CRL-1658), a dihydrofolate reductase-deficient cell line of Chinese hamster ovary cells (CHO cells, ATCC CCL-61) (Urlaub, G. and Chasin, L. A. Proc. Natl. Acad. Sci. U.S.A. (1980) 77, p. 4126-4220) and FreeStyle 293F cells (Invitrogen Corp.).

When prokaryotic cells are used as hosts, *Escherichia coli* or *Bacillus subtilis* can be used, for example.

An antibody gene of interest is introduced into these cells for transformation, and the transformed cells are then cultured *in vitro* to obtain an antibody. In the aforementioned culture, there are cases where yield is different depending on the sequence of the antibody, and thus, it is possible to select an antibody, which is easily produced as a medicament, from antibodies having equivalent binding activity, using the yield as an indicator. Accordingly, the antibody of the present invention also includes an antibody obtained by the above-described method for producing an antibody, which comprises a step of culturing the transformed host cells and a step of collecting an antibody of interest or a functional fragment of the antibody from the culture obtained in the aforementioned step.

It is known that the lysine residue at the carboxyl terminal of the heavy chain of an antibody produced by culturing mammalian cells is deleted (Journal of Chromatography A, 705: 129-134 (1995)), and also, it is known that the two amino acid residues at the heavy chain carboxyl terminus, glycine and lysine, are deleted, and that the proline residue newly positioned at the carboxyl terminus is amidated (Analytical Biochemistry, 360: 75-83 (2007)). However, such deletion and modification of these heavy chain sequences does not have an influence on the antigen-binding activity and effector function (activation of complement, antibody-dependent cellular cytotoxicity, etc.) of an antibody. Accordingly, the antibody according to the present invention also includes an antibody that has undergone the aforementioned modification, and a functional fragment of the antibody, and specific examples of such an antibody include a deletion mutant comprising a deletion of 1 or 2 amino acids at the heavy chain carboxyl terminus, and a deletion mutant formed by amidating the aforementioned deletion mutant (e.g., a heavy chain in which the proline residue at the carboxyl-terminal site is amidated). However, deletion mutants involving a deletion at the carboxyl terminus of the heavy chain of the antibody according to the present invention are not limited to the above-described deletion mutants, as long as they retain antigen-binding activity and effector function. Two heavy chains constituting the antibody according to the present invention may be any one type of heavy chain selected from the group consisting of a full-length antibody and the above-described deletion mutants, or may be a combination of any two types selected from the aforementioned group. The ratio of individual deletion mutants can be influenced by the types of cultured mammalian cells that produce the antibody according to the present invention, and the culture conditions. Examples of the main ingredient of the antibody according to the present invention can include antibodies where one amino acid residue is deleted at each of the carboxyl termini of the two heavy chains.

Examples of the isotype of the antibody of the present invention can include IgG (IgG1, IgG3 and IgG4). Among others, IgG1, IgG2 and IgG4 are preferable.

If IgG1 is used as the isotype of the antibody of the present invention, the effector function may be adjusted by substituting some amino acid residues in the constant region. Examples of variants of IgG1 with the effector function lowered or attenuated may include, but not limited to, IgG1 LALA (IgG1-L234A, L235A) and IgG1 LAGA (IgG1-L235A, G237A) (Journal of Virology, Vol. 75, No. 24(Dec. 15, 2001), pp. 12161-12168), and a preferred variant of IgG1 is IgG1 LALA. The L234A, L235A indicates substitution of leucine with alanine at the 234- and 235-positions specified by EU-index numbering (Proc. Natl. Acad. Sci. U.S.A., Vol. 63, No. 1 (May 15, 1969), pp. 78-85), and the G237A indicates substitution of glycine with alanine at the 237-position specified by EU-index numbering.

Typical examples of bioactivity of antibodies may include, but are not limited to, antigen-binding activity, activity to internalize in cells expressing an antigen by binding to the antigen, activity to neutralize antigen activity, activity to enhance antigen activity, antibody-dependent cellular cytotoxicity (ADCC), complement-dependent cytotoxicity (CDC), and antibody-dependent cellular phagocytosis (ADCP), and the function of the antibody according to the present invention is binding activity to CDH6, and preferably activity to internalize in CDH6-expression cells by binding to CDH6. In addition to cellular internalization activity, the antibody of the present invention may have activities of ADCC, CDC, and/or ADCP in combination.

The antibody obtained may be purified to a homogeneous state. For separation/purification of the antibody,

separation/purification methods commonly used for protein can be used. For example, the antibody may be separated/purified by appropriately selecting and combining column chromatography, filter filtration, ultrafiltration, salting-out, dialysis, preparative polyacrylamide gel electrophoresis, isoelectric focusing, and so on (Strategies for Protein Purification and Characterization: A Laboratory Course Manual, Daniel R. Marshak et al. eds., Cold Spring Harbor Laboratory Press (1996); and Antibodies: A Laboratory Manual. Ed Harlow and David Lane, Cold Spring Harbor Laboratory (1988)), but separation/purification methods are not limited thereto.

Examples of chromatography may include affinity chromatography, ion-exchange chromatography, hydrophobic chromatography, gel filtration chromatography, reversed-phase chromatography and absorption chromatography.

These chromatographies may be carried out using liquid chromatography such as HPLC or FPLC.

Examples of columns for affinity chromatography may include, but not limited to, a Protein A column and a Protein G column. Examples of columns that can be used as a Protein A column include Hyper D, POROS and Sepharose F. F. (Pharmacia).

Alternatively, the antibody may be purified by utilizing binding activity to an antigen with a carrier to which the antigen has been immobilized.

The present invention relates to a polynucleotide encoding the antibody of the present invention. The polynucleotide of the present invention is preferably a polynucleotide comprising the polynucleotide described in any one of the following (a) to (e):

(a) a combination of a polynucleotide encoding a heavy chain amino acid sequence and a polynucleotide encoding a light chain amino acid sequence of the CDH6 antibody of the present invention,

(b) a combination of a polynucleotide encoding a heavy chain amino acid sequence including the sequences of CDRH1 to CDRH3 and a polynucleotide encoding a light chain amino acid sequence including the sequences of CDRL1 to CDRL3 of any one of CDH6 antibodies of the present invention,

(c) a combination of a polynucleotide encoding a heavy chain amino acid sequence comprising the amino acid sequence of the heavy chain variable region and a polynucleotide encoding a light chain amino acid sequence comprising the amino acid sequence of the light chain variable region of the CDH6 antibody of the present invention,

(d) a polynucleotide that is hybridizable with nucleotides consisting of a polynucleotide complementary to the polynucleotide according to any one of (a) to (c) under stringent conditions and is encoding the amino acid sequence of the antibody capable of binding to CDH6, and

(e) a polynucleotide encoding the amino acid sequence of a polypeptide obtained by substituting, deleting, adding, or inserting 1 to 50, 1 to 45, 1 to 40, 1 to 35, 1 to 30, 1 to 25, 1 to 20, 1 to 15, 1 to 10, one to eight, one to six, one to five, one to four, one to three, one or two, or one amino acid in the polynucleotide according to any one of (a) to (c) and that is encoding the amino acid sequence of the antibody capable of binding to CDH6.

The present invention includes a nucleotide encoding the antibody of the present invention or a functional fragment of the antibody, or a modified variant of the antibody or functional fragment; a recombinant vector including the gene inserted therein; and a cell including the gene or the vector introduced therein.

The present invention includes a method for producing an antibody or a functional fragment of the antibody, or a modified variant of the antibody or functional fragment, the method including the steps of: culturing the cell; and collecting from the culture an antibody or a functional fragment of the antibody, or a modified variant of the antibody or functional fragment.

The amino acid sequences or nucleotide sequences of the antibodies of the present invention and the amino acid sequences or nucleotide sequences of the proteins used in the present invention are listed in Tables 1-1 to Tables 1-14.

[Table 1-1]

| SEQ ID NO | | Sequence |
|---|---|---|
| 1 | Amino acid sequence of human CDH6 ORF | MRTYRYFLLLFWVGQPYPTLSTPLSKRTSGFPAKKRALELSGNSKNELNRSKRSWMW<br>NQFFLLEEYTGSDYQYVGKLHSDQDRGDGSLKYILSGDGAGDLFIINENTGDIQATK<br>RLDREEKPVYILRAQAINRRTGRPVEPESEFIIKIHDINDNEPIFTKEVYTATVPEM<br>SDVGTFVVQVTATDADDPTYGNSAKVVYSILQGQPYFSVESETGIIKTALLNMDREN<br>REQYQVVIQAKDMGGQMGGLSGTTTVNITLTDVNDNPPRFPQSTYQFKTPESSPPGT<br>PIGRIKASDADVGENAEIEYSITDGEGLDMFDVITDQETQEGIITVKKLLDFEKKKV<br>YTLKVEASNPYVEPRFLYLGPFKDSATVRIVVEDVDEPPVFSKLAYILQIREDAQIN<br>TTIGSVTAQDPDAARNPVKYSVDRHTDMDRIFNIDSGNGSIFTSKLLDRETLLWHNI<br>TVIATEINNPKQSSRVPLYIKVLDVNDNAPEFAEFYETFVCEKAKADQLIQTLHAVD<br>KDDPYSGHQFSFSLAPEAASGSNFTIQDNKDNTAGILTRKNGYNRHEMSTYLLPVVI<br>SDNDYPVQSSTGTVTVRVCACDHHGNMQSCHAEALIHPTGLSTGALVAILLCIVILL<br>VTVVLFAALRRQRKKEPLIISKEDIRDNIVSYNDEGGGEEDTQAFDIGTLRNPEAIE<br>DNKLRRDIVPEALFLPRRTPTARDNTDVRDFINQRLKENDTDPTAPPYDSLATYAYE<br>GTGSVADSLSSLESVTTDADQDYDYLSDWGPRFKKLADMYGGVDSDKDS |
| 2 | HumanCDH6 EC1 | SWMWNQFFLLEEYTGSDYQYVGKLHSDQDRGDGSLKYILSGDGAGDLFIINENTGDI<br>QATKRLDREEKPVYILRAQAINRRTGRPVEPESEFIIKIHDINDNEPIF |
| 3 | HumanCDH6 EC2 | TKEVYTATVPEMSDVGTFVVQVTATDADDPTYGNSAKVVYSILQGQPYFSVESETGI<br>IKTALLNMDRENREQYQVVIQAKDMGGQMGGLSGTTTVNITLTDVNDNPPRF |
| 4 | HumanCDH6 EC3 | PQSTYQFKTPESSPPGTPIGRIKASDADVGENAEIEYSITDGEGLDMFDVITDQETQ<br>EGIITVKKLLDFEKKKVYTLKVEASNPYVEPRFLYLGPFKDSATVRIVVEDVDEPPV<br>F |
| 5 | HumanCDH6 EC4 | SKLAYILQIREDAQINTTIGSVTAQDPDAARNPVKYSVDRHTDMDRIFNIDSGNGSI<br>FTSKLLDRETLLWHNITVIATEINNPKQSSRVPLYIKVLDVNDNAP |
| 6 | HumanCDH6 EC5 | EFAEFYETFVCEKAKADQLIQTLHAVDKDDPYSGHQFSFSLAPEAASGSNFTIQDNK<br>DNTAGILTRKNGYNRHEMSTYLLPVVISDNDYPVQSSTGTVTVRVCACDHHGNMQSC<br>HAEALIHP |
| 7 | Amino acid sequence of cynomolgus CDH6 ORF | MRTYRYFLLLFWVGQPYPTLSTPLSKRTSGFPAKKRALELSGNSKNELNRSKRSWMW<br>NQFFLLEEYTGSDYQYVGKLHSDQDRGDGSLKYILSGDGAGDLFIINENTGDIQATK<br>RLDREEKPVYILRAQAINRRTGRPVEPESEFIIKIHDINDNEPIFTKEVYTATVPEM<br>SDVGTFVVQVTATDADDPTYGNSAKVVYSILQGQPYFSVESETGIIKTALLNMDREN<br>REQYQVVIQAKDMGGQMGGLSGTTTVNITLTDVNDNPPRFPQSTYQFKTPESSPPGT<br>PIGRIKASDADVGENAEIEYSITDGEGLDMFDVITDQETQEGIITVKKLLDFEKKKV<br>YTLKVEASNPHVEPRFLYLGPFKDSATVRIVVEDVDEPPVFSKLAYILQIREDAQIN<br>TTIGSVTAQDPDAARNPVKYSVDRHTDMDRIFNIDSGNGSIFTSKLLDRETLLWHNI<br>TVIATEINNPKQSSRVPLYIKVLDVNDNAPEFAEFYETFVCEKAKADQLIQTLRAVD<br>KDDPYSGHQFSFSLAPEAASGSNFTIQDNKDNTAGILTRKNGYNRHEMSTYLLPVVI<br>SDNDYPVQSSTGTVTVRVCACDHHGNMQSCHAEALIHPTGLSTGALVAILLCIVILL<br>VTVVLFAALRRQRKKEPLIISKEDIRDNIVSYNDEGGGEEDTQAFDIGTLRNPEAIE<br>DNKLRRDIVPEALFLPRRTPTARDNTDVRDFINQRLKENDTDPTAPPYDSLATYAYE<br>GTGSVADSLSSLESVTTDGDQDYDYLSDWGPRFKKLADMYGGVDSDKDS |
| 8 | Cynomolgus CDH6 primer 1 | CACCATGAGAACTTACCGCTACTTCTTGCTGCTC |

[Table 1-2]

| | | |
|---|---|---|
| 9 | Cynomolgus CDH6 primer 2 | TTAGGAGTCTTTGTCACTGTCCACTCCTCC |
| 10 | rG019 light-chain variable region amino acid sequence | DIQMTQSPSLLSASVGDRVTLNCKASQNIYKNLAWYQQKLGEGPKLLIYDANTLQTG<br>IPSRFSGSGSGSDFTLTISSLQPEDVATYFCQQYYSGWAFGGVTNLELKRA |

(continued)

| | | |
|---|---|---|
| 11 | rG019 light-chain variable region nucleotide sequence | GACATCCAGATGACCCAGTCTCCTTCACTCCTGTCTGCATCTGTGGGAGACAGAGTC ACTCTCAACTGCAAAGCAAGTCAGAATATTTATAAGAACTTAGCCTGGTATCAGCAA AAGCTTGGAGAAGGTCCCAAACTCCTGATTTATGATGCAAACACTTTGCAAACGGGC ATCCCATCAAGGTTCAGTGGCAGTGGATCTGGTTCAGATTTCACACTCACCATCAGC AGCCTGCAGCCTGAAGATGTTGCCACATATTTCTGCCAGCAGTACTATAGCGGGTGG GCGTTCGGTGGAGTCACCAACCTGGAATTGAAACGGGCT |
| 12 | rG019 CDRL1 | KASQNIYKNLA |
| 13 | rG019 CDRL2 | DANTLQT |
| 14 | rG019 CDRL3 | QQYYSGWA |
| 15 | rG019 heavy-chain variable region amino acid sequence | QVQLQQSGAELVKPGSSVKISCKASGYTFTRNFMHWIKQQPGNGLEWIGWIYCGDGE TEYNQKFNGKATLTADRSSSTAYMELSRLTSEDSAVYFCARGVYGGFAGGYFDFWGQ GVMVTVSS |
| 16 | rG019 heavy-chain variable region nucleotide sequence | CAGGTACAGCTGCAGCAATCTGGGGCTGAACTGGTGAAGCCTGGGTCCTCAGTGAAA ATTTCCTGCAAGGCTTCTGGCTACACCTTCACCAGGAACTTTATGCACTGGATAAAA CAGCAGCCTGGAAATGGCCTTGAGTGGATTGGGTGGATTTATTGTGGAGATGGTGAG ACAGAGTACAATCAAAAGTTCAATGGGAAGGCAACACTCACTGCGGACAGATCCTCC AGCACAGCCTATATGGAGCTCAGCAGACTGACATCTGAGGACTCTGCAGTCTATTTC TGTGCAAGAGGGGTTTACGGAGGGTTTGCCGGGGGCTACTTTGATTTCTGGGGCCAA GGAGTCATGGTCACAGTCTCCTCA |
| 17 | rG019 CDRH1 | GYTFTRNFMH |
| 18 | rG019 CDRH2 | WIYCGDGETE |
| 19 | rG019 CDRH3 | GVYGGFAGGYFDF |
| 20 | DNA fragment comprising DNA sequence encoding human light-chain signal sequence and human κ-chain constant region | gcctccggactctagagccaccATGGTGCTGCAGACCCAGGTGTTCATCTCCCTGCT GCTGTGGATCTCCGGCGCGTACGGCGATATCGTGATGATTAAACGTACGGTGGCCGC CCCCTCCGTGTTCATCTTCCCCCCCTCCGACGAGCAGCTGAAGTCCGGCACCGCCTC CGTGGTGTGCCTGCTGAATAACTTCTACCCCAGAGAGGCCAAGGTGCAGTGGAAGGT GGACAACGCCCTGCAGTCCGGGAACTCCCAGGAGAGCGTGACCGAGCAGGACAGCAA GGACAGCACCTACAGCCTGAGCAGCACCCTGACCCTGAGCAAAGCCGACTACGAGAA GCACAAGGTGTACGCCTGCGAGGTGACCCACCAGGGCCTGAGCTCCCCCGTCACCAA GAGCTTCAACAGGGGGGAGTGTtaggggcccgtttaaacgggggaggcta |

[Table 1-3]

| | | |
|---|---|---|
| 21 | DNA fragment comprising DNA sequence encoding human heavy-chain signal sequence and human IgG1 constant region | gcctccggactctagagccaccATGAAACACCTGTGGTTCTTCCTCCTGCTGGTGGC<br>AGCTCCCAGATGGGTGCTGAGCCAGGTGCAATTGTGCAGGCGGTTAGCTCAGCCTCC<br>ACCAAGGGCCCAAGCGTCTTCCCCCTGGCACCCTCCTCCAAGAGCACCTCTGGCGGC<br>ACAGCCGCCCTGGGCTGCCTGGTCAAGGACTACTTCCCCGAACCCGTGACCGTGAGC<br>TGGAACTCAGGCGCCCTGACCAGCGGCGTGCACACCTTCCCCGCTGTCCTGCAGTCC<br>TCAGGACTCTACTCCCTCAGCAGCGTGGTGACCGTGCCCTCCAGCAGCTTGGGCACC<br>CAGACCTACATCTGCAACGTGAATCACAAGCCCAGCAACACCAAGGTGGACAAGAGA<br>GTTGAGCCCAAATCTTGTGACAAAACTCACACATGCCCACCCTGCCCAGCACCTGAA<br>CTCCTGGGGGGACCCTCAGTCTTCCTCTTCCCCCCAAAACCCAAGGACACCCTCATG<br>ATCTCCCGGACCCCTGAGGTCACATGCGTGGTGGTGGACGTGAGCCACGAAGACCCT<br>GAGGTCAAGTTCAACTGGTACGTGGACGGCGTGGAGGTGCATAATGCCAAGACAAAG<br>CCCCGGGAGGAGCAGTACAACAGCACGTACCGGGTGGTCAGCGTCCTCACCGTCCTG<br>CACCAGGACTGGCTGAATGGCAAGGAGTACAAGTGCAAGGTCTCCAACAAAGCCCTC<br>CCAGCCCCCATCGAGAAAACCATCTCCAAAGCCAAAGGCCAGCCCCGGGAACCACAG<br>GTGTACACCCTGCCCCCATCCCGGGAGGAGATGACCAAGAACCAGGTCAGCCTGACC<br>TGCCTGGTCAAAGGCTTCTATCCCAGCGACATCGCCGTGGAGTGGGAGAGCAATGGC<br>CAGCCCGAGAACAACTACAAGACCACCCCTCCCGTGCTGGACTCCGACGGCTCCTTC<br>TTCCTCTACAGCAAGCTCACCGTGGACAAGAGCAGGTGGCAGCAGGGCAACGTCTTC<br>TCATGCTCCGTGATGCATGAGGCTCTGCACAACCACTACACCCAGAAGAGCCTCTCC<br>CTGTCTCCCGGCAAAtgagatatcgggcccgtttaaacgggggaggcta |
| 22 | DNA fragment comprising DNA sequence encoding chG019 light-chain | ccagcctccggactctagagccaccATGGTGCTGCAGACCCAGGTGTTCATCAGCCT<br>GCTGCTGTGGATCAGCGGCGCCTACGGCGACATCCAGATGACCCAGAGCCCTAGCCT<br>GCTGAGCGCCAGCGTGGGCGATAGAGTGACCCTGAACTGCAAGGCCAGCCAGAACAT<br>CTACAAGAACCTGGCCTGGTATCAGCAGAAGCTGGGCGAGGGCCCCAAGCTGCTGAT<br>CTACGACGCCAACACCCTGCAGACCGGCATCCCCAGCAGATTTTCTGGCAGCGGCAG<br>CGGCTCCGACTTCACCCTGACAATCAGCAGCCTGCAGCCCGAGGACGTGGCCACCTA<br>CTTTTGCCAGCAGTACTACAGCGGCTGGGCCTTCGGCGGCGTGACCAACCTGGAACT<br>GAAGAGAGCCGTGGCCGCTCCCTCCGTGTTCATCTTCCCACCTAGCGACGAGCAGCT<br>GAAGTCCGGCACAGCCTCTGTCGTGTGCCTGCTGAACAACTTCTACCCCCGCGAGGC<br>CAAGGTGCAGTGGAAGGTGGACAATGCCCTGCAGTCTGGCAACAGCCAGGAAAGCGT<br>GACCGAGCAGGACAGCAAGGACTCCACCTACAGCCTGAGCAGCACCCTGACCCTGAG<br>CAAGGCCGACTACGAGAAGCACAAGGTGTACGCCTGCGAAGTGACCCACCAGGGCCT<br>GTCTAGCCCCGTGACCAAGAGCTTCAACCGGGGCGAGTGTtgagtttaaacgggga<br>ggctaact |
| 23 | chG019 light chain full-length amino acid sequence | MVLQTQVFISLLLWISGAYGDIQMTQSPSLLSASVGDRVTLNCKASQNIYKNLAWYQ<br>QKLGEGPKLLIYDANTLQTGIPSRFSGSGSGSDFTLTISSLQPEDVATYFCQQYYSG<br>WAFGGVTNLELKRAVAAPSVFIFPPSDEQLKSGTASVVCLLNNFYPREAKVQWKVDN<br>ALQSGNSQESVTEQDSKDSTYSLSSTLTLSKADYEKHKVYACEVTHQGLSSPVTKSF<br>NRGEC |

[Table 1-4]

| | | |
|---|---|---|
| 24 | chG019 light chain full-length nucleotide sequence | ATGGTGCTGCAGACCCAGGTGTTCATCAGCCTGCTGCTGTGGATCAGCGGCGCCTAC<br>GGCGACATCCAGATGACCCAGAGCCCTAGCCTGCTGAGCGCCAGCGTGGGCGATAGA<br>GTGACCCTGAACTGCAAGGCCAGCCAGAACATCTACAAGAACCTGGCCTGGTATCAG<br>CAGAAGCTGGGCGAGGGCCCCAAGCTGCTGATCTACGACGCCAACACCCTGCAGACC<br>GGCATCCCCAGCAGATTTTCTGGCAGCGGCAGCGGCTCCGACTTCACCCTGACAATC<br>AGCAGCCTGCAGCCCGAGGACGTGGCCACCTACTTTTGCCAGCAGTACTACAGCGGC<br>TGGGCCTTCGGCGGCGTGACCAACCTGGAACTGAAGAGAGCCGTGGCCGCTCCCTCC<br>GTGTTCATCTTCCCACCTAGCGACGAGCAGCTGAAGTCCGGCACAGCCTCTGTCGTG<br>TGCCTGCTGAACAACTTCTACCCCCGCGAGGCCAAGGTGCAGTGGAAGGTGGACAAT<br>GCCCTGCAGTCTGGCAACAGCCAGGAAAGCGTGACCGAGCAGGACAGCAAGGACTCC<br>ACCTACAGCCTGAGCAGCACCCTGACCCTGAGCAAGGCCGACTACGAGAAGCACAAG<br>GTGTACGCCTGCGAAGTGACCCACCAGGGCCTGTCTAGCCCCGTGACCAAGAGCTTC<br>AACCGGGGCGAGTGT |

(continued)

| | | |
|---|---|---|
| 25 | chG019 light-chain variable region nucleotide sequence | GACATCCAGATGACCCAGAGCCCTAGCCTGCTGAGCGCCAGCGTGGGCGATAGAGTG<br>ACCCTGAACTGCAAGGCCAGCCAGAACATCTACAAGAACCTGGCCTGGTATCAGCAG<br>AAGCTGGGCGAGGGCCCCAAGCTGCTGATCTACGACGCCAACACCCTGCAGACCGGC<br>ATCCCCAGCAGATTTTCTGGCAGCGGCAGCGGCTCCGACTTCACCCTGACAATCAGC<br>AGCCTGCAGCCCGAGGACGTGGCCACCTACTTTTGCCAGCAGTACTACAGCGGCTGG<br>GCCTTCGGCGGCGTGACCAACCTGGAACTGAAGAGAGCC |
| 26 | chG019 heavy chain full-length amino acid sequence | MKHLWFFLLLVAAPRWVLSQVQLQQSGAELVKPGSSVKISCKASGYTFTRNFMHWIK<br>QQPGNGLEWIGWIYPGDGETEYNQKFNGKATLTADRSSSTAYMELSRLTSEDSAVYF<br>CARGVYGGFAGGYFDFWGQGVMVTVSSASTKGPSVFPLAPSSKSTSGGTAALGCLVK<br>DYFPEPVTVSWNSGALTSGVHTFPAVLQSSGLYSLSSVVTVPSSSLGTQTYICNVNH<br>KPSNTKVDKRVEPKSCDKTHTCPPCPAPELLGGPSVFLFPPKPKDTLMISRTPEVTC<br>VVVDVSHEDPEVKFNWYVDGVEVHNAKTKPREEQYNSTYRVVSVLTVLHQDWLNGKE<br>YKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEAL<br>HNHYTQKSLSLSPGK |

[Table 1-5]

| | | |
|---|---|---|
| 27 | chG019 heavy chain full-length nucleotide sequence | ATGAAACACCTGTGGTTCTTCCTCCTGCTGGTGGCAGCTCCCAGATGGGTGCTGAGC<br>CAGGTGCAGCTGCAGCAGTCTGGCGCCGAGCTCGTGAAGCCTGGCAGCAGCGTGAAG<br>ATCAGCTGCAAGGCCAGCGGCTACACCTTCACCCGGAACTTCATGCACTGGATCAAG<br>CAGCAGCCCGGCAACGGCCTGGAATGGATCGGCTGGATCTATCCCGGCGACGGCGAG<br>ACAGAGTACAACCAGAAGTTCAACGGCAAGGCCACCCTGACCGCCGACAGAAGCAGC<br>TCCACCGCCTACATGGAACTGAGCCGGCTGACCAGCGAGGACAGCGCCGTGTACTTT<br>TGCGCCAGAGGCGTGTACGGCGGCTTCGCTGGCGGCTACTTCGATTTTTGGGGCCAG<br>GGCGTGATGGTCACCGTCAGCTCAGCCTCCACCAAGGGCCCAAGCGTCTTCCCCCTG<br>GCACCCTCCTCCAAGAGCACCTCTGGCGGCACAGCCGCCCTGGGCTGCCTGGTCAAG<br>GACTACTTCCCCGAACCCGTGACCGTGAGCTGGAACTCAGGCGCCCTGACCAGCGGC<br>GTGCACACCTTCCCCGCTGTCCTGCAGTCCTCAGGACTCTACTCCCTCAGCAGCGTG<br>GTGACCGTGCCCTCCAGCAGCTTGGGCACCCAGACCTACATCTGCAACGTGAATCAC<br>AAGCCCAGCAACACCAAGGTGGACAAGAGAGTTGAGCCCAAATCTTGTGACAAAACT<br>CACACATGCCCACCCTGCCCAGCACCTGAACTCCTGGGGGGACCCTCAGTCTTCCTC<br>TTCCCCCCAAAACCCAAGGACACCCTCATGATCTCCCGGACCCCTGAGGTCACATGC<br>GTGGTGGTGGACGTGAGCCACGAAGACCCTGAGGTCAAGTTCAACTGGTACGTGGAC<br>GGCGTGGAGGTGCATAATGCCAAGACAAAGCCCCGGGAGGAGCAGTACAACAGCACG<br>TACCGGGTGGTCAGCGTCCTCACCGTCCTGCACCAGGACTGGCTGAATGGCAAGGAG<br>TACAAGTGCAAGGTCTCCAACAAAGCCCTCCCAGCCCCCATCGAGAAAACCATCTCC<br>AAAGCCAAAGGCCAGCCCCGGGAACCACAGGTGTACACCCTGCCCCCATCCCGGGAG<br>GAGATGACCAAGAACCAGGTCAGCCTGACCTGCCTGGTCAAAGGCTTCTATCCCAGC<br>GACATCGCCGTGGAGTGGGAGAGCAATGGCCAGCCCGAGAACAACTACAAGACCACC<br>CCTCCCGTGCTGGACTCCGACGGCTCCTTCTTCCTCTACAGCAAGCTCACCGTGGAC<br>AAGAGCAGGTGGCAGCAGGGCAACGTCTTCTCATGCTCCGTGATGCATGAGGCTCTG<br>CACAACCACTACACCCAGAAGAGCCTCTCCCTGTCTCCCGGCAAA |
| 28 | chG019 heavy chain variable region amino acid sequence | QVQLQQSGAELVKPGSSVKISCKASGYTFTRNFMHWIKQQPGNGLEWIGWIYPGDGE<br>TEYNQKFNGKATLTADRSSSTAYMELSRLTSEDSAVYFCARGVYGGFAGGYFDFWGQ<br>GVMVTVSS |
| 29 | chG019 heavy-chain variable sequence nucleotide sequence | CAGGTGCAGCTGCAGCAGTCTGGCGCCGAGCTCGTGAAGCCTGGCAGCAGCGTGAAG<br>ATCAGCTGCAAGGCCAGCGGCTACACCTTCACCCGGAACTTCATGCACTGGATCAAG<br>CAGCAGCCCGGCAACGGCCTGGAATGGATCGGCTGGATCTATCCCGGCGACGGCGAG<br>ACAGAGTACAACCAGAAGTTCAACGGCAAGGCCACCCTGACCGCCGACAGAAGCAGC<br>TCCACCGCCTACATGGAACTGAGCCGGCTGACCAGCGAGGACAGCGCCGTGTACTTT<br>TGCGCCAGAGGCGTGTACGGCGGCTTCGCTGGCGGCTACTTCGATTTTTGGGGCCAG<br>GGCGTGATGGTCACCGTCAGCTCA |
| 30 | chG019 CDRH2 | WIYPGDGETE |

(continued)

| | | |
|---|---|---|
| 31 | hL02 light chain full-length amino acid sequence | MVLQTQVFISLLLWISGAYGDIQMTQSPSSLSASVGDRVTITCKASQNIYKNLAWYQ<br>QKPGKAPKLLIYDANTLQTGVPSRFSGSGSGSDFTLTISSLQPEDFATYFCQQYYSG<br>WAFGQGTKVEIKRTVAAPSVFIFPPSDEQLKSGTASVVCLLNNFYPREAKVQWKVDN<br>ALQSGNSQESVTEQDSKDSTYSLSSTLTLSKADYEKHKVYACEVTHQGLSSPVTKSF<br>NRGEC |

[Table 1-6]

| | | |
|---|---|---|
| 32 | hL02 light chain full-length nucleotide sequence | ATGGTGCTGCAGACCCAGGTGTTCATCTCCCTGCTGCTGTGGATCTCCGGCGCGTAC<br>GGCGACATCCAGATGACCCAGAGCCCTAGCAGCCTGAGCGCCAGCGTGGGCGACAGA<br>GTGACCATCACATGCAAGGCCAGCCAGAACATCTACAAGAACCTGGCCTGGTATCAG<br>CAGAAGCCCGGCAAGGCCCCCAAGCTGCTGATCTACGACGCCAACACCCTGCAGACC<br>GGCGTGCCCAGCAGATTTTCTGGCAGCGGCAGCGGCTCCGACTTCACCCTGACAATC<br>AGCAGCCTGCAGCCCGAGGACTTCGCCACCTACTTTTGCCAGCAGTACTACAGCGGC<br>TGGGCCTTCGGCCAGGGCACCAAGGTGGAAATCAAGCGTACGGTGGCCGCCCCCTCC<br>GTGTTCATCTTCCCCCCCTCCGACGAGCAGCTGAAGTCCGGCACCGCCTCCGTGGTG<br>TGCCTGCTGAATAACTTCTACCCCAGAGAGGCCAAGGTGCAGTGGAAGGTGGACAAC<br>GCCCTGCAGTCCGGGAACTCCCAGGAGAGCGTGACCGAGCAGGACAGCAAGGACAGC<br>ACCTACAGCCTGAGCAGCACCCTGACCCTGAGCAAAGCCGACTACGAGAAGCACAAG<br>GTGTACGCCTGCGAGGTGACCCACCAGGGCCTGAGCTCCCCCGTCACCAAGAGCTTC<br>AACAGGGGGGAGTGT |
| 33 | hL02 light-chain variable region amino acid sequence | DIQMTQSPSSLSASVGDRVTITCKASQNIYKNLAWYQQKPGKAPKLLIYDANTLQTG<br>VPSRFSGSGSGSDFTLTISSLQPEDFATYFCQQYYSGWAFGQGTKVEIKRT |
| 34 | hL02 light-chain variable region nucleotide sequence | GACATCCAGATGACCCAGAGCCCTAGCAGCCTGAGCGCCAGCGTGGGCGACAGAGTG<br>ACCATCACATGCAAGGCCAGCCAGAACATCTACAAGAACCTGGCCTGGTATCAGCAG<br>AAGCCCGGCAAGGCCCCCAAGCTGCTGATCTACGACGCCAACACCCTGCAGACCGGC<br>GTGCCCAGCAGATTTTCTGGCAGCGGCAGCGGCTCCGACTTCACCCTGACAATCAGC<br>AGCCTGCAGCCCGAGGACTTCGCCACCTACTTTTGCCAGCAGTACTACAGCGGCTGG<br>GCCTTCGGCCAGGGCACCAAGGTGGAAATCAAGCGTACG |
| 35 | hL03 light chain full-length amino acid sequence | MVLQTQVFISLLLWISGAYGDIQMTQSPSSLSASVGDRVTITCKASQNIYKNLAWYQ<br>QKLGEGPKLLIYDANTLQTGVPSRFSGSGSGTDFTLTISSLQPEDFATYYCQQYYSG<br>WAFGQGTKVEIKRTVAAPSVFIFPPSDEQLKSGTASVVCLLNNFYPREAKVQWKVDN<br>ALQSGNSQESVTEQDSKDSTYSLSSTLTLSKADYEKHKVYACEVTHQGLSSPVTKSF<br>NRGEC |
| 36 | hL03 light chain full-length nucleotide sequence | ATGGTGCTGCAGACCCAGGTGTTCATCTCCCTGCTGCTGTGGATCTCCGGCGCGTAC<br>GGCGACATCCAGATGACCCAGAGCCCTAGCAGCCTGAGCGCCAGCGTGGGCGACAGA<br>GTGACCATCACATGCAAGGCCAGCCAGAACATCTACAAGAACCTGGCCTGGTATCAG<br>CAGAAGCTGGGCGAGGGCCCCAAGCTGCTGATCTACGACGCCAACACCCTGCAGACC<br>GGCGTGCCCAGCAGATTTTCTGGCAGCGGCTCCGGCACCGACTTCACCCTGACAATC<br>AGCAGCCTGCAGCCCGAGGACTTCGCCACCTACTACTGCCAGCAGTACTACAGCGGC<br>TGGGCCTTTGGCCAGGGCACCAAGGTGGAAATCAAGCGTACGGTGGCCGCCCCCTCC<br>GTGTTCATCTTCCCCCCCTCCGACGAGCAGCTGAAGTCCGGCACCGCCTCCGTGGTG<br>TGCCTGCTGAATAACTTCTACCCCAGAGAGGCCAAGGTGCAGTGGAAGGTGGACAAC<br>GCCCTGCAGTCCGGGAACTCCCAGGAGAGCGTGACCGAGCAGGACAGCAAGGACAGC<br>ACCTACAGCCTGAGCAGCACCCTGACCCTGAGCAAAGCCGACTACGAGAAGCACAAG<br>GTGTACGCCTGCGAGGTGACCCACCAGGGCCTGAGCTCCCCCGTCACCAAGAGCTTC<br>AACAGGGGGGAGTGT |
| 37 | hL03 light-chain variable region amino acid sequence | DIQMTQSPSSLSASVGDRVTITCKASQNIYKNLAWYQQKLGEGPKLLIYDANTLQTG<br>VPSRFSGSGSGTDFTLTISSLQPEDFATYYCQQYYSGWAFGQGTKVEIKRT |

[Table 1-7]

| | | |
|---|---|---|
| 38 | hL03 light-chain variable region nucleotide sequence | GACATCCAGATGACCCAGAGCCCTAGCAGCCTGAGCGCCAGCGTGGGCGACAGAGTG<br>ACCATCACATGCAAGGCCAGCCAGAACATCTACAAGAACCTGGCCTGGTATCAGCAG<br>AAGCTGGGCGAGGGCCCCAAGCTGCTGATCTACGACGCCAACACCCTGCAGACCGGC<br>GTGCCCAGCAGATTTTCTGGCAGCGGCTCCGGCACCGACTTCACCCTGACAATCAGC<br>AGCCTGCAGCCCGAGGACTTCGCCACCTACTACTGCCAGCAGTACTACAGCGGCTGG<br>GCCTTTGGCCAGGGCACCAAGGTGGAAATCAAGCGTACG |
| 39 | hH01 heavy chain full-length amino acid sequence | MKHLWFFLLLVAAPRWVLSEVQLVQSGAEVKKPGASVKVSCKASGYTFTRNFMHWVR<br>QAPGQGLEWMGWIYPGDGETEYAQKFQGRVTITADTSTSTAYMELSSLRSEDTAVYY<br>CARGVYGGFAGGYFDFWGQGTLVTVSSASTKGPSVFPLAPSSKSTSGGTAALGCLVK<br>DYFPEPVTVSWNSGALTSGVHTFPAVLQSSGLYSLSSVVTVPSSSLGTQTYICNVNH<br>KPSNTKVDKRVEPKSCDKTHTCPPCPAPELLGGPSVFLFPPKPKDTLMISRTPEVTC<br>VVVDVSHEDPEVKFNWYVDGVEVHNAKTKPREEQYNSTYRVVSVLTVLHQDWLNGKE<br>YKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEAL<br>HNHYTQKSLSLSPGK |
| 40 | hH01 heavy chain full-length nucleotide sequence | ATGAAACACCTGTGGTTCTTCCTCCTGCTGGTGGCAGCTCCCAGATGGGTGCTGAGC<br>GAAGTGCAGCTGGTGCAGTCTGGCGCCGAAGTGAAGAAACCAGGCGCCAGCGTGAAG<br>GTGTCCTGCAAGGCCAGCGGCTACACCTTTACCCGGAACTTCATGCACTGGGTGCGC<br>CAGGCTCCAGGCCAGGGACTGGAATGGATGGGCTGGATCTATCCCGGCGACGGCGAG<br>ACAGAGTACGCCCAGAAATTCCAGGGCAGAGTGACCATCACCGCCGACACCAGCACC<br>TCCACCGCCTACATGGAACTGAGCAGCCTGCGGAGCGAGGACACCGCCGTGTACTAT<br>TGTGCCAGAGGCGTGTACGGCGGCTTCGCTGGCGGCTACTTCGATTTTTGGGGCCAG<br>GGCACCCTCGTGACCGTCAGCTCAGCCTCCACCAAGGGCCCAAGCGTCTTCCCCCTG<br>GCACCCTCCTCCAAGAGCACCTCTGGCGGCACAGCCGCCCTGGGCTGCCTGGTCAAG<br>GACTACTTCCCCGAACCCGTGACCGTGAGCTGGAACTCAGGCGCCCTGACCAGCGGC<br>GTGCACACCTTCCCCGCTGTCCTGCAGTCCTCAGGACTCTACTCCCTCAGCAGCGTG<br>GTGACCGTGCCCTCCAGCAGCTTGGGCACCCAGACCTACATCTGCAACGTGAATCAC<br>AAGCCCAGCAACACCAAGGTGGACAAGAGAGTTGAGCCCAAATCTTGTGACAAAACT<br>CACACATGCCCACCCTGCCCAGCACCTGAACTCCTGGGGGGACCCTCAGTCTTCCTC<br>TTCCCCCCAAAACCCAAGGACACCCTCATGATCTCCCGGACCCCTGAGGTCACATGC<br>GTGGTGGTGGACGTGAGCCACGAAGACCCTGAGGTCAAGTTCAACTGGTACGTGGAC<br>GGCGTGGAGGTGCATAATGCCAAGACAAAGCCCCGGGAGGAGCAGTACAACAGCACG<br>TACCGGGTGGTCAGCGTCCTCACCGTCCTGCACCAGGACTGGCTGAATGGCAAGGAG<br>TACAAGTGCAAGGTCTCCAACAAAGCCCTCCCAGCCCCCATCGAGAAAACCATCTCC<br>AAAGCCAAAGGCCAGCCCCGGGAACCACAGGTGTACACCCTGCCCCCATCCCGGGAG<br>GAGATGACCAAGAACCAGGTCAGCCTGACCTGCCTGGTCAAAGGCTTCTATCCCAGC<br>GACATCGCCGTGGAGTGGGAGAGCAATGGCCAGCCCGAGAACAACTACAAGACCACC<br>CCTCCCGTGCTGGACTCCGACGGCTCCTTCTTCCTCTACAGCAAGCTCACCGTGGAC<br>AAGAGCAGGTGGCAGCAGGGCAACGTCTTCTCATGCTCCGTGATGCATGAGGCTCTG<br>CACAACCACTACACCCAGAAGAGCCTCTCCCTGTCTCCCGGCAAA |
| 41 | hH01 light-chain variable region amino acid sequence | EVQLVQSGAEVKKPGASVKVSCKASGYTFTRNFMHWVRQAPGQGLEWMGWIYPGDGE<br>TEYAQKFQGRVTITADTSTSTAYMELSSLRSEDTAVYYCARGVYGGFAGGYFDFWGQ<br>GTLVTVSS |

[Table 1-8]

| | | |
|---|---|---|
| 42 | hL01 heavy-chain variable region nucleotide sequence | GAAGTGCAGCTGGTGCAGTCTGGCGCCGAAGTGAAGAAACCAGGCGCCAGCGTGAAG<br>GTGTCCTGCAAGGCCAGCGGCTACACCTTTACCCGGAACTTCATGCACTGGGTGCGC<br>CAGGCTCCAGGCCAGGGACTGGAATGGATGGGCTGGATCTATCCCGGCGACGGCGAG<br>ACAGAGTACGCCCAGAAATTCCAGGGCAGAGTGACCATCACCGCCGACACCAGCACC<br>TCCACCGCCTACATGGAACTGAGCAGCCTGCGGAGCGAGGACACCGCCGTGTACTAT<br>TGTGCCAGAGGCGTGTACGGCGGCTTCGCTGGCGGCTACTTCGATTTTTGGGGCCAG<br>GGCACCCTCGTGACCGTCAGCTCA |
| 43 | hH02 heavy chain full-length amino acid sequence | MKHLWFFLLLVAAPRWVLSEVQLVQSGAEVKKPGASVKVSCKASGYTFTRNFMHWVR<br>QAPGQGLEWMGWIYPGDGETEYNQKFQGRVTITADRSTSTAYMELSSLRSEDTAVYF<br>CARGVYGGFAGGYFDFWGQGTLVTVSSASTKGPSVFPLAPSSKSTSGGTAALGCLVK<br>DYFPEPVTVSWNSGALTSGVHTFPAVLQSSGLYSLSSVVTVPSSSLGTQTYICNVNH<br>KPSNTKVDKRVEPKSCDKTHTCPPCPAPELLGGPSVFLFPPKPKDTLMISRTPEVTC<br>VVVDVSHEDPEVKFNWYVDGVEVHNAKTKPREEQYNSTYRVVSVLTVLHQDWLNGKE<br>YKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEAL<br>HNHYTQKSLSLSPGK |
| 44 | hH02 heavy chain full-length nucleotide sequence | ATGAAACACCTGTGGTTCTTCCTCCTGCTGGTGGCAGCTCCCAGATGGGTGCTGAGC<br>GAAGTGCAGCTGGTGCAGTCTGGCGCCGAAGTGAAGAAACCAGGCGCCAGCGTGAAG<br>GTGTCCTGCAAGGCCAGCGGCTACACCTTTACCCGGAACTTCATGCACTGGGTGCGC<br>CAGGCTCCAGGCCAGGGACTGGAATGGATGGGCTGGATCTATCCCGGCGACGGCGAG<br>ACAGAGTACAACCAGAAATTCCAGGGCAGAGTGACCATCACCGCCGACAGAAGCACC<br>AGCACCGCCTACATGGAACTGAGCAGCCTGCGGAGCGAGGATACCGCCGTGTACTTC<br>TGTGCCAGAGGCGTGTACGGCGGCTTCGCTGGCGGCTACTTCGATTTTTGGGGCCAG<br>GGCACCCTCGTGACCGTCAGCTCAGCCTCCACCAAGGGCCCAAGCGTCTTCCCCCTG<br>GCACCCTCCTCCAAGAGCACCTCTGGCGGCACAGCCGCCCTGGGCTGCCTGGTCAAG<br>GACTACTTCCCCGAACCCGTGACCGTGAGCTGGAACTCAGGCGCCCTGACCAGCGGC<br>GTGCACACCTTCCCCGCTGTCCTGCAGTCCTCAGGACTCTACTCCCTCAGCAGCGTG<br>GTGACCGTGCCCTCCAGCAGCTTGGGCACCCAGACCTACATCTGCAACGTGAATCAC<br>AAGCCCAGCAACACCAAGGTGGACAAGAGAGTTGAGCCCAAATCTTGTGACAAAACT<br>CACACATGCCCACCCTGCCCAGCACCTGAACTCCTGGGGGGACCCTCAGTCTTCCTC<br>TTCCCCCCAAAACCCAAGGACACCCTCATGATCTCCCGGACCCCTGAGGTCACATGC<br>GTGGTGGTGGACGTGAGCCACGAAGACCCTGAGGTCAAGTTCAACTGGTACGTGGAC<br>GGCGTGGAGGTGCATAATGCCAAGACAAAGCCCCGGGAGGAGCAGTACAACAGCACG<br>TACCGGGTGGTCAGCGTCCTCACCGTCCTGCACCAGGACTGGCTGAATGGCAAGGAG<br>TACAAGTGCAAGGTCTCCAACAAAGCCCTCCCAGCCCCCATCGAGAAAACCATCTCC<br>AAAGCCAAAGGCCAGCCCCGGGAACCACAGGTGTACACCCTGCCCCCATCCCGGGAG<br>GAGATGACCAAGAACCAGGTCAGCCTGACCTGCCTGGTCAAAGGCTTCTATCCCAGC<br>GACATCGCCGTGGAGTGGGAGAGCAATGGCCAGCCCGAGAACAACTACAAGACCACC<br>CCTCCCGTGCTGGACTCCGACGGCTCCTTCTTCCTCTACAGCAAGCTCACCGTGGAC<br>AAGAGCAGGTGGCAGCAGGGCAACGTCTTCTCATGCTCCGTGATGCATGAGGCTCTG<br>CACAACCACTACACCCAGAAGAGCCTCTCCCTGTCTCCCGGCAAA |
| 45 | hH02 heavy-chain variable region amino acid sequence | EVQLVQSGAEVKKPGASVKVSCKASGYTFTRNFMHWVRQAPGQGLEWMGWIYPGDGE<br>TEYNQKFQGRVTITADRSTSTAYMELSSLRSEDTAVYFCARGVYGGFAGGYFDFWGQ<br>GTLVTVSS |

[Table 1-9]

| | | |
|---|---|---|
| 46 | hH02 heavy-chain variable region nucleotide sequence | GAAGTGCAGCTGGTGCAGTCTGGCGCCGAAGTGAAGAAACCAGGCGCCAGCGTGAAG<br>GTGTCCTGCAAGGCCAGCGGCTACACCTTTACCCGGAACTTCATGCACTGGGTGCGC<br>CAGGCTCCAGGCCAGGGACTGGAATGGATGGGCTGGATCTATCCCGGCGACGGCGAG<br>ACAGAGTACAACCAGAAATTCCAGGGCAGAGTGACCATCACCGCCGACAGAAGCACC<br>AGCACCGCCTACATGGAACTGAGCAGCCTGCGGAGCGAGGATACCGCCGTGTACTTC<br>TGTGCCAGAGGCGTGTACGGCGGCTTCGCTGGCGGCTACTTCGATTTTTGGGGCCAG<br>GGCACCCTCGTGACCGTCAGCTCA |

(continued)

| | | |
|---|---|---|
| 47 | hH04 heavy chain full-length amino acid sequence | MKHLWFFLLLVAAPRWVLSQVQLVQSGAEVKKPGASVKVSCKASGYTFTRNFMHWIR<br>QAPGQGLEWMGWIYPGDGETEYAQKFQGRVTLTADRSTSTAYMELSSLRSEDTAVYY<br>CARGVYGGFAGGYFDFWGQGTLVTVSSASTKGPSVFPLAPSSKSTSGGTAALGCLVK<br>DYFPEPVTVSWNSGALTSGVHTFPAVLQSSGLYSLSSVVTVPSSSLGTQTYICNVNH<br>KPSNTKVDKRVEPKSCDKTHTCPPCPAPELLGGPSVFLFPPKPKDTLMISRTPEVTC<br>VVVDVSHEDPEVKFNWYVDGVEVHNAKTKPREEQYNSTYRVVSVLTVLHQDWLNGKE<br>YKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEAL<br>HNHYTQKSLSLSPGK |
| 48 | hH04 heavy chain full-length nucleotide sequence | ATGAAACACCTGTGGTTCTTCCTCCTGCTGGTGGCAGCTCCCAGATGGGTGCTGAGC<br>CAGGTGCAGCTGGTGCAGTCTGGCGCCGAAGTGAAGAAACCAGGCGCCAGCGTGAAG<br>GTGTCCTGCAAGGCCAGCGGCTACACCTTTACCCGGAACTTCATGCACTGGATCCGG<br>CAGGCCCCTGGACAGGGCCTGGAATGGATGGGCTGGATCTATCCCGGCGACGGCGAG<br>ACAGAGTACGCCCAGAAATTCCAGGGCAGAGTGACCCTGACCGCCGACAGAAGCACC<br>AGCACCGCCTACATGGAACTGAGCAGCCTGCGGAGCGAGGACACCGCCGTGTACTAT<br>TGTGCCAGAGGCGTGTACGGCGGCTTCGCTGGCGGCTACTTCGATTTTTGGGGCCAG<br>GGCACCCTCGTGACCGTCAGCTCAGCCTCCACCAAGGGCCCAAGCGTCTTCCCCCTG<br>GCACCCTCCTCCAAGAGCACCTCTGGCGGCACAGCCGCCCTGGGCTGCCTGGTCAAG<br>GACTACTTCCCCGAACCCGTGACCGTGAGCTGGAACTCAGGCGCCCTGACCAGCGGC<br>GTGCACACCTTCCCCGCTGTCCTGCAGTCCTCAGGACTCTACTCCCTCAGCAGCGTG<br>GTGACCGTGCCCTCCAGCAGCTTGGGCACCCAGACCTACATCTGCAACGTGAATCAC<br>AAGCCCAGCAACACCAAGGTGGACAAGAGAGTTGAGCCCAAATCTTGTGACAAAACT<br>CACACATGCCCACCCTGCCCAGCACCTGAACTCCTGGGGGGACCCTCAGTCTTCCTC<br>TTCCCCCCAAAACCCAAGGACACCCTCATGATCTCCCGGACCCCTGAGGTCACATGC<br>GTGGTGGTGGACGTGAGCCACGAAGACCCTGAGGTCAAGTTCAACTGGTACGTGGAC<br>GGCGTGGAGGTGCATAATGCCAAGACAAAGCCCCGGGAGGAGCAGTACAACAGCACG<br>TACCGGGTGGTCAGCGTCCTCACCGTCCTGCACCAGGACTGGCTGAATGGCAAGGAG<br>TACAAGTGCAAGGTCTCCAACAAAGCCCTCCCAGCCCCCATCGAGAAAACCATCTCC<br>AAAGCCAAAGGCCAGCCCCGGGAACCACAGGTGTACACCCTGCCCCCATCCCGGGAG<br>GAGATGACCAAGAACCAGGTCAGCCTGACCTGCCTGGTCAAAGGCTTCTATCCCAGC<br>GACATCGCCGTGGAGTGGGAGAGCAATGGCCAGCCCGAGAACAACTACAAGACCACC<br>CCTCCCGTGCTGGACTCCGACGGCTCCTTCTTCCTCTACAGCAAGCTCACCGTGGAC<br>AAGAGCAGGTGGCAGCAGGGCAACGTCTTCTCATGCTCCGTGATGCATGAGGCTCTG<br>CACAACCACTACACCCAGAAGAGCCTCTCCCTGTCTCCCGGCAAA |
| 49 | hH04 heavy-chain variable region amino acid sequence | QVQLVQSGAEVKKPGASVKVSCKASGYTFTRNFMHWIRQAPGQGLEWMGWIYPGDGE<br>TEYAQKFQGRVTLTADRSTSTAYMELSSLRSEDTAVYYCARGVYGGFAGGYFDFWGQ<br>GTLVTVSS |

[Table 1-10]

| | | |
|---|---|---|
| 50 | hH04 heavy-chain variable region nucleotide sequence | CAGGTGCAGCTGGTGCAGTCTGGCGCCGAAGTGAAGAAACCAGGCGCCAGCGTGAAG<br>GTGTCCTGCAAGGCCAGCGGCTACACCTTTACCCGGAACTTCATGCACTGGATCCGG<br>CAGGCCCCTGGACAGGGCCTGGAATGGATGGGCTGGATCTATCCCGGCGACGGCGAG<br>ACAGAGTACGCCCAGAAATTCCAGGGCAGAGTGACCCTGACCGCCGACAGAAGCACC<br>AGCACCGCCTACATGGAACTGAGCAGCCTGCGGAGCGAGGACACCGCCGTGTACTAT<br>TGTGCCAGAGGCGTGTACGGCGGCTTCGCTGGCGGCTACTTCGATTTTTGGGGCCAG<br>GGCACCCTCGTGACCGTCAGCTCA |
| 51 | NOVO0712 light chain full-length amino acid sequence | MVLQTQVFISLLLWISGAYGDIQMTQSPSSLSASVGDRVTITCRASQSISSYLNWYQ<br>QKPGKAPKLLIYAVSTLQSGVPSRFSGSGSGTDFTLTISSLQPEDFATYYCQQSGTF<br>PPTTFGQGTKVEIKRTVAAPSVFIFPPSDEQLKSGTASVVCLLNNFYPREAKVQWKV<br>DNALQSGNSQESVTEQDSKDSTYSLSSTLTLSKADYEKHKVYACEVTHQGLSSPVTK<br>SFNRGEC |

(continued)

| | | |
|---|---|---|
| 52 | Nucleotide sequence encoding amino acid sequence shown in SEQ ID NO: 51 | ATGGTGCTGCAGACCCAGGTGTTCATCTCCCTGCTGCTGTGGATCTCCGGCGCGTAC<br>GGCGACATCCAGATGACCCAGAGCCCTAGCAGCCTGAGCGCCAGCGTGGGCGACAGA<br>GTGACCATCACCTGTAGAGCCAGCCAGAGCATCAGCAGCTACCTGAACTGGTATCAG<br>CAGAAGCCCGGCAAGGCCCCCAAACTGCTGATCTACGCCGTGTCCACACTGCAGAGC<br>GGCGTGCCCAGCAGATTTTCTGGCAGCGGCTCCGGCACCGACTTCACCCTGACAATC<br>AGCAGCCTGCAGCCCGAGGACTTCGCCACCTACTACTGTCAGCAGTCCGGCACCTTC<br>CCCCCCACCACATTTGGCCAGGGCACCAAGGTGGAAATCAAGCGTACGGTGGCCGCC<br>CCCTCCGTGTTCATCTTCCCCCCCTCCGACGAGCAGCTGAAGTCCGGCACCGCCTCC<br>GTGGTGTGCCTGCTGAATAACTTCTACCCCAGAGAGGCCAAGGTGCAGTGGAAGGTG<br>GACAACGCCCTGCAGTCCGGGAACTCCCAGGAGAGCGTGACCGAGCAGGACAGCAAG<br>GACAGCACCTACAGCCTGAGCAGCACCCTGACCCTGAGCAAAGCCGACTACGAGAAG<br>CACAAGGTGTACGCCTGCGAGGTGACCCACCAGGGCCTGAGCTCCCCCGTCACCAAG<br>AGCTTCAACAGGGGGGAGTGT |
| 53 | NOVO0712 heavy chain full-length amino acid sequence | MKHLWFFLLLVAAPRWVLSQVQLLESGGGLVQPGGSLRLSCAASGFTFSSHGMHWVR<br>QAPGKGLEWVSVISGSGSNTGYADSVKGRFTISRDNSKNTLYLQMNSLRAEDTAVYY<br>CARQWGSYAFDSWGQGTLVTVSSASTKGPSVFPLAPSSKSTSGGTAALGCLVKDYFP<br>EPVTVSWNSGALTSGVHTFPAVLQSSGLYSLSSVVTVPSSSLGTQTYICNVNHKPSN<br>TKVDKRVEPKSCDKTHTCPPCPAPELLGGPSVFLFPPKPKDTLMISRTPEVTCVVVD<br>VSHEDPEVKFNWYVDGVEVHNAKTKPREEQYNSTYRVVSVLTVLHQDWLNGKEYKCK<br>VSNKALPAPIEKTISKAKGQPREPQVYTLPPSREEMTKNQVSLTCLVKGFYPSDIAV<br>EWESNGQPENNYKTTPPVLDSDGSFFLYSKLTVDKSRWQQGNVFSCSVMHEALHNHY<br>TQKSLSLSPGK |

[Table 1-11]

| | | |
|---|---|---|
| 54 | Nucleotide sequence encoding amino acid sequence shown in SEQ ID NO: 53 | ATGAAACACCTGTGGTTCTTCCTCCTGCTGGTGGCAGCTCCCAGATGGGTGCTGAGC<br>CAGGTGCAGCTGCTGGAATCTGGCGGAGGACTGGTGCAGCCTGGCGGCTCTCTGAGA<br>CTGTCTTGTGCCGCCAGCGGCTTCACCTTCAGCAGCCACGGAATGCACTGGGTGCGC<br>CAGGCCCCTGGAAAGGGACTGGAATGGGTGTCCGTGATCAGCGGCAGCGGCTCCAAT<br>ACCGGCTACGCCGATAGCGTGAAGGGCCGGTTCACCATCAGCCGGGACAACAGCAAG<br>AACACCCTGTACCTGCAGATGAACAGCCTGCGGGCCGAGGACACCGCCGTGTACTAT<br>TGTGCCAGACAGTGGGGCAGCTACGCCTTCGATTCTTGGGGCCAGGGCACCCTCGTG<br>ACCGTCAGCTCAGCCTCCACCAAGGGCCCAAGCGTCTTCCCCCTGGCACCCTCCTCC<br>AAGAGCACCTCTGGCGGCACAGCCGCCCTGGGCTGCCTGGTCAAGGACTACTTCCCC<br>GAACCCGTGACCGTGAGCTGGAACTCAGGCGCCCTGACCAGCGGCGTGCACACCTTC<br>CCCGCTGTCCTGCAGTCCTCAGGACTCTACTCCCTCAGCAGCGTGGTGACCGTGCCC<br>TCCAGCAGCTTGGGCACCCAGACCTACATCTGCAACGTGAATCACAAGCCCAGCAAC<br>ACCAAGGTGGACAAGAGAGTTGAGCCCAAATCTTGTGACAAAACTCACACATGCCCA<br>CCCTGCCCAGCACCTGAACTCCTGGGGGGACCCTCAGTCTTCCTCTTCCCCCCAAAA<br>CCCAAGGACACCCTCATGATCTCCCGGACCCCTGAGGTCACATGCGTGGTGGTGGAC<br>GTGAGCCACGAAGACCCTGAGGTCAAGTTCAACTGGTACGTGGACGGCGTGGAGGTG<br>CATAATGCCAAGACAAAGCCCCGGGAGGAGCAGTACAACAGCACGTACCGGGTGGTC<br>AGCGTCCTCACCGTCCTGCACCAGGACTGGCTGAATGGCAAGGAGTACAAGTGCAAG<br>GTCTCCAACAAAGCCCTCCCAGCCCCCATCGAGAAAACCATCTCCAAAGCCAAAGGC<br>CAGCCCCGGGAACCACAGGTGTACACCCTGCCCCCATCCCGGGAGGAGATGACCAAG<br>AACCAGGTCAGCCTGACCTGCCTGGTCAAAGGCTTCTATCCCAGCGACATCGCCGTG<br>GAGTGGGAGAGCAATGGCCAGCCCGAGAACAACTACAAGACCACCCCTCCCGTGCTG<br>GACTCCGACGGCTCCTTCTTCCTCTACAGCAAGCTCACCGTGGACAAGAGCAGGTGG<br>CAGCAGGGCAACGTCTTCTCATGCTCCGTGATGCATGAGGCTCTGCACAACCACTAC<br>ACCCAGAAGAGCCTCTCCCTGTCTCCCGGCAAA |
| 55 | hH11 heavy-chain variable region amino acid sequence | EVQLVQSGAEVKKPGASVKVSCKASGYTFTRNFMHWVRQAPGQGLEWMGWIAPGDGE<br>TEYAQKFQGRVTITADTSTSTAYMELSSLRSEDTAVYYCARGVYGGFAGGYFDFWGQ<br>GTLVTVSS |

(continued)

| | | |
|---|---|---|
| 56 | hH11 heavy-chain variable region nucleotide sequence | GAAGTGCAGCTGGTTCAGTCTGGCGCCGAAGTGAAAAAGCCTGGCGCCTCTGTGAAG<br>GTGTCCTGCAAGGCCTCTGGCTACACATTCACCCGGAACTTCATGCACTGGGTCCGA<br>CAGGCTCCAGGACAGGGACTTGAATGGATGGGATGGATTGCTCCCGGCGACGGCGAG<br>ACAGAGTACGCCCAGAAATTCCAGGGCAGAGTGACCATCACCGCCGACACCTCTACA<br>AGCACCGCCTACATGGAACTGAGCAGCCTGAGAAGCGAGGACACCGCCGTGTACTAT<br>TGTGCCAGAGGCGTGTACGGCGGATTCGCTGGCGGCTACTTTGATTTTTGGGGCCAG<br>GGCACCCTGGTCACCGTGAGCTCA |
| 57 | hH11 CDRH1 | GYTFTRNFMH |
| 58 | hH11 CDRH2 | WIAPGDGETE |
| 59 | hH11 CDRH3 | GVYGGFAGGYFDF |
| 60 | hH31 heavy-chain variable region amino acid sequence | EVQLVQSGAEVKKPGASVKVSCKASGYTFTRNFMHWVRQAPGQGLEWMGWIYPGDGE<br>TEYASKFQGRVTITADTSTSTAYMELSSLRSEDTAVYYCARGVYGGAAGGYFDFWGQ<br>GTLVTVSS |

[Table 1-12]

| | | |
|---|---|---|
| 61 | hH31 heavy-chain variable region nucleotide sequence | GAAGTGCAGCTGGTTCAGTCTGGCGCCGAAGTGAAAAAGCCTGGCGCCTCTGTGAAG<br>GTGTCCTGCAAGGCCTCTGGCTACACATTCACCCGGAACTTCATGCACTGGGTCCGA<br>CAGGCTCCAGGACAGGGACTTGAATGGATGGGCTGGATCTATCCCGGCGACGGCGAG<br>ACAGAGTACGCCAGCAAATTTCAGGGCAGAGTGACCATCACCGCCGACACCTCTACA<br>AGCACCGCCTACATGGAACTGAGCAGCCTGAGAAGCGAGGACACCGCCGTGTACTAT<br>TGTGCCAGAGGCGTTTACGGCGGAGCCGCTGGCGGCTACTTTGATTTTTGGGGCCAG<br>GGCACCCTGGTCACCGTGAGCTCA |
| 62 | hH31 CDRH1 | GYTFTRNFMH |
| 63 | hH31 CDRH2 | WIYPGDGETE |
| 64 | hH31 CDRH3 | GVYGGAAGGYFDF |
| 65 | hH01A heavy chain full-length amino acid sequence | MKHLWFFLLLVAAPRWVLSEVQLVQSGAEVKKPGASVKVSCKASGYTFTRNFMHWVR<br>QAPGQGLEWMGWIYPGDGETEYAQKFQGRVTITADTSTSTAYMELSSLRSEDTAVYY<br>CARGVYGGFAGGYFDFWGQGTLVTVSSASTKGPSVFPLAPSSKSTSGGTAALGCLVK<br>DYFPEPVTVSWNSGALTSGVHTFPAVLQSSGLYSLSSVVTVPSSSLGTQTYICNVNH<br>KPSNTKVDKRVEPKSCDKTHTCPPCPAPEAAGGPSVFLFPPKPKDTLMISRTPEVTC<br>VVVDVSHEDPEVKFNWYVDGVEVHNAKTKPREEQYNSTYRVVSVLTVLHQDWLNGKE<br>YKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEAL<br>HNHYTQKSLSLSPGK |

(continued)

| | | |
|---|---|---|
| 66 | hH01A heavy chain full-length nucleotide sequence | ATGAAACACCTGTGGTTCTTCCTCCTGCTGGTGGCAGCTCCCAGATGGGTGCTGAGC<br>GAAGTGCAGCTGGTTCAGTCTGGCGCCGAAGTGAAAAAGCCTGGCGCCTCTGTGAAG<br>GTGTCCTGCAAGGCCTCTGGCTACACATTCACCCGGAACTTCATGCACTGGGTCCGA<br>CAGGCTCCAGGACAGGGACTTGAATGGATGGGCTGGATCTATCCCGGCGACGGCGAG<br>ACAGAGTACGCCCAGAAATTCCAGGGCAGAGTGACCATCACCGCCGACACCTCTACA<br>AGCACCGCCTACATGGAACTGAGCAGCCTGAGAAGCGAGGACACCGCCGTGTACTAT<br>TGTGCCAGAGGCGTGTACGGCGGATTCGCTGGCGGCTACTTTGATTTTTGGGGCCAG<br>GGCACCCTGGTCACCGTGAGCTCAGCCTCCACCAAGGGCCCAAGCGTCTTCCCCCTG<br>GCACCCTCCTCCAAGAGCACCTCTGGCGGCACAGCCGCCCTGGGCTGCCTGGTCAAG<br>GACTACTTCCCCGAACCCGTGACCGTGAGCTGGAACTCAGGCGCCCTGACCAGCGGC<br>GTGCACACCTTCCCCGCTGTCCTGCAGTCCTCAGGACTCTACTCCCTCAGCAGCGTG<br>GTGACCGTGCCCTCCAGCAGCTTGGGCACCCAGACCTACATCTGCAACGTGAATCAC<br>AAGCCCAGCAACACCAAGGTGGACAAGAGAGTTGAGCCCAAATCTTGTGACAAAACT<br>CACACATGCCCACCCTGCCCAGCACCTGAAGCCGCGGGGGGACCCTCAGTCTTCCTC<br>TTCCCCCCAAAACCCAAGGACACCCTCATGATCTCCCGGACCCCTGAGGTCACATGC<br>GTGGTGGTGGACGTGAGCCACGAAGACCCTGAGGTCAAGTTCAACTGGTACGTGGAC<br>GGCGTGGAGGTGCATAATGCCAAGACAAAGCCCCGGGAGGAGCAGTACAACAGCACG<br>TACCGGGTGGTCAGCGTCCTCACCGTCCTGCACCAGGACTGGCTGAATGGCAAGGAG<br>TACAAGTGCAAGGTCTCCAACAAAGCCCTCCCAGCCCCCATCGAGAAAACCATCTCC<br>AAAGCCAAAGGCCAGCCCCGGGAACCACAGGTGTACACCCTGCCCCCATCCCGGGAG<br>GAGATGACCAAGAACCAGGTCAGCCTGACCTGCCTGGTCAAAGGCTTCTATCCCAGC<br>GACATCGCCGTGGAGTGGGAGAGCAATGGCCAGCCCGAGAACAACTACAAGACCACC<br>CCTCCCGTGCTGGACTCCGACGGCTCCTTCTTCCTCTACAGCAAGCTCACCGTGGAC<br>AAGAGCAGGTGGCAGCAGGGCAACGTCTTCTCATGCTCCGTGATGCATGAGGCTCTG<br>CACAACCACTACACCCAGAAGAGCCTCTCCCTGTCTCCCGGCAAA |

[Table 1-13]

| | | |
|---|---|---|
| 67 | hH11A heavy chain full-length amino acid sequence | MKHLWFFLLLVAAPRWVLSEVQLVQSGAEVKKPGASVKVSCKASGYTFTRNFMHWVR<br>QAPGQGLEWMGWIAPGDGETEYAQKFQGRVTITADTSTSTAYMELSSLRSEDTAVYY<br>CARGVYGGFAGGYFDFWGQGTLVTVSSASTKGPSVFPLAPSSKSTSGGTAALGCLVK<br>DYFPEPVTVSWNSGALTSGVHTFPAVLQSSGLYSLSSVVTVPSSSLGTQTYICNVNH<br>KPSNTKVDKRVEPKSCDKTHTCPPCPAPEAAGGPSVFLFPPKPKDTLMISRTPEVTC<br>VVVDVSHEDPEVKFNWYVDGVEVHNAKTKPREEQYNSTYRVVSVLTVLHQDWLNGKE<br>YKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEAL<br>HNHYTQKSLSLSPGK |

(continued)

| | | |
|---|---|---|
| 68 | hH11A heavy chain full-length nucleotide sequence | ATGAAACACCTGTGGTTCTTCCTCCTGCTGGTGGCAGCTCCCAGATGGGTGCTGAGC<br>GAAGTGCAGCTGGTTCAGTCTGGCGCCGAAGTGAAAAAGCCTGGCGCCTCTGTGAAG<br>GTGTCCTGCAAGGCCTCTGGCTACACATTCACCCGGAACTTCATGCACTGGGTCCGA<br>CAGGCTCCAGGACAGGGACTTGAATGGATGGGATGGATTGCTCCCGGCGACGGCGAG<br>ACAGAGTACGCCCAGAAATTCCAGGGCAGAGTGACCATCACCGCCGACACCTCTACA<br>AGCACCGCCTACATGGAACTGAGCAGCCTGAGAAGCGAGGACACCGCCGTGTACTAT<br>TGTGCCAGAGGCGTGTACGGCGGATTCGCTGGCGGCTACTTTGATTTTTGGGGCCAG<br>GGCACCCTGGTCACCGTGAGCTCAGCCTCCACCAAGGGCCCAAGCGTCTTCCCCCTG<br>GCACCCTCCTCCAAGAGCACCTCTGGCGGCACAGCCGCCCTGGGCTGCCTGGTCAAG<br>GACTACTTCCCCGAACCCGTGACCGTGAGCTGGAACTCAGGCGCCCTGACCAGCGGC<br>GTGCACACCTTCCCCGCTGTCCTGCAGTCCTCAGGACTCTACTCCCTCAGCAGCGTG<br>GTGACCGTGCCCTCCAGCAGCTTGGGCACCCAGACCTACATCTGCAACGTGAATCAC<br>AAGCCCAGCAACACCAAGGTGGACAAGAGAGTTGAGCCCAAATCTTGTGACAAAACT<br>CACACATGCCCACCCTGCCCAGCACCTGAAGCCGCGGGGGGACCCTCAGTCTTCCTC<br>TTCCCCCCAAAACCCAAGGACACCCTCATGATCTCCCGGACCCCTGAGGTCACATGC<br>GTGGTGGTGGACGTGAGCCACGAAGACCCTGAGGTCAAGTTCAACTGGTACGTGGAC<br>GGCGTGGAGGTGCATAATGCCAAGACAAAGCCCCGGGAGGAGCAGTACAACAGCACG<br>TACCGGGTGGTCAGCGTCCTCACCGTCCTGCACCAGGACTGGCTGAATGGCAAGGAG<br>TACAAGTGCAAGGTCTCCAACAAAGCCCTCCCAGCCCCCATCGAGAAAACCATCTCC<br>AAAGCCAAAGGCCAGCCCCGGGAACCACAGGTGTACACCCTGCCCCCATCCCGGGAG<br>GAGATGACCAAGAACCAGGTCAGCCTGACCTGCCTGGTCAAAGGCTTCTATCCCAGC<br>GACATCGCCGTGGAGTGGGAGAGCAATGGCCAGCCCGAGAACAACTACAAGACCACC<br>CCTCCCGTGCTGGACTCCGACGGCTCCTTCTTCCTCTACAGCAAGCTCACCGTGGAC<br>AAGAGCAGGTGGCAGCAGGGCAACGTCTTCTCATGCTCCGTGATGCATGAGGCTCTG<br>CACAACCACTACACCCAGAAGAGCCTCTCCCTGTCTCCCGGCAAA |
| 69 | hH31A heavy chain full-length amino acid sequence | MKHLWFFLLLVAAPRWVLSEVQLVQSGAEVKKPGASVKVSCKASGYTFTRNFMHWVR<br>QAPGQGLEWMGWIYPGDGETEYASKFQGRVTITADTSTSTAYMELSSLRSEDTAVYY<br>CARGVYGGAAGGYFDFWGQGTLVTVSSASTKGPSVFPLAPSSKSTSGGTAALGCLVK<br>DYFPEPVTVSWNSGALTSGVHTFPAVLQSSGLYSLSSVVTVPSSSLGTQTYICNVNH<br>KPSNTKVDKRVEPKSCDKTHTCPPCPAPEAAGGPSVFLFPPKPKDTLMISRTPEVTC<br>VVVDVSHEDPEVKFNWYVDGVEVHNAKTKPREEQYNSTYRVVSVLTVLHQDWLNGKE<br>YKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEAL<br>HNHYTQKSLSLSPGK |

[Table 1-14]

| | | |
|---|---|---|
| 70 | hH31A heavy chain full-length nucleotide sequence | ATGAAACACCTGTGGTTCTTCCTCCTGCTGGTGGCAGCTCCCAGATGGGTGCTGAGC<br>GAAGTGCAGCTGGTTCAGTCTGGCGCCGAAGTGAAAAAGCCTGGCGCCTCTGTGAAG<br>GTGTCCTGCAAGGCCTCTGGCTACACATTCACCCGGAACTTCATGCACTGGGTCCGA<br>CAGGCTCCAGGACAGGGACTTGAATGGATGGGCTGGATCTATCCCGGCGACGGCGAG<br>ACAGAGTACGCCAGCAAATTTCAGGGCAGAGTGACCATCACCGCCGACACCTCTACA<br>AGCACCGCCTACATGGAACTGAGCAGCCTGAGAAGCGAGGACACCGCCGTGTACTAT<br>TGTGCCAGAGGCGTTTACGGCGGAGCCGCTGGCGGCTACTTTGATTTTTGGGGCCAG<br>GGCACCCTGGTCACCGTGAGCTCAGCCTCCACCAAGGGCCCAAGCGTCTTCCCCCTG<br>GCACCCTCCTCCAAGAGCACCTCTGGCGGCACAGCCGCCCTGGGCTGCCTGGTCAAG<br>GACTACTTCCCCGAACCCGTGACCGTGAGCTGGAACTCAGGCGCCCTGACCAGCGGC<br>GTGCACACCTTCCCCGCTGTCCTGCAGTCCTCAGGACTCTACTCCCTCAGCAGCGTG<br>GTGACCGTGCCCTCCAGCAGCTTGGGCACCCAGACCTACATCTGCAACGTGAATCAC<br>AAGCCCAGCAACACCAAGGTGGACAAGAGAGTTGAGCCCAAATCTTGTGACAAAACT<br>CACACATGCCCACCCTGCCCAGCACCTGAAGCCGCGGGGGGACCCTCAGTCTTCCTC<br>TTCCCCCCAAAACCCAAGGACACCCTCATGATCTCCCGGACCCCTGAGGTCACATGC<br>GTGGTGGTGGACGTGAGCCACGAAGACCCTGAGGTCAAGTTCAACTGGTACGTGGAC<br>GGCGTGGAGGTGCATAATGCCAAGACAAAGCCCCGGGAGGAGCAGTACAACAGCACG<br>TACCGGGTGGTCAGCGTCCTCACCGTCCTGCACCAGGACTGGCTGAATGGCAAGGAG<br>TACAAGTGCAAGGTCTCCAACAAAGCCCTCCCAGCCCCCATCGAGAAAACCATCTCC<br>AAAGCCAAAGGCCAGCCCCGGGAACCACAGGTGTACACCCTGCCCCCATCCCGGGAG<br>GAGATGACCAAGAACCAGGTCAGCCTGACCTGCCTGGTCAAAGGCTTCTATCCCAGC<br>GACATCGCCGTGGAGTGGGAGAGCAATGGCCAGCCCGAGAACAACTACAAGACCACC<br>CCTCCCGTGCTGGACTCCGACGGCTCCTTCTTCCTCTACAGCAAGCTCACCGTGGAC<br>AAGAGCAGGTGGCAGCAGGGCAACGTCTTCTCATGCTCCGTGATGCATGAGGCTCTG<br>CACAACCACTACACCCAGAAGAGCCTCTCCCTGTCTCCCGGCAAA |
| 71 | DNA fragment comprising DNA sequence encoding amino acid sequence of human heavy chain signal sequence and human IgG1 LALA constant region | ccagcctccggactctagagccaccATGAAACACCTGTGGTTCTTCCTCCTGCTGGT<br>GGCAGCTCCCAGATGGGTGCTGAGCCAGGTGCAATTGTGCAGGCGGTTAGCTCAGCC<br>TCCACCAAGGGCCCAAGCGTCTTCCCCCTGGCACCCTCCTCCAAGAGCACCTCTGGC<br>GGCACAGCCGCCCTGGGCTGCCTGGTCAAGGACTACTTCCCCGAACCCGTGACCGTG<br>AGCTGGAACTCAGGCGCCCTGACCAGCGGCGTGCACACCTTCCCCGCTGTCCTGCAG<br>TCCTCAGGACTCTACTCCCTCAGCAGCGTGGTGACCGTGCCCTCCAGCAGCTTGGGC<br>ACCCAGACCTACATCTGCAACGTGAATCACAAGCCCAGCAACACCAAGGTGGACAAG<br>AGAGTTGAGCCCAAATCTTGTGACAAAACTCACACATGCCCACCCTGCCCAGCACCT<br>GAAGCCGCGGGGGGACCCTCAGTCTTCCTCTTCCCCCCAAAACCCAAGGACACCCTC<br>ATGATCTCCCGGACCCCTGAGGTCACATGCGTGGTGGTGGACGTGAGCCACGAAGAC<br>CCTGAGGTCAAGTTCAACTGGTACGTGGACGGCGTGGAGGTGCATAATGCCAAGACA<br>AAGCCCCGGGAGGAGCAGTACAACAGCACGTACCGGGTGGTCAGCGTCCTCACCGTC<br>CTGCACCAGGACTGGCTGAATGGCAAGGAGTACAAGTGCAAGGTCTCCAACAAAGCC<br>CTCCCAGCCCCCATCGAGAAAACCATCTCCAAAGCCAAAGGCCAGCCCCGGGAACCA<br>CAGGTGTACACCCTGCCCCCATCCCGGGAGGAGATGACCAAGAACCAGGTCAGCCTG<br>ACCTGCCTGGTCAAAGGCTTCTATCCCAGCGACATCGCCGTGGAGTGGGAGAGCAAT<br>GGCCAGCCCGAGAACAACTACAAGACCACCCCTCCCGTGCTGGACTCCGACGGCTCC<br>TTCTTCCTCTACAGCAAGCTCACCGTGGACAAGAGCAGGTGGCAGCAGGGCAACGTC<br>TTCTCATGCTCCGTGATGCATGAGGCTCTGCACAACCACTACACCCAGAAGAGCCTC<br>TCCCTGTCTCCCGGCAAAtgagatatcgggcccgtttaaacgggggaggctaac |

3. Anti-CDH6 antibody-drug conjugate

The antibody-drug conjugate of the present invention is represented by the following formula:

[Formula 34]

$$Ab \left[ (N297\ glycan) \left[ L - D \right]_{m^2} \right]_2$$

wherein

m$^2$ represents an integer of 1 or 2,
Ab represents an IgG antibody specifically binding to an amino acid sequence comprising the amino acid sequence shown in SEQ ID NO: 4 and having internalization ability that permits cellular uptake, or a functional fragment thereof
L represents a linker, linking the glycan (N297) bonding to N297 of Ab and D, and
D is any one of the following formulas:

[Formula 35]

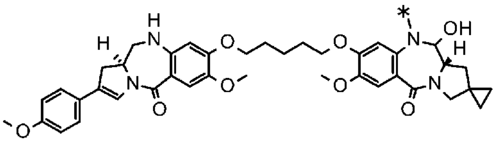
,
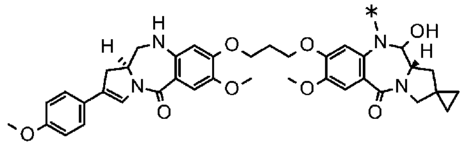
,
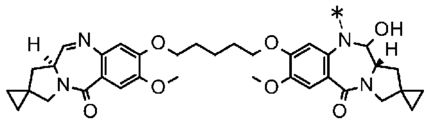
or
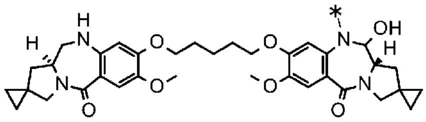

wherein the asterisk (*) represents bonding to L.

<Drug>

The anti-CDH6 antibody obtained in the above "2. Production of anti-CDH6 antibody" can be conjugated to a drug via a linker moiety to prepare an anti-CDH6 antibody-drug conjugate. The drug is not particularly limited as long as it has a substituent or a molecular part that can be connected to a linker The anti-CDH6 antibody-drug conjugate can be used for various purposes according to the conjugated drug. Examples of such a drug can include substances having antitumor activity, substances effective for blood diseases, substances effective for autoimmune diseases, anti-inflammatory substances, antimicrobial substances, antifungal substances, antiparasitic substances, antiviral substances, and anti-anesthetic substances.

<Antitumor compound>

Examples using an antitumor compound as the compound to be used in the anti-CDH6 antibody-drug conjugate of the present invention will be described below. The antitumor compound is not particularly limited as long as the compound has an antitumor effect and has a substituent or a partial structure that can be connected to a linker structure. Upon cleavage of a part or the whole of the linker in tumor cells, the antitumor compound moiety is released so that the antitumor compound exhibits an antitumor effect. As the linker is cleaved at a connecting position with the drug, the antitumor compound is released in its original structure to exert its original antitumor effect. The released compounds serving as the drug are, e.g., drugs 1 to 4 described in Examples 10-7 to 10-10.

The anti-CDH6 antibody obtained in the above "2. Production of anti-CDH6 antibody" can be conjugated to the antitumor compound via a linker structure moiety to prepare an anti-CDH6 antibody-drug conjugate.

The antitumor compound to be used in the present invention is represented by any one of the formulas below:

[Formula 36]

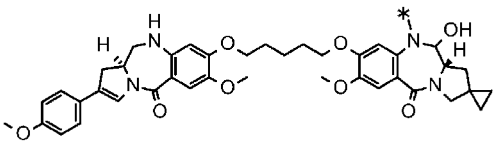
,
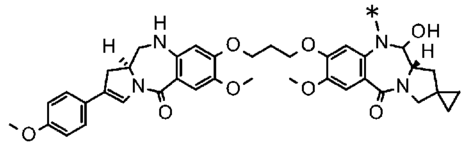
,
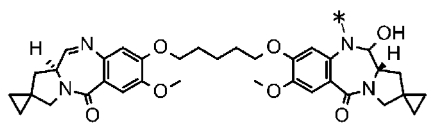
or
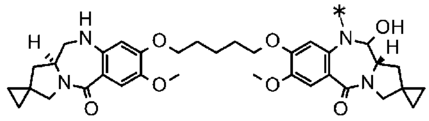

wherein the asterisk (*) represents bonding to L.

The PBD derivative of the present invention has an asymmetric carbon at the 11'-position, and thus there exist optical isomers. Herein, these isomers and a mixture of these isomers are all represented by a single formula. Accordingly, the PBD derivatives of the present invention include all the optical isomers and mixtures of the optical isomers at any ratio. The absolute steric configuration at the 11'-position of the PBD derivatives of the present invention can be determined through X-ray crystal structure analysis or NMR such as a Mosher method for its crystalline product or intermediate, or a derivative thereof. Then, the absolute steric configuration may be determined by using a crystalline product or intermediate derivatized with a reagent having an asymmetric center whose steric configuration is known. As desired, stereoisomers of the synthesized compound according to the present invention may be obtained by isolating with a common optical resolution method or separation method.

There may exist stereoisomers, optical isomers due to an asymmetric carbon atom, geometric isomers, tautomers, or optical isomers such as d-forms, 1-forms and atropisomers for the antibody-drug conjugate of the present invention, the free drug or production intermediates of the antibody-drug conjugate, and these isomers, optical isomers, and mixtures of them are all included in the present invention.

The antitumor compound to be used in the present invention is represented by any one of the formulas below:

[Formula 37]

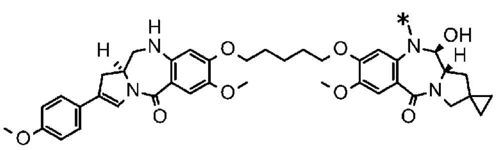
,
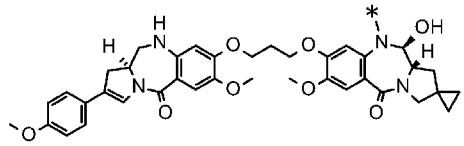
,
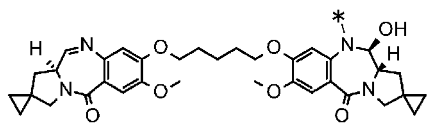
or
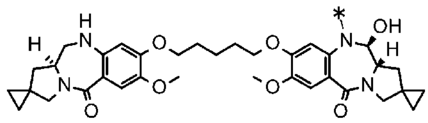

wherein the asterisk (*) represents bonding to L.

<Linker structure>

The linker structure to bond the antitumor drug to an antibody in the antibody-drug conjugate of the present invention will be described.

Linker L is represented by the following formula:

$$-Lb-La-Lp-NH-B-CH_2-O(C=O)-*.$$

The asterisk* represents bonding to the nitrogen atom at the N10'-position of the antitumor compound represented by D; Lb represents a spacer which connects La to the glycan or remodeled glycan of Ab.

B represents a phenyl group or a heteroaryl group, and is preferably a 1,4-phenyl group, a 2,5-pyridyl group, a 3,6-pyridyl group, a 2,5-pyrimidyl group, or a 2,5-thienyl group, and more preferably a 1,4-phenyl group.

Lp represents a linker consisting of an amino acid sequence cleavable *in vivo* or in a target cell. Lp is, for example, cleaved by the action of an enzyme such as an esterase or peptidase.

Lp is a peptide residue composed of two to seven (preferably, two to four) amino acids. That is, Lp is composed of an oligopeptide residue in which two to seven amino acids are connected via a peptide bond.

Lp is bound at the N terminal to the carbonyl group of La in Lb-La-, and forms at the C terminal an amide bond with the amino group (-NH-) of the part -NH-B-$CH_2$-O(C=O)- of the linker. The bond between the C terminal of Lp and -NH- is cleaved by an enzyme such as an esterase.

The amino acids constituting Lp are not limited to particular amino acids, and, for example are L- or D-amino acids, and preferably L-amino acids. The amino acids may be not only $\alpha$-amino acids, but may include an amino acid with structure, for example, of $\beta$-alanine, $\varepsilon$-aminocaproic acid, or $\gamma$-aminobutyric acid, and may further include a non-natural amino acid such as an N-methylated amino acid.

The amino acid sequence of Lp is not limited to a particular amino acid sequence, and examples of amino acids that constitute Lp may include, but are not limited to, glycine (Gly; G), valine (Val; V), alanine (Ala; A), phenylalanine (Phe; F), glutamic acid (Glu; E), isoleucine (Ile; I), proline (Pro; P), citrulline (Cit), leucine (Leu; L), serine (Ser; S), lysine (Lys; K) and aspartic acid (Asp; D). Preferred among them are glycine (Gly; G), valine (Val; V), alanine (Ala; A) and citrulline (Cit).

Any of these amino acids may appear multiple times, and Lp has an amino acid sequence including arbitrarily selected amino acids. Drug release pattern may be controlled via amino acid type.

Specific examples of linker Lp may include, but are not limited to, -GGVA-, -GG-(D-)VA-, -VA-, -GGFG-, -GGPI-, -GGVCit-, -GGVK-, -GG(D-)PI-, -GGPL-, -EGGVA, - PI-, -GGF-, DGGF-, (D-)D-GGF-, -EGGF-, -SGGF-, -KGGF-, - DGGFG-, -GGFGG-, -DDGGFG-, -KDGGFG- and -GGFGGGF-.

Here, "(D-)V" indicates D-valine, "(D)-P" indicates D-proline, and "(D-)D" indicates D-aspartic acid.

Linker Lp is preferably any of the following: - GGVA-, -GG-(D-)VA-, -VA-, -GGFG-, -GGPI-, -GGVCit-, - GGVK-, -GG(D-)PI- and -GGPL-,

Linker Lp is more preferably any of the following: - GGVA-, -GGVCit-, and -VA-.

Lb represents a spacer which connects La to the glycan or remodeled glycan of Ab.

La, which is not particularly limited, represents any one selected from the following group.

- C(=O)-$CH_2CH_2$-C(=O)-,
- C(=O)-$(CH_2CH_2)_2$-C(=O)-,
- C(=O)-$CH_2CH_2$-C(=O)-NH-$(CH_2CH_2)_2$-C(=O)-,
- C(=O)-$CH_2CH_2$-C(=O)-NH-$(CH_2CH_2O)_2$-$CH_2$-C(=O)-,
- C(=O)-$CH_2CH_2$-NH-C(=O)-$(CH_2CH_2O)_4$-$CH_2CH_2$-C(=O)-,
- $CH_2$-OC(=O)- and
- OC(=O)-.

La is more preferably, -C(=O)-$CH_2CH_2$-C(=O)- or - C(=O)-$(CH_2CH_2)_2$-C(=O)-.

Spacer Lb is not limited to a particular spacer, and for examples, a spacer represented by the following formulas are included.

[Formula 38]

(Lb−1)

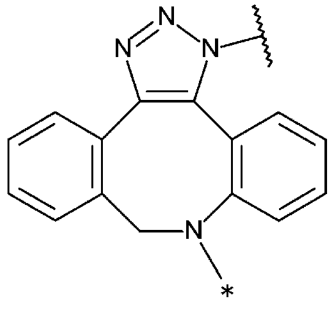

or

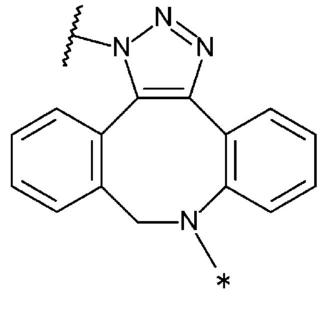

[Formula 39]

(Lb−2)

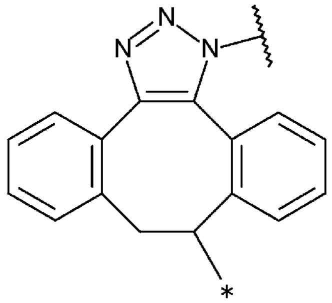

or

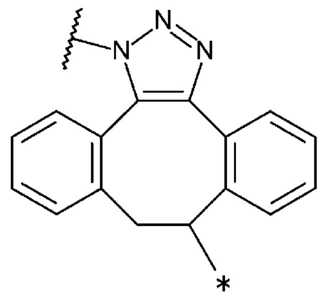

[Formula 40]

(Lb−3)

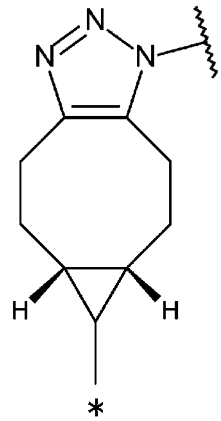

or

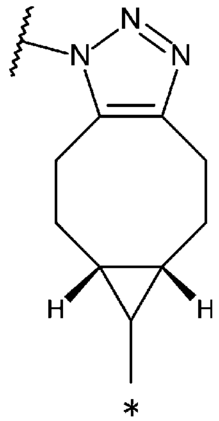

In the structural formulas for Lb shown above, each asterisk* represents bonding to the -(C=O) or $-(CH_2)n^4$ at the left end of La, and each wavy line represents bonding to the glycan or remodeled glycan of Ab.

In each structural formula for Lb (Lb-1, Lb-2 or Lb-3) shown above, the triazole ring site formed through click reaction of an azide group and a cyclooctynyl group provides structures of geometric isomers, and molecules of Lb exist as any one of the two structures or as a mixture of both of them. There exist two or four ($m^2$ is 1 or 2) "-L-D" moieties per molecule of the antibody-drug conjugate of the present invention, and either one of the two structures exist or both of them coexist as Lb (Lb-1, Lb-2, or Lb-3) in L of each of the "-L-D" moieties.

If Lb is i), L is preferably represented by $-Lb-La-Lp-NH-B-CH_2-O(C=O)-*$

L is selected from the following group:

- $Z^1-C(=O)-CH_2CH_2-C(=O)-GGVA-NH-B-CH_2-OC(=O)-$,
- $Z^1-C(=O)-CH_2CH_2-C(=O)-GG-(D-)VA-NH-B-CH_2-OC(=O)-$
- $Z^1-C(=O)-CH_2CH_2-C(=O)-VA-NH-B-CH_2-OC(=O)-$,
- $Z^1-C(=O)-(CH_2CH_2)_2-C(=O)-VA-NH-B-CH_2-OC(=O)-$,
- $Z^1-C(=O)-CH_2CH_2-C(=O)-GGPI-NH-B-CH_2-OC(=O)-$,
- $Z^1-C(=O)-CH_2CH_2-C(=O)-GGFG-NH-B-CH_2-OC(=O)-$,
- $Z^1-C(=O)-CH_2CH_2-C(=O)-GGVCit-NH-B-CH_2-OC(=O)-$,
- $Z^1-C(=O)-CH_2CH_2-C(=O)-GGVK-NH-B-CH_2-OC(=O)-$,
- $Z^{1-}C(=O)-CH_2CH_2-C(=O)-GGPL-NH-B-CH_2-OC(=O)-$,
- $Z^1-C(=O)-CH_2CH_2-C(=O)-NH-(CH_2CH_2)_2-C(=O)-VA-NH-B-CH_2-OC(=O)-$,
- $Z^1-C(=O)-CH_2CH_2-C(=O)-NH-(CH_2CH_2O)_2-CH_2-C(=O)-VA-NH-B-CH_2-OC(=O)-$,
- $Z^1-C(=O)-CH_2CH_2-NH-C(=O)-(CH_2CH_2O)_4-CH_2CH_2-C(=O)-VA-NH-B-CH_2-OC(=O)-$,
- $Z^2-OC(=O)-GGVA-NH-B-CH_2-OC(=O)-$ and
- $Z^3-CH_2-OC(=O)-GGVA-NH-B-CH_2-OC(=O)-$,

wherein $Z^1$ represents the following structural formula:

[Formula 41]

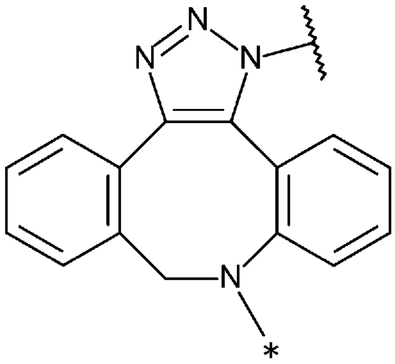

or

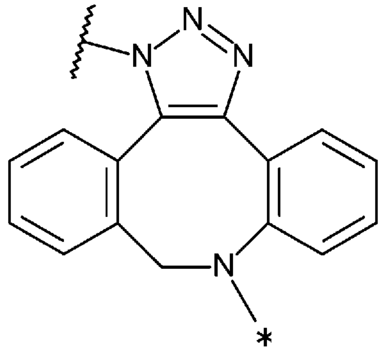

$Z^2$ represents the following structural formula:

[Formula 42]

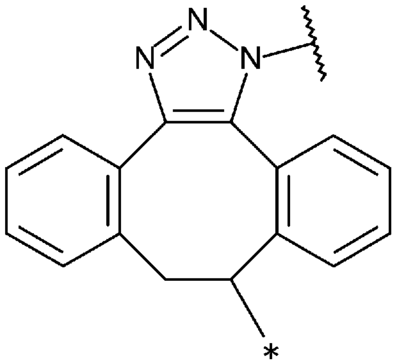

or

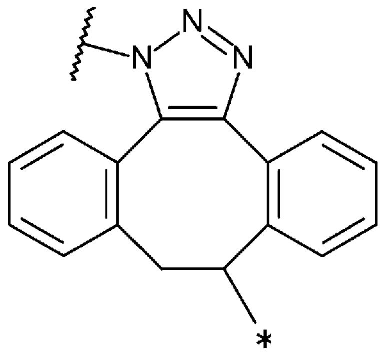

$Z^3$ represents the following structural formula:

[Formula 43]

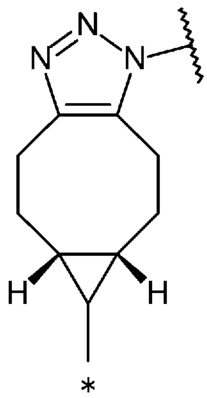

or

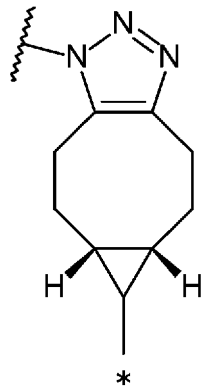

wherein, in the structural formulas $Z^1$, $Z^2$ and $Z^3$, the asterisk * represents bonding to the C(=O), O or $CH_2$ neighboring $Z^1$, $Z^2$ or $Z^3$ and the wavy line represents bonding to the glycan or remodeled glycan of Ab; and

B represents a 1,4-phenyl group.

The antibody-drug conjugate of the present invention is supposed to exhibit antitumor activity through a process in which most molecules of the antibody-drug conjugate migrate into tumor cells, and a linker portion (e.g., Lp) is then cleaved by an enzyme or the like to activate the antibody-drug conjugate, which releases the portion of drug D (hereinafter, referred to as the free drug (described later)).

Therefore, it is preferable that the antibody-drug conjugate of the present invention is stable outside tumor cells.

<Glycan Remodeling>

Recently has been reported a method for remodeling heterogeneous glycoproteins of an antibody by enzymatic reaction or the like to introduce a homogeneous glycan having a functional group (ACS Chemical Biology 2012, 7, 110, ACS Medicinal Chemistry Letters 2016, 7, 1005). In addition, an attempt with use of this glycan remodeling technique has been made to site-specifically introduce a drug to synthesize a homogeneous ADC (Bioconjugate Chemistry 2015, 26, 2233, Angew. Chem. Int. Ed. 2016, 55, 2361-2367, US 2016361436).

In the glycan remodeling of the present invention, using a hydrolase, heterogeneous glycans bonding to a protein (e.g., an antibody) are cleaved off to leave only GlcNAc at each terminus thereby producing a homogenous protein moiety with GlcNAc (hereinafter, referred to as an "acceptor"). Subsequently, an arbitrary glycan separately prepared (hereinafter, referred to as a "donor") is provided, and the acceptor and the donor are linked together by using a transglycosidase. Thereby, a homogeneous glycoprotein with an arbitrary glycan structure can be synthesized.

In the present invention, a "glycan" refers to a structural unit of two or more monosaccharides bonded together via glycosidic bonds. Specific monosaccharides and glycans are occasionally abbreviated, for example, as "GlcNAc-", "MSG-", and so on. When any of these abbreviations is used in a structural formula, the abbreviation is shown with an intention that an oxygen atom or nitrogen atom involved in a glycosidic bond at the reducing terminal to another structural unit is not included in the abbreviation indicating the glycan, unless specifically defined.

In the present invention, a monosaccharide as a basic unit of a glycan is indicated for convenience so that in the ring structure, the position of a carbon atom bonding to an oxygen atom constituting the ring and directly bonding to a hydroxy group (or an oxygen atom involved in a glycosidic bond) is defined as the 1-position (the 2-position only for sialic acids), unless otherwise specified. The names of compounds in the Examples are each provided in view of the chemical structure as a whole, and that rule is not necessarily applied.

When a glycan is indicated as a sign (e.g., GLY, SG, MSG, GlcNAc) in the present invention, the sign is intended, unless otherwise defined, to include carbon atoms ranging to the reducing terminal and not to include N or O involved in an N- or O-glycosidic bond.

In the present invention, unless specifically stated, a partial structure when a glycan is linking to a side chain of an amino acid is indicated in such a manner that the side chain portion is indicated in parentheses, for example, "(SG-)Asn".

Glycans in Ab of the present invention are N-linked glycans or O-linked glycans, and preferably N-linked glycans.

N-linked glycans and O-linked glycans bond to an amino acid side chain of an antibody via an N-glycosidic bond and an O-glycosidic bond, respectively.

IgG has a well conserved N-linked glycan on an asparagine residue at the 297-position of the Fc region of the heavy chain (hereinafter, referred to as "Asn297 or N297"), and the N-linked glycan is known to contribute to the activity and kinetics of the antibody molecule (Biotechnol. Prog., 2012, 28, 608-622, Sanglier-Cianferani, S., Anal. Chem., 2013, 85, 715-736).

The amino acid sequence in the constant region of IgG is well conserved, and each amino acid is specified by Eu index numbering in Edelman et al. (Proc. Natl. Acad. Sci. U.S.A., Vol. 63, No. 1 (May 15, 1969), pp. 78-85). For example, Asn297, to which an N-linked glycan is added in the Fc region, corresponds to the 297-position in Eu index numbering, and each amino acid is uniquely specified by Eu index numbering, even if the actual position of the amino acid has varied through fragmentation of the molecule or deletion of a region.

In the antibody-drug conjugate of the present invention, the antibody or functional fragment of the antibody more preferably bonds to L via a glycan bonding to a side chain of Asn297 thereof (hereinafter, referred to as "N297 glycan"), and the antibody or functional fragment of the antibody even more preferably bonds via the N297 glycan to L, wherein the N297 glycan is a remodeled glycan.

An antibody having the remodeled glycan is referred to as a glycan-remodeled antibody.

SGP, an abbreviation for sialyl glycopeptide, is a representative N-linked complex glycan. SGP can be separated/purified from the yolk of a hen egg, for example, by using a method described in WO 2011/0278681. Purified products of SGP are commercially available (Tokyo Chemical Industry Co., Ltd., FUSHIMI Pharmaceutical Co., Ltd.). For example, disialooctasaccharide (Tokyo Chemical Industry Co., Ltd.), a glycan formed by deleting one GlcNAc at the reducing terminus in the glycan moiety of SG (hereinafter, referred to as "SG (10)", is commercially available.

In the present invention, a glycan structure formed by deleting a sialic acid at a non-reducing terminal only in either one of the branched chains of β-Man in SG (10) refers to MSG (9), and a structure having a sialic acid only in the 1-3 branched chain is called as MSG1, and a structure having a sialic acid only in the 1-6 branched chain is called as MSG2.

The remodeled glycan of the present invention is N297-(Fuc)MSG1, N297-(Fuc)MSG2, or a mixture of N297-(Fuc)MSG1 and N297-(Fuc)MSG2, or N297-(Fuc)SG, and is preferably N297-(Fuc)MSG1, N297-(Fuc)MSG2, or N297-(Fuc)SG, and is more preferably N297-(Fuc)MSG1 or N297-(Fuc)MSG2.

N297-(Fuc)MSG1 is represented by the following structural formula or sequence formula:

[Formula 44]

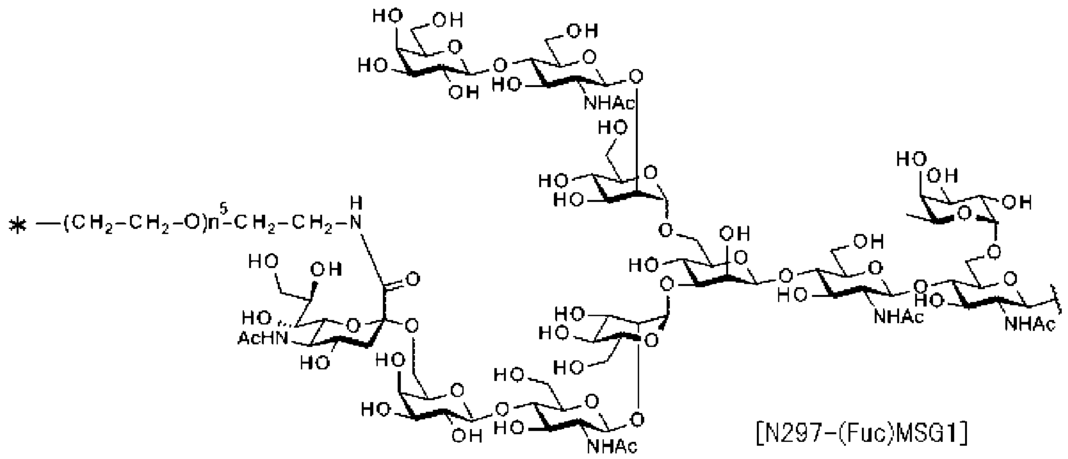

[N297-(Fuc)MSG1]

[Formula 45]

Fucα1
|
6

Galβ1-4GlcNAcβ1-2Manα1— 6

Manβ1-4GlcNAcβ1-4GlcNAcβ1

* —L(PEG)-NeuAcα2-6Galβ1-4GlcNAcβ1-2Manα1— 3

[N297-(Fuc)MSG1]

In the formulas, each wavy line represents bonding to Asn297 of the antibody,

L (PEG) represents $-(CH_2CH_2-O)n^5-CH_2CH_2-NH-$, wherein the amino group at the right end represents amide-bonding to the carboxylic acid at the 2-position of the sialic acid at the non-reducing terminal in the 1-3 branched chain of the β-Man in the N297 glycan,
Each asterisk* represents bonding to linker L, in particular, the nitrogen atom at the 1- or 3-position of the 1,2,3-triazole ring of Lb in linker L, and
$n^5$ represents an integer of 2 to 10, and preferably an integer of 2 to 5.

N297-(Fuc)MSG2 is represented by the following structural formula or sequence formula.

[Formula 46]

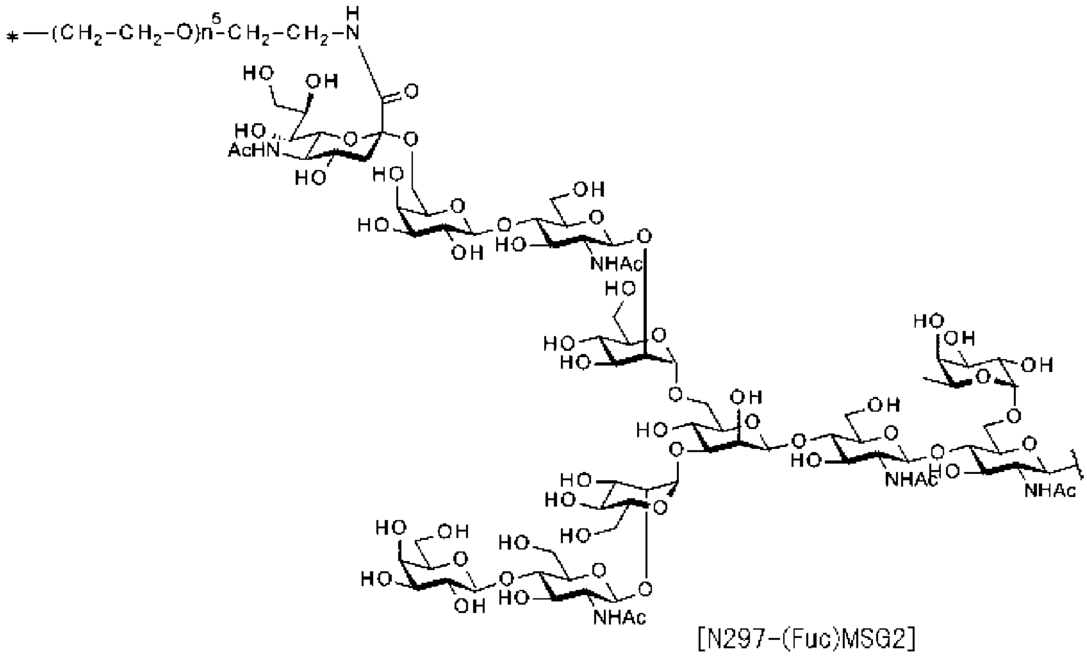

[N297-(Fuc)MSG2]

[Formula 47]

Fucα1
|
6

* - L(PEG)-NeuAcα2-6Galβ1-4GlcNAcβ1-2Manα1 — 6
Manβ1-4GlcNAcβ1-4GlcNAcβ1-
Galβ1-4GlcNAcβ1-2Manα1 — 3

[N297-(Fuc)MSG2]

In the formulas, each wavy line represents bonding to Asn297 of the antibody,

L(PEG) represents $-(CH_2CH_2-O)n^5-CH_2CH_2-NH-$, wherein the amino group at the right end represents amide-bonding to the carboxylic acid at the 2-position of the sialic acid at the non-reducing terminal in the 1-6 branched chain of the β-Man in the N297 glycan,
The asterisk* represents bonding to linker L, in particular, the nitrogen atom at the 1- or 3-position of the 1,2,3-triazole ring of Lb in linker L, and
$n^5$ is an integer of 2 to 10, and preferably an integer of 2 to 5.

N297-(Fuc)SG is represented by the following structural formula or sequence formula.

[Formula 48]

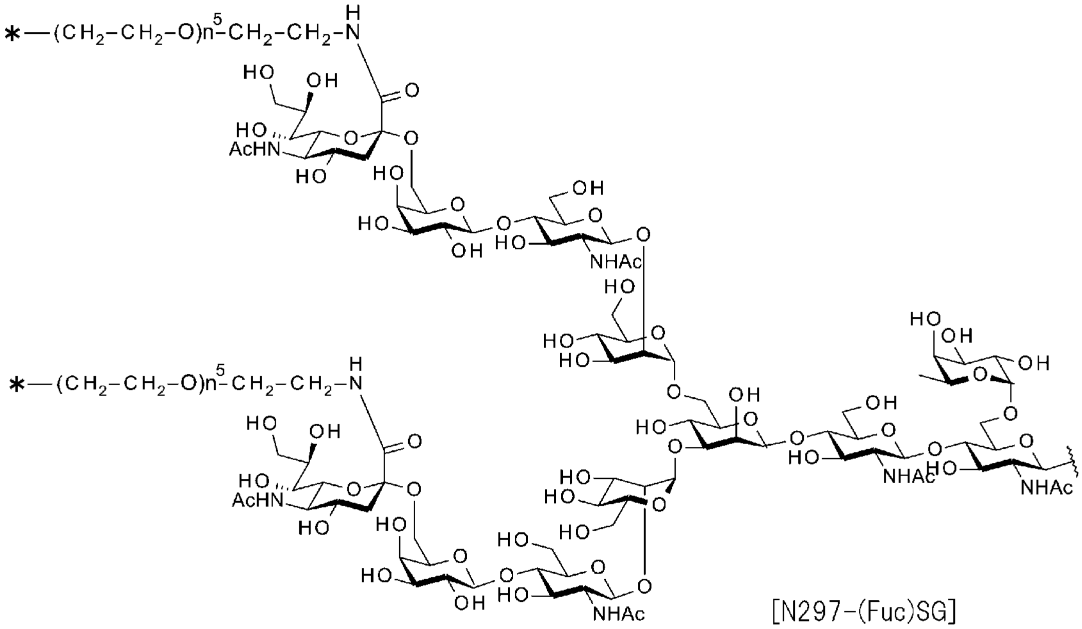

[N297-(Fuc)SG]

[Formula 49]

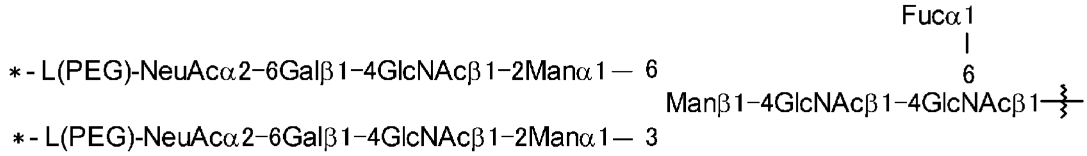

[N297-(Fuc)SG]

In the formulas, each wavy line represents bonding to Asn297 of the antibody,

L (PEG) represents - $(CH_2CH_2\text{-}O)n^5\text{-}CH_2CH_2\text{-}NH\text{-}$, wherein the amino group at the right end represents amide-bonding to the carboxylic acid at the 2-position of the sialic acid at the non-reducing terminal in each of the 1-3 and 1-6 branched chains of the β-Man in the N297 glycan,
each asterisk* represents bonding to linker L, in particular, the nitrogen atom at the 1- or 3-position of the 1,2,3-triazole ring of Lb in linker L, and
$n^5$ is an integer of 2 to 10, and preferably an integer of 2 to 5.

If the N297 glycan of the antibody in the antibody-drug conjugate of the present invention is N297-(Fuc)MSG1, N297-(Fuc)MSG2, or a mixture of them, the antibody-drug conjugate is a molecule to which two molecules of linker L and two molecules of drug D have been conjugated ($m^2$ = 1) since the antibody is a dimer.

<Preparation of antibody>

A glycan-remodeled antibody can be produced by using a method as illustrated in the following formula, for example, according to a method described in WO2013/120066.

[Formula 50]

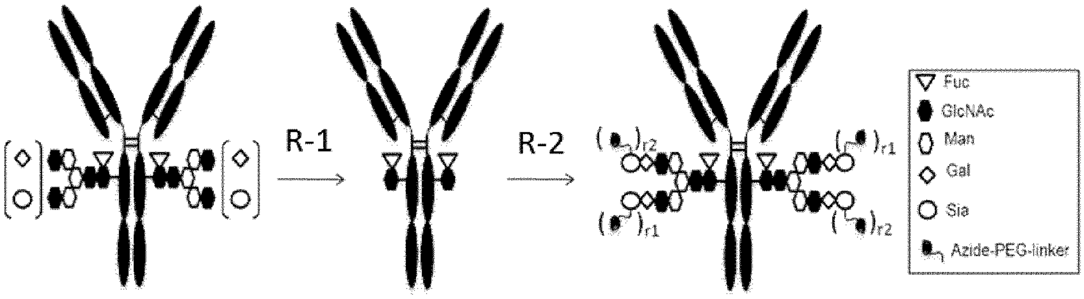


(r1, r2) is any one of (1, 0), (0, 1) and (1,1)

Step R-1: Hydrolysis of glycosidic bond at GlcNAcβ1-4GlcNAc of chitobiose structure at reducing terminal

This is a step of preparing a glycan-truncated antibody by cleaving N-linked glycan bonding to asparagine at the 297-position of the amino acid sequence of a targeted antibody (N297-linked glycan) with use of a known enzymatic reaction.

A targeted antibody (20 mg/mL) in buffer solution (e.g., 50 mM phosphate buffer solution) is subjected to a hydrolysis reaction of the glycosidic bond between GlcNAcβ1 and 4GlcNAc in the chitobiose structure at the reducing terminal with use of hydrolase such as the enzyme EndoS at 0°C to 40°C. The reaction time is 10 minutes to 72 hours, and preferably 1 hour to 6 hours. The amount of the wild-type EndoS to be used is 0.1 to 10 mg, preferably 0.1 to 3 mg, to 100 mg of the antibody. After the completion of the reaction, purification with affinity chromatography and/or purification with a hydroxyapatite column, each described later, are/is carried out to produce a (Fucα1,6)GlcNAc antibody with the glycan hydrolyzed between GlcNAcβ1 and 4GlcNAc.

Step R-2: Transglycosylation reaction

This is a step of producing a glycan-remodeled antibody by bonding the (Fucα1,6)GlcNAc antibody to MSG-(MSG1-, MSG2-) or SG-type glycan oxazoline (hereinafter, referred to as "azide glycan oxazoline ") having a PEG linker including an azide group with use of an enzymatic reaction.

The glycan-truncated antibody in buffer solution (e.g., phosphate buffer solution) is subjected to transglycosylation reaction by reacting with an azide glycan oxazoline in the presence of a catalytic amount of transglycosidase such as EndoS (D233Q/Q303L) at 0°C to 40°C. The reaction time is 10 minutes to 72 hours, and preferably 1 hour to 6 hours. The amount of the EndoS (D233Q/Q303L) to be used is 1 to 10 mg, preferably 1 to 3 mg, to 100 mg of the antibody, and the amount of the azide glycan oxazoline to be used is 2 equivalents to an excess equivalent, preferably 2 equivalents to 20 equivalents.

After the completion of the reaction, purification with affinity chromatography and purification with a hydroxyapatite column are carried out to afford a purified glycan-remodeled antibody.

The azide glycan oxazoline form may be prepared according to methods described in Example 11. By using a reaction known in the field of synthetic organic chemistry (e.g., a condensation reaction), $N_3$-$(CH_2CH_2$-$O)n_5$-$CH_2CH_2$-$NH_2$, a PEG linker including an azide group ($N_3$-L(PEG)), may be introduced to MSG1. Specifically, carboxylic acid at the 2-position of a sialic acid and the amino group at the right-hand end of $N_3$-$(CH_2CH_2$-$O)n_5$-$CH_2CH_2$-$NH_2$ undergo a condensation reaction to form an amide bond.

Examples of the condensing agent in using condensation reaction may include, but not limited to, N, N'-dicyclohexylcarbodiimide (DCC), 1-ethyl-3-(3-dimethylaminopropyl)carbodiimide (EDCI), carbonyldiimidazole (CDI), 2-(2H-benzotriazol-2-yl)-4-(1, 1, 3, 3-tetramethylbutyl)phenol (BOP), 1H-benzotriazol-1-yloxytripyrrolidinophosphonium hexafluorophosphate (PyBOP) and O-(7-azabenzotriazol-1-yl)-N, N, N', N'-tetramethyluronium hexafluorophosphate (HATU), and examples of the solvent for the reaction may include, but not limited to, dichloromethane, DMF, THF, ethyl acetate and mixed solvent thereof.

The reaction temperature is typically -20°C to 100°C or the boiling point of the solvent, and preferably in the range of -5°C to 50°C. As necessary, an organic base such as triethylamine, diisopropylethylamine, N-methylmorpholine, and 4-dimethylaminopyridine or an inorganic base such as potassium carbonate, sodium carbonate, potassium hydrogen carbonate, and sodium hydrogen carbonate may be added. Further, for example, 1-hydroxybenzotriazole or N-hydroxysuccinimide may be added as a reaction accelerator.

MSG1 may be obtained by hydrolysis of the separated/purified (MSG-)Asn (in Example 11) with hydrolase

such as EndoM.

Oxazolination may be prepared from GlcNAc at the reducing terminal of MSG1 according to a known article (J. Org Chem., 2009, 74(5), 2210-2212. Helv. Chim. Acta, 2012, 95, 1928-1936.).

For the enzyme for the hydrolysis reaction of N297 glycan, for example, EndoS or an enzyme variant retaining hydrolysis activity may be used.

By reacting the (Fucα1,6)GlcNAc-antibody obtained in the above hydrolysis reaction, as a glycan acceptor molecule, and an MSG- (MSG1-, MSG2-) or SG-type glycan donor molecule by use of a glycosyltransferase (e.g., WO 2017010559) such as an EndoS D233Q or EndoS D233Q/Q303L variant, an antibody of the above-described structure including MSG- (MSG1-, MSG2-) or SG type N297 glycan can be obtained.

If the number of conjugated drug molecules per antibody molecule, $m^2$, in the antibody-drug conjugate is 1, a glycan donor molecule having MSG, MSG1, or MSG2 as a glycan is employed. For such a glycan, commercially available monosialo-Asn free (1S2G/1G2S-10NC-Asn, GlyTech, Inc., hereinafter, referred to as "(MSG-)Asn") as a raw material may be separated in accordance with a method described in Example 11 to obtain (MSG-)Asn1 or (MSG2-)Asn, which may each be employed, or a mixture of them may be employed without separation.

If the number of conjugated drug molecules per antibody molecule, $m^2$, in the antibody-drug conjugate is 2, a glycan donor molecule including SG (10) as glycan is used for the transglycosylation reaction. For such SG (10) glycan, for example, that obtained from SGP through hydrolysis or the like may be used, or SG (10) glycan such as commercially available disialooctasaccharide (Tokyo Chemical Industry Co., Ltd.) may be used.

MSG- (MSG1-, MSG2-) or SG-type glycan included in the donor molecule has a PEG linker having an azide group ($N_3$-L(PEG)) at the 2-position of a sialic acid therein. To introduce a PEG linker having an azide group ($N_3$-L(PEG)) to the 2-position of a sialic acid, a reaction known in the field of synthetic organic chemistry (e.g., a condensation reaction) may be used for MSG (MSG (9)), MSG1, or MSG2, or disialooctasaccharide (SG (10)) and the PEG linker having an azide group ($N_3$-L(PEG)) $N_3$-$(CH_2CH_2$-$O)n_5$-$CH_2CH_2$-$NH_2$, wherein $n_5$ is an integer of 2 to 10, and preferably represents an integer of 2 to 5. Specifically, the carboxylic acid at the 2-position of a sialic acid and the amino group at the right-hand end of $N_3$-$(CH_2CH_2$-$O)n_5$-$CH_2CH_2$-$NH_2$ undergo a condensation reaction to from an amide bond.

Alternatively, MSG (MSG1, MSG2) or SG-type glycan may be obtained by introducing a PEG linker having an azide group ($N_3$-$(CH_2CH_2$-$O)n_5$-$CH_2CH_2$-$NH_2$) to the carboxylic acid at the 2-position of a sialic acid of a raw material, such as (MSG1-)Asn, (MSG2-)Asn and (SG-)Asn (GlyTech, Inc.) with an α-amino group optionally protected or modified, and to carboxylic acid of the Asn with use of a condensation reaction, and utilizing a hydrolase such as EndoM and EndoRp. Examples of protective groups for α-amino groups include, but not limited to, an acetyl (Ac) group, a t-butoxycarbonyl (Boc) group, a benzoyl (Bz) group, a benzyl (Bzl) group, a carbobenzoxy (Cbz) group and a 9-fluorenylmethoxycarbonyl (Fmoc) group. The protective group for α-amino groups is preferably an Fmoc group.

Examples of modifying groups for α-amino groups include modifying groups that enhance solubility in water with a hydroxyacetyl group, a PEG structure, or the like.

An α-amino group of (MSG1-)Asn, (MSG-2)Asn, or (SG-)Asn is preferably protected with any of the protective groups. If an α-amino group is protected with a protective group (e.g., an Fmoc group), the protective group may be removed, as necessary, after introduction of a PEG linker having an azide group and before causing action of a hydrolase.

It is preferred to use an activated form such as an oxazoline formed by treatment with 2-chloro-1,3-dimethyl-1H-benzimidazol-3-ium-chloride for GlcNAc at the reducing terminal of MSG (MSG1, MSG2) or SG-type glycan included in the molecule.

Various enzymes for use in a transglycosylation reaction (transglycosidases) may be employed that have the activity of transferring a complex glycan to N297 glycan; however, EndoS D233Q, a modified product for which a hydrolysis reaction is suppressed by substituting Asp at the 233-position of EndoS with Gln, is a preferred transglycosidase. A transglycosylation reaction using EndoS D233Q is described, for example, in WO 2013/120066. Alternatively, a modified enzyme such as EndoS D233Q/Q303L (WO 2017010559), which is obtained by further adding a mutation to EndoS D233Q, may be used.

The purification operation for the antibody after the glycan remodeling for the antibody (glycohydrolysis and the transglycosylation reaction) is intended to separate low-molecular-weight compounds and enzymes used for the reaction, and gel filtration chromatography, ionexchange chromatography, affinity chromatography, and so on are typically used for such purification, and additional purification with a hydroxyapatite column may be further carried out. That is, the present invention provides a method for producing a drug-conjugate form, the method including, subsequent to the step of purifying an intermediate from the reaction solution after glycohydrolysis of an antibody, the additional step of purifying with a hydroxyapatite column. According to an example of reports on glycan remodeling (J. Am. Chem. Soc. 2012, 134, 12308-12318., Angew. Chem. Int. Ed. 2016, 55, 2361-2367), a reaction solution after treatment of an antibody with hydrolase can be purified with a Protein A column (affinity chromatography column) only; however, this purification method has been proved to be incapable of completely removing hydrolase (e.g., EndoS), which affects the subsequent transglycosylation reaction because of the residual enzyme. In view of such a result, purification methods were examined, resulting in finding that when purification of a reaction solution after treatment of an antibody with hydrolase was carried

out using a Protein A column and a hydroxyapatite column (CHT column, Bio-Rad Laboratories, Inc.) in the order presented, the reaction efficiency of the subsequent glycosylation reaction was enhanced, without the influence of any residual enzyme.

In preparing the glycan-remodeled antibody, the concentration of an aqueous solution of an antibody, measurement of concentration, and buffer exchange may be carried out according to common operations A to C in the following.

(Common operation A: Concentration of aqueous solution of antibody)

A solution of an antibody or antibody-drug conjugate was placed in a container of an Amicon Ultra (30,000 to 50,000 MWCO, Millipore Corporation), and the solution of an antibody or antibody-drug conjugate, which is described later, was concentrated through a centrifugation operation (centrifugation at 2000 G to 4000 G for 5 to 20 minutes) using a centrifuge (Allegra X-15R, Beckman Coulter, Inc.).

(Common operation B: Measurement of antibody concentration)

Measurement of antibody concentration was carried out by using a UV measurement apparatus (Nanodrop 1000, Thermo Fisher Scientific Inc.) according to a method specified by the manufacturer. Then, 280 nm absorption coefficients, being different among antibodies (1.3 mL $mg^{-1}$ $cm^{-1}$ to 1.8 mL $mg^{-1}$ $cm^{-1}$), were used.

(Common operation C: Buffer exchange for antibody)

A buffer solution (e.g., phosphate buffered saline (pH 6.0), phosphate buffer (pH 6.0)) was added to an aqueous solution of an antibody, which was concentrated according to common operation A. This operation was carried out several times, and the antibody concentration was then measured by using common operation B, and adjusted to 10 mg/mL with a buffer solution (e.g., phosphate buffered saline (pH 6.0), phosphate buffer (pH 6.0)).

<Conjugation>

This production method is a method for producing an antibody-drug conjugate by conjugating the above-described glycan-remodeled antibody to production intermediate (2) through SPAAC (strain-promoted alkyne azide cycloaddition: J. AM. CHEM. SOC. 2004, 126, 15046-15047) reaction.

[Formula 51]

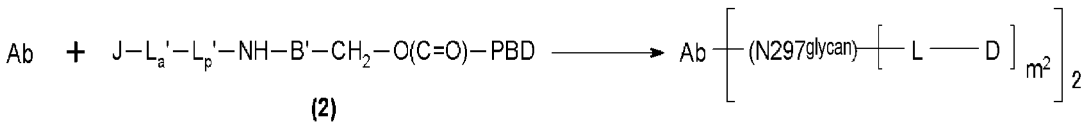

(2)

wherein Ab represents the glycan-remodeled antibody,
La', Lp' and B' are the same as defined in La, Lp and B, respectively, and
J represents any of these structural formulas, wherein the asterisk* represents bonding to La'.

[Formula 52]

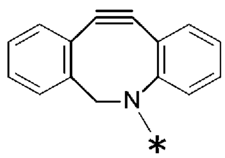
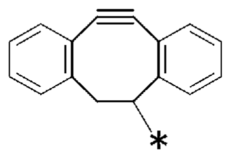
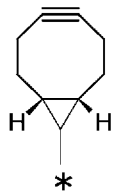

J-La'-Lp'-NH-B'-$CH_2$-O(C=O)-PBD can be synthesized by the method described in any one of Examples 10-1 to 10-6.

SPAAC reaction proceeds by mixing a buffer solution (sodium acetate solution, sodium phosphate, sodium borate solution, or the like, or a mixture thereof) of antibody Ab and a solution dissolving compound (2) in an appropriate

solvent (dimethyl sulfoxide (DMSO), dimethylformamide (DMF), dimethylacetamide (DMA), N-methyl-2-pyridone (NMP), propylene glycol (PG), or the like, or a mixture thereof).

The amount of moles of compound (2) to be used is 2 mol to an excess amount of moles, preferably 1 mol to 30 mol, per mole of the antibody, and the ratio of the organic solvent is preferably 1 to 200% v/v to the buffer of the antibody. The reaction temperature is 0°C to 37°C, and preferably 10°C to 25°C, and the reaction time is 1 to 150 hours, and preferably 6 hours to 100 hours. The pH in the reaction is preferably 5 to 9.

Antibody-drug conjugate compounds (ADCs) can be identified from each other through buffer exchange, purification, and measurement of antibody concentration and average number of conjugated drug molecules per antibody molecule (DAR:Drug to Antibody Ratio) according to common operations A to C described above and common operations D to F described later.

(Common operation D: Purification of antibody-drug conjugate)

An NAP-25 column was equilibrated with acetate buffer solution (10 mM, pH 5.5; herein, referred to as ABS) containing commercially available sorbitol (5%). To this NAP-25 column, an aqueous reaction solution of an antibody-drug conjugate (about 1.5 to 2.5 mL) was applied, and eluted with a buffer in an amount specified by the manufacturer to separate and collect an antibody fraction. The fraction separated and collected was again applied to the NAP-25 column, and a gel filtration purification operation to elute with a buffer was repeated twice or three times in total to afford the antibody-drug conjugate with an unbound drug-linker, dimethyl sulfoxide, and propylene glycol removed. As necessary, the concentration of the solution of the antibody-drug conjugate was adjusted through common operations A to C.

(Common operation E: Measurement of antibody concentration of antibody-drug conjugate)

The concentration of the conjugated drug in an antibody-drug conjugate can be calculated by using Lambert-Beer's law shown below. Equation (I) using Lambert-Beer's law is as follows:
[Expression 1]

$$\underset{\text{Absorbance}}{A_{280}} = \underset{\text{Molar absorption coefficient}}{\varepsilon_{280}(L \cdot mol^{-1} \cdot cm^{-1})} \cdot \underset{\text{Molar concentration}}{C(mol \cdot L^{-1})} \cdot \underset{\text{Optical path length}}{l(cm)} \quad \text{Expression (I)}$$

Here, A280 denotes absorbance of an aqueous solution of an antibody-drug conjugate at 280 nm, ε280 denotes the molar absorption coefficient of an antibody-drug conjugate at 280 nm, and C ($mol \cdot L^{-1}$) denotes the molarity of an antibody-drug conjugate. From expression (I), the molarity of an antibody-drug conjugate, C ($mol \cdot L^{-1}$), can be determined by using expression (II) below.
[Expression 2]

$$C(mol \cdot L^{-1}) = \frac{A_{280}}{\varepsilon_{280}(L \cdot mol^{-1} \cdot cm^{-1}) \cdot l(cm)} \quad \text{Expression (II)}$$

Further, the both sides are multiplied by the molar mass of the antibody-drug conjugate, MW ($g \cdot mol^{-1}$), to determine the weight concentration of the antibody-drug conjugate, C' ($mg \cdot mL^{-1}$) (expression (III)).
[Expression 3]

$$C'(mg \cdot mL^{-1}) = MW(g \cdot mol^{-1}) \cdot C(mol \cdot L^{-1}) = \frac{A_{280} \cdot MW(g \cdot mol^{-1})}{\varepsilon_{280}(L \cdot mol^{-1} \cdot cm^{-1}) \cdot l(cm)} \quad \text{Expression (III)}$$

Values used for the expression and applied to Examples will be described.

The absorbance A280 used was a measured value of UV absorbance of an aqueous solution of an antibody-drug conjugate at 280 nm. For molar mass, MW ($g \cdot mol^{-1}$), an estimated value of the molecular weight of an antibody was calculated from the amino acid sequence of the antibody, and used as an approximate value of the molar mass of an antibody-drug conjugate. The optical path length, 1 (cm), used in measurement was 1 cm.

The molar absorption coefficient, ε280, of the antibody-drug conjugate can be determined by using expression (IV) below:

[Expression 4]

$$\varepsilon_{280} = \text{Molar absorption coefficient of antibody } \varepsilon_{Ab,\ 280} + \text{Molar absorption coefficient of drug } \varepsilon_{DL,\ 280} \times \text{Number of drug molecules conjugated} \qquad \text{Expression (IV)}$$

Here, $\varepsilon_{Ab, 280}$ denotes the molar absorption coefficient of an antibody at 280 nm and $\varepsilon_{DL, 280}$ denotes the molar absorption coefficient of a drug at 280 nm.

By using a known calculation method (Protein Science, 1995, vol. 4, 2411-2423), $\varepsilon_{Ab, 280}$ can be estimated from the amino acid sequence of an antibody. In the Examples, the molar absorption coefficient of H01L02 antibody used was $\varepsilon_{Ab, 280}$ = 223400 (calculated estimated value). The molar absorption coefficient of H01L02A antibody used was $\varepsilon_{Ab, 280}$ = 223674 (calculated estimated value), the molar absorption coefficient of H31L02A antibody used was $\varepsilon_{Ab, 280}$ = 223314 (calculated estimated value), the molar absorption coefficient of H11L02A antibody used was $\varepsilon_{Ab, 280}$ =220490 (calculated estimated value), and the molar absorption coefficient of LPS antibody used was $\varepsilon_{Ab, 280}$ = 230300 (calculated estimated value).

$\varepsilon_{DL, 280}$ was calculated for use from a measured value obtained in each UV measurement. Specifically, the absorbance of a solution dissolving a conjugate precursor (drug) with a certain molarity was measured, and expression (I), Lambert-Beer's law, was applied thereto, and the resulting value was used.

(Common operation F: Measurement of average number of conjugated drug molecules per antibody molecule in antibody-drug conjugate

The average number of conjugated drug molecules per antibody molecule in an antibody-drug conjugate can be determined through high-performance liquid chromatography (HPLC) with the following method.

(F-1 Preparation of sample for HPLC analysis (reduction of antibody-drug conjugate))

A solution of an antibody-drug conjugate (about 1 mg/mL, 60 μL) is mixed with an aqueous solution of dithiothreitol (DTT) (100 mM, 15 μL). The mixture is incubated at 37°C for 30 minutes to prepare a sample in which the disulfide bond between the L chain and H chain of the antibody-drug conjugate cleaved, and this sample is used for HPLC analysis.

(F-2. HPLC analysis)

HPLC analysis is carried out under the following conditions.

HPLC system: Agilent 1290 HPLC system (Agilent Technologies)
Detector: Ultraviolet absorption spectrometer (measurement wavelength: 280 nm, 329 nm)
Column: BEH Phenyl (2.1 × 50 mm, 1.7 μm, Waters Acquity)
Column temperature: 75°C
Mobile phase A: 0.1% trifluoroacetic acid (TFA) -15% isopropyl alcohol aqueous solution
Mobile phase B: 0.075% TFA-15% isopropyl alcohol acetonitrile solution
Gradient program: 14%-36% (0 min to 15 min), 36%-80% (15 min to 17 min), 80%-14% (17 min to 17.1 min), 14%-14% (17.1 min to 23 min)
Sample injection volume: 5μL

(F-3. Data analysis)

(F-3-1) An H chain with a conjugated drug molecule(s) (H chain with one conjugated drug molecule: $H_1$, H chain with two conjugated drug molecules: $H_2$) have hydrophobicity increased in proportion to the number of conjugated drug molecules and have longer retention time as compared to the L chain ($L_0$) and H chain ($H_0$) of an antibody without any conjugated drug molecule, and hence $L_0$, $H_0$, $H_1$, and $H_2$ are eluted in the presented order. Therefore, through comparison of retention time between $L_0$ and $H_0$, each peak detected can be assigned to $L_0$, $H_0$, $H_1$, or $H_2$. Whether a drug is conjugated or not can be confirmed by checking absorption at a wavelength of 329 nm characteristic to the drug.

(F-3-2) Since each drug-linker absorbs UV, peak area values are corrected by using the following expression

with the molar absorption coefficients of an H chain and drug-linker according to the number of conjugated drug-linker molecules.

[Expression 5]

$$\text{H-chain peak-area correction value }(Hi) = \text{Peak area} \times \frac{\text{H-chain molar absorption coefficient}}{\text{H-chain molar absorption coefficient + number of drug molecules connected} \times \text{drug-linker molar absorption coefficient}}$$

Here, for the molar absorption coefficients (280 nm) of the L chain and H chain of each antibody, values estimated from the amino acid sequences of the L chain and H chain of the antibody by using a known calculation method (Protein Science, 1995, vol. 4, 2411-2423). In the case of H01L02 antibody, 81480 was used as the molar absorption coefficient of the H chain estimated from the amino acid sequence. In the case of the H01L02A antibody, similarly, 79829 was used as the molar absorption coefficient of the H chain; in the case of the H31L02A antibody, 80131 was used as the molar absorption coefficient of the H chain; in the case of the H11L02A antibody, 78696 was used as the molar absorption coefficient of the H chain; in the case of the LPS antibody, 77470 was used as the molar absorption coefficient of the H chain; and the molar absorption coefficient (280 nm) measured for drug-linker 1-4, as a conjugate precursor, was used as the molar absorption coefficient (280 nm) of each drug-linker.

(F-3-3) The peak area ratios (%) of each chain to the total of corrected peak areas are calculated by using the following expression.

[Expression 6]

$$\text{H-chain peak-area correction value} = \frac{A_{Hi}}{A_{H0} + A_{H1} + A_{H2}} \times 100$$

$A_{Hi}$ : ***Hi*** Corrected peak area value of each chain

(F-3-4) The average number of conjugated drug molecules per antibody molecule in an antibody-drug conjugate is calculated by using the following expression.

[Expression 7]

Average number of drug molecules conjugated = ($H_0$ peak area ratio × 0 + $H_1$ peak area ratio × 1 + $H_2$ peak area ratio × 2)/100 × 2

4. Medicament

Since the anti-CDH6 antibody of the present invention or the functional fragment of the antibody described in the above section "2. Production of anti-CDH6 antibody" and the Examples binds to CDH6 on the surface of tumor cells and has internalization activity, it can be used as a medicament, and in particular, as a therapeutic agent for cancer such as renal cell tumor or ovarian tumor, for example, renal cell carcinoma, clear renal cell carcinoma, papillary renal cell carcinoma, ovarian cancer, ovarian serous adenocarcinoma, thyroid cancer, bile duct cancer, lung cancer (e.g., small-cell lung cancer or non-small cell lung cancer), glioblastoma, mesothelioma, uterine cancer, pancreatic cancer, Wilms' tumor or neuroblastoma, either alone or in combination with an additional drug.

Furthermore, the anti-CDH6 antibody of the present invention or the functional fragment of the antibody can be used in the detection of cells expressing CDH6.

Moreover, since the anti-CDH6 antibody of the present invention or the functional fragment of the antibody has internalization activity, it can be applied as the antibody in an antibody-drug conjugate.

When a drug having antitumor activity such as cytotoxic activity is used as the drug, the anti-CDH6 antibody-drug conjugate of the present invention described in the above section "3. Anti-CDH6 antibody-drug conjugate" and the Examples is a conjugate of the anti-CDH6 antibody and/or the functional fragment of the antibody having internalization

activity, and the drug having antitumor activity such as cytotoxic activity. Since this anti-CDH6 antibody-drug conjugate exhibits antitumor activity against cancer cells expressing CDH6, it can be used as a medicament, and in particular, as a therapeutic agent and/or a prophylactic agent for cancer.

The anti-CDH6 antibody-drug conjugate of the present invention may absorb moisture or have adsorption water, for example, to turn into a hydrate when it is left in air or subjected to recrystallization or purification procedures. Such a compound or a pharmacologically acceptable salt containing water is also included in the present invention.

When the anti-CDH6 antibody-drug conjugate of the present invention has a basic group such as an amino group, it can form a pharmacologically acceptable acidaddition salt, if desired. Examples of such an acidaddition salt can include: hydrohalides such as hydrofluoride, hydrochloride, hydrobromide, and hydroiodide; inorganic acid salts such as nitrate, perchlorate, sulfate, and phosphate; lower alkanesulfonates such as methanesulfonate, trifluoromethanesulfonate, and ethanesulfonate; arylsulfonates such as benzenesulfonate and p-toluenesulfonate; organic acid salts such as formate, acetate, trifluoroacetate, malate, fumarate, succinate, citrate, tartrate, oxalate, and maleate; and amino acid salts such as ornithine salt, glutamate, and aspartate.

When the anti-CDH6 antibody-drug conjugate of the present invention has an acidic group such as a carboxy group, it can form a pharmacologically acceptable baseaddition salt, if desired. Examples of such a baseaddition salt can include: alkali metal salts such as a sodium salt, a potassium salt, and lithium salt; alkaline earth metal salts such as a calcium salt and a magnesium salt; inorganic salts such as an ammonium salt; and organic amine salts such as a dibenzylamine salt, a morpholine salt, a phenylglycine alkyl ester salt, an ethylenediamine salt, an N-methylglucamine salt, a diethylamine salt, a triethylamine salt, a cyclohexylamine salt, a dicyclohexylamine salt, an N,N'-dibenzylethylenediamine salt, a diethanolamine salt, an N-benzyl-N-(2-phenylethoxy)amine salt, a piperazine salt, tetramethylammonium salt, and a tris(hydroxymethyl)aminomethane salt.

The present invention can also include an anti-CDH6 antibody-drug conjugate in which one or more atoms constituting the antibody-drug conjugate are replaced with isotopes of the atoms. There exist two types of isotopes: radioisotopes and stable isotopes. Examples of the isotope can include isotypes of hydrogen (2H and 3H), isotopes of carbon (11C, 13C and 14C), isotopes of nitrogen (13N and 15N), isotopes of oxygen (150, 170 and 180), and isotopes of fluorine (18F). A composition comprising the antibody-drug conjugate labeled with such an isotope is useful as, for example, a therapeutic agent, a prophylactic agent, a research reagent, an assay reagent, a diagnostic agent, and an *in vivo* diagnostic imaging agent. Each and every antibody-drug conjugate labeled with an isotope, and mixtures of antibody-drug conjugates labeled with an isotope at any given ratio are included in the present invention. The antibody-drug conjugate labeled with an isotope can be produced, for example, by using a starting material labeled with an isotope, instead of a starting material for the production method of the present invention mentioned later, according to a method known in the art.

*In vitro* cytotoxicity can be measured based on the activity of suppressing the proliferative responses of cells, for example. For example, a cancer cell line overexpressing CDH6 is cultured, and the anti-CDH6 antibody-drug conjugate is added at different concentrations to the culture system. Thereafter, its suppressive activity against focus formation, colony formation and spheroid growth can be measured. In this context, for example, by using a renal cell tumor- or ovarian tumor-derived cancer cell line, cell growth inhibition activity against renal cell tumor or ovarian tumor can be examined.

*In vivo* therapeutic effects on cancer in an experimental animal can be measured, for example, by administering the anti-CDH6 antibody-drug conjugate to a nude mouse into which a tumor cell line highly expressing CDH6 has been inoculated, and then measuring a change in the cancer cells. In this context, for example, by using an animal model derived from an immunodeficient mouse by the inoculation of renal cell carcinoma-, renal clear cell carcinoma-, papillary renal cell carcinoma-, ovarian cancer-, ovarian serous adenocarcinoma- or thyroid cancer-derived cells, therapeutic effects on renal cell carcinoma, renal clear cell carcinoma, papillary renal cell carcinoma, ovarian cancer, ovarian serous adenocarcinoma or thyroid cancer can be measured.

The type of cancer to which the anti-CDH6 antibody-drug conjugate of the present invention is applied is not particularly limited as long as CDH6 is expressed in cancer cells to be treated. Examples thereof can include renal cell carcinoma (e.g., renal clear cell carcinoma or papillary renal cell carcinoma), ovarian cancer, ovarian serous adenocarcinoma, thyroid cancer, bile duct cancer, lung cancer (e.g., small-cell lung cancer or non-small cell lung cancer), glioblastoma, mesothelioma, uterine cancer, pancreatic cancer, Wilms' tumor and neuroblastoma, though the cancer is not limited thereto as long as the cancer expresses CDH6. More preferred examples of the cancer can include renal cell carcinoma (e.g., renal clear cell carcinoma and papillary renal cell carcinoma) and ovarian cancer.

The anti-CDH6 antibody-drug conjugate of the present invention can preferably be administered to a mammal, and more preferably to a human.

A substances used in a pharmaceutical composition comprising the anti-CDH6 antibody-drug conjugate of the present invention can be appropriately selected from pharmaceutical additives and others usually used in this field, in terms of the applied dose or the applied concentration, and then used.

The anti-CDH6 antibody-drug conjugate of the present invention can be administered as a pharmaceutical

composition comprising one or more pharmaceutically compatible components. For example, the pharmaceutical composition typically comprises one or more pharmaceutical carriers (e.g., sterilized liquids (e.g., water and oil (including petroleum oil and oil of animal origin, plant origin, or synthetic origin (e.g., peanut oil, soybean oil, mineral oil, and sesame oil))). Water is a more typical carrier when the pharmaceutical composition is intravenously administered. An aqueous saline solution, an aqueous dextrose solution, and an aqueous glycerol solution can also be used as a liquid carrier, in particular, for an injection solution. Suitable pharmaceutical vehicles are known in the art. If desired, the composition may also comprise a trace amount of a moisturizing agent, an emulsifying agent, or a pH buffering agent. Examples of suitable pharmaceutical carriers are disclosed in "Remington's Pharmaceutical Sciences" by E. W. Martin. The prescription corresponds to an administration mode.

Various delivery systems are known, and they can be used for administering the anti-CDH6 antibody-drug conjugate of the present invention. Examples of the administration route can include, but are not limited to, intradermal, intramuscular, intraperitoneal, intravenous and subcutaneous routes. The administration can be made by, e.g., injection or bolus injection, for example. According to a specific preferred embodiment, the administration of the above-described antibody-drug conjugate is performed by injection. Parenteral administration is a preferred administration route.

According to a representative embodiment, the pharmaceutical composition is prescribed, as a pharmaceutical composition suitable for intravenous administration to a human, according to conventional procedures. The composition for intravenous administration is typically a solution in a sterile and isotonic aqueous buffer solution. If necessary, the medicament may also contain a solubilizing agent and a local anesthetic to alleviate pain at an injection area (e.g., lignocaine). In general, the above-described ingredients are provided, either separately or together in a mixture in unit dosage form, as a freeze-dried powder or an anhydrous concentrate contained in a container which is obtained by sealing in, for example, an ampoule or a sachet indicating the amount of the active agent. When the medicament is to be administered by injection, it may be administered using, for example, an injection bottle containing water or saline of sterile pharmaceutical grade. When the medicament is to be administered by injection, an ampoule of sterile water or saline for injection may be provided such that the above-described ingredients are admixed with one another before administration.

The pharmaceutical composition of the present invention may be a pharmaceutical composition comprising only the anti-CDH6 antibody-drug conjugate of the present application, or may be a pharmaceutical composition comprising the anti-CDH6 antibody-drug conjugate and at least one other therapeutic agent for cancer. The anti-CDH6 antibody-drug conjugate of the present invention can also be administered together with an additional therapeutic agent for cancer, and can thereby enhance an anticancer effect. The additional anticancer agent used for such a purpose may be administered to an individual, simultaneously, separately, or continuously, together with the antibody-drug conjugate. Otherwise, the additional anticancer agent and the anti-CDH6 antibody-drug conjugate may each be administered to the subject at different administration intervals. Examples of such a therapeutic agent for cancer can include tyrosine kinase inhibitors including imatinib, sunitinib, and regorafenib, CDK4/6 inhibitors including palbociclib, HSP90 inhibitors including TAS-116, MEK inhibitors including MEK162, and immune checkpoint inhibitors including nivolumab, pembrolizumab, and ipilimumab, though the therapeutic agent for cancer is not limited thereto as long as the drug has antitumor activity.

Such a pharmaceutical composition can be prepared as a formulation having a selected composition and a necessary purity in the form of a freeze-dried formulation or a liquid formulation. The pharmaceutical composition prepared as a freeze-dried formulation may be a formulation containing an appropriate pharmaceutical additive used in this field. Likewise, the liquid formulation can be prepared such that the liquid formulation contains various pharmaceutical additives used in this field.

The composition and concentration of the pharmaceutical composition also vary depending on the administration method. With regard to the affinity of the anti-CDH6 antibody-drug conjugate comprised in the pharmaceutical composition of the present invention for the antigen, i.e., the dissociation constant (Kd value) of the anti-CDH6 antibody-drug conjugate to the antigen, as the affinity increases (i.e., the Kd value is low), the pharmaceutical composition can exert medicinal effects, even if the applied dose thereof is decreased. Accordingly, the applied dose of the antibody-drug conjugate can also be determined by setting the applied dose based on the status of the affinity of the antibody-drug conjugate for the antigen. When the antibody-drug conjugate of the present invention is administered to a human, it may be administered at a dose of, for example, from approximately 0.001 to 100 mg/kg once or a plurality of times at intervals of 1 to 180 days. It can be administered preferably at a dose of from 0.1 to 50 mg/kg and more preferably 1 to 50 mg/kg, 1 to 30 mg/kg, 1 to 20 mg/kg, 1 to 15 mg/kg, 2 to 50 mg/kg, 2 to 30 mg/kg, 2 to 20 mg/kg or 2 to 15 mg/kg a plurality of times at intervals of 1 to 4 weeks, preferably 2 to 3 weeks.

## Examples

Hereinafter, the present invention will be specifically described in the following examples. However, the present invention is not limited to these. Furthermore, these examples should not be construed in a limited manner by any means.

It is to be noted that, in the following examples, unless otherwise specified, individual operations regarding genetic manipulation have been carried out according to the method described in "Molecular Cloning" (Sambrook, J., Fritsch, E. F. and Maniatis, T., published by Cold Spring Harbor Laboratory Press in 1989) or other methods described in experimental manuals used by persons skilled in the art, or when commercially available reagents or kits have been used, the examples have been carried out in accordance with the instructions included in the commercially available products. In the present description, reagents, solvents and starting materials are readily available from commercially available sources, unless otherwise specified.

[Example 1: Obtaining of rat anti-human CDH6 antibody having internalization activity]

1)-1 Construction of human and cynomolgus monkey CDH6 expression vectors

Using a human CDH6 protein (NP_004923)-encoding cDNA expression vector (OriGene Technologies Inc., RC217889), the cDNA was incorporated into a vector for mammalian expression according to a method known to a person skilled in the art to produce human CDH6 expression vector pcDNA3.1-hCDH6. The amino acid sequence of the human CDH6 ORF (open reading frame) is shown in SEQ ID No: 1.

cDNA encoding cynomolgus monkey CDH6 protein was cloned with cDNA synthesized from total RNA of the cynomolgus monkey kidney as a template using primer 1 (5'-CACCATGAGAACTTACCGCTACTTCTTGCTGCTC-3') (SEQ ID No: 8) and primer 2 (5'-TTAGGAGTCTTTGTCACTGTCCACTCCTCC-3') (SEQ ID No: 9). It was confirmed that the obtained sequence corresponded to the extracellular region of cynomolgus monkey CDH6 (NCBI, XP_005556691.1). It was also confirmed that the sequence corresponded to the full-length sequence of cynomolgus monkey CDH6 (EHH54180.1) registered in EMBL. The cDNA was incorporated into a vector for mammalian expression according to a method known to a person skilled in the art to produce a cynomolgus monkey CDH6 expression vector, pcDNA3.1-cynoCDH6. The amino acid sequence of the cynomolgus monkey CDH6 ORF is shown in SEQ ID No: 7.

EndoFree Plasmid Giga Kit (Qiagen N.V.) was used for mass production of the produced plasmid DNA.

1) -2 Immunization

For immunization, WKY/Izm female rats (Japan SLC, Inc.) were used. First, the lower limbs of each rat were pre-treated with hyaluronidase (Sigma-Aldrich Co. LLC), and thereafter, the human CDH6 expression vector pcDNA3.1-hCDH6 produced in Example 1)-1 was intramuscularly injected into the same sites. Subsequently, employing ECM830 (BTX), *in vivo* electroporation was carried out on the same sites using a two-needle electrode. Approximately once every two weeks, the same *in vivo* electroporation was repeated, and thereafter, lymph nodes or the spleen were collected from the rat, and used for producing hybridomas.

1) -3 Production of hybridomas

The lymph node cells or the spleen cells were fused with mouse myeloma SP2/0-ag14 cells (ATCC, No. CRL-1 581) according to electrical cell fusion, using a LF301 Cell Fusion Unit (BEX Co., Ltd.), and the cells were then suspended and diluted with ClonaCell-HY Selection Medium D (StemCell Technologies Inc.), and then cultured under conditions of 37°C and 5% $CO_2$. Individual hybridoma colonies that appeared in the culture medium were collected as monoclonal hybridomas, then suspended in ClonaCell-HY Selection Medium E (StemCell Technologies Inc.), and then cultured under conditions of 37°C and 5% $CO_2$. After moderate proliferation of cells, frozen stocks of individual hybridoma cells were produced, while the obtained hybridoma culture supernatant was used to screen for anti-human CDH6 antibody-producing hybridomas.

1) -4 Antibody-producing hybridoma screening according to Cell-ELISA method

1) -4-1 Preparation of antigen gene-expressing cells for use in Cell-ELISA

293α cells (a stable expression cell line derived from HEK293 cells expressing integrin αv and integrin β3) were prepared at 5 x $10^5$ cells/mL in DMEM medium supplemented with 10% FBS. In accordance with transduction procedures for using Lipofectamine 2000 (Thermo Fisher Scientific Inc.), DNA of pcDNA3.1-hCDH6 or pcDNA3.1-cynoCDH6, or pcDNA3.1 as a negative control was introduced into the 293α cells, and the cells were dispensed in an amount of 100 μL/well onto a 96-well plate (Corning Inc.). Thereafter, the cells were cultured under conditions of 37°C and 5% $CO_2$ in DMEM medium supplemented with 10% FBS for 24 to 27 hours. The obtained transfected cells were used for Cell-ELISA in an adhesive state.

1)-4-2 Cell-ELISA

The culture supernatant of the 293α cells transfected with the expression vector prepared in Example 1)-4-1 was removed, and the culture supernatant from each hybridoma was then added to the 293α cells transfected with pcDNA3.1-hCDH6 or pcDNA3.1-cynoCDH6, or pcDNA3.1. The cells were left standing at 4°C for 1 hour. The cells in the wells were washed once with PBS (+) supplemented with 5% FBS, and thereafter, Anti-Rat IgG-Peroxidase antibody produced in rabbit (Sigma-Aldrich Co. LLC) that had been 500-fold diluted with PBS (+) supplemented with 5% FBS was added to the wells. The cells were left standing at 4°C for 1 hour. The cells in the wells were washed three times with PBS (+) supplemented with 5% FBS, and thereafter, OPD coloring solution (which had been prepared by dissolving o-phenylenediamine dihydrochloride (Wako Pure Chemical Industries, Ltd.) and $H_2O_2$ in an OPD solution (0.05 M trisodium citrate, 0.1 M disodium hydrogen phosphate 12-water; pH 4.5), so that the substances became 0.4 mg/ml and 0.6% (v/v), respectively) was added in an amount of 100 μL/well to the wells. A coloring reaction was carried out with occasional stirring. Thereafter, 1 M HCl was added to the plate (100 μL/well) to terminate the coloring reaction, followed by measurement of the absorbance at 490 nm using a plate reader (ENVISION: PerkinElmer, Inc.). Hybridomas that produced a culture supernatant exhibiting higher absorbance in the 293α cells transfected with the pcDNA3.1-hCDH6 or pcDNA3.1-cynoCDH6 expression vector than that in the 293α cells transfected with the control pcDNA3.1 were selected as hybridomas producing antibodies binding to human CDH6 and cynomolgus monkey CDH6.

1)-5 Selective screening for antibody binding to cynomolgus monkey CDH6 according to flow cytometry

1)-5-1 Preparation of antigen gene-expressing cells for use in flow cytometry analysis

293T cells were seeded in a 225-$cm^2$ flask (Sumitomo Bakelite Co., Ltd.) at $5 \times 10^4$ cells/$cm^2$, and the cells were then cultured overnight under conditions of 37°C and 5% $CO_2$ in DMEM medium supplemented with 10% FBS. pcDNA3.1-cynoCDH6 or pcDNA3.1 as a negative control was introduced into the 293T cells using Lipofectamine 2000, and the cells were further cultured overnight under conditions of 37°C and 5% $CO_2$. The 293T cells transfected with each vector were treated with TrypLE Express (Thermo Fisher Scientific Corp.), and the cells were washed with DMEM supplemented with 10% FBS, and then suspended in PBS supplemented with 5% FBS. The obtained cell suspension was used in flow cytometry analysis.

1)-5-2 Flow cytometry analysis

The binding specificity to cynomolgus monkey CDH6 of an antibody produced from the human CDH6- and cynomolgus monkey CDH6-binding antibody-producing hybridomas that had been selected by Cell-ELISA in Example 1)-4 was further confirmed by flow cytometry. The suspension of the transiently expressing 293T cells prepared in Example 1)-5-1 was centrifuged, and the supernatant was then removed. Thereafter, the cells were suspended by the addition of the culture supernatant from each hybridoma. The cells were left standing at 4°C for 1 hour. The cells were washed twice with PBS supplemented with 5% FBS, and thereafter, the cells were suspended by the addition of Anti-Rat IgG FITC conjugate (Sigma-Aldrich Co. LLC) that had been 500-fold diluted with PBS supplemented with 5% FBS. The cells were left standing at 4°C for 1 hour. The cells were washed twice with PBS supplemented with 5% FBS, and then re-suspended in PBS supplemented with 5% FBS and 2 μg/ml 7-aminoactinomycin D (Molecular Probes, Inc.), followed by detection using a flow cytometer (FC500; Beckman Coulter, Inc.). The data was analyzed using FlowJo (TreeStar, Inc.). After dead cells were removed from analysis by gating out 7-aminoactinomycin D-positive cells, a histogram of the FITC fluorescence intensity of live cells was generated. Hybridomas producing antibodies specifically binding to cynomolgus monkey CDH6 expressed on the cell membrane surface were selected based on results where the histogram for the antibody shifted to the strong fluorescence intensity side in the 293T cells transfected with pcDNA3.1-cynoCDH6 compared with the 293T cells transfected with the control pcDNA3.1.

1)-6 Determination of isotype of rat monoclonal antibody

Clone rG019, which appeared to bind specifically and strongly to human CDH6 and monkey CDH6, was selected from among the rat anti-CDH6 antibody-producing hybridomas selected in Example 1)-5, and the isotype of each antibody was identified. The heavy chain subclass and the light chain type of the antibody were determined using a RAT MONOCLONAL ANTIBODY ISOTYPING TEST KIT (DS Pharma Biomedical Co., Ltd.). As a result, it was confirmed that rG019 had a heavy chain of IgG2b subclass and a light chain of κ chain type.

1)-7 Preparation of rat anti-human CDH6 antibody

1)-7-1 Production of culture supernatant

The rat anti-human CDH6 monoclonal antibody was purified from the hybridoma culture supernatant. First, the volume of each rat anti-CDH6 monoclonal antibody-producing hybridoma was sufficiently increased with ClonaCell-HY Selection Medium E (StemCell Technologies Inc.), and thereafter, the medium was exchanged with Hybridoma SFM (Thermo Fisher Scientific Corp.) to which 20% of Ultra Low IgG FBS (Thermo Fisher Scientific Corp.) had been added. Thereafter, the hybridoma was cultured for 4 to 5 days. The resulting culture supernatant was harvested, and insoluble matter was removed therefrom by passing through a 0.8-$\mu$m filter, and through a 0.2-$\mu$m filter.

1)-7-2 Purification of rat anti-CDH6 antibody

An antibody (rat anti-CDH6 antibody (rG019)) was purified from the culture supernatant of hybridomas prepared in Example 1)-7-1 according to Protein G affinity chromatography. The antibody was adsorbed on a Protein G column (GE Healthcare Biosciences Corp.), the column was then washed with PBS, and the antibody was then eluted with a 0.1 M glycine/HCl aqueous solution (pH 2.7). 1 M Tris-HCl (pH 9.0) was added to the eluate, so that the pH was adjusted to pH 7.0 to 7.5. Thereafter, using Centrifugal UF Filter Device VIVASPIN20 (molecular weight cutoff: UF30K, Sartorius Inc.), the buffer was replaced with HBSor (25 mM histidine/5% sorbitol, pH 6.0), while the antibody was concentrated, so that the concentration of the antibody was adjusted to 1 mg/mL. Finally, the antibody was filtrated through a Minisart-Plus filter (Sartorius Inc.) to obtain a purified sample.

[Example 2: *In-vitro* evaluation of rat anti-CDH6 antibody]

2)-1 Evaluation of binding ability of rat anti-CDH6 antibody by flow cytometry

The human CDH6-binding activity of the rat anti-CDH6 antibody produced in Example 1)-7 was evaluated by flow cytometry. Using Lipofectamine 2000 (Thermo Fisher Scientific Inc.), pcDNA3.1-hCDH6 produced in Example 1)-1 was transiently introduced into 293T cells (ATCC). The cells were cultured overnight under conditions of 37°C and 5% $CO_2$, treated with TrypLE Express (Thermo Fisher Scientific Inc.), and thereafter, a cell suspension was prepared. The suspension of the transfected 293T cells was centrifuged, and the supernatant was then removed. Thereafter, the cells were suspended by the addition of the rat anti-CDH6 monoclonal antibody (clone No: rG019), which had been prepared in Example 1)-7, or rat IgG control (R&D Systems, Inc.) (final concentration: 10 ng/mL). The cells were left standing at 4°C for 1 hour. The cells were washed twice with PBS supplemented with 5% FBS, and then suspended by the addition of Anti-Rat IgG (whole molecule)-FITC antibody produced in rabbit (Sigma-Aldrich Co. LLC) that had been 50-fold diluted with PBS supplemented with 5% FBS. The cells were left standing at 4°C for 1 hour. The cells were washed twice with PBS supplemented with 5% FBS, followed by detection using a flow cytometer (FC500; Beckman Coulter, Inc.). The data was analyzed using FlowJo (TreeStar, Inc.). The results are shown in Figure 1. In the histogram of Figure 1, the abscissa depicts FITC fluorescence intensity indicating the amount of the antibody bound, and the ordinate depicts a cell count. The shaded histogram shows that negative control 293T cells untransfected with hCDH6 were used, and the open solid line histogram shows that hCDH6-transfected 293T cells were used. As seen, fluorescence intensity was enhanced by the binding of the antibody to hCDH6 on the cell surface. The rat IgG control binds to neither of the cells. As a result, it was confirmed that the 4 produced rat anti-CDH6 monoclonal antibodies bind to 293T cells transfected with pcDNA3.1-hCDH6.

2)-2 Analysis of CDH6-binding site of rat anti-CDH6 antibody by flow cytometry

2)-2-1 Construction of expression vector for each domain deletion mutant of human CDH6

The full-length extracellular region of human CDH6 has five extracellular domains, EC1 (SEQ ID NO: 2), EC2 (SEQ ID NO: 3), EC3 (SEQ ID NO: 4), EC4 (SEQ ID NO: 5), and EC5 (SEQ ID NO: 6). A gene to be expressed such that each one of the five EC domains could be deleted from full-length human CDH6 was synthesized by GeneArt, and incorporated into p3xFLAG-CMV-9 vectors for mammalian expression (Sigma-Aldrich Co. LLC) according to a method known to a person skilled in the art in order to produce an expression vector for each domain deletion mutant lacking any one of EC1 to EC5.

2)-2-2 Epitope analysis of rat anti-CDH6 antibody by flow cytometry using domain deletion mutant

The epitopes to which the rat anti-human CDH6 antibodies bound were identified by flow cytometry analysis using a 293α cell line transfected with each EC domain deletion vector. Using Lipofectamine 2000 (Thermo Fisher Scientific Inc.), each domain deletion mutant expression vector produced in Example 2)-2-1, or pcDNA3.1-hCDH6 for the expression of full-length human CDH6 was transiently introduced into a 293α cell line, which was a cell line derived from HEK293 cells by stable transfection with integrin αv and integrin β3 expression vectors. The cells were cultured overnight under conditions of 37°C and 5% $CO_2$, treated with TrypLE Express (Thermo Fisher Scientific Inc.), and thereafter, a cell suspension was prepared. The suspension of the transfected 293α cells was centrifuged, and a supernatant was then removed. Thereafter, the cells were suspended by the addition of the anti-CDH6 monoclonal antibody (clone No: rG019), which had been prepared in Example 1)-7, or rat IgG control (R&D Systems, Inc.) (final concentration: 20 nM). The cells were left standing at 4°C for 1 hour. The cells were washed twice with PBS supplemented with 5% FBS, and then suspended by the addition of Anti-Rat IgG (whole molecule)-FITC antibody produced in rabbit (Sigma-Aldrich Co. LLC) that had been 50-fold diluted with PBS supplemented with 5% FBS. The cells were left standing at 4°C for 1 hour. The cells were washed twice with PBS supplemented with 5% FBS, followed by detection using a flow cytometer (Canto II; BD Biosciences). The data was analyzed using FlowJo (TreeStar, Inc.). The results are shown in Figures 2-1 to 2-2. In the histograms of Figures 2-1 to 2-2, the abscissa depicts FITC fluorescence intensity indicating the amount of the antibody bound, and the ordinate depicts cell count. The shaded histogram shows that negative control untransfected 293α cells were used, and the open solid line histogram shows that 293 cells expressing full-length hCDH6 or each EC domain deletion mutant were used. Fluorescence intensity is enhanced when the antibody binds to full-length hCDH6 or each EC domain deletion mutant on the surface of cells. The rat IgG control binds to none of the transfected cells. rG019 binds to the full-length hCDH6, the EC1 deletion mutant, the EC2 deletion mutant, the EC4 deletion mutant, and the EC5 deletion mutant, but does not bind to the EC3 deletion mutant. From this result, it was demonstrated that rG019 specifically binds to hCDH6 with EC3 as an epitope.

2)-3 Confirmation of CDH6 expression in human tumor cell line

In order to select a CDH6-positive human tumor cell line for use in the evaluation of the obtained antibodies, CDH6 expression information was retrieved from a known database, and the expression of CDH6 on the cell membrane surface was evaluated by flow cytometry. Human ovarian tumor cell lines NIH:OVCAR-3, PA-1, OV-90 and human renal cell tumor cell line 786-O and Caki-1 (all obtained from ATCC) were each cultured under conditions of 37°C and 5% $CO_2$, treated with TrypLE Express (Thermo Fisher Scientific Inc.), and thereafter, a cell suspension was prepared. The cells were centrifuged, and the supernatant was then removed. Thereafter, the cells were suspended by the addition of a commercially available anti-human CDH6 antibody (MABU2715, R&D Systems, Inc.) or mouse IgG1 (BD Pharmingen) as a negative control (final concentration: 50 μg/mL). The cells were left standing at 4°C for 1 hour. The cells were washed twice with PBS supplemented with 5% FBS, and then suspended by the addition of F(ab')2 Fragment of FITC-conjugated Goat Anti-mouse immunoglobulins (Dako) that had been 50-fold diluted with PBS supplemented with 5% FBS. The cells were left standing at 4°C for 1 hour. The cells were washed twice with PBS supplemented with 5% FBS, followed by detection using a flow cytometer (Canto II; BD Biosciences). The data was analyzed using FlowJo (TreeStar, Inc.). The results are shown in Figure 3. In the histogram of Figure 3, the abscissa depicts FITC fluorescence intensity indicating the amount of the antibody bound, and the ordinate depicts cell count. The shaded histogram shows that the negative control mIgG1 was used in staining, and the open solid line histogram shows that the anti-human CDH6 antibody was used in staining. As seen, fluorescence intensity was enhanced by the binding of the antibody to hCDH6 on the surface of cells. The mIgG1 control binds to none of the cells. As a result, it was confirmed that the NIH:OVCAR-3, PA-1, OV-90, 786-O and Caki-1 cell lines endogenously express CDH6 on the cell surface.

[Example 3: Amplification and sequencing of rG019 heavy-chain variable region and light-chain variable region gene fragments

3)-1 Preparation of total RNA from rG019 antibody-producing hybridoma

In order to amplify cDNA encoding each variable region of rG019, total RNA was prepared from G019 antibody-producing hybridoma using TRIzol Reagent (Ambion, Inc.).

3)-2 Amplification of cDNA encoding rG019 heavy-chain variable region by 5'-RACE PCR and determination of nucleotide sequence

cDNA encoding the heavy chain variable region was amplified using approximately 1 μg of the total RNA

prepared in Example 3)-1 and a SMARTer RACE cDNA Amplification Kit (Clontech Laboratories, Inc.). As primers used to amplify the cDNA of the variable region of the rG019 heavy chain gene according to PCR, UPM (Universal Primer A Mix: included with SMARTer RACE cDNA Amplification Kit) and primers designed from the sequences of the constant regions of known rat heavy chains were used.

The heavy chain variable region-encoding cDNA amplified by 5'-RACE PCR was cloned into a plasmid, and thereafter, the nucleotide sequence of the cDNA of the heavy chain variable region was subjected to sequence analysis.

The determined nucleotide sequence of the cDNA encoding the heavy chain variable region of rG019 is shown in SEQ ID NO: 16, and the amino acid sequence thereof is shown in SEQ ID NO: 15.

3)-3 Amplification of cDNA encoding rG019 light-chain variable region by 5'-RACE PCR and determination of nucleotide sequence

Amplification and sequencing were carried out by the same method as that applied in Example 3)-2. However, as primers used to amplify the cDNA of the variable region of the rG019 light chain gene according to PCR, UPM (Universal Primer A Mix: included with SMARTer RACE cDNA Amplification Kit) and primers designed from the sequences of the constant regions of known rat light chains were used.

The determined nucleotide sequence of the cDNA encoding the light chain variable region of rG019 is shown in SEQ ID NO: 11, and the amino acid sequence thereof is shown in SEQ ID NO: 10.

[Example 4: Production of human chimeric anti-CDH6 antibody chG019]

4)-1 Construction of human chimeric anti-CDH6 antibody chG019 expression vector

4)-1-1 Construction of chimeric and humanized light-chain expression vector pCMA-LK

An approx. 5.4-kb fragment, which had been obtained by digesting plasmid pcDNA3.3-TOPO/LacZ (Invitrogen Corp.) with the restriction enzymes XbaI and PmeI, was bound to a DNA fragment comprising a DNA sequence (SEQ ID NO: 20) encoding a human light chain signal sequence and a human κ chain constant region, using an In-Fusion Advantage PCR cloning kit (Clontech Laboratories, Inc.), to produce pcDNA3.3/LK.

A neomycin expression unit was removed from pcDNA3.3/LK to construct pCMA-LK.

4)-1-2 Construction of chimeric and humanized IgG1 type heavy-chain expression vector pCMA-G1

A DNA fragment, which had been obtained by digesting pCMA-LK with XbaI and PmeI to remove the DNA sequence encoding the light chain signal sequence and the human κ chain constant region therefrom, was bound to a DNA fragment comprising a DNA sequence (SEQ ID NO: 21) encoding a human heavy chain signal sequence and a human IgG1 constant region, using an In-Fusion Advantage PCR cloning kit (Clontech Laboratories, Inc.), to construct pCMA-G1.

4)-1-3 Construction of chG019 heavy-chain expression vector

A DNA fragment from nucleotide positions 36 to 440 in the nucleotide sequence of the chG019 heavy chain shown in SEQ ID NO: 27 was synthesized (GENEART). Using an In-Fusion HD PCR cloning kit (Clontech Laboratories, Inc.), the synthesized DNA fragment was inserted into a site of pCMA-G1 that had been cleaved with the restriction enzyme BlpI, so as to construct a chG019 heavy chain expression vector. It is to be noted that, for the chG019 heavy chain, a CDR sequence with cysteine substituted with proline was used in order to prevent unpredictable disulfide bonds.

4)-1-4 Construction of chG019 light-chain expression vector

A DNA fragment comprising a DNA sequence (SEQ ID NO: 22) encoding the chG019 light chain was synthesized (GENEART). Using an In-Fusion HD PCR cloning kit (Clontech Laboratories, Inc.), the synthesized DNA fragment was bound to a DNA fragment, which had been obtained by digesting pCMA-LK with XbaI and PmeI to remove the DNA sequence encoding the light chain signal sequence and the human κ chain constant region therefrom, so as to construct a chG019 light chain expression vector.

4)-2 Production and purification of human chimeric anti-CDH6 antibody chG019

4)-2-1 Production of chG019

In accordance with the manual, FreeStyle 293F cells (Invitrogen Corp.) were cultured and passaged. $1.2 \times 10^9$ FreeStyle 293F cells (Invitrogen Corp.) in the logarithmic growth phase were seeded on a 3-L Fernbach Erlenmeyer Flask (Corning Inc.), then diluted with FreeStyle 293 expression medium (Invitrogen Corp.) at $2.0 \times 10^6$ cells/mL. To 40 ml of Opti-Pro SFM medium (Invitrogen Corp.), 0.24 mg of the heavy chain expression vector, 0.36 mg of the light chain expression vector and 1.8 mg of Polyethyleneimine (Polyscience #24765) were added, and the obtained mixture was gently stirred. After incubation for 5 minutes, the mixture was added to the FreeStyle 293F cells. The cells were shake-cultured at 90 rpm in an 8% $CO_2$ incubator at 37°C for 4 hours, and thereafter, 600 mL of EX-CELL VPRO medium (SAFC Biosciences Inc.), 18 mL of GlutaMAX I (GIBCO), and 30 mL of Yeastolate Ultrafiltrate (GIBCO) were added to the culture. The cells were further shake-cultured at 90 rpm in an 8% $CO_2$ incubator at 37°C for 7 days. The obtained culture supernatant was filtrated through a Disposable Capsule Filter (Advantec #CCS-045-E1H).

4)-2-2 Purification of chG019

An antibody was purified from the culture supernatant obtained in Example 4)-2-1 by a one-step process according to rProtein A affinity chromatography. The culture supernatant was applied to a column that had been packed with MabSelectSuRe (GE Healthcare Biosciences Corp.) equilibrated with PBS, and thereafter, the column was washed with PBS in an amount of two or more times the volume of the column. Subsequently, the antibody was eluted with a 2 M arginine hydrochloride solution (pH 4.0), so that a fraction containing an antibody was collected. The fraction was dialyzed (Thermo Fisher Scientific Inc., Slide-A-Lyzer Dialysis Cassette), so that the buffer was replaced with HBSor (25 mM histidine/5% sorbitol, pH 6.0). Using a Centrifugal UF Filter Device VIVASPIN20 (molecular weight cutoff: UF10K, Sartorius Inc.), the antibody was concentrated, so that the concentration of IgG was adjusted to 5 mg/ml or more. Finally, the antibody was filtrated through a Minisart-Plus filter (Sartorius Inc.) to obtain a purified sample.

4)-3 Evaluation of binding activity of human chimeric anti-CDH6 antibody chG019

The CDH6-binding activity of the human chimeric anti-CDH6 antibody chG019 purified in 4)-2 was confirmed by flow cytometry. Using Lipofectamine 2000, pcDNA3.1-hCDH6 or pcDNA3.1-cynoCDH6 produced in Example 1)-1, or pcDNA3.1 was transiently introduced into 293α cells. The cells were cultured overnight under conditions of 37°C and 5% $CO_2$, treated with TrypLE Express (Thermo Fisher Scientific Inc.), and thereafter, a cell suspension was prepared. chG019 was added to the suspension of each of these cells. The cells were left standing at 4°C for 1 hour. Thereafter, the cells were washed twice with PBS supplemented with 5% FBS, and then suspended by the addition of PE-labeled F(ab')2 Fragment anti-human IgG, Fcγ antibody (Jackson ImmunoResearch Laboratories, Inc.) that had been 500-fold diluted with PBS supplemented with 5% FBS. The cells were left standing at 4°C for 1 hour. The cells were washed twice with PBS supplemented with 5% FBS, and then re-suspended in PBS supplemented with 5% FBS, followed by detection using a flow cytometer (Canto II; BD Biosciences). The data was analyzed using FlowJo (TreeStar, Inc.). As shown in Figure 4, chG019 did not bind to the 293T cells transfected with pcDNA3.1 as a negative control, but did bind to the 293T cells transfected with pcDNA3.1-hCDH6 or pcDNA3.1-cynoCDH6 in an antibody concentration-dependent manner. In Figure 4, the abscissa depicts antibody concentration, and the ordinate depicts the amount of the antibody bound, based on mean fluorescence intensity. It is evident from this result that chG019 specifically binds to human CDH6 and cynomolgus monkey CDH6 with almost equivalent binding activity.

[Example 5: Production of humanized anti-CDH6 antibody]

5)-1 Design of humanized form of anti-CDH6 antibody

5)-1-1 Molecular modeling of chG019 variable region

The molecular modeling of the variable regions of chG019 exploited a method known as homology modeling (Methods in Enzymology, 203, 121-153, (1991)). The commercially available protein three-dimensional structure analysis program BioLuminate (manufactured by Schrodinger, LLC) was employed using, as a template, a structure (PDB ID: 2I9L) registered in Protein Data Bank (Nuc. Acid Res. 35, D301-D303 (2007)) with a high sequence identity to the heavy chain and light chain variable regions of chG019.

5)-1-2 Design of amino acid sequence of humanized hG019

chG019 was humanized by CDR grafting (Proc. Natl. Acad. Sci. USA 86, 10029-10033 (1989)). The consensus sequences of human gamma chain subgroup 1 and kappa chain subgroup 1 determined by KABAT et al. (Sequences of Proteins of Immunological Interest, 5th Ed. Public Health Service National Institutes of Health, Bethesda, MD. (1991)) had high identity to the framework regions of chG019, and based on this, they were selected as acceptors for the heavy chain and the light chain, respectively. Donor residues to be grafted onto the acceptors were selected by analyzing three-dimensional models with reference to, for example, the criteria given by Queen et al. (Proc. Natl. Acad. Sci. USA 86, 10029-10033 (1989)).

5)-2 Humanization of chG019 heavy chain

The three heavy chains thus designed were named hH01, hH02 and hH04. The full-length amino acid sequence of the hH01 heavy chain is shown in SEQ ID NO: 39. The nucleotide sequence encoding the amino acid sequence of SEQ ID NO: 39 is shown in SEQ ID NO: 40. The full-length amino acid sequence of the heavy chain hH02 is shown in SEQ ID NO: 43. The nucleotide sequence encoding the amino acid sequence of SEQ ID NO: 43 is shown in SEQ ID NO: 44. The full-length amino acid sequence of the heavy chain hH04 is shown in SEQ ID NO: 47. The nucleotide sequence encoding the amino acid sequence of SEQ ID NO: 47 is shown in SEQ ID NO: 48.

5)-3 Humanization of chG019 light chain

Two light chains thus designed were named hL02 and hL03. The full-length amino acid sequence of the hL02 light chain is shown in SEQ ID NO: 31. The nucleotide sequence encoding the amino acid sequence of SEQ ID NO: 31 is shown in SEQ ID NO: 32. The full-length amino acid sequence of the light chain hL03 is shown in SEQ ID NO: 35. The nucleotide sequence encoding the amino acid sequence of SEQ ID NO: 35 is shown in SEQ ID NO: 36.

5)-4 Design of humanized hG019 by combination of heavy chain and light chain

An antibody consisting of hH01 and hL02 was named "H01L02 antibody" or "H01L02". An antibody consisting of hH02 and hL02 was named "H02L02 antibody" or "H02L02". An antibody consisting of hH02 and hL03 was named "H02L03 antibody" or "H02L03". An antibody consisting of hH04 and hL02 was named "H04L02 antibody" or "H04L02".

5)-5 Expression of humanized anti-CDH6 antibody

5)-5-1 Construction of humanized hG019 heavy-chain expression vector

5)-5-1-1 Construction of hH01 heavy-chain expression vector

A DNA fragment from nucleotide positions 36 to 440 in the nucleotide sequence of the hH01 heavy chain shown in SEQ ID NO: 40 was synthesized (GENEART). A hH01 heavy chain expression vector was constructed by the same method as that applied in Example 4)-1-3.

5)-5-1-2 Construction of hH02 heavy-chain expression vector

A DNA fragment from nucleotide positions 36 to 440 in the nucleotide sequence of the hH02 heavy chain shown in SEQ ID NO: 44 was synthesized (GENEART). A hH02 heavy chain expression vector was constructed by the same method as that applied in Example 4)-1-3.

5)-5-1-3 Construction of hH04 heavy-chain expression vector

A DNA fragment from nucleotide positions 36 to 440 in the nucleotide sequence of the hH04 heavy chain shown in SEQ ID NO: 48 was synthesized (GENEART). A hH04 heavy chain expression vector was constructed by the same method as that applied in Example 4)-1-3.

5)-5-2 Construction of humanized hG019 light-chain expression vector

5)-5-2-1 Construction of hL02 light-chain expression vector

A DNA fragment comprising a hL02 light chain variable region-encoding DNA sequence from nucleotide positions 37 to 399 in the nucleotide sequence of the hL02 light chain shown in SEQ ID NO: 32 was synthesized (GENEART). Using an In-Fusion HD PCR cloning kit (Clontech Laboratories, Inc.), the synthesized DNA fragment was inserted into a site of pCMA-LK that had been cleaved with the restriction enzyme BsiWI, so as to construct a hL02 light chain expression vector.

5)-5-2-2 Construction of hL03 light-chain expression vector

A DNA fragment comprising a hL03 light chain variable region-encoding DNA sequence from nucleotide positions 37 to 399 in the nucleotide sequence of the hL03 light chain shown in SEQ ID NO: 36 was synthesized (GENEART). A hL03 light chain expression vector was constructed by the same method as that applied in Example 5)-5-2-1.

5)-5-3 Preparation of humanized hG019

5)-5-3-1 Production of H01L02, H02L02, H02L03 and H04L02

The antibodies were produced by the same method as that applied in Example 4)-2-1. H01L02, H02L02, H02L03 and H04L02 were produced by the combination of the heavy chain and the light chain shown in Example 5)-4.

5)-5-3-2 Two-step purification of H01L02, H02L02, H02L03 and H04L02

The antibody was purified from the culture supernatant obtained in Example 5)-5-3-1, by a two-step process, namely, by rProtein A affinity chromatography and ceramic hydroxyapatite. The culture supernatant was applied to a column that had been packed with MabSelectSuRe (manufactured by GE Healthcare Biosciences Corp.) equilibrated with PBS, and thereafter, the column was washed with PBS in an amount of two or more times the volume of the column. Subsequently, the antibody was eluted using a 2 M arginine hydrochloride solution (pH 4.0). A fraction containing the antibody was dialyzed (Thermo Fisher Scientific Inc., Slide-A-Lyzer Dialysis Cassette), so that the buffer was replaced with PBS. The antibody solution was 5-fold diluted with a buffer of 5 mM sodium phosphate/50 mM MES/pH 7.0, and then applied to a ceramic hydroxyapatite column (Bio-Rad Laboratories, Inc., Bio-Scale CHT Type-1 Hydroxyapatite Column) that had been equilibrated with a buffer of 5 mM NaPi/50 mM MES/30 mM NaCl/pH 7.0. Elution was carried out on a linear concentration gradient of sodium chloride, so that a fraction containing an antibody was collected. This fraction was dialyzed (Thermo Fisher Scientific Inc., Slide-A-Lyzer Dialysis Cassette), so that the buffer was replaced with HBSor (25 mM histidine/5% sorbitol, pH 6.0). The antibody was concentrated with Centrifugal UF Filter Device VIVASPIN20 (molecular weight cutoff: UF10K, Sartorius Inc.), thereby adjusting the IgG concentration to 20 mg/ml. Finally, the antibody was filtrated through a Minisart-Plus filter (Sartorius Inc.) to obtain a purified sample.

[Reference Example 1: Production of anti-CDH6 antibody NOV0712]

The anti-CDH6 antibody NOV0712 used in the Examples was produced with reference to the light chain full-length and heavy chain full-length amino acid sequences (SEQ ID NO: 235 and SEQ ID NO: 234, respectively, in International Publication No. WO 2016/024195) of NOV0712 described in International Publication No. WO 2016/024195.

Reference Example 1)-1 Anti-CDH6 antibody NOV0712

Reference Example 1)-1-1 Construction of anti-CDH6 antibody NOV0712 heavy-chain expression vector

A NOV0712 heavy chain variable region-encoding DNA fragment from nucleotide positions 36 to 428 in the nucleotide sequence of the NOV0712 heavy chain shown in SEQ ID NO: 54 was synthesized (GENEART). A NOV0712 heavy chain expression vector was constructed by the same method as that applied in Example 4)-1-3. The amino acid sequence of the NOV0712 heavy chain expressed by the NOV0712 heavy chain expression vector is shown in SEQ ID NO: 53. In the amino acid sequence shown in SEQ ID NO: 53, the amino acid sequence consisting of the amino acid residues at positions 1 to 19 is a signal sequence.

Reference Example 1)-1-2 Construction of anti-CDH6 antibody NOV0712 light-chain expression vector

A DNA fragment comprising a NOV0712 light chain variable region-encoding DNA sequence from nucleotide positions 37 to 405 in the nucleotide sequence of the NOV0712 light chain shown in SEQ ID NO: 52 was synthesized (GENEART). A NOV0712 light chain expression vector was constructed by the same method as that applied in Example 5)-5-2-1. The amino acid sequence of the NOV0712 light chain expressed by the NOV0712 light chain expression vector is shown in SEQ ID NO: 51. In the amino acid sequence shown in SEQ ID NO: 51, the amino acid sequence consisting of the amino acid residues at positions 1 to 20 is a signal sequence.

Reference Example 1)-2 Preparation of anti-CDH6 antibody NOV0712

Reference Example 1)-2-1 Production of anti-CDH6 antibody NOV0712

NOV0712 was produced by the same method as that applied in Example 4)-2-1.

Reference Example 1)-2-2 One-step purification of anti-CDH6 antibody NOV0712

The anti-CDH6 antibody NOV0712 was purified from the culture supernatant obtained in Reference Example 1)-2-1 by the same method as that applied in Example 4)-2-2 (antibody concentration: 5 mg/l HBSor).

[Example 6: *in vitro* evaluation of humanized hG019 and NOV0712]

6)-1 Evaluation of binding activity of humanized hG019

The dissociation constant between the antibody and the antigen (Recombinant Human CDH6 Fc His chimera, R&D Systems, Inc.) was measured by using Biacore T200 (GE Healthcare Biosciences Corp.), according to a capture method, which comprises capturing the antigen as a ligand with the immobilized anti-His antibody and then measuring the dissociation constant using an antibody as an analyte. Approximately 1000 RU of the anti-histidine antibody (His capture kit, GE Healthcare Biosciences Corp.) was covalently bound to sensor chip CM5 (GE Healthcare Biosciences Corp.) by the amine coupling method. The antibody was also immobilized onto reference cells in the same manner as above. HBS-P+ (10 mM HEPES pH 7.4, 0.15 M NaCl, 0.05% Surfactant P20) supplemented with 1 mM $CaCl_2$ was used as a running buffer. The antigen was added onto the anti-histidine antibody-immobilized chip for 60 seconds, and a dilution series solution (0.391 to 100 nM) of the antibody was then added at a flow rate of 30 μl/min for 300 seconds. Subsequently, the dissociation phase was monitored for 600 seconds. As a regeneration solution, a glycine solution (pH 1.5) supplemented with 5 M $MgCl_2$ was added twice at a flow rate of 10 μl/min for 30 seconds. A Steady State Affinity model in analysis software (BIAevaluation software, version 4.1) was used in data analysis, and the dissociation constant (KD) was calculated. The results are shown in Table 2.

[Table 2]

| | Antibody | KD (M) |
|---|---|---|
| 1 | H01L02 | 1.5E-09 |
| 2 | H02L02 | 1.1E-09 |
| 3 | H02L03 | 1.4E-09 |
| 4 | H04L02 | 1.1E-09 |

6)-2 Analysis of CDH6-binding sites of humanized hG019 and NOV0712

6)-2-1 Epitope Analysis using domain defective mutant

Using Lipofectamine 2000 (Thermo Fisher Scientific Inc.), each domain deletion mutant expression vector produced in Example 2)-2-1, or pcDNA3.1-hCDH6 for the expression of full-length human CDH6 was transiently introduced into cells. The cells were cultured overnight under conditions of 37°C and 5% $CO_2$, and thereafter, a cell suspension was prepared. The suspension of the transfected 293α cells was centrifuged, and the supernatant was then removed. Thereafter, the cells were suspended by the addition of each of the 4 humanized hG019 antibodies (clone Nos: H01L02, H02L02, H02L03 and H04L02), which had been prepared in Example 5)-5-3, or the anti-CDH6 antibody NOV0712,

which had been prepared in Reference Example 1, or human IgG1 (Calbiochem) as a negative control. The cells were left standing at 4°C for 1 hour. The cells were washed twice with PBS supplemented with 5% FBS, and then suspended by the addition of APC-anti-human IgG goat F(ab')2 (Jackson ImmunoResearch Laboratories, Inc.) that had been 500-fold diluted with PBS supplemented with 5% FBS. The cells were left standing at 4°C for 1 hour. The cells were washed twice with PBS supplemented with 5% FBS, followed by detection using a flow cytometer (Canto II; BD Biosciences). The data was analyzed using FlowJo (TreeStar, Inc.). The results are shown in Figures 5-1 to 5-6. In the histograms of Figures 5-1 to 5-6, the abscissa depicts APC fluorescence intensity indicating the amount of the antibody bound, and the ordinate depicts cell count. The shaded histogram shows that negative control untransfected 293α cells were used, and the open solid line histogram shows that 293α cells expressing full-length hCDH6 or each EC domain deletion mutant were used. Fluorescence intensity is enhanced when the antibody binds to full-length hCDH6 or each EC domain deletion mutant on the cell surface. The human IgG1 control binds to none of the transfected cells. The 4 humanized hG019 antibodies (clone Nos: H01L02, H02L02, H02L03 and H04L02) bind to the full-length hCDH6, the EC1 deletion mutant, the EC2 deletion mutant, the EC4 deletion mutant, and the EC5 deletion mutant, but do not bind to the EC3 deletion mutant. Specifically, it was demonstrated that the 4 humanized hG019 antibodies specifically bind to hCDH6 with EC3 as an epitope. On the other hand, the anti-CDH6 antibody NOV0712 binds to the full-length hCDH6, the EC1 deletion mutant, the EC2 deletion mutant, the EC3 deletion mutant, and the EC4 deletion mutant, but does not bind to the EC5 deletion mutant. Specifically, it was demonstrated that the anti-CDH6 antibody NOV0712 specifically binds to hCDH6 with EC5 as an epitope. This is consistent with epitope information on NOV0712 described in International Publication No. WO 2016/024195. From this result, it was demonstrated that the 4 humanized hG019 antibodies obtained in the present description are anti-CDH6 antibodies that exhibit properties different from those of NOV0712.

6)-2-2 Binding competition assay of antibodies

6)-2-2-1 Production of 786-O/hCDH6 stably expressing cell line

The 786-O/hCDH6 stably expressing cell line was produced by infecting 786-O cells (ATCC) with a recombinant retrovirus for full-length human CDH6 expression. A human CDH6 expression retrovirus vector (pQCXIN-hCDH6) was produced by using a human CDH6 protein (NP_004923)-encoding cDNA expression vector (OriGene Technologies Inc., RC217889), and incorporating the cDNA into retrovirus vector pQCXIN (Clontech Laboratories, Inc.) according to a method known to a person skilled in the art. Using FuGene HD (Promega Corp.), pQCXIN-hCDH6 was transiently introduced into retrovirus packaging cells RetroPack PT67 (Clontech Laboratories, Inc.). After 48 hours, a culture supernatant containing recombinant retrovirus was recovered, and then added to the 786-O cell culture system, so that the cells were infected. From 3 days after the infection, the infected cells were cultured under conditions of 37°C and 5% $CO_2$ in a medium supplemented with G418 (Gibco) (final concentration: 50 mg/mL) and screened with the drug, so as to establish cell line 786-O/hCDH6 stably expressing human CDH6. The high expression of human CDH6 in the stably expressing line was confirmed by flow cytometry in the same manner as that applied in Example 2)-3-1 (Figure 6). Goat anti-Mouse IgG1 Secondary Antibody Alexa Fluor 647 (Thermo Fisher Scientific Inc.) that had been 500-fold diluted with PBS supplemented with 5% FBS was used as an antibody for detection. The results are shown in Figure 6. In the histogram of Figure 8, the abscissa depicts Alexa Fluor 647 fluorescence intensity indicating the amount of the antibody bound, and the ordinate depicts cell count. The shaded histogram shows that the negative control mIgG1 was used in staining, and the open solid line histogram shows that the anti-human CDH6 antibody was used in staining. As seen, fluorescence intensity was enhanced by the binding of the antibody to hCDH6 on cell surface. The mIgG1 control binds to none of the cells. As a result, it was demonstrated that the 786-O/hCDH6 stably expressing cell line more highly expresses human CDH6 than the parent line 786-O cells.

6)-2-2-2 Binding Competition assay using labeled H01L02 and labeled NOV0712

Labeled H01L02 and labeled NOV0712 were produced using an Alexa Fluor 488 Monoclonal Antibody Labeling Kit (Thermo Fisher Scientific Inc.). The cell suspension of the 786-O/hCDH6 stably expressing cell line produced in 6)-2-2-1 was centrifuged, and the supernatant was then removed. Thereafter, the cells were suspended by the addition of labeled NOV0712 or labeled H01L02 (final concentration: 5 nM) and, further, the addition of each of the 4 humanized hG019 antibodies (clone Nos: H01L02, H02L02, H02L03 and H04L02), which had been prepared in Example 5)-5-3, or the anti-CDH6 antibody NOV0712, which had been prepared in Reference Example 1, or human IgG1 (Calbiochem) as a negative control (final concentration: as shown in the abscissa of Figure 7). The cells were left standing at 4°C for 1 hour. Thereafter, the cells were washed twice with PBS supplemented with 5% FBS, followed by detection using a flow cytometer (Canto II; BD Biosciences). The data was analyzed using FlowJo (TreeStar, Inc.). The results are shown in Figure 7. The abscissa depicts the final concentration of the added unlabeled antibody, and the ordinate depicts the amount of the antibody bound based on mean fluorescence intensity. When unlabeled NOV0712 is added to cells

supplemented with labeled NOV0712, the amount of the labeled antibody bound is decreased by replacement with the unlabeled antibody in an addition concentration-dependent manner because they compete with each other for binding to the same epitope. On the other hand, even if each of the 4 humanized hG019 antibodies or human IgG1 as a negative control is added to cells supplemented with labeled NOV0712, there is no change in the amount of the labeled antibody bound, indicating that these antibodies differ in epitope and thus do not compete with each other for binding. Likewise, when each of the 4 unlabeled humanized hG019 antibodies is added to cells supplemented with labeled H01L02, the amount of the labeled antibody bound is decreased by replacement with the unlabeled antibody in an addition concentration-dependent manner because they compete with each other for binding to the same epitope. On the other hand, even if NOV0712 or human IgG1 as a negative control is added to cells supplemented with labeled H01L02, there is no change in the amount of the labeled antibody bound, indicating that these antibodies differ in epitope and thus do not compete with each other for binding.

6)-3 Evaluation of internalization activity of humanized hG019 and NOV0712

The internalization activity of humanized hG019 and NOV0712 was evaluated using an anti-human IgG reagent Hum-ZAP (Advanced Targeting Systems) conjugated with a toxin (saporin) inhibiting protein synthesis. Specifically, human CDH6-positive ovarian tumor cell line NIH:OVCAR-3 (ATCC) was seeded at 4 x $10^3$ cells/well on a 96-well plate, and then cultured overnight under conditions of 37°C and 5% $CO_2$. Human CDH6-positive renal cell tumor cell line 786-O (ATCC) was seeded at 1 x $10^3$ cells/well on a 96-well plate, and then cultured overnight. Human CDH6-positive ovarian tumor cell line PA-1 (ATCC) was seeded at 1 x $10^3$ cells/well on a 96-well plate, and then cultured overnight under conditions of 37°C and 5% $CO_2$. On the next day, each anti-CDH6 antibody (final concentration: 1 nM) or human IgG1 antibody (Calbiochem) as a negative control antibody was added to the plate. Hum-ZAP (final concentration: 0.5 nM) or F(ab')2 Fragment Goat Anti-human IgG, Fc (gamma) Fragment Specific (Jackson ImmunoResearch Laboratories, Inc.) unconjugated with the toxin (final concentration: 0.5 nM) as a negative control was further added to the plate, and the cells were cultured under conditions of 37°C and 5% $CO_2$ for 3 days. The number of live cells was measured by the quantification of ATP activity (RLU) using CellTiter-Glo(TM) Luminescent Cell Viability Assay. In this evaluation, Hum-ZAP is taken up into cells in a manner dependent on the internalization activity of the humanized anti-CDH6 antibody, so that saporin, which inhibits protein synthesis, is released into the cells, so as to suppress cell growth. A cell growth inhibition effect brought about by the addition of the anti-CDH6 antibody was indicated by a relative survival rate when the number of live cells in a well supplemented with the negative control instead of Hum-ZAP was defined as 100%. Figure 8 shows a table of the cell survival rate. In this experiment, an antibody having strong internalization activity is considered to offer a low cell survival rate. As a result, the 4 humanized hG019 antibodies have an internalization rate of approximately 50 to 75% predicted from the cell survival rates for all of the 3 cell lines. Thus, the 4 humanized hG019 antibodies exhibit very high internalization activity and exhibit much higher internalization activity than that of NOV0712. From the mechanism of the medicinal effects of ADC, an antibody having higher internalization activity is considered to be more suitable as an ADC antibody.

[Example 7: Production of humanized hG019 variant]

7)-1 Design of humanized hG019 variant

7)-1-1 Design of variant having modified H01L02 variable region

A heavy chain designed by substituting the 71st tyrosine in the hH01 amino acid sequence described in Example 5)-2, with alanine was designated as hH11 and a heavy chain designed by substituting the 81st glutamine in the sequence with serine and the 123rd phenylalanine with alanine was designated as hH31. The amino acid sequence of the hH11 heavy-chain variable region is shown in SEQ ID No: 55. The nucleotide sequence encoding the amino acid sequence of SEQ ID No: 55 is shown in SEQ ID No: 56. The amino acid sequence of the hH31 heavy-chain variable region is shown in SEQ ID No: 60. The nucleotide sequence encoding the amino acid sequence of SEQ ID No: 60 is shown in SEQ ID No: 61.

7)-1-2 Design of LALA of human heavy chain

A heavy chain was designed by substituting leucine residues at positions 234 and 235 (specified by the EU index) of the hH01 amino acid sequence described in Example 5)-2 with alanine residues (referred to herein as "hH01A"). A heavy chain having the hH11 variable region designed in Example 7)-1-1, i.e., an isotype of IgG1, was designed by substituting leucine residues at position 234 and 235 (specified by the EU index) with alanine residues (referred to herein as "hH11A"). A heavy chain having the hH31 variable region (designed in Example 7)-1-1), i.e., an isotype of IgG1, was

designed by substituting leucine residues at positions 234 and 235 (specified by the EU index) with alanine residues (referred to herein as "hH31A").

7)-2 Design of variant of humanized hG019 changed in binding affinity by using heavy chain and light chain in combination

The antibody having hH01A and hL02 is designated as "H01L02A antibody" or "H01L02A". The antibody having hH11A and hL02 is designated as "H11L02A antibody" or "H11L02A". The antibody having hH31A and hL02 is designated as "H31L02A antibody" or "H31L02A".

7)-3 Expression of variant of humanized hG019 changed in binding affinity

7)-3-1 Construction of humanized IgG1 LALA type heavy-chain expression vector pCMA-G1LALA

Using a DNA fragment comprising the DNA sequence encoding amino acid sequence of the human heavy chain signal sequence shown in SEQ ID No: 71 and the human IgG1 LALA constant region, pCMA-G1LALA was constructed in the same manner as in Example 4)-1-2.

7)-3-2 Construction of hH01A heavy-chain expression vector

A DNA fragment from nucleotide positions 36 to 440 in the nucleotide sequence of the hH01A shown in SEQ ID No: 66 was synthesized (GENEART). Using an In-Fusion HD PCR cloning kit (Clontech Laboratories, Inc.), the synthesized DNA fragment was inserted into a site of pCMA-G1LALA that had been cleaved with the restriction enzyme BlpI, so as to construct a hH01A expression vector.

7)-3-3 Construction of hH11A heavy-chain expression vector

A DNA fragment from nucleotide positions 36 to 440 in the nucleotide sequence of the hH11A shown in SEQ ID No: 68 was synthesized (GENEART). An hH11A expression vector was constructed in the same manner as in Example 7)-3-2.

7)-3-4 Construction of hH31A heavy-chain expression vector

A DNA fragment from nucleotide positions 36 to 440 in the nucleotide sequence of the hH31A shown in SEQ ID No: 70 was synthesized (GENEART). An hH31A expression vector was constructed in the same manner as in Example 7)-3-2.

7)-4 Preparation of variant of humanized hG019 changed in binding affinity

7)-4-1 Production of H01L02A, H11L02A and H31L02A

Production was carried out in the same manner as in Example 4)-2-1. Using the light-chain expression vector constructed in Example 5)-5-2-1 and the heavy-chain expression vector constructed in Example 7)-3, combinations of an H chain expression vector and an L chain expression vector, i.e., H01L02A, H11L02A, H31L02A, which correspond to the combinations of H and L chains described in Example 7)-2, were obtained.

7)-4-2 Two-step purification of H01L02A, H11L02A and H31L02A

Purification was carried out in the same manner as in Example 5)-5-3-2 using the culture supernatant obtained in Example 7)-4-1.

[Example 8: *in vitro* evaluation of humanized hG019 variant changed in binding affinity]

8)-1 Evaluation of binding activity of H01L02A, H11L02A, H31L02A

The dissociation constant of a construct of each of H01L02A, H11L02A and H31L02A prepared in Example 7)-4 and human CDH6 was measured by using Biacore T200 (GE Healthcare Biosciences Corp.), according to a capture method, which comprises capturing the antigen as a ligand with Anti-histidine antibody immobilized by means of a His Capture Kit (manufactured by GE Healthcare Bioscience) and then measuring the dissociation constant using an antibody

as an analyte. As the sensor chip, CM5 (manufactured by GE Healthcare Bioscience) was used. As the running buffer, buffer containing 20 mM Tris-HCl, 150 mM NaCl, 1 mM $CaCl_2$ and 0.05% Surfactant P20 (pH7.4), was used. On the chip, a 1 μg/mL Recombinant Human Cadherin-6 (KCAD)/Fc Chimera (RD-SYSTEMS) was added at a rate of 10 μL/minute for 60 seconds, and a dilution series solution (0.11 to 81 μg/mL) of the antibody prepared in Example 7)-4 was then added at a flow rate of 30 μl/min for 180 seconds. Subsequently, the dissociation phase was monitored for 180 seconds. As a regeneration solution, a Glycine 1.5 (GE Healthcare Bioscience made by the company) was added at a flow rate of 20 μl/min for 30 seconds. A Steady State Affinity model was used in data analysis, and the dissociation constant (KD) was calculated. The results are shown in Table 3.

[Table 3]

| Name | KD (nM) |
|---|---|
| H01L02A | 2.8 |
| H11L02A | 43.1 |
| H31L02A | 11.1 |

[Example 9: Production of anti-LPS antibody as control] An anti-LPS antibody was produced with reference to WO2015/046505. The isotope of the anti-LPS antibody used in the Example is IgG1 (also referred to as an anti-LPS antibody). The amino acid sequences of a light chain and heavy chain of the anti-LPS antibody used in the Example are shown in SEQ ID No: 72 and SEQ ID No: 73, respectively.

[Example 10: Synthesis of production intermediate]

[Example 10-1: Intermediate 1]

[Formula 53]

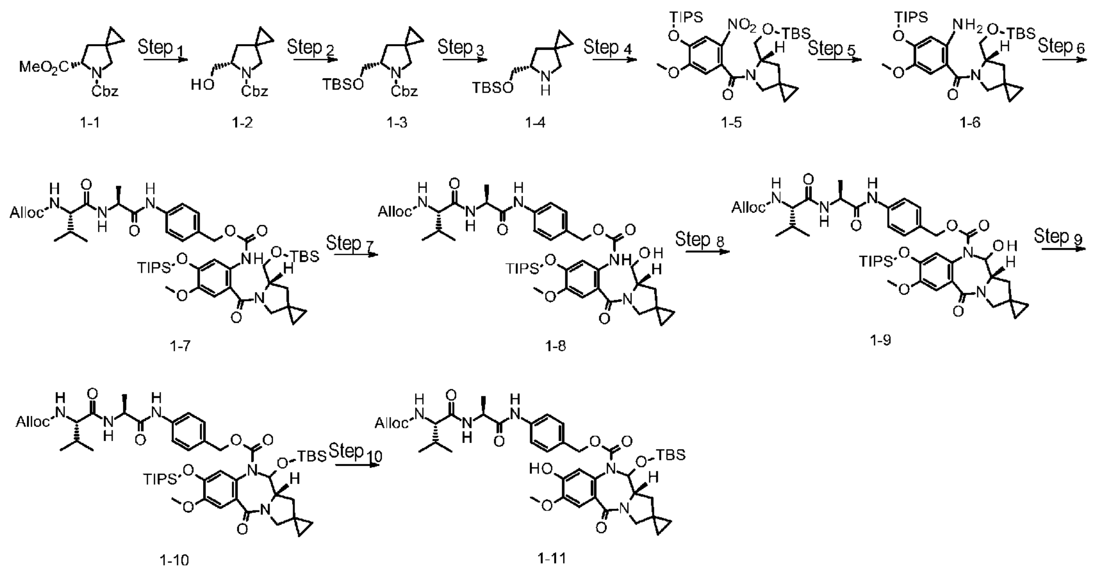


Step 1: Benzyl (6S)-6-(hydroxymethyl)-5-azaspiro[2.4]heptane-5-carboxylate (1-2)

To a solution of 5-benzyl 6-methyl(6S)-5-azaspiro[2.4]heptane-5,6-dicarboxylate (1-1) (104 mmol, WO 2012087596) in THF (500 mL), lithium borohydride (4.30 g, 178 mmol) was added in small portions at 0°C. The solution was stirred at 0°C for 30 minutes, and then stirred at room temperature for 2 hours. Water (180 mL) and 2 N hydrochloric acid (186 mL) were added at 0°C, and the resultant was distilled under reduced pressure. The resulting residue was extracted with ethyl acetate four times, and the organic layer was washed with brine and then dried over anhydrous sodium sulfate. The resultant was distilled under reduced pressure, and the resulting residue (1-2) (27.9 g, 90%) was

directly used for the subsequent reaction.

Step 2: Benzyl (6S)-6-({[tert-butyl(dimethyl)silyl]oxy}methyl)-5-azaspiro[2.4]heptan e-5-carboxylate (1-3)

To a solution of the compound obtained in step 1 (1-2) (27.9 g, 107 mmol) and imidazole (14.5 g, 214 mmol) in dichloromethane (300 mL), tert-butyldimethylsilyl chloride (24.2 g, 160 mmol) was added at room temperature, and the resultant was stirred at room temperature for 18 hours. The reaction solution was washed with a saturated aqueous citric acid, a saturated aqueous sodium hydrogen carbonate, and brine, dried over anhydrous sodium sulfate, and then distilled under reduced pressure. The resulting residue was purified by silica gel column chromatography [hexane:ethyl acetate = 100:0 (v/v) to 50:50 (v/v)] to afford the desired compound (1-3) (32.5 g, 81%).
1H-NMR (CDC13) δ: 7.39-7.34 (5H, m), 5.23-5.11 (2H, m), 4.10-3.48 (4H, m), 3.16-3.14 (1H, m), 2.15-2.04 (1H, m), 1.81-1.77 (1H, m), 0.91-0.88 (9H, m), 0.65-0.55 (4H, m), 0.08-0.01 (6H, m).
MS (APCI) m/z: 376 (M+H)+
Step 3: (6S)-6-({[tert-Butyl(dimethyl)silyl]oxy}methyl)-5-azaspiro[2.4]heptane (1-4)
To a solution of the compound obtained in step 2 (1-3) (32.5 g, 86.5 mmol) in ethanol (400 mL), 7.5% palladium carbon catalyst (moisture content: 54%, 5.00 g) was added at room temperature, and the resultant was stirred under a hydrogen atmosphere at room temperature for 6 hours. The reaction solution was filtered through Celite, and the filtrate was distilled under reduced pressure to afford the desired compound (1-4) (21.3 g, quantitative).
1H-NMR (CDC13) δ: 3.79-3.77 (1H, m), 3.71-3.69 (1H, m), 3.65-3.60 (1H, m), 3.01-2.98 (2H, m), 1.81-1.71 (2H, m), 0.90 (9H, s), 0.65-0.57 (4H, m), 0.08 (3H, s), 0.07 (3H, s) .
MS (APCI, ESI) m/z: 242 (M+H)+

Step 4: [(6S)-6-({[tert-Butyl(dimethyl)silyl]oxy}methyl)-5-azaspiro[2.4]hept-5 - yl](5-methoxy-2-nitro-4-{[tri(propan-2-yl)silyl]oxy}phenyl)methanone (1-5)

To a solution of 5-methoxy-2-nitro-4-{tri(propan-2-yl)silyl]oxy}benzoic acid (52.2 g, 141 mmol, US 20150283262) and 1-hydroxybenzotriazole monohydrate (23.8 g, 155 mmol) in dichloromethane (500 mL), N,N'-dicyclohexylcarbodiimide (35.0 g, 170 mmol) was added under ice-cooling. The reaction mixture was stirred at room temperature. After the carboxylic acid disappeared, a solution of the compound obtained in step 3 (1-4) (34.1 g, 141 mmol) and triethylamine (29.4 mL, 212 mmol) in dichloromethane (100 mL) was slowly added dropwise thereto. After the reaction solution was stirred at room temperature overnight, saturated aqueous sodium hydrogen carbonate was added to the reaction mixture, and the reaction mixture was extracted with chloroform. The organic layer was washed with water and brine, and dried over anhydrous magnesium sulfate. The resultant was distilled under reduced pressure, and to the resulting residue ethyl acetate and diethyl ether were added, and the solid contents were removed through filtration, and the filtrate was distilled under reduced pressure, and the resulting residue was purified by silica gel column chromatography [hexane:ethyl acetate = 100:0 (v/v) to 25:75 (v/v)] to afford the desired compound (1-5) (55.0 g, 66%).
1H-NMR (CDC13) δ: 7.72-7.66 (1H, m), 6.80-6.73 (1H, m), 4.53-4.49 (1H, m), 4.04-3.95 (1H, m), 3.91-3.88 (3H, m), 3.59-3.54 (1H, m), 3.36-3.25 (0.5H, m), 3.01-2.96 (1.5H, m), 2.24-2.20 (0.3H, m), 2.09-2.05 (0.7H, m), 2.00-1.97 (0.7H, m), 1.69-1.67 (0.3H, m), 1.32-1.24 (3H, m), 1.12-1.05 (18H, m), 0.93-0.91 (6H, m), 0.79-0.77 (3H, m), 0.71-0.62 (2H, m), 0.57-0.40 (2H, m), 0.12-0.10 (4H, m), 0.11-0.15 (2H, m).
MS (APCI, ESI) m/z: 593 (M+H)+

Step 5: (2-Amino-5-methoxy-4-{[tri(propan-2-yl)silyl]oxy}phenyl)[(6S)-6-({[tert-butyl(dimethyl)silyl]oxy}methyl)-5-azaspiro[2.4]hept-5 - yl]methanone (1-6)

To a solution of the compound obtained in step 4 (1-5) (55.0 g, 92.8 mmol) in ethanol (300 mL), 7.5% palladium carbon (10.0 g) was added under a nitrogen atmosphere. The nitrogen balloon was immediately replaced with a hydrogen balloon, and the reaction mixture was vigorously stirred under a hydrogen atmosphere at room temperature. After the raw materials disappeared, the reaction mixture was filtered, and the filtrate was distilled under reduced pressure to afford the desired compound (1-6) (52.2 g, 100%), which was directly used for the subsequent reaction.
1H-NMR (CDC13) δ: 6.71 (1H, s), 6.25 (1H, s), 4.55-4.28 (2H, m), 3.97 (1H, m), 3.75-3.62 (3H, m), 3.70 (3H, s), 3.09-3.07 (1H, m), 2.24-2.19 (1H, m), 1.81-1.68 (1H, m), 1.27-1.22 (3H, m), 1.09-1.05 (18H, m), 0.90 (9H, s), 0.65-0.46 (4H, m), 0.07-0.03 (6H, m).
MS (APCI, ESI) m/z: 563 (M+H)+
Step 6: N-[(Prop-2-en-1-yloxy)carbonyl]-L-valyl-N-[4-({[(2-{[(6S)-6-({[tert-butyl(dimethyl)silyl]oxy}methyl)-5-azaspiro[2.4]hept-5 - yl]carbonyl}-4-methoxy-5-{ [tri(propan-2-yl)silyl]oxy}phenyl)carbamoyl]oxy}methyl)phenyl]-L-alaninamide (1-7)
To a solution of the compound obtained in step 5 (1-6) (18.6 g, 33.0 mmol) and triethylamine (6.26 mL, 45.2

mmol) in THF (300 mL), triphosgene (4.22 g, 14.2 mmol) was slowly added on an ethanol-ice bath. After the addition, a solution of N-[(prop-2-en-1-yloxy)carbonyl]-L-valyl-N-[4-(hydroxymethyl)phenyl]-L-alaninamide (11.4 g, 30.2 mmol, WO 2011130598) and triethylamine (6.26 mL, 45.2 mmol) in THF (100 mL) and N,N-dimethylformamide (30 mL) was slowly added dropwise to the ice-cooled reaction mixture. After the dropwise addition, the ice bath was removed, and the reaction mixture was stirred under a nitrogen atmosphere at 40°C. After the raw materials disappeared, water was added to the reaction mixture, and the reaction mixture was extracted with ethyl acetate. The organic layer was washed with brine, and dried over anhydrous sodium sulfate. After filtration followed by distillation under reduced pressure, the resulting residue was purified by silica gel column chromatography [hexane:ethyl acetate = 100:0 (v/v) to 40:60 (v/v)] to afford the desired compound (1-7) (23.5 g, 74%).
1H-NMR (CDC13) δ: 8.99 (1H, m), 8.58 (1H, s), 7.80 (1H, s), 7.55-7.53 (2H, m), 7.34-7.32 (2H, m), 6.77-6.75 (2H, m), 5.94-5.87 (1H, m), 5.40-5.38 (1H, m), 5.33-5.29 (1H, m), 5.23-5.21 (1H, m), 5.13 (1H, m), 5.10 (2H, m), 4.69-4.64 (1H, m), 4.62-4.52 (2H, m), 4.06-4.03 (1H, m), 3.98 (1H, m), 3.76-3.65 (6H, m), 3.04 (1H, m), 2.28-2.26 (1H, m), 2.18-2.13 (1H, m), 1.46 (3H, m), 1.32-1.25 (3H, m), 1.11-1.09 (18H, m), 0.99-0.84 (15H, m), 0.65-0.40 (4H, m), 0.08-0.00 (6H, m).
MS (APCI, ESI) m/z: 966 (M+H)+

Step 7: N-[(Prop-2-en-1-yloxy)carbonyl]-L-valyl-N-[4-({[(2-{[(6S)-6-(hydroxymethyl)-5-azaspiro[2.4]hept-5-yl]carbonyl}-4-methoxy-5-{ [tri(propan-2-yl)silyl]oxy}phenyl)carbamoyl]oxy}methyl)phenyl]-L-alaninamide (1-8)

To a solution of the compound obtained in step 6 (1-7) (23.5 g, 24.3 mmol) in THF (50 mL), methanol (50 mL) and water (44 mL), acetic acid (200 mL) was added at room temperature. The reaction mixture was stirred at room temperature. After the raw materials disappeared, the reaction mixture was extracted with ethyl acetate. The organic layer was washed with water and brine, and dried over anhydrous sodium sulfate. After filtration followed by distillation under reduced pressure, the resulting residue was purified by silica gel column chromatography [hexane:ethyl acetate = 100:0 (v/v) to 0:100 (v/v)] to afford the desired compound (1-8) (18.0 g, 87%).
1H-NMR (CDC13) δ: 8.64-8.62 (1H, m), 8.50 (1H, m), 7.69 (1H, m), 7.55-7.53 (2H, m), 7.34-7.32 (2H, m), 6.79-6.75 (3H, m), 5.91-5.89 (1H, m), 5.39 (1H, m), 5.32-5.29 (1H, m), 5.23-5.21 (1H, m), 4.68-4.54 (4H, m), 4.31 (1H, m), 4.06-4.04 (1H, m), 3.81-3.79 (3H, m), 3.76 (3H, s), 3.63-3.61 (1H, m), 3.13-3.11 (1H, m), 2.16-2.13 (1H, m), 1.87-1.81 (2H, m), 1.46-1.43 (3H, m), 1.30-1.24 (3H, m), 1.12-1.08 (18H, m), 0.98-0.91 (6H, m), 0.63-0.45 (4H, m).
MS (APCI, ESI) m/z: 852 (M+H)+

Step 8: N-[(Prop-2-en-1-yloxy)carbonyl]-L-valyl-N-{4-[({[(11a'S)-11'-hydroxy-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (1-9)

To a solution of dimethyl sulfoxide (3.75 mL, 52.8 mmol) in dichloromethane (300 mL), oxalyl chloride (2.17 mL, 25.3 mmol) was slowly added dropwise under a nitrogen atmosphere at -78°C. After the dropwise addition, the reaction mixture was stirred at -78°C. A solution of the compound obtained in step 7 (1-8) (18.0 g, 21.1 mmol) in dichloromethane (50.0 mL) was slowly added to the reaction mixture. Triethylamine (14.6 mL, 105 mmol) was added to the reaction solution at -78°C. After the addition, the cool bath was removed, and the temperature was slowly raised to room temperature. After the raw materials disappeared, water was added to the reaction mixture, and the reaction mixture was extracted with chloroform (200 mL). The organic layer was washed with water and brine, and dried over anhydrous magnesium sulfate. After filtration followed by distillation under reduced pressure, the resulting residue was purified by silica gel column chromatography [hexane:ethyl acetate = 100:0 (v/v) to 0:60 (v/v)] to afford the desired compound (1-9) (16.5 g, 92%).
1H-NMR (CDC13) δ: 8.51-8.36 (1H, m), 7.54-7.38 (2H, m), 7.22-7.07 (3H, m), 6.73-6.64 (1H, m), 5.94-5.87 (2H, m), 5.33-5.22 (3H, m), 5.09 (1H, m), 4.97 (1H, m), 4.64-4.58 (4H, m), 4.02-4.00 (1H, m), 3.86-3.83 (3H, m), 3.75-3.70 (1H, m), 3.61-3.54 (2H, m), 3.38-3.29 (1H, m), 2.40 (1H, m), 2.16-2.14 (1H, m), 1.74-1.71 (1H, m), 1.44 (3H, m), 1.18-1.16 (3H, m), 1.05-1.00 (18H, m), 0.97-0.92 (6H, m), 0.72-0.60 (4H, m).
MS (APCI, ESI) m/z: 850 (M+H)+
Step 9: N-[(Prop-2-en-1-yloxy)carbonyl]-L-valyl-N-{4-[({[(11a'S)-11'-{[tert-butyl(dimethyl)silyl]oxy}-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alaninamide (1-10)
To a solution of the compound obtained in step 8 (1-9) (12.0 g, 14.1 mmol) and 2,6-lutidine (6.58 mL, 56.5 mmol) in dichloromethane (200 mL), tert-butyldimethylsilyl trifluoromethylsulfonate (9.73 mL, 42.3 mmol) was slowly added dropwise under a nitrogen atmosphere at 0°C. After stirring under ice-cooling for 10 minutes, the ice bath was removed, and the reaction mixture was stirred at room temperature. After the raw materials disappeared, water was added to the reaction mixture, which was extracted with chloroform. The organic layer was washed with water and brine, and dried over anhydrous sodium sulfate. After filtration followed by distillation under reduced pressure, the resulting

residue was purified by silica gel column chromatography [hexane:ethyl acetate = 100:0(v/v) to 25:75(v/v)] to afford the desired compound (1-10) (8.12 g, 60%).
1H-NMR (CDC13) δ: 8.67-8.45 (1H, m), 7.50-7.44 (2H, m), 7.19 (1H, s), 7.13 (2H, m), 6.95 (2H, m), 6.62-6.57 (2H, m), 6.01 (1H, m), 5.95-5.86 (1H, m), 5.33-5.13 (3H, m), 4.82 (1H, m), 4.65-4.54 (3H, m), 4.03-4.01 (1H, m), 3.84-3.82 (3H, m), 3.73-3.66 (1H, m), 3.50-3.48 (1H, m), 3.27 (1H, m), 2.37-2.33 (1H, m), 2.19-2.13 (1H, m), 1.54-1.43 (3H, m), 1.22-1.13 (3H, m), 1.10-1.00 (18H, m), 0.97-0.91 (6H, m), 0.81 (9H, s), 0.76-0.59 (4H, m), 0.19--0.09 (6H, m) .
MS (APCI, ESI) m/z: 964 (M+H)+

Step 10: N-[(Prop-2-en-1-yloxy)carbonyl]-L-valyl-N-{4-[({[(11a'S)-11'-{[tert-butyl(dimethyl)silyl]oxy}-8'-hydroxy-7'-methoxy-5'-oxo-11',11a'-dihydro-1'H-spiro[cyclopropane-1,2'-pyrrolo[2,1-c] [1,4]benzodiazepine]-10' (5'H)-yl]carbonyl}oxy)methyl]phenyl}-L-alaninamide (1-11)

To a solution of the compound obtained in step 9 (1-10) (8.12 g, 8.42 mmol) in N,N-dimethylformamide (90 mL) and water (2 mL), lithium acetate (0.611 g, 9.26 mmol) was added, and the resultant was stirred at room temperature. After the raw materials disappeared, water was added to the reaction mixture, which was extracted with ethyl acetate. The organic layer was washed with water and brine, and dried over anhydrous sodium sulfate. After filtration followed by distillation under reduced pressure, the resulting residue was purified by silica gel column chromatography [hexane:ethyl acetate = 100:0 (v/v) to 0:100 (v/v)] to afford the desired compound (1-11) (5.48 g, 81%).
1H-NMR (CDC13) δ: 8.76-8.60 (1H, m), 8.02-7.56 (1H, m), 7.45-7.44 (2H, m), 7.21 (1H, s), 7.10-7.09 (2H, m), 6.81-6.74 (1H, m), 6.65 (1H, s), 6.23 (1H, s), 6.01-5.99 (1H, m), 5.95-5.84 (1H, m), 5.41-5.20 (2H, m), 5.16 (1H, m), 4.84 (1H, m), 4.67-4.54 (4H, m), 4.05-4.03 (1H, m), 3.87 (3H, s), 3.71 (1H, m), 3.55-3.51 (1H, m), 3.26 (1H, m), 2.35 (1H, m), 2.18-2.12 (1H, m), 1.55-1.42 (3H, m), 0.97-0.92 (6H, m), 0.81 (9H, s), 0.76-0.61 (4H, m), 0.20--0.06 (6H, m).
MS (APCI, ESI) m/z: 808 (M+H)+

[Example 10-2: Intermediate 2]

[Formula 54]

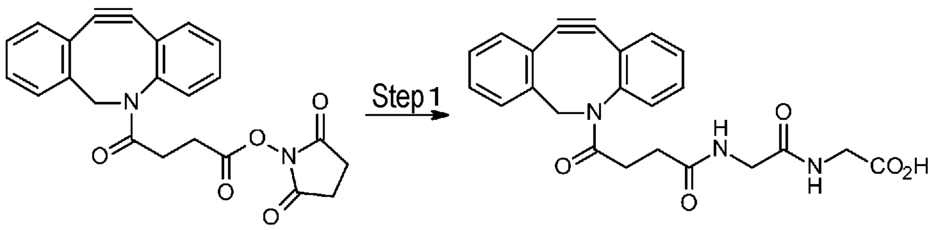


2-1 2-2

Step 1: N-[4-(11,12-Didehydrodibenzo[b,f]azocin-5(6H)-yl)-4-oxobutanoyl]glycylglycine (2-2)

To a solution of glycylglycine (0.328 g, 2.49 mmol), N,N-diisopropylethylamine (0.433 mL, 2.49 mmol) in N,N-dimethylformamide(20 mL), 1-{[4-(11,12-didehydrodibenzo[b,f]azocin-5(6H)-yl)-4-oxobutanoyl]oxy}pyrrolidin-2,5-dione (2-1) (1.00 g, 2.49 mmol, Click Chemistry Tools) and water (10 mL) were added at room temperature, and the resultant was stirred at room temperature overnight. The resultant was distilled under reduced pressure, and the resulting residue was purified by silica gel column chromatography [chloroform:CMW = 100:0(v/v) to 0:100(v/v)] to afford the desired compound (0.930 g, 89%).
1H-NMR (DMSO-D6) δ: 12.58 (1H, s), 8.14-8.12 (1H, m), 8.08-8.07 (1H, m), 7.69-7.68 (1H, m), 7.62-7.61 (1H, m), 7.53-7.45 (3H, m), 7.40-7.29 (3H, m), 5.05-5.01 (1H, m), 3.73-3.72 (2H, m), 3.66-3.60 (3H, m), 2.66-2.60 (1H, m), 2.33-2.24 (1H, m), 2.08-2.04 (1H, m), 1.81-1.77 (1H, m). MS (APCI, ESI) m/z: 420 [(M+H)+].

[Example 10-3: Drug-linker 1]

[Formula 55]

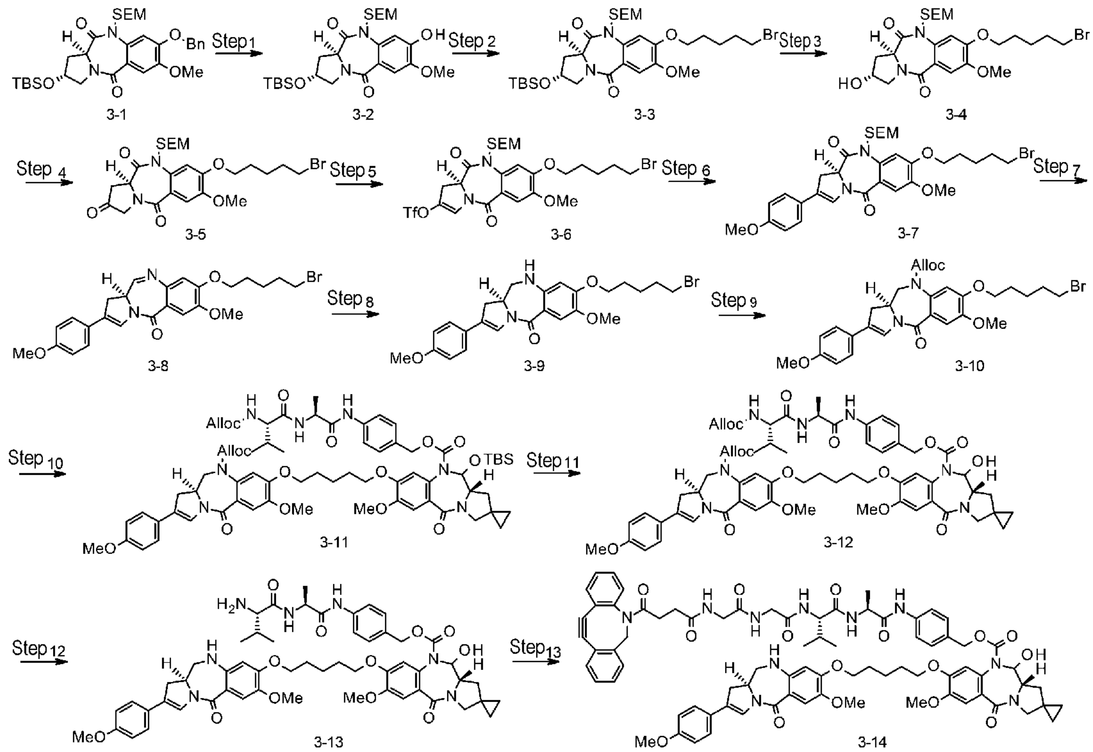

Step 1: (2R,11aS)-2-{[tert-Butyl(dimethyl)silyl]oxy}-8-hydroxy-7-methoxy-10-{[2-(trimethylsilyl)ethoxy]methyl}-2,3-dihydro-1H-pyrrolo[2,1-c][1,4]benzodiazepin-5,11(10H,11aH)-dione (3-2)

To a solution of (2R,11aS)-8-(benzyloxy)-2-{[tert-butyl(dimethyl)silyl]oxy}-7-methoxy-10-{[2-(trimethylsilyl)ethoxy]methyl}-2,3-dihydro-1H-pyrrolo[2,1-c][1,4]benzodiazepin-5,11(10H,11aH)-dione (3-1) (25.5 g, 41.6 mmol, WO 2016149546) in THF (150 mL) and ethanol (150 mL), 5% palladium carbon (moisture content: 54%, 10.0 g) was added under a nitrogen atmosphere, and the reaction solution was then stirred under a hydrogen atmosphere at room temperature for 3 days. Chloroform was added to the reaction solution, which was filtered through Celite, and the filtrate was then distilled under reduced pressure. The resulting residue was purified by silica gel column chromatography [hexane:ethyl acetate = 100:0 (v/v) to 50:50 (v/v)] to afford the desired compound (3-2) (19.4 g, 89%).
1H-NMR (CDC13) δ: 7.36 (1H, s), 7.25 (1H, s), 6.01 (1H, s), 5.45-5.43 (1H, m), 4.69-4.67 (1H, m), 4.60-4.55 (1H, m), 4.23-4.21 (1H, m), 3.96 (3H, s), 3.76-3.68 (2H, m), 3.63-3.61 (1H, m), 3.56-3.53 (1H, m), 2.88-2.83 (1H, m), 2.03-2.00 (1H, m), 1.00-0.98 (2H, m), 0.87 (9H, s), 0.10 (6H, s), 0.02 (9H, s).
MS (APCI, ESI) m/z: 523 (M+H)+

Step 2: (2R,11aS)-8-[(5-Bromopentyl)oxy]-2-{[tert-butyl(dimethyl)silyl]oxy}-7-methoxy-10-{[2-(trimethylsilyl)ethoxy]methyl}-2,3-dihydro-1H-pyrrolo[2,1-c][1,4]benzodiazepin-5,11(10H,11aH)-dione (3-3)

To a solution of the compound obtained in step 1 (3-2) (10.8 g, 20.7 mmol) in N,N-dimethylformamide (30 mL), 1,5-dibromopentane (23.8 g, 103 mmol) and potassium carbonate (3.43 g, 24.8 mmol) were added at room temperature. After stirring at room temperature for 3 hours, water was added to the reaction solution, which was extracted with ethyl acetate. The organic layer obtained was washed with brine and dried over sodium sulfate, and distilled under reduced pressure. The resulting residue was purified by silica gel column chromatography [hexane:ethyl acetate = 90:10 (v/v) to 50:50 (v/v)] to afford the desired compound (3-3) (14.5 g, quantitative).
1H-NMR (CDC13) δ: 7.34 (1H, s), 7.21 (1H, s), 5.52-5.49 (1H, m), 4.63-4.62 (1H, m), 4.58-4.55 (1H, m), 4.24-4.22 (1H, m), 4.07-4.04 (2H, m), 3.92 (3H, s), 3.82-3.64 (3H, m), 3.56-3.53 (1H, m), 3.45-3.43 (2H, m), 2.86-2.84 (1H, m), 2.04-2.00 (1H, m), 1.97-1.87 (4H, m), 1.66-1.62 (2H, m), 1.01-0.98 (2H, m), 0.87 (9H, s), 0.10 (6H, s), 0.04 (9H, s).
MS (APCI, ESI) m/z: 673 [81Br, (M+H)+], 671 [79Br, (M+H)+] .

Step 3: (2R,11aS)-8-[(5-Bromopentyl)oxy]-2-hydroxy-7-methoxy-10-{ [2-(trimethylsilyl)ethoxy]methyl}-2,3-dihydro-1H-pyrrolo[2,1-c] [1,4]benzodiazepin-5,11(10H,11aH)-dione (3-4)

To a solution of the compound obtained in step 2 (3-3) (21.5 mmol) in THF (40 mL), a 1 mol/L THF solution of tetrabutylammonium fluoride (28.0 mL, 28.0 mmol) was added at 0°C. After stirring at room temperature for 30 minutes, water was added to the reaction solution, which was extracted with ethyl acetate, and the organic layer obtained was washed with brine. The resultant was dried over sodium sulfate, and then distilled under reduced pressure. The resulting residue was purified by silica gel column chromatography [chloroform:methanol = 97.5:2.5 (v/v) to 92.5:7.5 (v/v)] to afford the desired compound (3-4) (11.3 g, 94%).
1H-NMR (CDC13) δ: 7.34 (1H, s), 7.21 (1H, s), 5.53-5.50 (1H, m), 4.69-4.64 (2H, m), 4.32-4.30 (1H, m), 4.10-4.00 (2H, m), 3.91 (3H, s), 3.88-3.75 (2H, m), 3.73-3.64 (2H, m), 3.45-3.44 (2H, m), 2.99-2.96 (1H, m), 2.15-2.09 (1H, m), 1.99-1.85 (5H, m), 1.68-1.62 (2H, m), 1.01-0.95 (2H, m), 0.04 (9H, s).
MS (APCI, ESI) m/z: 559 [81Br, (M+H)+], 557 [79Br, (M+H)+].

Step 4: (11aS)-8-[(5-Bromopentyl)oxy]-7-methoxy-10-{[2-(trimethylsilyl)ethoxy]methyl}-1H-pyrrolo[2,1-c][1,4]benzodiazepin-2,5,11(3H,10H,11aH)-trione (3-5)

The compound obtained in step 3 (3-4) (11.3 g, 20.2 mmol), tetrabutylammonium bromide (0.325 g, 1.01 mmol), and potassium bromide (0.240 g, 2.02 mmol) were dissolved in a saturated aqueous sodium hydrogen carbonate (60 mL)/dichloromethane (60 mL), to which nor-AZADO (0.0279 g, 0.202 mmol) and sodium hypochlorite pentahydrate (2.03 g, 27.2 mmol) were added at 0°C, and the resultant was stirred at 0°C for 30 minutes. Because the raw materials remained, sodium hypochlorite pentahydrate (1.00 g, 13.4 mmol) was added thereto at 0°C, and the resultant was stirred at 0°C for 15 minutes. Sodium hypochlorite pentahydrate (0.300 g, 4.03 mmol) was further added thereto at 0°C, and the resultant was stirred at 0°C for 15 minutes, and the disappearance of the raw materials was confirmed by TLC. An aqueous solution of sodium thiosulfate was added to the reaction solution, which was extracted with chloroform, and the organic layer obtained was dried over sodium sulfate. The resultant was distilled under reduced pressure, and the resulting residue was purified by silica gel column chromatography [hexane:ethyl acetate = 75:25(v/v) to 40:60(v/v)] to afford the desired compound (3-5) (9.74 g, 87%).
1H-NMR (CDC13) δ: 7.33 (1H, s), 7.24 (1H, s), 5.56-5.53 (1H, m), 4.71-4.69 (1H, m), 4.66-4.63 (1H, m), 4.27-4.22 (1H, m), 4.12-4.02 (2H, m), 3.93-3.88 (4H, m), 3.82-3.75 (1H, m), 3.69-3.67 (1H, m), 3.61-3.56 (1H, m), 3.46-3.44 (2H, m), 2.82-2.77 (1H, m), 1.97-1.89 (4H, m), 1.68-1.64 (2H, m), 1.05-0.93 (2H, m), 0.04 (9H, s).
MS (APCI, ESI) m/z: 557 [81Br, (M+H)+], 555 [79Br, (M+H)+].

Step 5: (11aS)-8-[(5-Bromopentyl)oxy]-7-methoxy-5,11-dioxo-10-{ [2-(trimethylsilyl)ethoxy]methyl}-5,10,11,11a-tetrahydro-1H-pyrrolo[2,1-c][1,4]benzodiazepin-2-yl trifluoromethanesulfonate (3-6)

To a solution of the compound obtained in step 4 (3-5) (9.74 g, 17.5 mmol) in dichloromethane (160 mL), 2,6-lutidine (8.17 mL, 70.1 mmol) was added at -40°C, and the resultant was stirred at -40°C for 10 minutes. Anhydrous trifluoromethanesulfonic acid (8.85 mL, 52.6 mmol) was added to the reaction solution at -40°C, and the resultant was stirred at -40°C for 30 minutes. To the reaction solution, a 10% aqueous solution of citric acid was added, which was extracted with chloroform, and the organic layer obtained was dried over sodium sulfate. The resultant was distilled under reduced pressure, and the resulting residue was purified by silica gel column chromatography [hexane:ethyl acetate = 95:5 (v/v) to 70:35 (v/v)] and then purified by NH2 silica gel chromatography [hexane:ethyl acetate = 95:5 (v/v) to 65:35 (v/v)] to afford the desired compound (3-6) (7.10 g, 59%).
1H-NMR (CDC13) δ: 7.32 (1H, s), 7.24 (1H, s), 7.15-7.14 (1H, m), 5.56-5.53 (1H, m), 4.70-4.68 (1H, m), 4.66-4.63 (1H, m), 4.11-4.01 (2H, m), 3.94-3.90 (4H, m), 3.84-3.75 (1H, m), 3.73-3.68 (1H, m), 3.46-3.44 (2H, m), 3.18-3.14 (1H, m), 1.96-1.88 (4H, m), 1.69-1.61 (2H, m), 1.02-0.92 (2H, m), 0.04 (9H, s).
MS (APCI, ESI) m/z: 689 [81Br, (M+H)+], 687 [79Br, (M+H)+].

Step 6: (11aS)-8-[(5-Bromopentyl)oxy]-7-methoxy-2-(4-methoxyphenyl)-10-{ [2-(trimethylsilyl)ethoxy]methyl}-1H-pyrrolo[2,1-c] [1,4]benzodiazepin-5,11(10H,11aH)-dione (3-7)

To a mixture of the compound obtained in step 5 (3-6) (2.00 g, 2.91 mmol), 4-methoxyphenylboronic acid (0.884 g, 5.82 mmol), tetrakis(triphenylphosphine)palladium (0) (0.336 g, 0.291 mmol) and sodium carbonate (1.23 g, 11.6 mmol), toluene (20 mL), ethanol (10 mL) and water (10 mL) were added at room temperature. The reaction solution was stirred at room temperature for 30 minutes, and the reaction solution was then extracted with ethyl acetate, and the extract was washed with water and brine. The organic layer was dried over sodium sulfate, and then distilled under reduced pressure. The resulting residue was purified by silica gel column chromatography [hexane:ethyl acetate = 90:10

(v/v) to 50:50 (v/v)] to afford the desired compound (3-7) (1.71 g, 91%).1H-NMR (CDC13) δ: 7.38-7.37 (3H, m), 7.33 (1H, s), 7.25 (1H, s), 6.89-6.88 (2H, m), 5.56-5.54 (1H, m), 4.71-4.68 (1H, m), 4.65-4.62 (1H, m), 4.09-4.04 (2H, m), 3.96-3.91 (4H, m), 3.85-3.66 (5H, m), 3.46-3.45 (2H, m), 3.16-3.12 (1H, m), 1.99-1.94 (4H, m), 1.69-1.64 (2H, m), 1.00-0.98 (2H, m), 0.04 (9H, s).
MS (APCI, ESI) m/z: 647 [81Br, (M+H)+], 645 [79Br, (M+H)+].

Step 7: (11aS)-8-[(5-Bromopentyl)oxy]-7-methoxy-2-(4-methoxyphenyl)-1, 11a-dihydro-5H-pyrrolo[2,1-c][1,4]benzodiazepin-5-one (3-8)

The compound obtained in step 6 (3-7) (0.789 g, 1.22 mmol) was dissolved in ethanol (10 mL) and THF (10 mL), and 2.0 M tetrahydrofuran solution of lithium borohydride (6.11 mL, 12.2 mmol) was added thereto at 0°C, and the resultant was stirred at 0°C for 3 hours. Water was added to the reaction solution, which was extracted with chloroform, and the organic layer obtained was dried over sodium sulfate. The resultant was distilled under reduced pressure, and the resulting residue was dissolved in dichloromethane (10 mL), ethanol (20 mL) and water (10 mL), to which silica gel (4 g) was added at room temperature, and the resultant was stirred at room temperature for 4 days. The silica gel was removed through filtration, and water was added thereto, and the resultant was extracted with chloroform. The organic layer obtained was dried over sodium sulfate. The resultant was distilled under reduced pressure, and the resulting residue was purified by silica gel column chromatography [hexane:ethyl acetate = 60:40 (v/v) to 25:75 (v/v)] to afford the desired compound (3-8) (0.496 g, 81%).
1H-NMR (CDC13) δ: 7.90-7.89 (1H, m), 7.53 (1H, s), 7.40-7.40 (1H, m), 7.35-7.34 (2H, m), 6.92-6.90 (2H, m), 6.83-6.81 (1H, m), 4.43-4.40 (1H, m), 4.13-4.06 (2H, m), 3.96 (3H, s), 3.84 (3H, s), 3.61-3.57 (1H, m), 3.47-3.36 (3H, m), 2.00-1.92 (4H, m), 1.67-1.63 (2H, m).
MS (APCI, ESI) m/z: 501 [81Br, (M+H)+], 499 [79Br, (M+H)+] .

Step 8: (11aS)-8-[(5-Bromopentyl)oxy]-7-methoxy-2-(4-methoxyphenyl)-1,10, 11,11a-tetrahydro-5H-pyrrolo[2, 1-c][1,4]benzodiazepin-5-one (3-9)

To a solution of the compound obtained in step 7 (3-8) (0.496 g, 0.992 mmol) in dichloromethane (20 mL), sodium triacetoxyborohydride (0.421 g, 1.99 mmol) was added at 0°C. After stirring at room temperature for 2 hours, a saturated aqueous sodium hydrogen carbonate was added thereto, and the resultant was extracted with chloroform. The organic layer was dried over sodium sulfate, and distilled under reduced pressure, and the resulting residue was then purified by silica gel column chromatography [hexane:ethyl acetate = 60:40 (v/v) to 25:75 (v/v)] to afford the desired compound (3-9) (0.426 g, 86%).
1H-NMR (CDC13) δ: 7.53-7.53 (2H, m), 7.32-7.30 (2H, m), 6.89-6.87 (2H, m), 6.05 (1H, s), 4.33-4.27 (2H, m), 4.00-3.98 (2H, m), 3.86 (3H, s), 3.82 (3H, s), 3.57-3.55 (2H, m), 3.42-3.38 (3H, m), 2.76-2.72 (1H, m), 1.96-1.88 (4H, m), 1.65-1.62 (2H, m).
MS (APCI, ESI) m/z: 503 [81Br, (M+H)+], 501 [79Br, (M+H)+].

Step 9: Prop-2-en-1-yl (11aS)-8-[(5-bromopentyl)oxy]-7-methoxy-2-(4-methoxyphenyl)-5-oxo-11,11a-dihydro-1H-pyrrolo[2,1-c] [1,4]benzodiazepin-10(5H)-carboxylate (3-10)

To a solution of the compound obtained in step 8 (3-9) (0.426 g, 0.849 mmol) in dichloromethane (30 mL), pyridine (0.102 mL 1.27 mmol) and allyl chloroformate (0.374 mL, 3.54 mmol) were added at 0°C, and the resultant was stirred at 0°C for 15 minutes. To the reaction solution, a 10% aqueous solution of citric acid was added, which was extracted with chloroform, and the organic layer obtained was washed with a saturated aqueous sodium hydrogen carbonate, and then dried over sodium sulfate. The resultant was distilled under reduced pressure, and the resulting residue was purified by silica gel column chromatography [hexane:ethyl acetate = 90:10 (v/v) to 50:50 (v/v)] to afford the desired compound (3-10) (0.465 g, 94%).
1H-NMR (CDC13) δ: 7.38 (1H, s), 7.31-7.29 (2H, m), 7.26-7.25 (1H, m), 6.89-6.87 (2H, m), 6.71 (1H, s), 5.80-5.78 (1H, m), 5.14-5.11 (2H, m), 4.65-4.62 (1H, m), 4.39-4.26 (3H, m), 4.03-4.01 (2H, m), 3.92 (3H, s), 3.82 (3H, s), 3.66-3.64 (1H, m), 3.46-3.44 (2H, m), 3.30-3.27 (1H, m), 2.72-2.68 (1H, m), 1.96-1.88 (4H, m), 1.68-1.60 (2H, m). MS (APCI, ESI) m/z: 587 [81Br, (M+H)+], 585 [79Br, (M+H)+].

Step 10: N-[(Prop-2-en-1-yloxy)carbonyl]-L-valyl-N-{4-[({[(11a'S)-11'-{[tert-butyl(dimethyl)silyl]oxy}-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c] [1,4]benzodiazepin-8-yl}oxy)pentyl]oxy}-5'-oxo-11',11a'-dihydro-1'H-spiro[cyclopropane-1,2'-pyrrolo[2,1-c] [1,4]benzodiazepine]-10' (5'H)-yl]carbonyl}oxy)methyl]phenyl}-L-alaninamide (3-11)

To a solution of the compound obtained in Step 10 of Example 10-1 (1-11) (0.130 g, 0.161 mmol) and the compound obtained in step 9 (3-10) (0.104 g, 0.177 mmol) in N,N-dimethylformamide (3 mL), potassium carbonate (0.0266 g, 0.193 mmol) was added at room temperature, and the resultant was stirred at room temperature overnight. The reaction solution was diluted with ethyl acetate, and washed with water and brine, and then dried over sodium sulfate. The resultant was distilled under reduced pressure, and the resulting residue was then purified by NH2-silica gel column chromatography [hexane:ethyl acetate = 70:30 (v/v) to 0:100 (v/v)] to afford the desired compound (3-11) (0.184 g, 87%).
1H-NMR (CDCl3) δ: 8.76 (1H, s), 7.58-7.56 (2H, m), 7.39 (1H, s), 7.32-7.30 (2H, m), 7.26-7.24 (2H, m), 7.19-7.17 (3H, m), 6.90-6.88 (2H, m), 6.78 (1H, s), 6.68-6.66 (1H, m), 6.37 (1H, s), 5.99-5.93 (3H, m), 5.34-5.20 (6H, m), 4.66-4.01 (11H, m), 3.90 (3H, s), 3.89 (3H, s), 3.78-3.54 (9H, m), 3.31-3.28 (2H, m), 2.73-2.69 (1H, m), 2.38-2.35 (1H, m), 2.19-2.13 (1H, m), 1.82-1.80 (2H, m), 1.46-1.29 (6H, m), 0.98-0.90 (6H, m), 0.83 (9H, s), 0.69-0.63 (4H, m), 0.19-0.16 (6H, m).
MS (APCI, ESI) m/z: 1312 (M+H)+

Step 11: N-[(Prop-2-en-1-yloxy)carbonyl]-L-valyl-N-{4-[({[(11a'S)-11'-hydroxy-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c] [1,4]benzodiazepin-8-yl}oxy)pentyl]oxy}-5'-oxo-11',11a'-dihydro-1'H-spiro[cyclopropane-1,2'-pyrrolo[2,1-c] [1,4]benzodiazepine]-10' (5'H)-yl]carbonyl}oxy)methyl]phenyl}-L-alaninamide (3-12)

To a solution of the compound obtained in step 10 (3-11) (0.1837 g, 0.140 mmol) and acetic acid (0.048 mL, 0.840 mmol) in THF (5.00 mL), a 1 mol/L tetrahydrofuran solution of tetrabutylammonium fluoride (0.700 mL, 0.700 mmol) was added at room temperature, and the resultant was stirred at room temperature for 3 hours. The reaction solution was diluted with ethyl acetate, and the organic layer was washed with a saturated aqueous sodium hydrogen carbonate and brine, and then dried over sodium sulfate. The resultant was distilled under reduced pressure, and the resulting residue was purified by silica gel chromatography [chloroform:methanol = 99.5:0.5(v/v) to 95:5(v/v)] to afford the desired compound (3-12) (0.178 g, quantitative).
1H-NMR (CDC13) δ: 8.86 (1H, s), 7.60-7.59 (2H, m), 7.39 (1H, s), 7.32-7.20 (7H, m), 6.90-6.88 (2H, m), 6.78 (1H, s), 6.68 (1H, s), 6.38 (1H, s), 5.90-5.87 (3H, m), 5.39-5.22 (6H, m), 4.72-4.02 (11H, m), 3.90 (3H, s), 3.88 (3H, s), 3.83 (3H, s), 3.70-3.63 (6H, m), 3.32-3.29 (3H, m), 2.73-2.69 (1H, m), 2.43-2.40 (1H, m), 2.12-2.06 (1H, m), 1.77-1.74 (2H, m), 1.39-1.25 (6H, m), 0.96-0.89 (6H, m), 0.73-0.66 (4H, m).
MS (APCI, ESI) m/z: 1198 (M+H)+

Step 12: L-Valyl-N-{4-[({[(11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c] [1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (3-13)

To a solution of the compound obtained in step 11 (3-12) (0.140 mmol) in dichloromethane (2 mL), pyrrolidine (0.0579 mL, 0.700 mmol) and tetrakis(triphenylphosphine)palladium (0) (0.0162 g, 0.0140 mmol) were added at room temperature, and the resultant was stirred at room temperature for 15 minutes. After distillation under reduced pressure, the resulting residue was purified by silica gel chromatography [chloroform:methanol = 99.5:0.5(v/v) to 92.5:7.5(v/v)] to afford the desired compound (3-13) (0.143 g, 99%).
1H-NMR (CDC13) δ: 9.12 (1H, s), 7.94-7.92 (1H, m), 7.57-7.53 (4H, m), 7.33-7.31 (2H, m), 7.20-7.18 (3H, m), 6.90-6.88 (2H, m), 6.36 (1H, s), 6.07 (1H, s), 5.91-5.88 (1H, m), 5.47-5.44 (1H, m), 5.21-5.13 (1H, m), 4.66-4.58 (3H, m), 4.32 (1H, s), 4.03-3.49 (17H, m), 3.38-3.29 (4H, m), 3.15-3.14 (1H, m), 2.77-2.73 (1H, m), 2.57 (2H, s), 2.43-2.40 (1H, m), 2.32-2.27 (1H, m), 1.81-1.39 (8H, m), 0.98-0.96 (3H, m), 0.85-0.83 (3H, m), 0.75-0.62 (4H, m).
MS (APCI, ESI) m/z: 1030 (M+H)+

Step 13: N-[4-(11,12-Didehydrodibenzo[b,f]azocin-5(6H)-yl)-4-oxobutanoyl]glycylglycyl-L-valyl-N-{4-[({[(11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c] [1,4]benzodiazepin-8-yl]oxy}pentyl)oxy]-5'-oxo-11',11a'-dihydro-1'H-spiro[cyclopropane-1,2'-pyrrolo[2,1-c] [1,4]benzodiazepine]-10' (5'H)-yl]carbonyl}oxy)methyl]phenyl}-L-alaninamide (3-14)

To a mixture of the compound obtained in step 1 of Example 10-2 (2-2) (0.0640 g, 0.153 mmol) and N-ethoxycarbonyl-2-ethoxy-1,2-dihydroquinoline (0.0446 g, 0.180 mmol), dichloromethane (2 mL) was added at room temperature, and the resultant was stirred at room temperature for 15 minutes. To the reaction solution, a solution of the compound obtained in step 12 (3-13) (0.143 g, 0.139 mmol) in dichloromethane (2 mL) was added, and the resultant was stirred at room temperature for 5 hours, and then distilled under reduced pressure. The resulting residue was purified by silica gel column chromatography [chloroform:methanol = 99.5:0.5 (v/v) to 92.5:7.5 (v/v)] to afford the desired compound (3-14) (0.103 g, 52%).

1H-NMR (DMSO-D6) δ: 9.93 (1H, s), 8.21-8.16 (2H, m), 8.07-8.04 (1H, m), 7.83-7.64 (2H, m), 7.60-7.55 (3H, m), 7.51-7.28 (10H, m), 7.19-7.16 (2H, m), 7.10-7.04 (1H, m), 6.92-6.90 (2H, m), 6.76-6.70 (1H, m), 6.39 (1H, s), 5.77-5.75 (1H, m), 5.21-5.18 (1H, m), 5.03-4.99 (1H, m), 4.82-4.79 (1H, m), 4.37-4.35 (1H, m), 4.21-4.20 (2H, m), 4.02-3.24 (26H, m), 3.16-3.13 (1H, m), 2.79-2.59 (2H, m), 2.39-2.28 (2H, m),
2.05-1.97 (2H, m), 1.91-1.77 (4H, m), 1.57-1.54 (3H, m), 1.28-1.23 (3H, m),
0.85-0.80 (6H, m), 0.67-0.61 (4H, m).
MS (APCI, ESI) m/z: 1431 (M+H)+

[Example 10-4: Drug-linker 2]

[Formula 56]

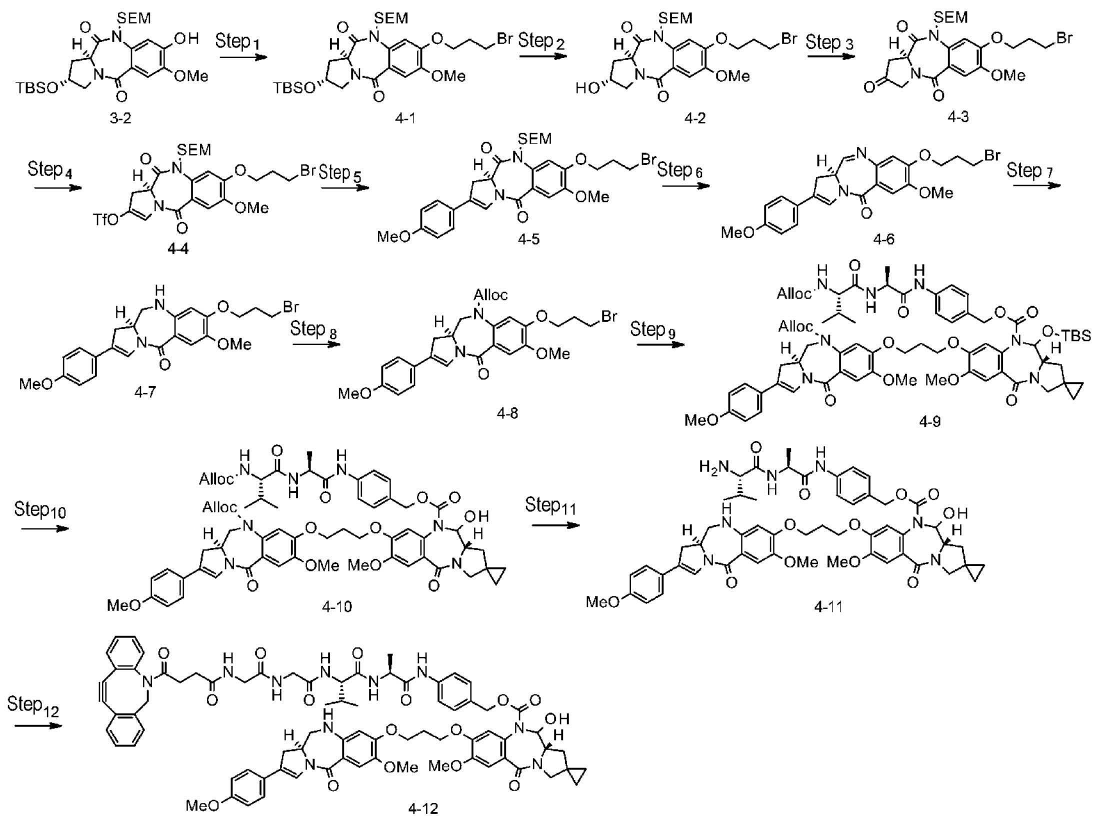


Step 1: (2R,11aS)-8-(3-Bromopropoxy)-2-{[tert-butyl(dimethyl)silyl]oxy}-7-methoxy-10-{[2-(trimethylsilyl)ethoxy]methyl}-2,3-dihydro-1H-pyrrolo[2,1-c][1,4]benzodiazepin-5,11(10H,11aH)-dione (4-1)

The compound obtained in step 1 of Example 10-3 (3-2) (5.06 g, 9.67 mmol) was reacted with 1,3-dibromopropane (4.93 mL, 48.4 mmol) in the same manner as in step 2 of Example 10-3 to afford the desired compound (4-1) (4.85 g, 78%).

1H-NMR (CDC13) δ: 7.35 (1H, s), 7.26 (1H, s), 5.52-5.50 (1H, m), 4.65-4.63 (1H, m), 4.61-4.55 (1H, m), 4.25-4.14 (3H, m), 3.92 (3H, s), 3.82-3.62 (5H, m), 3.57-3.54 (1H, m), 2.86-2.84 (1H, m), 2.41-2.39 (2H, m), 2.06-1.99 (1H, m), 1.03-0.97 (2H, m), 0.87 (9H, s), 0.10 (6H, s), 0.04 (9H, s).
MS (APCI, ESI) m/z: 645 [81Br, (M+H)+], 643 [79Br, (M+H)+].

Step 2: (2R,11aS)-8-(3-Bromopropoxy)-2-hydroxy-7-methoxy-10-{[2-(trimethylsilyl)ethoxy]methyl}-2,3-dihydro-1H-pyrrolo[2,1-c] [1,4]benzodiazepin-5,11(10H,11aH)-dione (4-2)

The compound obtained in step 1 (4-1) (4.85 g, 7.54 mmol) was reacted in the same manner as in step 3 of Example 10-3 to afford the desired compound (4-2) (4.05 g, quantitative).
1H-NMR (CDC13) δ: 7.35 (1H, s), 7.26 (1H, s), 5.53-5.51 (1H, m), 4.66-4.61 (2H, m), 4.32-4.30 (1H, m), 4.21-4.16 (2H, m), 3.91-3.85 (4H, m), 3.82-3.74 (1H, m), 3.71-3.59 (4H, m), 2.99-2.96 (1H, m), 2.43-2.37 (2H, m), 2.15-2.09 (2H, m), 1.04-0.96 (2H, m), 0.04 (9H, s).
MS (APCI, ESI) m/z: 531 [81Br, (M+H)+], 529 [79Br, (M+H)+].

Step 3: (11aS)-8-(3-Bromopropoxy)-7-methoxy-10-{[2-(trimethylsilyl)ethoxy]methyl}-1H-pyrrolo[2,1-c] [1,4]benzodiazepin-2,5,11(3H,10H,11aH)-trione (4-3)

The compound obtained in step 2 (4-2) (7.54 mmol) was reacted in the same manner as in step 4 of Example 10-3 to afford the desired compound (4-3) (3.73 g, 93%). 1H-NMR (CDC13) δ: 7.34 (1H, s), 7.29 (1H, s), 5.56-5.53 (1H, m), 4.72-4.69 (1H, m), 4.67-4.61 (1H, m), 4.23-4.17 (3H, m), 3.97-3.88 (4H, m), 3.82-3.75 (1H, m), 3.74-3.56 (4H, m), 2.82-2.77 (1H, m), 2.43-2.38 (2H, m), 1.06-0.94 (2H, m), 0.08-0.00 (9H, m).

Step 4: (11aS)-8-(3-Bromopropoxy)-7-methoxy-5,11-dioxo-10-{[2-(trimethylsilyl)ethoxy]methyl}-5,10,11,11a-tetrahydro-1H-pyrrolo[2,1-c] [1,4]benzodiazepin-2-yl trifluoromethanesulfonate (4-4)

The compound obtained in step 3 (4-3) (3.73 g, 7.08 mmol) was reacted in the same manner as in step 5 of Example 10-3 to afford the desired compound (4-4) (3.27 g, 70%).
1H-NMR (CDC13) δ: 7.33 (1H, s), 7.29 (1H, s), 7.15-7.15 (1H, m), 5.56-5.54 (1H, m), 4.70-4.65 (2H, m), 4.21-4.18 (2H, m), 3.94-3.91 (4H, m), 3.81-3.79 (1H, m), 3.70-3.64 (3H, m), 3.19-3.15 (1H, m), 2.47-2.38 (2H, m), 1.02-1.00 (2H, m), 0.04 (9H, s).
MS (APCI, ESI) m/z: 661 [81Br, (M+H)+], 659 [79Br, (M+H)+].

Step 5: (11aS)-8-(3-Bromopropoxy)-7-methoxy-2-(4-methoxyphenyl-10-{[2-(trimethylsilyl)ethoxy]methyl}-1H-pyrrolo[2,1-c][1,4]benzodiazepin-5,11(10H,11aH)-dione (4-5)

The compound obtained in step 4 (4-4) (3.27 g, 4.96 mmol) was reacted in the same manner as in step 6 of Example 10-3 to afford the desired compound (4-5) (2.49 g, 81%).
1H-NMR (DMSO-D6) δ: 7.49-7.47 (2H, m), 7.40 (1H, s), 7.31-7.24 (2H, m), 6.93-6.88 (2H, m), 5.33-5.31 (1H, m), 5.25-5.18 (1H, m), 4.81-4.80 (1H, m), 4.23-4.10 (2H, m), 3.85 (3H, s), 3.77 (3H, s), 3.70-3.59 (3H, m), 3.52-3.40 (2H, m), 3.15-3.08 (1H, m), 2.33-2.27 (2H, m), 0.86-0.74 (2H, m), -0.07 (9H, s).
MS (APCI, ESI) m/z: 619 [81Br, (M+H)+], 617 [79Br, (M+H)+] .

Step 6: (11aS)-8-(3-Bromopropoxy)-7-methoxy-2-(4-methoxyphenyl)-1, 11a-dihydro-5H-pyrrolo[2,1-c][1,4]benzodiazepin-5-one (4-6)

The compound obtained in step 5 (4-5) (2.49 g, 4.04 mmol) was reacted in the same manner as in step 7 of Example 10-3 to afford the desired compound (4-6) (1.59 g, 84%).
MS (APCI, ESI) m/z: 473 [81Br, (M+H)+], 471 [79Br, (M+H)+] .

Step 7: (11aS)-8-(3-Bromopropoxy)-7-methoxy-2-(4-methoxyphenyl)-1,10,11,11a-tetrahydro-5H-pyrrolo[2,1-c][1,4]benzodiazepin-5-one (4-7)

The compound obtained in step 6 (4-6) (1.59 g, 3.38 mmol) was reacted in the same manner as in step 8 of Example 10-3 to afford the desired compound (4-7) (1.39 g, 87%).
1H-NMR (CDCl3) δ: 7.54 (1H, s), 7.54-7.51 (1H, m), 7.32-7.29 (2H, m), 6.89-6.87 (2H, m), 6.10 (1H, s), 4.32-4.28 (2H, m), 4.14-4.13 (2H, m), 3.85 (3H, s), 3.82 (3H, s), 3.63-3.62 (2H, m), 3.57-3.55 (2H, m), 3.40-3.36 (1H, m), 2.76-2.72 (1H, m), 2.40-2.37 (2H, m).
MS (APCI, ESI) m/z: 475 [81Br, (M+H)+], 473 [79Br, (M+H)+] .

Step 8: Prop-2-en-1-yl(11aS)-8-(3-bromopropoxy)-7-methoxy-2-(4-methoxyphenyl)-5-oxo-11,11a-dihydro-1H-pyrrolo[2,1-c][1,4]benzodiazepin-10(5H)-carboxylate (4-8)

The compound obtained in step 7 (4-7) (1.40 g, 0.2.95 mmol) was reacted in the same manner as in step 9 of Example 10-3 to afford the desired compound (4-8) (0.885 g, 54%).
1H-NMR (CDCl3) δ: 7.34 (1H, s), 7.27-7.25 (2H, m), 7.22 (1H, s), 6.86-6.84 (2H, m), 6.73 (1H, s), 5.76-5.74 (1H, m), 5.11-5.09 (2H, m), 4.62-4.59 (2H, m), 4.33-4.31 (1H, m), 4.16-4.13 (3H, m), 3.88 (3H, s), 3.79 (3H, s), 3.60-3.59 (3H, m), 3.27-3.23 (1H, m), 2.69-2.65 (1H, m), 2.37-2.34 (2H, m).
MS (APCI, ESI) m/z: 559 [81Br, (M+H)+], 557 [79Br, (M+H)+] .

Step 9: N-{[(Prop-2-en-1-yl)oxy]carbonyl}-L-valyl-N-[4-({[(11'aS)-11'-{[tert-butyl(dimethyl)silyl]oxy}-7'-methoxy-8'-(3-{[(11aS)-7-methoxy-2-(4-methoxyphenyl)-5-oxo-10-{[(prop-2-en-1-yl)oxy)carbonyl}-5,10,11,11a-tetrahydro-1H-pyrrolo[2,1-c][1,4]benzodiazepin-8-yl]oxy}propoxy)-5'-oxo-11',11'a-dihydro-1'H, 3'H-spiro[cyclopropane-1,2'-pyrrolo[2,1-c][1,4]benzodiazepine]-10'(5'H)-carbonyl]oxy}methyl)phenyl]-L-alaninamide (4-9)

The compound obtained in step 8 (4-8) (0.0381 g, 0.0683 mmol) was reacted with the compound obtained in the step 10 of Example 10-1 (1-11) (0.0552 g, 0.0683 mmol) in the same manner as in step 10 of Example 10-3 to afford the desired compound (4-9) (0.0712 g, 81%).
MS (APCI, ESI) m/z: 1284 (M+H)+.

Step 10: N-{[(Prop-2-en-1-yl)oxy]carbonyl}-L-valyl-N-[4-({[(11'aS)-11'-hydroxy-7'-methoxy-8'-(3-{[(11aS)-7-methoxy-2-(4-methoxyphenyl)-5-oxo-10-{[(prop-2-en-1-yl)oxy]carbonyl}-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10' (5'H)-carbonyl]oxy}methyl)phenyl]-L-alaninamide (4-10)

The compound obtained in step 9 (4-9) (0.0712 g, 0.0554 mmol) was reacted in the same manner as in step 11 of Example 10-3 to afford the desired compound (4-10) (0.0671g, quantitative).
MS (APCI, ESI) m/z: 1170 (M+H)+.

Step 11: L-Valyl-N-[4-({[(11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-11)

The compound obtained in step 10 (4-10) (0.0571 mmol) was reacted in the same manner as in step 12 of Example 10-3 to afford the desired compound (4-11) (0.0574 g, 99%).
1H-NMR (CDCl3) δ: 9.16 (1H, s), 7.93-7.91 (1H, m), 7.55-7.52 (1H, m), 7.50-7.47 (3H, m), 7.35-7.32 (2H, m), 7.21 (1H, s), 7.13-7.11 (2H, m), 6.90-6.87 (2H, m), 6.40 (1H, s), 6.08 (1H, s), 5.90-5.87 (1H, m), 5.37-5.34 (1H, m), 4.73-4.53 (3H, m), 4.23-4.08 (5H, m), 3.89 (3H, s), 3.82 (3H, s), 3.78-3.72 (5H, m), 3.57-3.51 (3H, m), 3.38-3.30 (3H, m), 2.76-2.71 (1H, m), 2.36-2.24 (4H, m), 1.78-1.42 (6H, m), 1.00-0.98 (3H, m), 0.87-0.84 (3H, m), 0.74-0.62 (4H, m).
MS (APCI, ESI) m/z: 1002 (M+H)+.

Step 12: N-[4-(11,12-Didehydrodibenzo[b,f]azocin-5(6H)-yl)-4-oxobutanoyl]glycylglycyl-L-valyl-N-[4-({[(11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-12)

The compound obtained in step 11 (4-11) (0.189 g, 0.189 mmol) was reacted with the compound obtained in the step 1 of Example 10-2 (2-2) (0.087 g, 0.207 mmol) in the same manner as in step 13 of Example 10-3 to afford the desired compound (4-12) (0.169 g, 64%).
MS (APCI, ESI) m/z: 1402 (M+H)+.

[Example 10-5: Drug-linker 3]

[Formula 57]

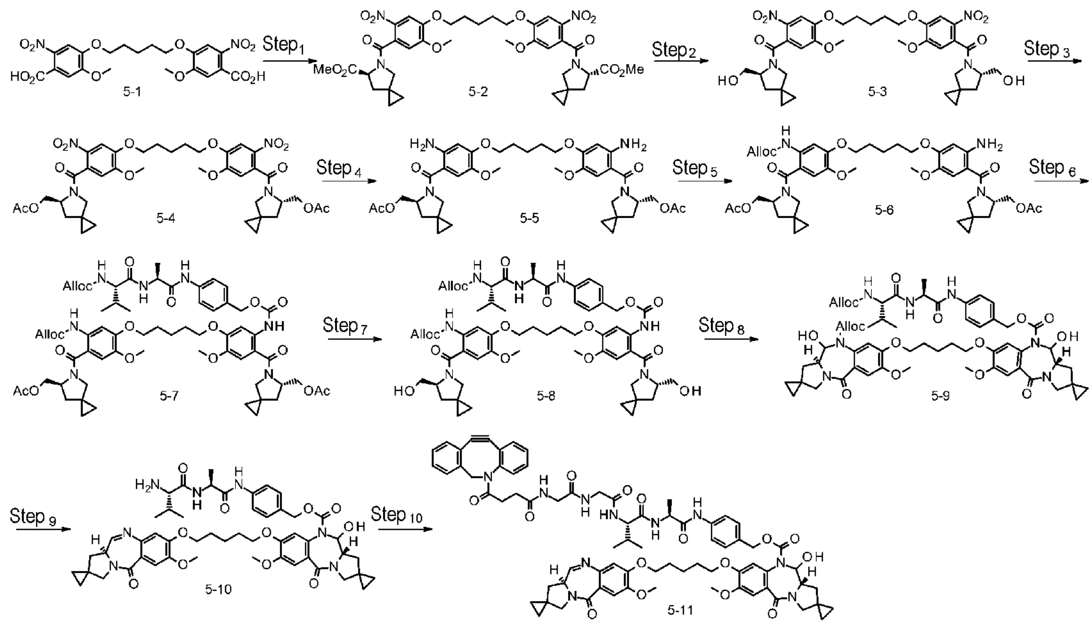


Step 1: Dimethyl(6S,6'S)-5,5'-{1,5-pentanediylbis[oxy(5-methoxy-2-nitrobenzen-4,1-diyl)carbonyl] }bis(5-azaspiro[2.4]heptane-6-carboxylate) (5-2)

To a solution of 4,4'-[1,5-pentanediylbis(oxy)]bis (5-methoxy-2-nitro benzoic acid) (5-1) (5.41 g, 10.9 mmol, Journal of Medicinal Chemistry 2004, 47, 1161) in dichloromethane (50 mL), oxalyl chloride (5.63 mL, 65.7 mmol) was added at 0°C, and N,N-dimethylformamide (0.0844 mL, 1.09 mmol) was added dropwise thereto. The temperature of the reaction solution was raised to room temperature, and the reaction solution was stirred for 2 hours. The resultant was distilled under reduced pressure, and the resulting residue was dissolved in dichloromethane (100 mL), which was added dropwise to dichloromethane solution (100 mL) of methyl (6S)-5-azaspiro[2.4]heptane-6-carboxylate hydrochloride (4.28 g, 24.1 mmol, Tetrahedron Letters 2012. 53. 3847) and triethylamine (6.07 mL, 43.8 mmol) under a nitrogen atmosphere at -40°C. The temperature of the reaction solution was raised to 0°C, and the reaction solution was stirred for 2 hours. To the reaction mixture, 1 N hydrochloric acid (100 mL) was added, and the organic layer was washed with water and brine, and dried over anhydrous sodium sulfate. The resultant was distilled under reduced pressure to afford the desired compound (5-2) (8.40 g, quantitative).
1H-NMR (DMSO-D6) $\delta$: 7.71 (2H, s), 6.88 (2H, s), 4.63 (2H, m), 4.15-4.12 (4H, m), 3.94 (6H, s), 3.71 (6H, s), 3.25 (2H, m), 3.10 (2H, m), 2.31-2.28 (2H, m), 1.90-1.83 (6H, m), 1.60-1.58 (2H, m), 0.71-0.49 (8H, m).
MS (APCI, ESI) m/z: 769 (M+H)+.

Step 2: {1,5-Pentanediylbis[oxy(5-methoxy-2-nitrobenzen-4,1-diyl)]}bis{[(6S)-6-(hydroxymethyl)-5-azaspiro[2.4]hept-5-yl]methanone} (5-3)

To a solution of the compound obtained in step 1 (5-2) (8.40 g, 10.9 mmol) in THF (100 mL), lithium borohydride (714 mg, 32.8 mmol) was added, and the resultant was stirred at 0°C for 30 minutes, and the temperature was raised to room temperature, and stirring was performed for 1 hour. After 1 N hydrochloric acid was added at 0°C, the resultant was extracted with ethyl acetate, and washed with brine, and then dried over anhydrous sodium sulfate. The solvent was distilled off under reduced pressure to afford the desired compound (5-3) (7.70 g, 99%).
1H-NMR (DMSO-D6) $\delta$: 7.67 (2H, s), 7.05 (2H, s), 4.86-4.74 (2H, m), 4.22-4.12 (6H, m), 3.92 (6H, s), 3.83-3.73 (2H, m), 3.62-3.51 (2H, m), 3.29 (1H, m), 3.11 (2H, m), 2.96 (1H, m), 2.12-2.03 (2H, m), 1.82-1.77 (6H, m), 1.59-1.56 (2H, m), 0.67-0.41 (8H, m).
MS (APCI, ESI) m/z: 713 (M+H)+.

Step 3: Pentane-1,5-diylbis[oxy(5-methoxy-2-nitrobenzen-4,1-diyl)carbonyl (6S)-5-azaspiro[2.4]heptan-5,6-diylmethanediyl] diazetate (5-4)

The compound obtained in step 2 (5-3) (7.70 g, 10.8 mmol) was dissolved in pyridine (20 mL) and acetic

anhydride (10 mL, 105.9 mmol), which was stirred at room temperature. The resultant was distilled under reduced pressure to afford the desired compound (5-4) (8.38 g, 97%) .
1H-NMR (DMSO-D6) δ: 7.68 (2H, s), 7.03 (2H, s), 4.47-4.46 (2H, m), 4.36-4.27 (4H, m), 4.13-4.11 (6H, m), 3.92 (6H, s), 3.16 (2H, m), 2.98 (2H, m), 2.17 (1H, m), 2.06 (6H, s), 1.84 (4H, m), 1.68 (1H, m), 1.58 (2H, m), 0.64-0.45 (8H, m).
MS (APCI, ESI) m/z: 797 (M+H)+.

Step 4: 1,5-Pentanediylbis[oxy(2-amino-5-methoxybenzen-4,1-diyl)carbonyl (6S)-5-azaspiro[2.4]heptan-5,6-diylmethanediyl] diacetate (5-5)

To a solution of the compound obtained in step 3 (5-4) (8.28 g, 10.4 mmol) in N,N-dimethylformamide (100 mL), 5% palladium carbon (moisture content: 54%, 1.00 g) was added, and the reaction solution was then vigorously stirred under a hydrogen atmosphere at room temperature for 6 hours. The resultant was filtered through Celite, and the filtrate was then distilled under reduced pressure, and the resulting residue was purified by silica gel column chromatography [chloroform:methanol = 100:0(v/v) to 90:10(v/v)] to afford the desired compound (5-5) (5.05 g, 66%).
1H-NMR (DMSO-D6) δ: 6.66 (2H, s), 6.36 (2H, s), 5.11 (4H, s), 4.49 (2H, s), 4.19 (4H, m), 3.90 (4H, m), 3.62 (6H, s), 3.48-3.46 (2H, m), 3.33 (2H, s), 3.23-3.20 (2H, m), 2.01 (6H, s), 1.78-1.74 (6H, m), 1.55 (2H, m), 0.61-0.58 (4H, m), 0.49-0.48 (4H, m).
MS (APCI, ESI) m/z: 737 (M+H)+.

Step 5: {(65)-5-[4-({5-[4-({(6*S*)-6-[(Acetyloxy)methyl]-5-azaspiro[2.4]hept-5-yl}carbonyl)-5-amino-2-methoxyphenoxy]pentyl}oxy)-5-methoxy-2-{ [(prop-2-en-1-yloxy)carbonyl]amino}benzoyl]-5-azaspiro[2.4]hept-6-yl}methyl acetate (monoallyloxycarbonyl form) (5-6)

To a solution of the compound obtained in step 4 (5-5) (5.05 g, 6.85 mmol) in dichloromethane (100 mL), pyridine (1.10 mL, 13.7 mmol) was added, and allyl chloroformate (0.725 mL, 6.85 mmol) was added thereto under a nitrogen atmosphere at -78°C, and the resultant was stirred for 2 hours. The resultant was distilled under reduced pressure, and the resulting residue was purified by silica gel column chromatography [hexane:ethyl acetate = 70:30 (v/v) to 100:0 (v/v), chloroform:methanol = 100:0 (v/v) to 90:10 (v/v)] to afford the bisallyloxycarbonyl form (5-6b) (1.36 g, 22%) and monoallyloxycarbonyl form (5-6) (2.63 g, 47%) as the desired compound.

Pentane-1,5-diylbis[oxy(5-methoxy-2-{[(prop-2-en-1-yloxy)carbonyl]amino}benzen-4,1-diyl) carbonyl(6*S*)-5-azaspiro[2.4]heptan-5,6-diylmethanediyl] diacetate (bisallyloxycarbonyl form) (5-6b):
1H-NMR (DMSO-D6) δ: 9.14 (2H, s), 7.14 (2H, s), 6.85 (2H, s), 5.94 (2H, m), 5.33 (2H, m), 5.21 (2H, m), 4.55 (4H, m), 4.47 (1H, s), 4.23 (3H, s), 3.96 (4H, m), 3.74 (6H, s), 3.34 (6H, s), 3.31 (2H, m), 3.21 (2H, m), 2.04 (6H, s), 1.79 (4H, m), 1.67 (2H, m), 1.56 (2H, m), 0.56-0.48 (8H, m).
MS (APCI, ESI) m/z: 905 (M+H)+. Monoallyloxycarbonyl form (5-6): 1H-NMR (DMSO-D6) δ: 9.14 (1H, s), 7.14 (1H, s), 6.85 (1H, s), 6.65 (1H, s), 6.35 (1H, s), 5.95 (1H, m), 5.33 (1H, m), 5.22 (1H, m), 5.11 (2H, s), 4.55 (2H, m), 4.48 (2H, s), 4.23-4.14 (4H, m), 3.96 (2H, m), 3.90 (2H, m), 3.74 (3H, s), 3.63 (3H, s), 3.49 (1H, m), 3.38-3.30 (4H, m), 3.21 (1H, m), 2.04 (3H, s), 2.01 (3H, s), 1.77 (5H, m), 1.68 (1H, m), 1.56 (2H, m), 0.63-0.48 (8H, m).
MS (APCI, ESI) m/z: 821 (M+H)+.

Step 6: N-[(2-Propen-1-yloxy)carbonyl]-L-valyl-N-{4-[({[2-({(6S)-6-[(acetyloxy)methyl]-5-azaspiro[2.4]hept-5-yl}carbonyl)-5-({5-[4-({(6S)-6-[(acetyloxy)methyl]-5-azaspiro[2.4]hept-5-yl}carbonyl)-2-methoxy-5-{[(2-propen-1-yloxy)carbonyl]amino}phenoxy]pentyl}oxy)-4-methoxyphenyl]carbamoyl}oxy)methyl]phenyl}-L-alaninamide (5-7)

The monoallyloxycarbonyl form obtained in step 5 (5-6) (2.00 g, 2.44 mmol) was reacted with N-[(prop-2-en-1-yloxy)carbonyl]-L-valyl-N-[4-(hydroxymethyl)phenyl]-L-alaninamide (1.10 g, 2.92 mmol, WO2011130598) in the same manner as in step 6 of Example 10-1 to afford the desired compound (5-7) (2.64 g, 89%).
1H-NMR (DMSO-D6) δ: 10.02 (1H, s), 9.14 (2H, s), 8.18 (1H, m), 7.59 (2H, m), 7.33 (2H, m), 7.27 (1H, m), 7.14 (2H, s), 6.85 (2H, s), 5.99-5.86 (2H, m), 5.31 (2H, n), 5.19 (2H, m), 5.03 (2H, s), 4.55 (2H, m), 4.48 (2H, n), 4.41 (2H, m), 4.23-4.21 (3H, m), 3.94-3.91 (4H, m), 3.88-3.86 (2H, m), 3.74 (3H, s), 3.74 (3H, s), 3.34 (4H, s), 3.32-3.30 (2H, m), 3.20-3.18 (2H, m), 2.03 (6H, s), 1.96 (1H, m), 1.79 (4H, s), 1.66 (1H, m), 1.55 (2H, s), 1.30 (3H, m), 0.88 (3H, m), 0.83 (3H, m), 0.54-0.49 (8H, m). MS (APCI, ESI) m/z: 1224 (M+H)+.

Step 7: N-[(2-Propen-1-yloxy)carbonyl]-L-valyl-N-[4-({[(2-{[(6S)-6-(hydroxymethyl)-5-azaspiro[2.4]hept-5-yl]carbonyl}-5-{[5-(4-{[(6S)-6-(hydroxymethyl)-5-azaspiro[2.4]hept-5-yl]carbonyl}-2-methoxy-5-{[(2-propen-1-yloxy)carbonyl]amino}phenoxy)pentyl]oxy}-4-methoxyphenyl)carbamoyl]oxy}methyl)phenyl]-L-alaninamide (5-8)

To a solution of the compound obtained in step 6 (5-7) (2.64 g, 2.16 mmol) in methanol (10 mL), potassium

carbonate (1.49 g, 10.8 mmol) was added, and the resultant was stirred at room temperature for 3 hours. A saturated aqueous ammonium chloride (100 mL) was added to the reaction mixture, which was extracted with ethyl acetate. The organic layer was dried over anhydrous sodium sulfate. The resultant was distilled under reduced pressure to afford the desired compound (5-8) (2.21 g, 90%).
1H-NMR (DMSO-D6) δ: 10.04 (1H, s), 9.18 (1H, s), 8.18 (1H, m), 7.59 (2H, m), 7.33 (2H, m), 7.26 (1H, m), 7.22 (1H, s), 7.14 (2H, s), 6.89 (2H, s), 5.98-5.86 (2H, m), 5.31 (2H, m), 5.19 (2H, m), 5.04 (2H, s), 4.80 (2H, m), 4.55 (2H, m), 4.48 (2H, m), 4.41 (1H, m), 4.26 (2H, s), 3.96-3.94 (4H, m), 3.90-3.85 (1H, m), 3.74 (6H, s), 3.59 (2H, m), 3.33 (6H, s), 3.09 (1H, m), 1.93-1.83 (8H, m), 1.57-1.54 (2H, m), 1.30 (3H, m), 0.88 (3H, m), 0.83 (3H, m), 0.52-0.43 (8H, m).
MS (APCI, ESI) m/z: 1140 (M+H)+.

Step 8: N-[(2-Propen-1-yloxy)carbonyl]-L-valyl-N-{4-[({[(11a'S)-11'-hydroxy-8'-{[5-({(11a'S)-11'-hydroxy-7'-methoxy-5'-oxo-10'-[(2-propen-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-7'-methoxy-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (5-9)

To a solution of the compound obtained in step 7 (5-8) (2.03 g, 1.78 mmol) in dichloromethane (50 mL), Dess-Martin periodinane (1.59 g, 3.74 mmol) was added, and the resultant was stirred at room temperature overnight. A saturated aqueous sodium hydrogen carbonate (100 mL) was added to the reaction mixture, which was extracted with chloroform. The organic layer was dried over anhydrous sodium sulfate. The resultant was distilled under reduced pressure, and the resulting residue was purified by silica gel column chromatography [chloroform:methanol = 100:0(v/v) to 90:10(v/v)] to afford the desired compound (5-9) (2.05 g, quantitative).
1H-NMR (DMSO-D6) δ: 9.99 (1H, s), 8.16 (1H, m), 7.54 (2H, m), 7.32-7.22 (3H, m), 7.08-7.04 (2H, m), 6.80-6.72 (2H, m), 6.55 (2H, s), 5.94-5.86 (2H, m), 5.75 (2H, m), 5.31-5.04 (2H, m), 4.81 (1H, m), 4.62 (1H, m), 4.48-4.38 (4H, m), 4.00-3.87 (4H, m), 3.79-3.76 (7H, m), 3.54 (2H, m), 3.42-3.40 (2H, m), 3.33 (4H, s), 3.14 (2H, m), 2.35 (2H, m), 1.80-1.78 (4H, m), 1.59-1.56 (4H, m), 1.29 (3H, m), 0.87 (3H, m), 0.83 (3H, m), 0.70-0.59 (8H, m).
MS (APCI, ESI) m/z: 1136 (M+H)+.

Step 9: L-Valyl-N-{4-[({[(11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (5-10)

The compound obtained in step 8 (5-9) (2.05 g, 1.80 mmol) was reacted in the same manner as in step 12 of Example 10-3 to afford the desired compound (5-10) (1.02 g, 60%).
1H-NMR (DMSO-D6) δ: 10.08 (1H, s), 7.57 (2H, m), 7.32-7.20 (3H, m), 7.05 (2H, s), 6.68-6.60 (3H, m), 5.74 (1H, m), 4.99-4.58 (4H, m), 3.99-3.94 (4H, m), 3.78-3.73 (6H, m), 3.66-3.38 (4H, m), 3.15-3.01 (3H, m), 2.40-2.34 (3H, m), 1.89-1.81 (6H, m), 1.57-1.53 (4H, m), 1.28 (3H, m), 0.88 (3H, m), 0.78 (3H, m), 0.64-0.55 (8H, m).
MS (APCI, ESI) m/z: 950 (M+H)+.

Step 10: N-[4-(11,12-Didehydrodibenzo[b,f]azocin-5(6H)-yl)-4-oxobutanoyl]glycylglycyl-L-valyl-N-{4-[({[(11a'S)-11'-hydroxy-7'-methoxy-8'-[(5-{[(11a'S)-7'-methoxy-5'-oxo-5', 11a'-dihydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (5-11)

The compound obtained in step 9 (5-10) (0.710 g, 0.747 mmol) and the compound obtained in step 1 of Example 10-2 (2-2) (0.313 g, 0.747 mmol) were dissolved in mixed solvent of dichloromethane (1.5 mL) and methanol (0.1 mL). Thereto, 4-(4,6-dimethoxy-1,3,5-triazin-2-yl)-4-methylmorpholinium chloride (0.264 g, 0.897 mmol) was added, and the resultant was stirred at room temperature for 1 hour. The resultant was distilled under reduced pressure, and the resulting residue was purified by silica gel column chromatography [chloroform:methanol = 100:0 (v/v) to 80:20 (v/v)] to afford the desired compound (5-11) (0.671 g, 66%).
1H-NMR (DMSO-D6) δ: 9.91 (1H, s), 8.32 (1H, s), 8.23-7.91 (3H, m), 7.81-7.19 (14H, m), 7.04 (1H, m), 6.80-6.62 (3H, m), 5.77-5.75 (1H, m), 5.20 (1H, m), 5.01 (1H, m), 4.79 (1H, m), 4.46-4.35 (1H, m), 4.04 (4H, m), 3.86-3.38 (18H, m), 3.22-3.15 (2H, m), 2.67-2.63 (1H, m), 2.46-2.23 (3H, m), 2.09-1.91 (2H, m), 1.80-1.78 (5H, m), 1.57 (3H, m), 1.27 (3H, s), 1.11-1.04 (1H, m), 0.87-0.79 (6H, m), 0.63-0.55 (6H, m).
MS (APCI, ESI) m/z: 1351 (M+H)+.

[Example 10-6: Drug-linker 4]

[Formula 58]

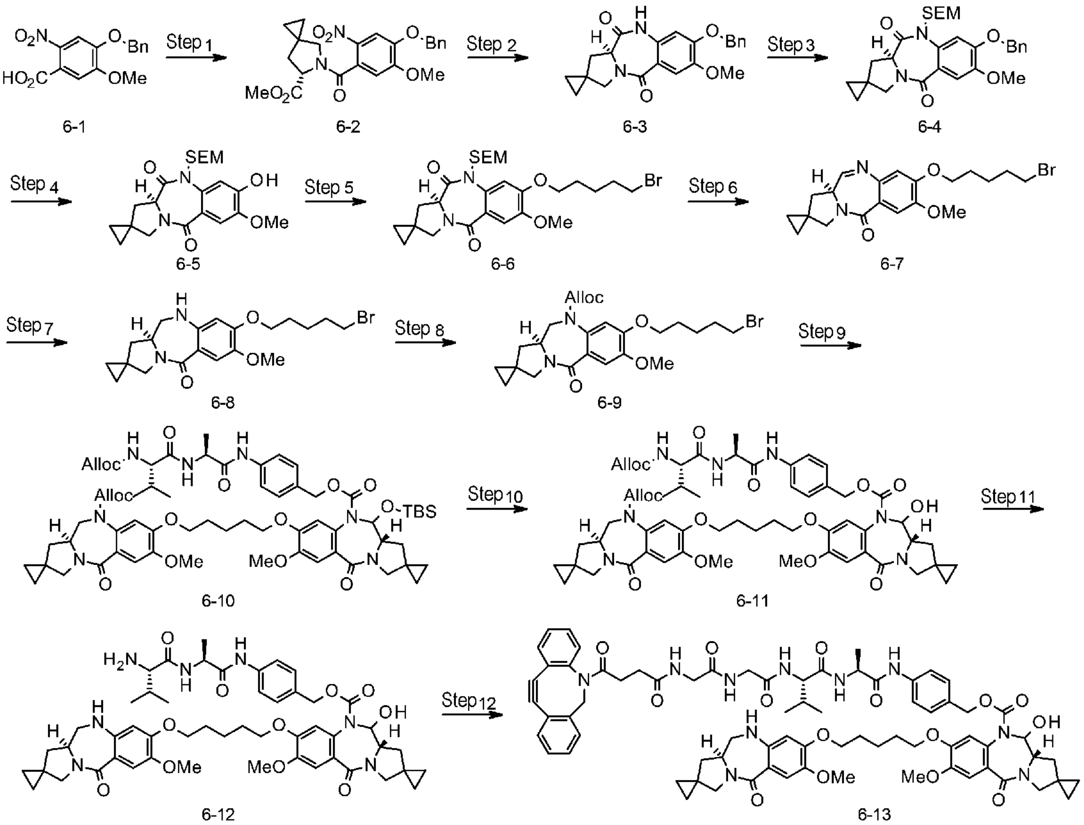


6-1, 6-2, 6-3, 6-4, 6-5, 6-6, 6-7, 6-8, 6-9, 6-10, 6-11, 6-12, 6-13

Step 1: Methyl(6S)-5-[4-(benzyloxy)-5-methoxy-2-nitrobenzoyl]-5-azaspiro[2.4]heptane-6-carboxylate (6-2)

To a solution of 4-(benzyloxy)-5-methoxy-2-nitrobenzoic acid (6-1) (6.07 g, 20.0 mmol, Tetrahedron 1995, 51, 5617) and N,N-dimethylformamide (1.08 mL, 13.9 mmol) in dichloromethane (100 mL), oxalyl chloride (3.43 mL, 40.0 mmol) was added dropwise under ice-cooling over 5 minutes. The reaction solution was stirred at room temperature for 5 hours, and then distilled under reduced pressure, and the resulting residue was dissolved in dichloromethane (20 mL), which was distilled under reduced pressure. After this operation was repeated three times, the residue was suspended in dichloromethane (5 mL), to which excess amounts of diethyl ether and hexane were added, and the following filtration and drying under reduced pressure afforded the crude acyl chloride. The acyl chloride obtained was dissolved in dichloromethane and cooled to -40°C (dry ice-acetonitrile bath), to which methyl (6S)-5-azaspiro[2.4]heptane-6-carboxylate hydrochloride (4.22 g, 22.0 mmol, Tetrahedron Letters 2012. 53. 3847) and triethylamine (3.36 mL, 24.2 mmol) were slowly added. The temperature of the reaction mixture was raised to room temperature overnight. To the reaction mixture, 1 N hydrochloric acid was added, and the reaction mixture was extracted with dichloromethane. The organic layer was washed with water, a saturated aqueous sodium hydrogen carbonate, and brine, and dried over anhydrous sodium sulfate. The resultant was distilled under reduced pressure, and the resulting residue was purified by silica gel column chromatography [hexane:ethyl acetate = 100:0 to 50:50] to afford the desired compound (6-2) (6.55 g, 80%).
MS (APCI, ESI) m/z: 441 (M+H)+

Step 2: (11a'S)-8'-(Benzyloxy)-7'-methoxy-1'H-spiro[cyclopropane-1,2'-pyrrolo[2,1-c][1,4]benzodiazepine]-511'(10'H,1la'H)-dione (6-3)

To a solution of the compound obtained in step 1 (6-2) (6.55 g, 16.0 mmol) in ethanol (150 mL) and THF (150 mL), Raney nickel (7.00 g) was added under a nitrogen atmosphere. Hydrazine monohydrate (7 mL) was added to the reaction mixture, and the temperature was gradually raised to 50°C. After stirring at 50°C for 2 hours, Raney nickel (3.00 g) and hydrazine monohydrate (3 mL) were added thereto, and the resultant was stirred for 1 hour. THF (100 mL) was added to the reaction mixture, which was filtered through Celite. The resultant was distilled under reduced pressure, and the resulting residue was purified by silica gel column chromatography [hexane:ethyl acetate = 100:0(v/v) to 25:75(v/v)] to afford the desired compound (6-3) (4.42 g, 73%).

1H-NMR (CDCl3) δ: 7.82 (1H, s), 7.48 (1H, s), 7.42-7.35 (4H, m), 7.32-7.31 (1H, m), 6.44 (1H, s), 5.16 (2H, s), 4.16-4.10 (1H, m), 3.93 (3H, s), 3.78-3.76 (1H, m), 3.39-3.37 (1H, m), 2.45-2.43 (1H, m), 2.24-2.21 (1H, m), 0.83-0.61 (4H, m).
MS (APCI, ESI) m/z: 379 (M+H)+

Step 3: (11a'S)-8'-(Benzyloxy)-7'-methoxy-10'-{[2-(trimethylsilyl)ethoxy]methyl}-1'H-spiro[cyclopropane-1,2'-pyrrolo[2,1-c][1,4]benzodiazepine]-5',11'(10'H,11a'H)-dione (6-4)

To a solution of the compound obtained in step 2 (6-3) (10.0 g, 26.4 mmol) in THF (150 mL), 2.6 mol/L normalhexane solution of normal-butyllithium (12.0 mL, 31.8 mmol) was added slowly dropwise at -40°C. The reaction solution was stirred at -40°C for 15 minutes, and 2-(chloromethoxy)ethyltrimethylsilane (5.57 mL, 31.7 mmol) was then added slowly dropwise thereto. After the reaction solution was stirred at room temperature for 3 hours, water was added thereto, and the resultant was extracted with ethyl acetate. The organic layer was washed with water and brine, and dried over anhydrous sodium sulfate. After distillation under reduced pressure, the resulting residue was purified by silica gel column chromatography [hexane:ethyl acetate = 100:0(v/v) to 30:70(v/v)] to afford the desired compound (6-4) (11.8 g, 88%).
1H-NMR (CDCl3) δ: 7.45-7.44 (2H, m), 7.37-7.32 (4H, m), 7.28 (1H, s), 5.48-5.46 (1H, m), 5.21 (2H, s), 4.50-4.48 (1H, m), 4.22-4.20 (1H, m), 3.95 (3H, s), 3.73-3.70 (2H, m), 3.62-3.60 (1H, m), 3.41-3.38 (1H, m), 2.45-2.43 (1H, m), 2.23-2.20 (1H, m), 0.98-0.96 (2H, m), 0.83-0.68 (4H, m), 0.04 (9H, s).
MS (APCI, ESI) m/z: 509 (M+H)+

Step 4: (11a'S)-8'-Hydroxy-7'-methoxy-10'-{[2-(trimethylsilyl)ethoxy]methyl}-1'H-spiro[cyclopropane-1,2'-pyrrolo[2,1-c][1,4]benzodiazepine]-5',11'(10'H,11a'H)-dione (6-5)

To a solution of the compound obtained in step 3 (6-4) (18.7 g, 36.8 mmol) in THF (50 mL) and ethanol (100 mL), 5% palladium carbon catalyst (5.00 g) was added under a nitrogen atmosphere. The nitrogen balloon was immediately replaced with a hydrogen balloon, and the reaction mixture was stirred under a hydrogen atmosphere for 6 hours. The reaction mixture was diluted by addition of chloroform and filtered through Celite, and the filtrate was then distilled under reduced pressure, and the resulting residue was purified by silica gel column chromatography [hexane:ethyl acetate = 100:0(v/v) to 25:75(v/v)] to afford the desired compound (6-5) (15.1 g, 98%).
1H-NMR (CDCl3) δ: 7.38 (1H, s), 7.28 (1H, s), 6.01 (1H, s), 5.49-5.47 (1H, m), 4.70-4.68 (1H, m), 4.24-4.22 (1H, m), 3.96 (3H, s), 3.76-3.71 (2H, m), 3.66-3.64 (1H, m), 3.42-3.39 (1H, m), 2.47-2.45 (1H, m), 2.23-2.21 (1H, m), 1.01-0.99 (2H, m), 0.89-0.63 (4H, m), 0.03 (9H, s).
MS (APCI, ESI) m/z: 419 (M+H)+

Step 5: (11a'S)-8'-[(5-Bromopentyl)oxy]-7'-methoxy-10'-{[2-(trimethylsilyl)ethoxy]methyl}-1'H-spiro[cyclopropane-1,2'-pyrrolo[2,1-c][1,4]benzodiazepine]-5',11'(10'H,11a'H)-dione (6-6)

The compound obtained in step 4 (6-5) (2.77 g, 6.62 mmol) was reacted in the same manner as in step 2 of Example 10-3 to afford the desired compound (6-6) (3.31 g, 88%).
1H-NMR (CDCl3) δ: 7.36 (1H, s), 7.25 (1H, s), 5.55 (1H, m), 4.65 (1H, m), 4.24-4.23 (1H, m), 4.11-4.03 (2H, m), 3.93 (3H, s), 3.85-3.78 (1H, m), 3.72-3.69 (2H, m), 3.46-3.39 (3H, m), 2.47-2.44 (1H, m), 2.25-2.22 (1H, m), 1.95-1.91 (4H, m), 1.67-1.59 (1H, m), 1.03-0.95 (2H, m), 0.90-0.85 (1H, m), 0.70-0.66 (4H, m), 0.05 (9H, s).

Step 6: (11a'S)-8'-[(5-Bromopentyl)oxy]-7'-methoxy-1',11a'-dihydro-5'H-spiro[cyclopropane-1,2'-pyrrolo[2,1-c][1,4]benzodiazepine]-5'-one (6-7)

The compound obtained in step 5 (6-6) (3.31 g, 5.83 mmol) was reacted in the same manner as in step 7 of Example 10-3 to afford the desired compound (6-7) (1.11 g, 45%).
1H-NMR (CDCl3) δ: 7.81 (1H, m), 7.53 (1H, s), 6.82 (1H, s), 4.13-4.06 (2H, m), 3.97 (3H, s), 3.88-3.83 (1H, m), 3.69 (1H, m), 3.52-3.39 (3H, m), 2.55-2.52 (1H, m), 2.06-1.89 (5H, m), 1.67-1.63 (2H, m), 0.76-0.72 (4H, m).

Step 7: (11a'S)-8'-[(5-Bromopentyl)oxy]-7'-methoxy-1',10',11',11a'-tetrahydro-5'H-spiro[cyclopropane-1,2'-pyrrolo[2,1-c][1,4]benzodiazepine]-5'-one (6-8)

The compound obtained in step 6 (6-7) (2.56 g, 6.08 mmol) was reacted in the same manner as in step 8 of Example 10-3 to afford the desired compound (6-8) (1.15 g, 45%).
1H-NMR (CDCl3) δ: 7.60 (1H, s), 6.07 (1H, s), 4.11-4.04 (1H, m), 3.99 (2H, m), 3.87-3.84 (1H, m), 3.85 (3H, s), 3.73 (1H, m), 3.58-3.53 (2H, m), 3.47-3.42 (3H, m), 2.03-1.78 (6H, m), 1.65-1.63 (2H, m), 0.77-0.56 (4H, m).

Step 8: Prop-2-en-1-yl(11a'S)-8'-[(5-bromopentyl)oxy]-7'-methoxy-5'-oxo-11', 11a'-dihydro-1'H-spiro[cyclopropane-1,2'-pyrrolo[2,1-c][1,4]benzodiazepine]-10'(5'H)-carboxylate (6-9)

The compound obtained in step 7 (6-8) (1.15 g, 2.72 mmol) was reacted in the same manner as in step 9 of Example 10-3 to afford the desired compound (6-9) (1.14 g, 82%).
1H-NMR (CDCl3) δ: 7.23 (1H, s), 6.69 (1H, s), 5.79 (1H, s), 5.13-5.10 (2H, m), 4.68-4.66 (1H, m), 4.48-4.45 (2H, m), 4.01 (2H, m), 3.92 (3H, s), 3.76 (1H, m), 3.54-3.37 (3H, m), 2.39 (1H, m), 1.95-1.90 (4H, m), 1.68-1.61 (3H, m), 1.44 (1H, m), 0.75-0.66 (4H, m).

Step 9: N-[(Prop-2-en-1-yloxy)carbonyl]-L-valyl-N-{4-[({[(11a'S)-11'-{[tert-butyl(dimethyl)silyl]oxy}-7'-methoxy-8'-{ [5-({(11a'S)-7'-methoxy-5'-oxo-10'-[(prop-2-en-1-yloxy)carbonyl]-5',10',11',11a'-tetrahydro'H-spiro[cyclopropane-1,2'-pyrrolo[2,1-c][1,4]benzodiazepine]-8'-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-10)

The compound obtained in step 8 (6-9) (0.374 g, 0.737 mmol) was reacted with the compound obtained in step 10 of Example 10-1 (1-11) (0.452 g, 0.56 mmol) in the same manner as in step 10 of Example 10-3 to afford the desired compound (6-10) (0.589 g, 65%).
MS (APCI, ESI) m/z: 1234 (M+H)+

Step 10: N-[(Prop-2-en-1-yloxy)carbonyl]-L-valyl-N-{4-[({[(11a'S)-11'-hydroxy-7'-methoxy-8'-{[5-({(11a'S)-7'-methoxy-5'-oxo-10'-[(prop-2-en-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-11)

The compound obtained in step 9 (6-10) (0.589 g, 0.477 mmol) was reacted in the same manner as in step 11 of Example 10-3 to afford the desired compound (6-11) (0.382 g, 71%).
1H-NMR (CDCl3) δ: 8.90 (1H, s), 7.55 (2H, m), 7.25-7.21 (2H, m), 6.74 (2H, m), 6.38 (1H, s), 5.90-5.87 (5H, m), 5.33-5.09 (8H, m), 4.66-4.60 (8H, m), 3.98-3.91 (10H, m), 3.77-3.30 (12H, m), 2.42-2.36 (2H, m), 1.77-1.39 (6H, m), 0.91-0.70 (14H, m).

Step 11: L-Valyl-N-{4-[({[(11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-12)

The compound obtained in step 10 (6-11) (0.382 g, 0.341 mmol) was reacted in the same manner as in step 12 of Example 10-3 to afford the desired compound (6-12) (0.200 g, 62%).
MS (APCI, ESI) m/z: 952 (M+H)+

Step 12: N-[4-(11,12-Didehydrodibenzo[b,f]azocin-5(6H)-yl)-4-oxobutanoyl]glycylglycyl-L-valyl-N-{4-[({[(11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-13)

The compound obtained in step 11 (6-12) (0.0560 g, 0.0588 mmol) was reacted with the compound obtained in step 1 of Example 10-2 (2-2) (0.022 g, 0.053 mmol) in the same manner as in step 13 of Example 10-3 to afford the desired compound (6-13) (0.0500 g, 63%).
MS (APCI, ESI) m/z: 1354 (M+H)+

[Synthesis of drug moiety]

[Example 10-7: Drug 1]

[Formula 59]

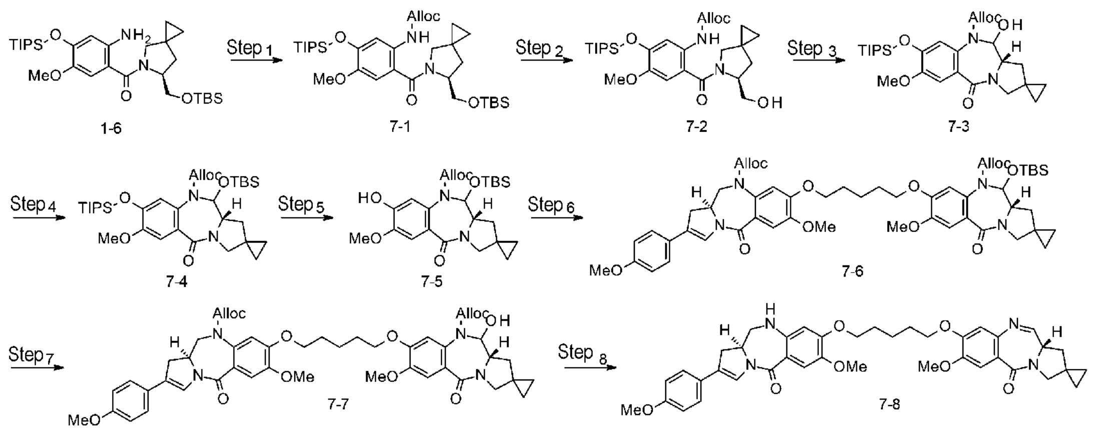


Step 1: Prop-2-en-1-yl(2-{[(6S)-6-({[tert-butyl(dimethyl)silyl]oxy}methyl)-5-azaspiro[2.4]hept-5 - yl]carbonyl}-4-methoxy-5-{[tri(propan-2-yl)silyl]oxy}phenyl)carbamate (7-1)

The compound obtained in step 5 of Example 1 (1-6) (4.59 g, 8.15 mmol) was reacted in the same manner as in step 9 of Example 10-3 to afford the desired compound (7-1) (4.86 g, 92%).
1H-NMR (CDCl3) δ: 8.97 (1H, s), 7.77 (1H, s), 6.77 (1H, s), 5.97-5.94 (1H, m), 5.39-5.21 (2H, m), 4.67-4.59 (3H, m), 4.00-3.98 (1H, m), 3.74-3.66 (5H, m), 3.05-3.03 (1H, m), 2.30-2.28 (1H, m), 1.72-1.70 (1H, m), 1.30-1.27 (3H, m), 1.11-1.05 (18H, m), 0.99-0.91 (9H, m), 0.61-0.53 (4H, m), 0.10-0.06 (6H, m).MS (APCI, ESI) m/z: 647 (M+H)+

Step 2: Prop-2-en-1-yl(2-{[(6S)-6-(hydroxymethyl)-5-azaspiro[2.4]hept-5-yl]carbonyl}-4-methoxy-5-{[tri(propan-2-yl)silyl]oxy}phenyl)carbamate (7-2)

The compound obtained in step 1 (7-1) (4.86 g, 7.51 mmol) was reacted in the same manner as in step 7 of Example 10-1 to afford the desired compound (7-2) (3.42 g, 86%).
1H-NMR (CDCl3) δ: 8.52 (1H, s), 7.71 (1H, s), 6.77 (1H, s), 6.00-5.94 (1H, m), 5.35-5.27 (2H, m), 4.65-4.64 (3H, m), 4.33-4.31 (1H, m), 3.82-3.77 (5H, m), 3.68-3.66 (1H, m), 3.15-3.13 (1H, m), 1.89-1.86 (2H, m), 1.30-1.26 (3H, m), 1.14-1.10 (18H, m), 0.66-0.51 (4H, m). MS (APCI, ESI) m/z: 533 (M+H)+

Step 3: Prop-2-en-1-yl(11a'S)-11'-hydroxy-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10'(5'H)-carboxylate (7-3)

The compound obtained in step 2 (7-2) (6.68 g, 12.5 mmol) was reacted in the same manner as in step 8 of Example 10-1 to afford the desired compound (7-3) (6.44 g, 97%).
1H-NMR (CDCl3) δ: 7.20 (1H, s), 6.69 (1H, s), 5.89-5.78 (2H, m), 5.18-5.15 (2H, m), 4.62-4.60 (1H, m), 4.49-4.47 (1H, m), 3.85 (3H, s), 3.74-3.71 (1H, m), 3.59-3.57 (1H, m), 3.33-3.30 (2H, m), 2.43-2.40 (1H, m), 1.76-1.73 (1H, m), 1.28-1.20 (3H, m), 1.09-1.07 (18H, m), 0.74-0.65 (4H, m).
MS (APCI, ESI) m/z: 531 (M+H)+

Step 4: Prop-2-en-1-yl(11a'S)-11'-{[tert-butyl(dimethyl)silyl]oxy}-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10'(5'H)-carboxylate (7-4)

The compound obtained in step 3 (7-3) (3.24 g, 6.10 mmol) was reacted in the same manner as in step 9 of Example 10-1 to afford the desired compound (7-4) (3.86 g, 98%).
1H-NMR (CDCl3) δ: 7.20 (1H, s), 6.67 (1H, s), 6.01-5.98 (1H, m), 5.79-5.73 (1H, m), 5.14-5.10 (2H, m), 4.64-4.61 (1H, m), 4.37-4.34 (1H, m), 3.86 (3H, s), 3.72-3.69 (1H, m), 3.52-3.50 (1H, m), 3.29-3.26 (1H, m), 2.38-2.34 (1H, m), 1.55-1.51 (1H, m), 1.28-1.24 (3H, m), 1.15-1.07 (18H, m), 0.81-0.66 (13H, m), 0.21 (3H, s), 0.18 (3H, s).
MS (APCI, ESI) m/z: 645 (M+H)+

Step 5: Prop-2-en-1-yl(11a'S)-11'-{[tert-butyl(dimethyl)silyl]oxy}-8'-hydroxy-7'-methoxy-5'-oxo - 11',11a'-dihydro-1'H-spiro[cyclopropane-1,2'-pyrrolo[2,1-c][1,4]benzodiazepine]-10'(5'H)-carboxylate (7-5)

The compound obtained in step 4 (7-4) (4.49 g, 6.96 mmol) was reacted in the same manner as in step 10 of Example 10-1 to afford the desired compound (7-5) (3.24 g, 95%).
1H-NMR ($CDCl_3$) δ: 7.25 (1H, s), 6.73 (1H, s), 6.02-6.00 (1H, m), 5.91 (1H, s), 5.77-5.75 (1H, m), 5.11-5.09 (2H, m), 4.64-4.62 (1H, m), 4.41-4.40 (1H, m), 3.95 (3H, s), 3.72-3.70 (1H, m), 3.54-3.53 (1H, m), 3.29-3.26 (1H, m), 2.36-2.34 (1H, m), 1.56-1.54 (1H, m), 0.79-0.67 (13H, m), 0.21 (3H, s), 0.20 (3H, s).
MS (APCI, ESI) m/z: 489 (M+H)+

Step 6: Prop-2-en-1-yl(11a'S)-11'-{[tert-butyl(dimethyl)silyl]oxy}-7'-methoxy-8'-{[5-({(11aS)-7-methoxy-2-(4-methoxyphenyl)-5-oxo-10-[(prop-2-en-1-yloxy)carbonylJ-5,10,11,11a-tetrahydro-1H-pyrrolo[2,1-c][1,4]benzodiazepin-8-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-carboxylate (7-6)

The compound obtained in step 5 (7-5) (0.080 g, 0.164 mmol) was reacted with the compound obtained in step 9 of Example 10-3 (3-10) (0.095 g, 0.163 mmol) in the same manner as in step 10 of Example 10-3 to afford the desired compound (7-6) (0.160 g, 98%).
1H-NMR (DMSO-D6) δ: 7.44-7.42 (3H, m), 7.12-7.10 (2H, m), 7.05-7.03 (1H, m), 6.92-6.90 (2H, m), 6.61-6.59 (1H, m), 5.87-5.81 (3H, m), 5.10-5.07 (4H, m), 4.66-4.55 (3H, m), 4.43-4.39 (2H, m), 4.21-3.94 (5H, m), 3.83 (3H, s), 3.81 (3H, s), 3.76 (3H, s), 3.65-3.62 (1H, m), 3.56-3.54 (1H, m), 3.42-3.39 (1H, m), 3.22-3.14 (2H, m), 2.77-2.73 (1H, m), 2.42-2.33 (1H, m), 1.81-1.79 (4H, m), 1.55-1.44 (3H, m), 0.82 (9H, s), 0.72-0.53 (4H, m), 0.19 (3H, s), 0.17 (3H, s).
MS (APCI, ESI) m/z: 993 (M+H)+

Step 7: Prop-2-en-1-yl(11a'S)-11'-hydroxy-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c][1,4]benzodiazepin-8-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-carboxylate (7-7)

The compound obtained in step 6 (7-6) (160 mg, 0.161 mmol) was reacted in the same manner as in step 11 of Example 10-3 to afford the desired compound (7-7) (141 mg, quantitative).
1H-NMR (DMSO-D6) δ: 7.44-7.42 (3H, m), 7.08-7.06 (3H, m), 6.92-6.90 (2H, m), 6.82-6.79 (1H, m), 6.56-6.54 (1H, m), 5.77-5.74 (3H, m), 5.09 (4H, s), 4.58-4.55 (3H, m), 4.43-4.41 (2H, m), 4.16-4.01 (5H, m), 3.81-3.81 (6H, m), 3.76 (3H, s), 3.64 (1H, s), 3.56-3.53 (1H, m), 3.42-3.38 (1H, m), 3.25-3.13 (2H, m), 2.74-2.70 (1H, m), 2.37-2.34 (1H, m), 1.82-1.79 (4H, m), 1.59-1.56 (3H, m), 0.66-0.62 (4H, m).
MS (APCI, ESI) m/z: 879 (M+H)+

Step 8: (11a'S)-7'-Methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-1',11a'-dihydro-5'H-spiro[cyclopropane-1,2'-pyrrolo[2,1-c][1,4]benzodiazepine]-5'-one (7-8)

The compound obtained in step 7 (7-7) (141 mg, 0.161 mmol) was reacted in the same manner as in step 12 of Example 10-3 to afford the desired compound (7-8) (109.8 mg, 99%).
1H-NMR (DMSO-D6) δ: 7.92-7.91 (1H, m), 7.45 (1H, s), 7.39-7.37 (2H, m), 7.33 (1H, s), 7.29 (1H, s), 6.92-6.89 (2H, m), 6.85 (1H, s), 6.56-6.54 (1H, m), 6.31 (1H, s), 4.19-4.12 (2H, m), 4.05-3.99 (1H, m), 3.95-3.93 (2H, m), 3.82-3.79 (4H, m), 3.76 (3H, s), 3.66 (3H, s), 3.52-3.46 (3H, m), 3.30-3.21 (2H, m), 2.78-2.74 (1H, m), 2.45-2.42 (1H, m), 2.06-2.05 (1H, m), 1.89-1.82 (4H, m), 1.60-1.58 (2H, m), 0.80-0.63 (4H, m).
MS (APCI, ESI) m/z: 693 (M+H)+

[Example 10-8: Drug 2]

[Formula 60]

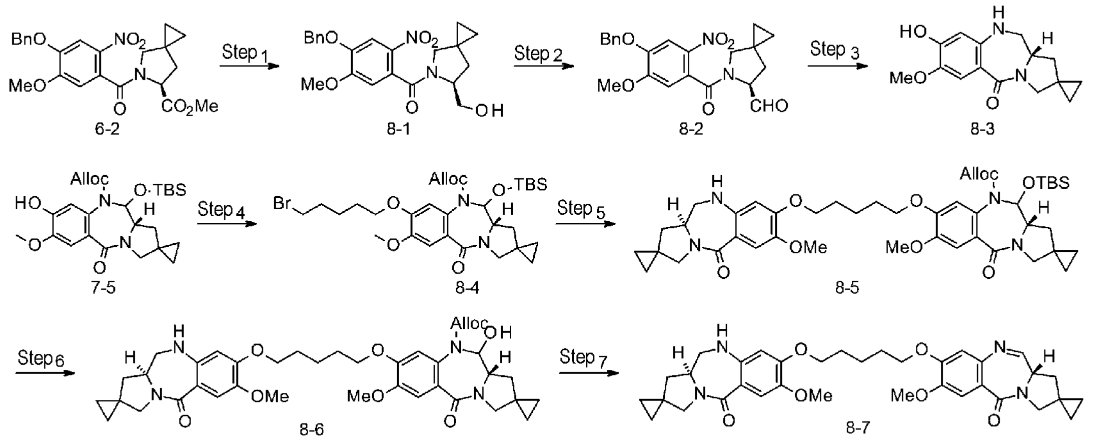


Step 1: [4-(Benzyl)-5-methoxy-2-nitrophenyl][(6S)-6-(hydroxymethyl)-5-azaspiro[2.4]hept-5-yl]methanone (8-1)

To a solution of the compound obtained in step 1 of Example 6 (6-2) (6.49 g, 14.7 mmol) in tetrahydrofuran (147 mL), lithium borohydride (0.642 g, 29.5 mmol) was added at 0°C, and the resultant was stirred at room temperature for 2 hours. To the reaction solution, 1 N hydrochloric acid was added, which was extracted with ethyl acetate. The organic layer obtained was washed with brine and dried over magnesium sulfate, and then distilled under reduced pressure. The crude product obtained (8-1) (6.94 g, quantitative) was used for the subsequent step.
MS (APCI, ESI) m/z: 413 (M+H)+

Step 2: (6S)-5-[4-(Benzyloxy)-5-methoxy-2-nitrobenzoyl]-5-azaspiro[2.4]heptane-6-carbaldehyde (8-2)

The compound obtained in step 1 (8-1) (4.50 g, 11.0 mmol) was reacted in the same manner as in step 8 of Example 10-1 to afford the desired compound (8-2) (1.94 g, 43%).
MS (APCI, ESI) m/z: 411 (M+H)+

Step 3: (11a'S)-8'-Hydroxy-7'-methoxy-1',10',11',11a'-tetrahydro-5'H-spiro[cyclopropane-1,2'-pyrrolo[2,1-c][1,4]benzodiazepine]-5'-one (8-3)

To a solution of the compound obtained in step 2 (8-2) (1.94 g, 4.73 mmol) in tetrahydrofuran (25 mL), ethyl acetate (25 mL), and methanol (25 mL), 5% palladium carbon (moisture content: 54%, 1.0 g) was added under a nitrogen atmosphere, and the reaction solution was then stirred under a hydrogen atmosphere at room temperature for 22 hours. After the reaction solution was filtered through Celite, the filtrate was distilled under reduced pressure. The resulting residue was purified by silica gel column chromatography [hexane:ethyl acetate = 80:20 (v/v) to 0:100 (v/v)] to afford the desired compound (8-3) (1.20 g, 93%).
1H-NMR (CDCl3) δ: 7.55 (1H, s), 6.16 (1H, s), 5.86 (1H, s), 4.08-4.02 (2H, m), 3.86 (3H, s), 3.72-3.69 (1H, m), 3.57-3.37 (3H, m), 2.04-2.01 (1H, m), 1.78-1.75 (1H, m), 0.79-0.53 (4H, m).
MS (APCI, ESI) m/z: 275 (M+H)+

Step 4: Prop-2-en-1-yl(11a'S)-8'-[(5-bromopentylo)oxy]-11'-{[tert-butyl(dimethyl)silyl]oxy}-7'-methoxy-5'-oxo-11',11a'-dihydro-1'H-spiro[cyclopropane-1,2'-pyrrolo[2,1-c][1,4]benzodiazepine]-10'(5'H)-carboxylat (8-4)

The compound obtained in step 5 of Example 10-7 (7-5) (0.300 g, 0.614 mmol) was reacted in the same manner as in step 2 of Example 10-3 to afford the desired compound (8-4) (0.388 g, 99%).
1H-NMR (CDCl3) δ: 7.24 (1H, s), 6.60 (1H, s), 6.02-5.98 (1H, m), 5.80-5.75 (1H, m), 5.11-5.06 (2H, m), 4.68-4.64 (1H, m), 4.40-4.38 (1H, m), 4.02-3.98 (2H, m), 3.92 (3H, s), 3.72-3.69 (1H, m), 3.54-3.52 (1H, m), 3.46-3.41 (2H, m), 3.29-3.26 (1H, m), 2.38-2.34 (1H, m), 1.94-1.87 (4H, m), 1.65-1.62 (2H, m), 1.55-1.55 (1H, m), 0.86 (9H, s), 0.75-0.67 (4H, m), 0.24-0.22 (6H, m).
MS (APCI, ESI) m/z: 639 [81Br, (M+H)+], 637 [79Br, (M+H)+] .

Step 5: Prop-2-en-1-yl(11a'S)-11'-{[tert-butyl(dimethyl)silyl]oxy}-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-carboxylate (8-5)

The compound obtained in step 4 (8-4) (0.203 g, 0.318 mmol) was reacted with the compound obtained in step 3 (8-3) (0.131 g, 0.478 mmol) in the same manner as in step 10 of Example 10-3 to afford the desired compound (8-5) (0.0880 g, 33%).
MS (APCI, ESI) m/z: 831 (M+H)+

Step 6: Prop-2-en-1-yl(11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-carboxylate (8-6)

The compound obtained in step 5 (8-5) (0.0880 g, 0.106 mmol) was reacted in the same manner as in step 11 of Example 10-3 to afford the desired compound (8-6) (0.0500 g, 66%).
MS (APCI, ESI) m/z: 717 (M+H)+

Step 7: (11a'S)-7'-Methoxy-8'-[(5-{[(11a'S)-7'-methoxy-5'-oxo-5', 11a'-dihydro-1'H-spiro[cyclopropane-1,2'-pyrrolo[2,1-c][1,4]benzodiazepine]-8'-yl]oxy}pentyl)oxy]-1',10',11',11a'-tetrahydro-5'H-spiro[cyclopropane-1,2'-pyrrolo[2,1-c][1,4]benzodiazepine]-5'-one (8-7)

The compound obtained in step 6 (8-6) (0.0500 g, 0.0698 mmol) was reacted in the same manner as in step 12 of Example 10-3 to afford the desired compound (8-7) (0.0330 g, 77%).
1H-NMR (CDCl3) δ: 7.80 (1H, m), 7.58 (1H, s), 7.52 (1H, s), 6.81 (1H, s), 6.05 (1H, s), 4.17-3.97 (5H, m), 3.94 (3H, s), 3.87 (1H, m), 3.84 (3H, s), 3.72-3.68 (3H, m), 3.51-3.45 (5H, m), 2.54-2.51 (1H, m), 2.03-1.90 (6H, m), 1.75-1.68 (2H, m), 0.66 (8H, m).
MS (APCI, ESI) m/z: 615 (M+H)+

[Example 10-9: Drug 3]

[Formula 61]

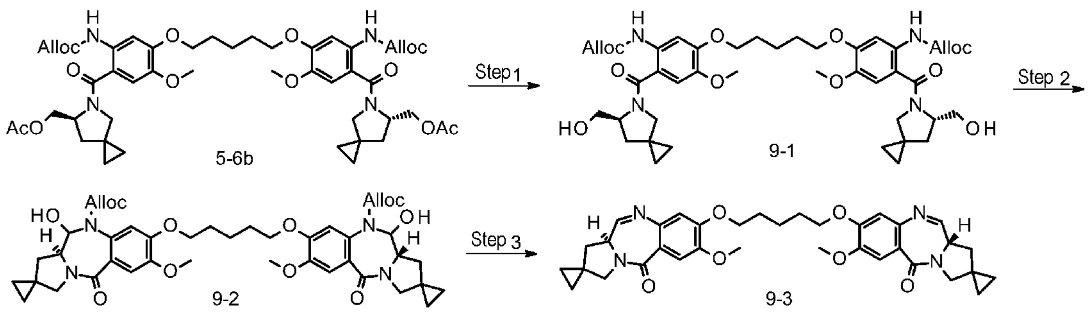


Step 1: Di-2-propen-1-yl{1,5-pentanediylbis[oxy(6-{[(6S)-6-(hydroxymethyl)-5-azaspiro[2.4]hept-5-yl]carbonyl}-4-methoxybenzen-3, 1-diyl)]}biscarbamate (9-1)

Bisallyloxycarbonyl form (5-6b) (0.460 g, 0.508 mmol), obtained in step 5 of Example 10-5, was reacted in the same manner as in step 7 of Example 10-5 to afford the desired compound (9-1) (0.421 g, quantitative). 1H-NMR (DMSO-D6) δ: 9.19 (2H, s), 7.22 (2H, s), 6.89 (2H, s), 5.97-5.92 (2H, m), 5.33 (2H, m), 5.22 (2H, m), 4.81 (2H, m), 4.55 (4H, m), 4.26 (2H, s), 3.96 (4H, m), 3.74 (6H, s), 3.62 (2H, m), 3.56 (2H, s), 3.37 (2H, m), 3.11 (2H, m), 1.88-1.78 (8H, m), 1.56-1.54 (2H, m), 0.54-0.43 (8H, m).
MS (APCI, ESI) m/z: 821 (M+H)+.

Step 2: Diprop-2-en-1-yl (11a'S, 11a''''S)-8',8" - [pentan-1,5-diylbis(oxy)]bis(11'-hydroxy-7'-methoxy-5'-oxo-11',11a'-dihydro-1'H-spiro[cyclopropane-1, 2'-pyrrolo[2,1-c][1,4]benzodiazepine]-10'(5'H)-carboxylate) (9-2)

The compound obtained in step 1 (9-1) (0.421 g, 0.513 mmol) was reacted in the same manner as in step 8 of Example 10-5 to afford the desired compound (9-2) (0.326 g, 78%).
1H-NMR (DMSO-D6) δ: 7.07 (2H, s), 6.80 (2H, s), 6.55 (2H, m), 5.84-5.81 (2H, m), 5.75 (2H, m), 5.09-5.05 (4H, m), 4.62 (2H, mz), 4.40 (2H, m), 3.98 (4H, m), 3.81 (6H, s), 3.54 (2H, m), 3.43-3.37 (2H, m), 3.14 (2H, m), 2.35 (2H, m), 1.81-1.79 (4H, m), 1.59-1.56 (4H, m), 0.70-0.59 (8H, m).MS (APCI, ESI) m/z: 817 (M+H)+.

Step 3: (11a'S,11a''''S)-8',8"-[1,5-Pentanediylbis(oxy)]bis(7'-methoxy-1',11a'-dihydro-5'H-spiro[cyclopropane-1,2'-pyrrolo[2,1-c][1,4]benzodiazepine]-5'-one) (9-3)

The compound obtained in step 2 (9-2) (0.326 g, 0.399 mmol) was reacted in the same manner as in step 12 of Example 10-3 to afford the desired compound (9-3) (0.208 g, 85%).
1H-NMR (DMSO-D6) δ: 7.91 (2H, m), 7.32 (2H, s), 6.84 (2H, s), 4.11 (2H, m), 4.06 (2H, m), 3.82 (6H, s), 3.51-3.31 (6H, m), 2.43 (2H, m), 2.05 (2H, m), 1.82-1.80 (4H, m), 1.60-1.58 (2H, m), 0.79-0.77 (2H, m), 0.68-0.64 (6H, m).MS (APCI, ESI) m/z: 613 (M+H)+.

[Example 10-10: Drug 4]

[Formula 62]

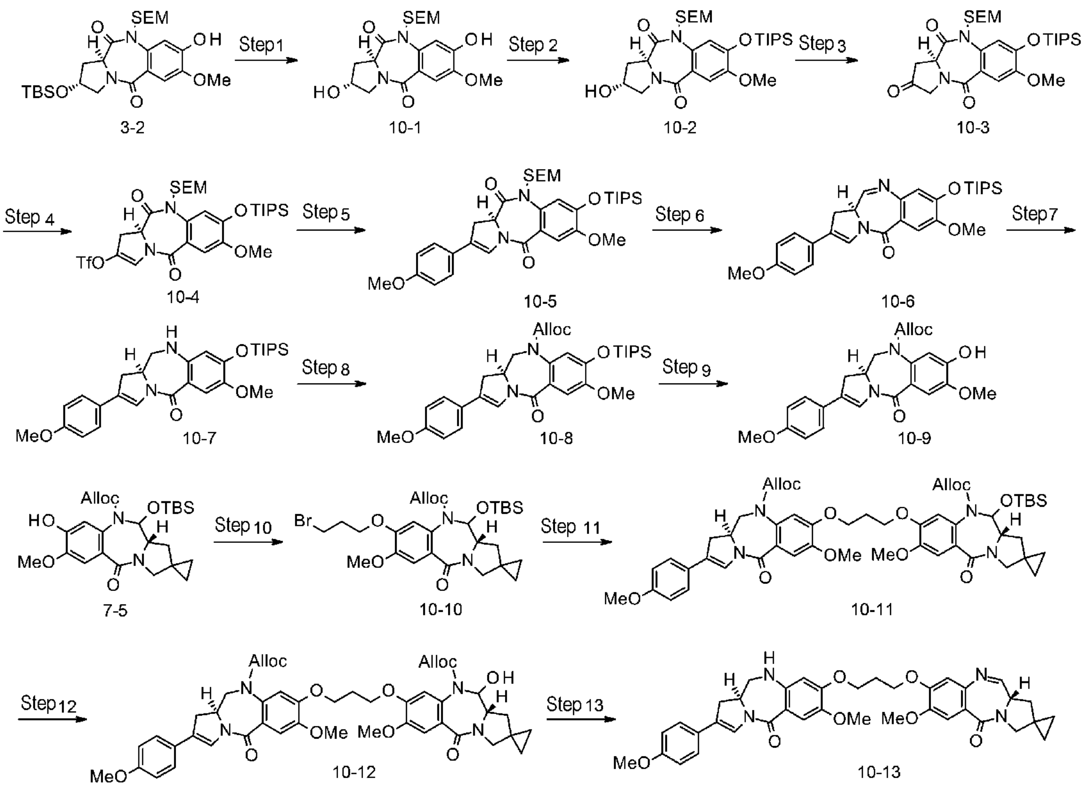


Step 1: (2R,11aS)-2,8-Dihydroxy-7-methoxy-10-{[2-(trimethylsilyl)ethoxy]methyl}-2,3-dihydro-1H-pyrrolo[2,1-c][1,4]benzodiazepine-5,11(10H,11aH)-dione (10-1)

The compound obtained in step 1 of Example 10-3 (3-2) (5.00g, 9.66 mmol) was reacted in the same manner as in step 3 of Example 10-3 to afford the desired compound (10-1) (3.95 g, 100%).
MS (APCI, ESI) m/z: 409 (M+H)+

Step 2: (2R,11aS)-2-Hydroxy-7-methoxy-10-{[2-(trimethylsilyl)ethoxy]methyl}-8-{[tri(propan-2-yl)silyl]oxy}-2,3-dihydro-1H-pyrrolo[2,1-c][1,4]benzodiazepine-5,11(10H,11aH)-dione (10-2)

To a solution of the compound obtained in step 1 (10-1) (3.95g, 9.67mmol) in dichloromethane (97 mL), imidazole (1.65 g, 24.2 mmol), triisopropylsilyl chloride (2.46 mL, 11.6 mmol) and dimethylformamide (5 mL) were added, and the resultant was stirred at room temperature for 21 hours. Water was added to the reaction solution, which was extracted with chloroform, and the organic layer obtained was washed with water, and then distilled under reduced pressure. The resulting residue was purified by silica gel chromatography [hexane:ethyl acetate = 100:0 (v/v) to 20:80 (v/v)] to afford the desired compound (10-2) (4.78 g, 87%).
MS (APCI, ESI) m/z: 565 (M+H)+

Step 3: (11aS)-7-Methoxy-10-{[2-(trimethylsilyl)ethoxy]methyl}-8-{[tri(propan-2-yl)silyl]oxy}-1H-pyrrolo[2,1-c][1,4]benzodiazepine-2,5,11(3H,10H,11aH)-trione

The compound obtained in step 2 (4.78 g, 8.43 mmol) was reacted in the same manner as in step 4 of Example 10-3 to afford the desired compound (10-3) (2.36 g, 50%). MS (APCI, ESI) m/z: 563 (M+H)+

Step 4: (11aS)-7-Methoxy-5,11-dioxo-10-{[2-(trimethylsilyl)ethoxy]methyl}-8-{[tri(propan-2-yl)silyl]oxy}-5,10,11,11a-tetrahydro-1H-pyrrolo[2,1-c][1,4]benzodiazepin-2-yl trifluoromethane sulfonate (10-4)

The compound obtained in step 3 (10-3) (1.53 g, 2,72 mmol) was reacted in the same manner as in step 5 of Example 10-3 to afford the desired compound (1.27 g, 69%) .
1H-NMR (CDCl3) δ: 7.31 (2H, s), 7.15 (1H, m), 5.52 (1H, m), 4.65 (1H, m), 4.57 (1H, m), 3.95-3.89 (1H, m), 3.87 (3H, s), 3.75-3.58 (2H, m), 3.18-3.14 (1H, m), 1.33-1.25 (3H, m), 1.10 (18H, m), 1.00-0.96 (2H, m), 0.03 (9H, s).

Step 5: (11aS)-7-Methoxy-2-(4-methoxyphenyl)-10-{[2-(trimethylsilyl)ethoxy]methyl}-8-{[tri(propan-2-yl)silyl]oxy}-1H-pyrrolo[2,1-c][1,4]benzodiazepine-5,11(10H,11aH)-dione (10-5)

The compound obtained in step 4 (10-4) (0.519 g, 0.747 mmol) was reacted in the same manner as in step 6 of Example 10-3 to afford the desired compound (10-5) (0.511 g, quantitative).
1H-NMR (CDCl3) δ: 7.41-7.31 (5H, m), 6.91-6.85 (2H, m), 5.52 (1H, m), 4.64 (1H, m), 4.57 (1H, m), 3.97-3.90 (1H, m), 3.88 (3H, s), 3.83 (3H, s), 3.75-3.56 (2H, m), 3.19-3.09 (1H, m), 1.36-1.23 (3H, m), 1.11 (18H, m), 1.02-0.97 (2H, m), 0.03 (9H, s).
MS (APCI, ESI) m/z: 653 [(M+H)+]

Step 6: (11aS)-7-Methoxy-2-(4-methoxyphenyl)-8-{[tri(propan-2-yl)silyl]oxy}-1, 11a-dihydro-5H-pyrrolo[2,1-c][1,4]benzodiazepine-5-one (10-6)

The compound obtained in step 5 (10-5) (0.178 g, 0.272 mmol) was reacted in the same manner as in step 7 of Example 10-3 to afford the desired compound (10-6) (0.094 g, 68%).
1H-NMR (CDCl3) δ: 7.87 (1H, m), 7.51 (1H, s), 7.41-7.39 (1H, m), 7.36-7.33 (2H, m), 6.93-6.89 (2H, m), 6.86 (1H, s), 4.44-4.38 (1H, m), 3.90 (3H, s), 3.83 (3H, s), 3.61-3.53 (1H, m), 3.41-3.34 (1H, m), 1.33-1.25 (3H, m), 1.11-1.06 (18H, m).
MS (APCI, ESI) m/z: 507 [(M+H)+]

Step 7: (11aS)-7-Methoxy-2-(4-methoxyphenyl)-8-{[tri(propan-2-yl)silyl]oxy}-1,10,11,11a-tetrahydro-5H-pyrrolo[2,1-c][1,4]benzodiazepine-5-one (10-7)

The compound obtained in step 6 (10-6) (0.063 g, 0.124 mmol) was used and reacted in the same manner as in step 8 of Example 10-3 to afford the desired compound (10-7) (0.046 g, 72%).
1H-NMR (CDCl3) δ: 7.53-7.48 (2H, m), 7.33-7.29 (2H, m), 6.90-6.86 (2H, m), 6.13-6.11 (1H, m), 4.36-4.29 (1H, m), 4.11 (1H, s), 3.82 (3H, s), 3.79 (3H, s), 3.59-3.50 (2H, m), 3.40-3.31 (1H, m), 2.78-2.68 (1H, m), 1.31-1.20 (3H, m), 1.13-1.02 (18H, m) .MS (APCI, ESI) m/z: 509 [(M+H)+]

Step 8: Prop-2-en-1-yl(11aS)-7-methoxy-2-(4-methoxyphenyl)-5-oxo-8-{[tri(propan-2-yl)silyl]oxy}-11,11a-dihydro-1H-pyrrolo[2,1-c][1,4]benzodiazepine-10 (5H)-carboxylate (10-8)

The compound obtained in step 7 (10-7) (0.046 g, 0.090 mmol) was used and reacted in the same manner as in step 9 of Example 10-3 to afford the desired compound (10-8) (0.03 g, 56%).

1H-NMR (CDCl3) δ: 7.39-7.36 (1H, m), 7.31-7.28 (2H, m), 7.22 (1H, s), 6.90-6.86 (3H, m), 6.75-6.72 (1H, m), 5.82-5.69 (1H, m), 5.18-5.08 (2H, m), 4.59-4.52 (1H, m), 4.48-4.39 (1H, m), 4.39-4.29 (1H, m), 4.23-4.12 (1H, m), 3.86 (3H, s), 3.82 (3H, s), 3.64-3.58 (1H, m), 3.32-3.25 (1H, m), 2.73-2.65 (1H, m), 1.30-1.20 (2H, m), 1.12-1.06 (18H, m).
MS (APCI, ESI) m/z: 593 [(M+H)+]

Step 9: Prop-2-en-1-yl(11aS)-8-hydroxy-7-methoxy-2-(4-methoxyphenyl)-5-oxo-11,11a-dihydro-1H-pyrrolo[2, 1-c][1,4]benzodiazepine-10 (5H)-carboxylate (10-9)

The compound obtained in step 8 (10-8) (0.030 g, 0.050 mmol) was reacted in the same manner as in step 10 of Example 10-1 to afford the desired compound (10-9) (0.015 g, 0.034 mmol).
1H-NMR (CDCl3) δ: 7.39-7.25 (4H, m), 6.92-6.78 (3H, m), 6.03-5.92 (1H, m), 5.86-5.68 (1H, m), 5.20-5.07 (2H, m), 4.66-4.57 (1H, m), 4.52-4.40 (1H, m), 4.40-4.27 (1H, m), 4.27-4.16 (1H, m), 3.95 (3H, s), 3.82 (3H, s), 3.66-3.59 (1H, m), 3.32-3.21 (1H, m), 2.74-2.64 (1H, m).
MS (APCI, ESI) m/z: 437 [(M+H)+]

Step 10: Prop-2-en-1-yl(11a'S)-8'-(3-bromopropoxy)-11'-{[tert-butyl(dimethyl)silyl]oxy}-7'-methoxy-5'-oxo-11',11a'-dihydro-1'H-spiro[cyclopropane-1,2'-pyrrolo[2,1-c][1,4]benzodiazepine]-10'(5'H)-carboxylate (10-10)

The compound obtained in step 5 of Example 10-7 (0.131 g, 0.268 mmol) was reacted in the same manner as in step 1 of Example 10-4 to afford the desired compound (10-10) (0.086 g, 52%).
1H-NMR (CDCl3) δ: 7.24 (1H, s), 6.65 (1H, s), 6.02 (1H, m), 5.87-5.71 (1H, m), 5.15-5.04 (2H, m), 4.72-4.62 (1H, m), 4.44-4.32 (1H, m), 4.23-4.07 (3H, m), 3.92 (3H, s), 3.77-3.47 (4H, m), 3.28 (1H, m), 2.37 (3H, m), 1.57-1.52 (1H, m), 0.86 (9H, s), 0.82-0.57 (4H, m), 0.21 (6H, m). MS (APCI, ESI) m/z: 611 [81Br, (M+H)+], 609 [79Br,

(M+H)+]

Step 11: Prop-2-en-1-yl(11a'S)-11'-{[tert-butyl(dimethyl)silyl]oxy}-7'-methoxy-8'-[3-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c][1,4]benzodiazepine-8-yl}oxy)propoxy]-5'-oxo-11',11a'-dihydro-1'H-spiro[cyclopropane-1,2'-pyrrolo[2,1-c][1,4]benzodiazepine]-10'(5'H)-carboxylate (10-11)

The compound obtained in step 10 (10-10) (0.015 g, 0.034 mmol) and the compound obtained in step 9 (10-9) (0.030 g, 0.048 mmol) were reacted in the same manner as in step 10 of Example 10-3 to afford the desired compound (10-11) (0.032 g, 96%).
MS (APCI, ESI) m/z: 965 [(M+H)+]

Step 12: Prop-2-en-1-yl(11a' S)-11'-hydroxy-7'-methoxy-8'-[3-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c][1,4]benzodiazepine-8-yl}oxy)propoxy]-5'-oxo-11',11a'-dihydro-1'H-spiro[cyclopropane-1,2'-pyrrolo[2,1-c][1,4]benzodiazepine]-10'(5'H)-carboxylate (10-12)

The compound obtained in step 11 (10-11) (0.031 g, 0.032 mmol) was reacted in the same manner as in step 11 of Example 10-3 to afford the desired compound (10-12) (0.026 g, 95%).
MS (APCI, ESI) m/z: 851 [(M+H)+]

Step 13: (11a'S)-7'-Methoxy-8'-(3-{[(11aS)-7-methoxy-2-(4-methoxyphenyl)-5-oxo-5,10,11,11a-tetrahydro-1H-pyrrolo[2,1-c][1,4]benzodiazepine-8-yl]oxy}propoxy)-1',11a'-dihydro-5'H-spiro[cyclopropane-1,2'-pyrrolo[2,1-c][1,4]benzodiazepine]-5'-one (10-13)

The compound obtained in step 12 (10-12) (0.026 g, 0.030 mmol) was reacted in the same manner as in step 12 of Example 10-3 to afford the desired compound (10-13) (0.018 g, 88%).
1H-NMR (CDCl3) δ: 7.80 (1H, m), 7.54-7.51 (3H, m), 7.33-7.29 (2H, m), 6.91-6.85 (3H, m), 6.14 (1H, s), 4.35-4.17 (6H, m), 3.95 (3H, s), 3.85 (3H, s), 3.82 (3H, s), 3.76-3.25 (5H, m), 2.79-2.69 (1H, m), 2.52 (1H, m), 2.45-2.35 (1H, m), 2.03-1.96 (1H, m), 1.28-1.23 (2H, m), 0.78-0.69 (4H, m).
MS (APCI, ESI) m/z: 665 [(M+H)+]

[Example 11: [$N_3$-PEG (3)]-MSG1-Ox]

Synthesis of [$N_3$-PEG (3)]-MSG1

[Formula 63]

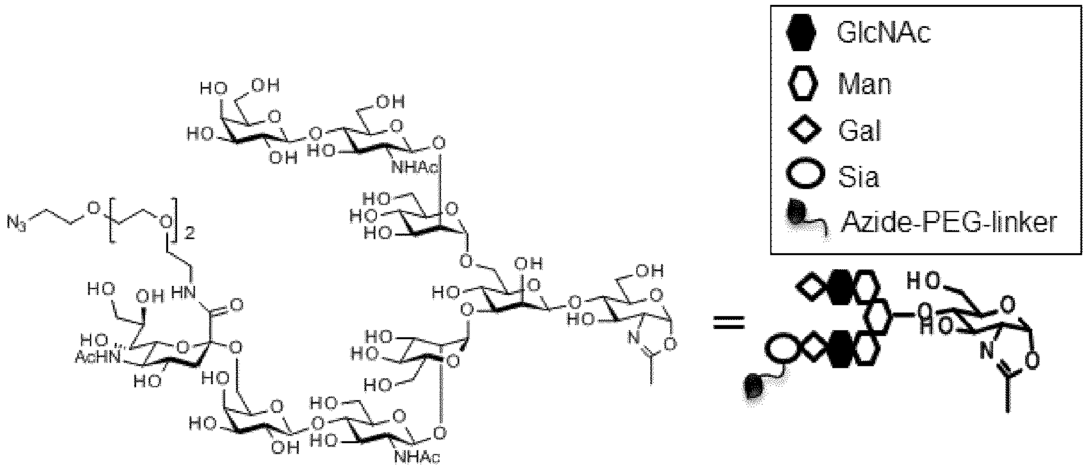


(In the figure, the schematic diagram in the right of the structural formula represents the corresponding structure in the schematic diagram of an intermediate as represented by the reaction formula of each of Examples 12, 13, 14, 15.)

Step 1: (MSG1-)Asn

The commercially available product monosialo-Asn free (1S2G/1G2S-10NC-Asn, produced by GlyTech, Inc.) (referred to as "(MSG-)Asn") (500 mg) was subjected to separation/purification by reversed-phase HPLC under the conditions described below, to separate into (MSG1-)Asn eluted as the 1st main peak (retention time: around 15 to 19 min) and (MSG2-)Asn eluted as the 2nd main peak (retention time: around 21 to 26 min). The eluent used was a 0.1% formic acid aqueous solution, the apparatus used was an ELS-PDA trigger preparative system (produced by JASCO Corporation), the column used was an Inertsil ODS-3 (10 um, 30ϕ × 250 mm, produced by GL Sciences, Inc.), and the flow rate was 30 mL/min. Fractions belonging to the first peak UV-detected (210 nm) during the elution were collected together, and freeze-dried to afford the desired compound (238 mg).

Step 2: MSG1

The compound obtained in step 1 (229 mg) was dissolved in 200 mM phosphate buffer solution (pH 6.25) (1145 μL), to which an aqueous solution (100 μL) of EndoM (produced by Tokyo Chemical Industry Co., Ltd., 1 U/mL)) was added, and the resultant was incubated at 35°C for 6 days. After the completion of the reaction, the reaction solution was subjected to ultrafiltration with a VIVASPIN 15R (Hydrosart membrane, 30K, 6,000 g), and the filtered solution obtained was subjected to separation/purification by reversed-phase HPLC. The eluent used was a 0.1% trifluoroacetic acid aqueous solution, the apparatus used was an ELS-PDA trigger preparative system (produced by JASCO Corporation), and the column used was an Inertsil ODS-3 (produced by GL Sciences, Inc.). Fractions containing the desired compound UV-detected (210 nm) during the elution were collected together, and freeze-dried to afford the desired compound (117 mg).

Step 3: [$N_3$-PEG (3)]-MSG1

Into a 5 mL sampling tube (Ina-Optica Co., Ltd.), an aqueous solution (1.2 mL) of 11-azide-3,6,9-trioxaundecane-1-amine (0.108 mL, 0.541 mmol) and MSG1 obtained in step 2 (117 mg, 0.068 mmol) were added, and the resultant was stirred for 1 hour and then freeze-dried. Into the 5 mL sampling tube after freeze-drying, an N,N-dimethylformamide solution (1.2 mL) of O-(7-azabenzotriazol-1-yl)-N,N,N',N'-tetramethyluronium hexafluorophosphate (103 mg, 0.27 mmol) and diisopropylethylamine (0.046 mL, 0.27 mmol) were added, followed by stirring at 37°C for 3 hours. After the completion

of the reaction, the reaction solution was transferred into a centrifuge tube (50 mL) into which diethyl ether (20 mL) had been added in advance. The solid matter was precipitated by using a small centrifuge (Hitachi Koki Co., Ltd., CF16RX) and the supernatant was removed. Further, diethyl ether (10 mL) was added, and the resultant was centrifuged and decanted. Subsequently, acetonitrile (10 mL) was added, and the resultant was centrifuged and decanted. This operation was repeated twice, and then the resultant was dried under reduced pressure to afford a crude product. The resulting solid matter was subjected to purification by reversed-phase HPLC in the same conditions as in step 2 to afford the desired compound (94.2 mg).

Step 4: [$N_3$-PEG (3)]-MSG1-Ox

Into a 5 mL sampling tube (produced by Ina-Optica Co., Ltd), the compound synthesized in step 3 (100 mg) and an aqueous solution (520 μL) of 2-chloro-1,3-dimethyl-1H-benzimidazol-3-ium-chloride (produced by FUSHIMI Pharmaceutical Co., Ltd. 56 mg, 0.257 mmol) was added. To the reaction solution after being ice-cooled, an aqueous solution (520 μL) of tripotassium phosphate (165 mg, 0.78 mmol) was added, followed by stirring under ice-cooling for 3 hours. The resulting reaction solution was subjected to ultrafiltration with an Amicon Ultra (Ultracel 30K, produced by Merck Millipore) to remove the solid matter. The filtered solution was purified by gel filtration chromatography. The apparatus used was a Purif-Rp2 (produced by Shoko Scientific Co., Ltd.), the column used was a HiPrep 26/10 Desalting (produced by GE Healthcare), the mobile phase used was 0.03%-$NH_3$ aqueous solution, and the flow rate was 10 mL/min and the fraction volume was 10 mL. Fractions containing the desired compound UV-detected (220 nm) during the elution were collected together, to which a 1 N aqueous solution of sodium hydroxide (104 μL, 0.104 mmol) was added, and the resultant was freeze-dried to afford the desired compound (84 mg).

In Examples 12 to 16, preparation of a glycanremodeled antibody will be described.

[Example 12: H01L02 antibody-[MSG1-$N_3$]$_2$]

[Formula 64]

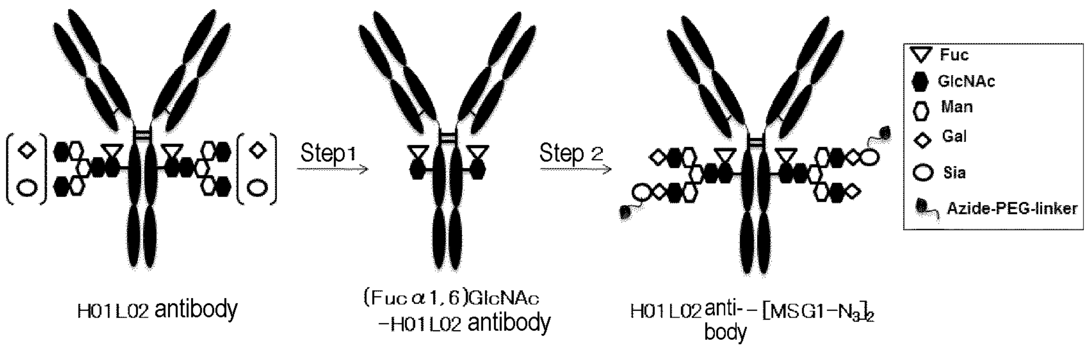


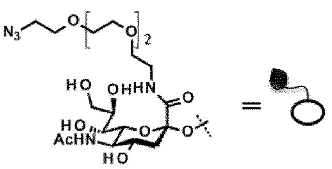

(This formula represents a linker structure in which an azide group has been introduced to a sialic acid at the non-reducing terminal of an MSG1-type N297 glycan. In Example 12, linker structures of intermediates formed by introducing an azide group to an N297 glycan are the same as the structure represented by the formula.)

Step 1: Preparation of (Fucα1,6)GlcNAc-H01L02 antibody

To H01L02 antibody solution (ca. 21.1 mg/mL) (50 mM phosphate buffer (pH 6.0)) (4.07 ml), 0.233 mL of wildtype EndoS solution (7.7 mg/mL, PBS) was added, and the solutions were incubated at 37°C for 4 hours. The progress of the reaction was checked by an Experion electrophoresis station (produced by Bio-Rad Laboratories, Inc.). After completion of the reaction, purification by affinity chromatography and purification with a hydroxyapatite column

were performed in accordance with the following methods.

(1) Purification by affinity chromatography

Purification apparatus: AKTA avant (produced by GE Healthcare)

Column: HiTrap rProtein A FF (5 mL)(produced by GE Healthcare)
Flow rate: 5 ml/min (1.25 ml/min in charging)

Each reaction solution obtained above was purified in multiple separate operations. In application of the sample, the reaction solution was added to the upper part of the column, and 4 CV of binding buffer (20 mM phosphate buffer (pH 6.0)) was flowed at 1.25 mL/min and 5 CV (column volume) thereof was further flowed at 5 mL/min. In an intermediate washing step, 15 CV of washing solution (20 mM phosphate buffer (pH 7.0), 0.5 M sodium chloride solution) was flowed. In elution, 6 CV of elution buffer (ImmunoPure IgG Eution buffer, produced by Pierce) was flowed. The eluate was immediately neutralized with 1 M Tris buffer (pH 9.0). Fractions containing the desired compound were subjected to buffer exchange to 5 mM phosphate buffer/50 mM 2-morpholinoethanesulfonic acid (MES) solution (pH 6.8) by using common operation C.

(2) Purification by hydroxyapatite chromatography

Purification apparatus: AKTA avant (produced by GE Healthcare)

Column: Bio-Scale Mini CHT Type I cartridge (5 mL) (produced by Bio-Rad Laboratories, Inc.)
Flow rate: 5 mL/min (1.25 mL/min in charging)

The solution obtained in (1) was applied to the upper part of the column, and 4 CV of solution A (5 mM phosphate buffer, 50 mM 2-morpholinoethanesulfonic acid (MES) solution (pH 6.8)) was flowed at 1.25 mL/min and 3 CV thereof was further flowed at 5 mL/min. Thereafter, elution was performed with solution A and solution B (5 mM phosphate buffer, 50 mM 2-morpholinoethanesulfonic acid (MES)(pH 6.8) and 2 M sodium chloride). The elution conditions were solution A:solution B = 100:0 to 0:100 (15 CV) . Further, 5 CV of washing solution (500 mM phosphate buffer (pH 6.5)) was flowed.

Fractions containing the desired compound were subjected to buffer exchange by using common operation C to afford a 11.4 mg/mL (Fucα1,6)GlcNAc-H01L02 antibody solution (50 mM phosphate buffer (pH 6.0)) (5.00 mL).

Step 2: Preparation of H01L02 antibody-[MSG1-$N_3$]$_2$

To the 11.4 mg/mL (Fucα1,6)GlcNAc-H01L02 antibody solution (50 mM phosphate buffer (pH 6.0)) obtained in step 1 (5.00 mL), a solution (0.180 mL) of the glycan synthesized in step 4 of Example 11 (9.00 mg) in 50 mM phosphate buffer (pH 6.0) and 5.10 mg/mL EndoS D233Q/Q303L solution (PBS) (0.230 mL) were added, and the resultant was incubated at 30°C for 3 hours. The progress of the reaction was checked by using an Experion electrophoresis station (produced by Bio-Rad Laboratories, Inc.). After the reaction, purification by affinity chromatography and purification by hydroxyapatite chromatography were performed as in step 1, and fractions containing the desired compound were then subjected to buffer exchange to phosphate buffered saline (pH 6.0) by using common operation C to afford a 9.91 mg/mL H01L02 antibody-[MSG1-$N_3$]$_2$ solution (phosphate buffered saline (pH 6.0)) (4.00 mL).

[Example 13: H01L02A antibody-[MSG1-$N_3$]$_2$]

[Formula 65]

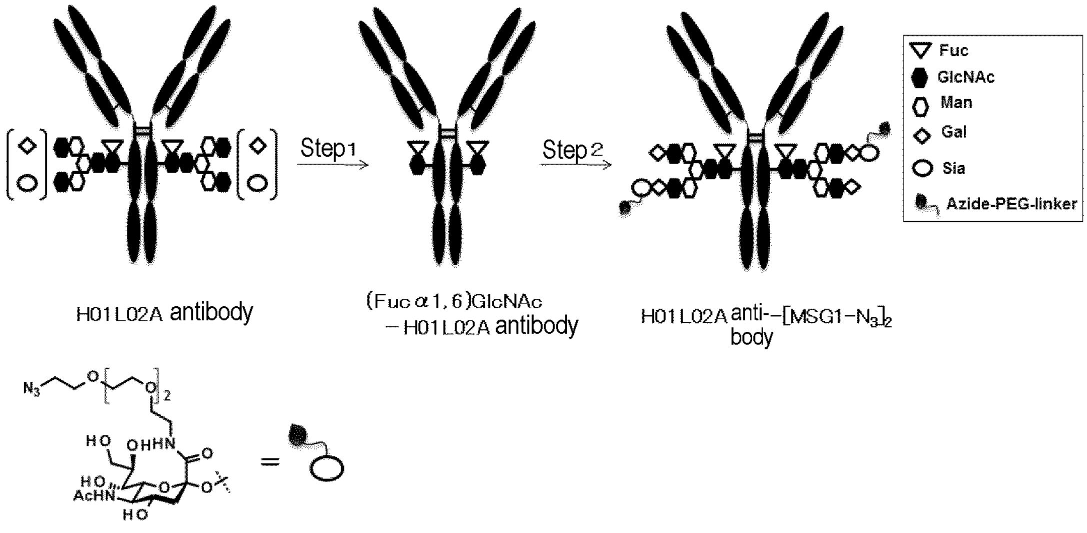


Step 1: Preparation of (Fucα1,6)GlcNAc-H01L02A antibody

The operation in step 1 of Example 12 was carried out with a ca. 21.6 mg/mL H01L02A antibody solution (50 mM phosphate buffer (pH 6.0)) (1.85 mL) to afford a 14.6 mg/mL (Fucα1,6)GlcNAc-H01L02A antibody solution (50 mM phosphate buffer (pH 6.0)) (2.0 mL).

Step 2: Preparation of H01L02A antibody-[MSG1-$N_3$]$_2$]

The operation in step 2 in Example 12 was carried out with the 14.6 mg/mL (Fucα1,6)GlcNAc-H01L02A antibody solution (50 mM phosphate buffer (pH 6.0)) (2.0 mL) obtained in step 1 to afford a 10.0 mg/mL H01L02A antibody-[MSG1-$N_3$]$_2$ solution (acetate buffer (pH 5.5, containing sorbitol)) (2.5 mL).

[Example 14: H31L02A antibody-[MSG1-$N_3$]$_2$]

[Formula 66]

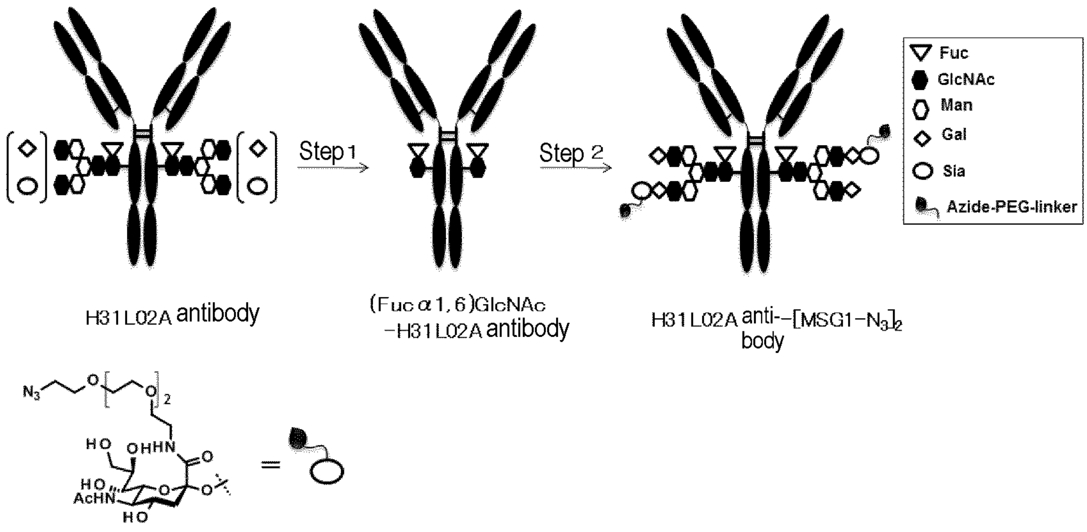

Step 1: Preparation of (Fucα1,6)GlcNAcH31L02A antibody

The operation in step 1 of Example 12 was carried out with a ca. 23.2 mg/mL H31L02A antibody solution (50 mM phosphate buffer (pH 6.0)) (3.45 mL) to afford a 8.43 mg/mL (Fucα1,6)GlcNAc-H31L02A antibody solution (50 mM phosphate buffer (pH 6.0)) (6.1 mL).

Step 2: Preparation of H31L02A antibody-[MSG1-$N_3$]$_2$]

The operation in step 2 in Example 12 was carried out with the 8.43 mg/mL (Fucα1,6)GlcNAc-H31L02A antibody solution (50 mM phosphate buffer (pH 6.0)) (5.00 mL) obtained in step 1 to afford a 6.52 mg/mL H31L02A antibody-[MSG1-$N_3$]$_2$ solution (phosphate buffer saline (pH 6.0)) (4.00 mL).

[Example 15: H11L02A antibody-[MSG1-$N_3$]$_2$]

[Formula 67]

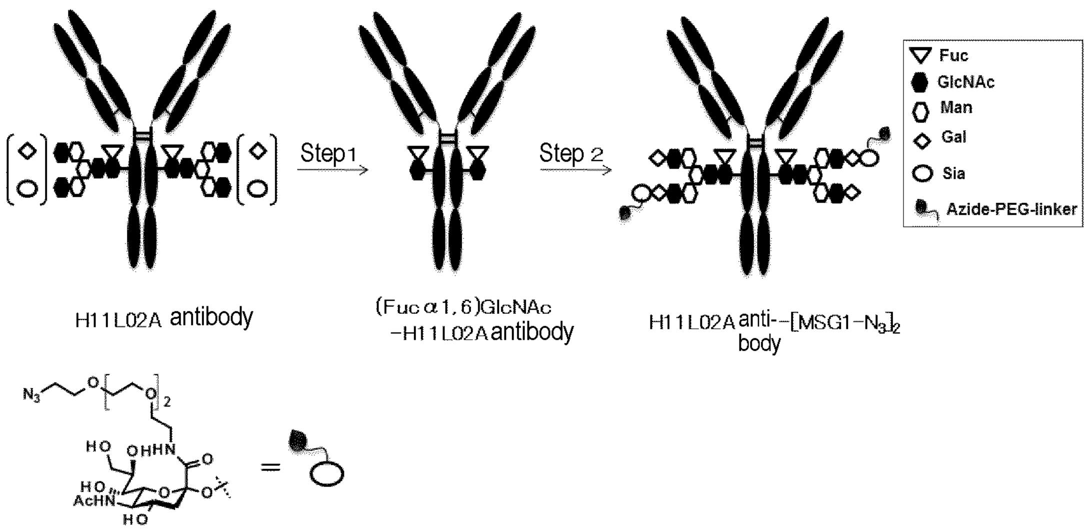


Step 1: Preparation of (Fucα1,6)GlcNAc-H11L02A antibody

The operation in step 1 of Example 12 was carried out with a ca. 24.2 mg/mL H11L02A antibody solution (50 mM phosphate buffer (pH 6.0)) (3.0 mL) to afford a 20.42 mg/mL (Fucα1,6)GlcNAc-H11L02A antibody solution (50 mM phosphate buffer (pH 6.0)) (2.7 mL).

Step 2: Preparation of H11L02A antibody-[MSG1-$N_3$]$_2$]

The operation in step 2 in Example 12 was carried out with the 20.39 mg/mL (Fucα1,6)GlcNAc-H11L02A antibody solution (50 mM phosphate buffer (pH 6.0)) (1.55 mL) obtained in step 1 to afford a 10.26 mg/mL H11L02A antibody-[MSG1-$N_3$]$_2$ solution (acetate buffer (pH 5.5, containing sorbitol)) (2.6 mL).

[Example 16: Anti-LPS antibody-[MSG1-$N_3$]$_2$]]

[Formula 68]

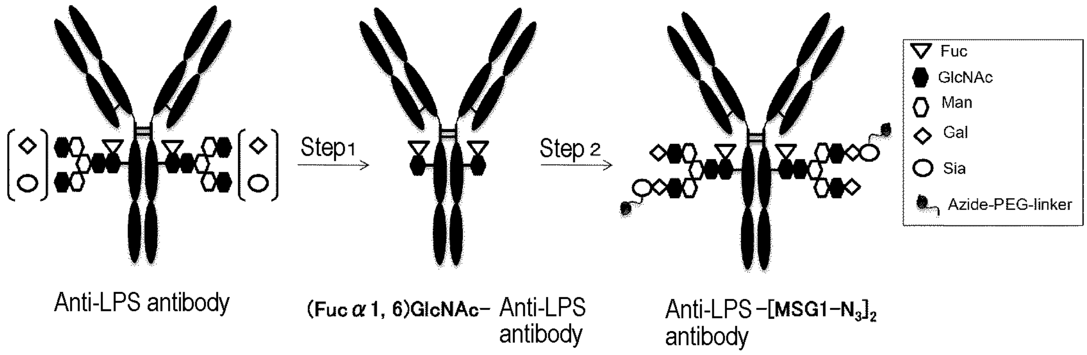


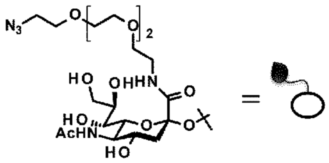

Step 1: Preparation of (Fucα1,6)GlcNAc-anti-LPS antibody

The operation in step 1 of Example 12 was performed using a ca. 17 mg/mL anti-LPS antibody solution (25 mM histidine solution (pH 6.0), 5% sorbitol solution) (6.6 mL) to afford a 21.03 mg/mL (Fucα1,6)GlcNAc-anti-LPS antibody solution (50 mM phosphate buffer (pH 6.0)) (5.4 mL) .

Step 2: Preparation of anti-LPS antibody-[MSG1-$N_3$]$_2$]

The operation in step 2 of Example 12 was performed using the 21.03 mg/mL (Fucα1,6)GlcNAc-anti-LPS antibody solution (50 mM phosphate buffer (pH 6.0)) obtained in step 1 (5.4 mL) to afford a 9.89 mg/mL anti-LPS antibody-[MSG1-$N_3$]$_2$ solution (phosphate buffered saline (pH 6.0)) (7.9 mL).

In Examples 17 to 27, synthesis of ADCs will be described.

[Example 17: ADC1]

The ADC described in Example 17 was synthesized, as illustrated in the following reaction formula, by conjugating the antibody obtained in step 2 of Example 12 with the drug-linker obtained in step 13 of Example 10-3 (3-14). In the formula, R represents the drug-linker used in the Example.

[Formula 69]

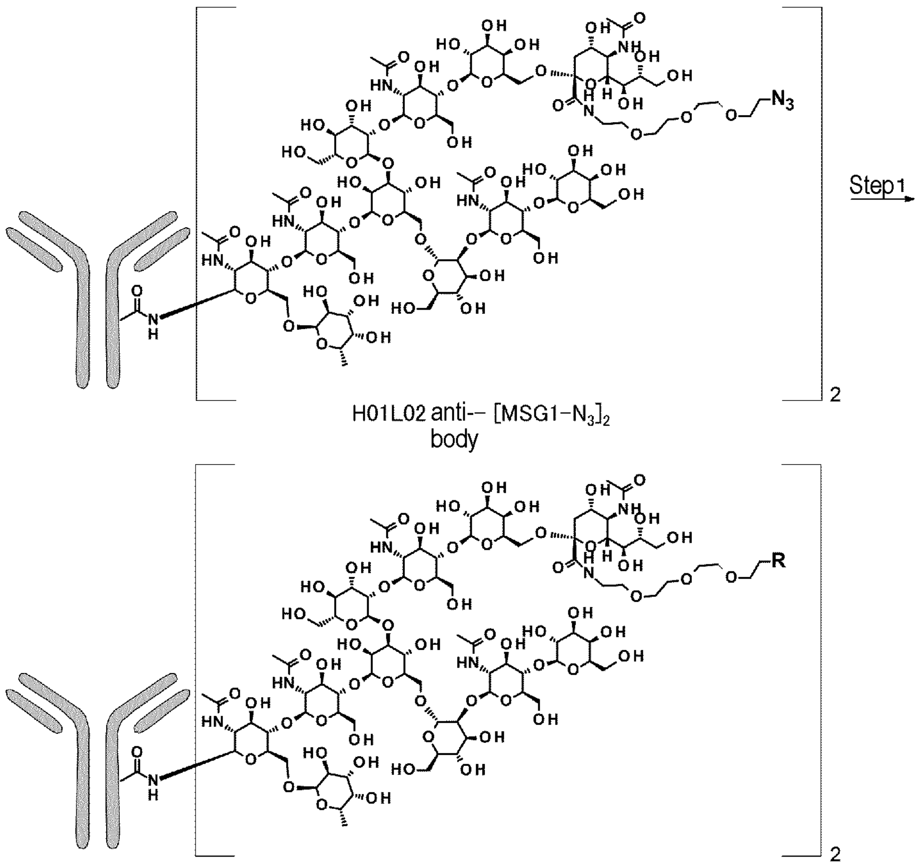


H01L02 anti-body — $[MSG1\text{-}N_3]_2$

[Formula 70]

R =

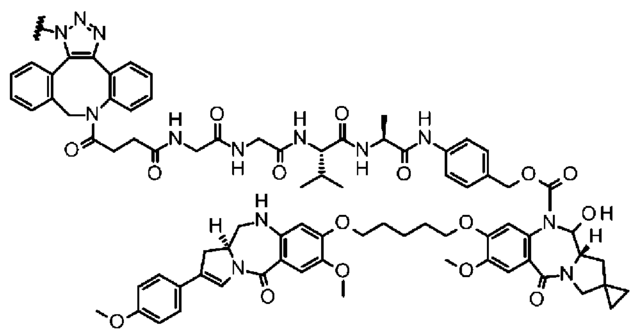

or

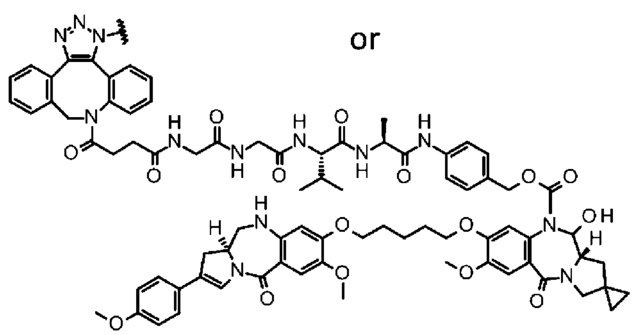

The triazole ring to be formed in step 1 has geometric isomers, and the compound obtained in step 1 of Example 17 has a linker as a mixture of the two structures shown above as R.

Step 1: Conjugation of antibody and drug-linker

To a phosphate buffered saline (pH 6.0) solution of the antibody (6.76 mg/mL, 1.50 mL) obtained in step 2 of Example 12, 1,2-propanediol (1.42 mL) and a 10 mM dimethyl sulfoxide solution of the compound obtained in step 13 of Example 10-3 (3-14) (0.0836 mL; 12 equivalents per antibody molecule) were added at room temperature, and the resultant was reacted using a tube rotator (MTR-103, AS ONE Corporation) at room temperature for 48 hours.

Purification operation: The solution was purified by using common operation D to afford 6.00 mL of a solution of the desired compound.

Characterization: The following characteristic values were obtained by using common operations E and F.

Antibody concentration: 1.37 mg/mL, antibody yield: 8.23 mg (81%), average number of conjugated drug molecules per antibody molecule (n): 1.7

[Example 18 ADC2]

The ADC described in Example 18 was synthesized, as illustrated in the following reaction formula, by conjugating the antibody obtained in step 2 of Example 13 with the drug-linker obtained in step 12 of Example 10-4 (4-12). In the formula, R represents the drug-linker used in the Example.

[Formula 71]

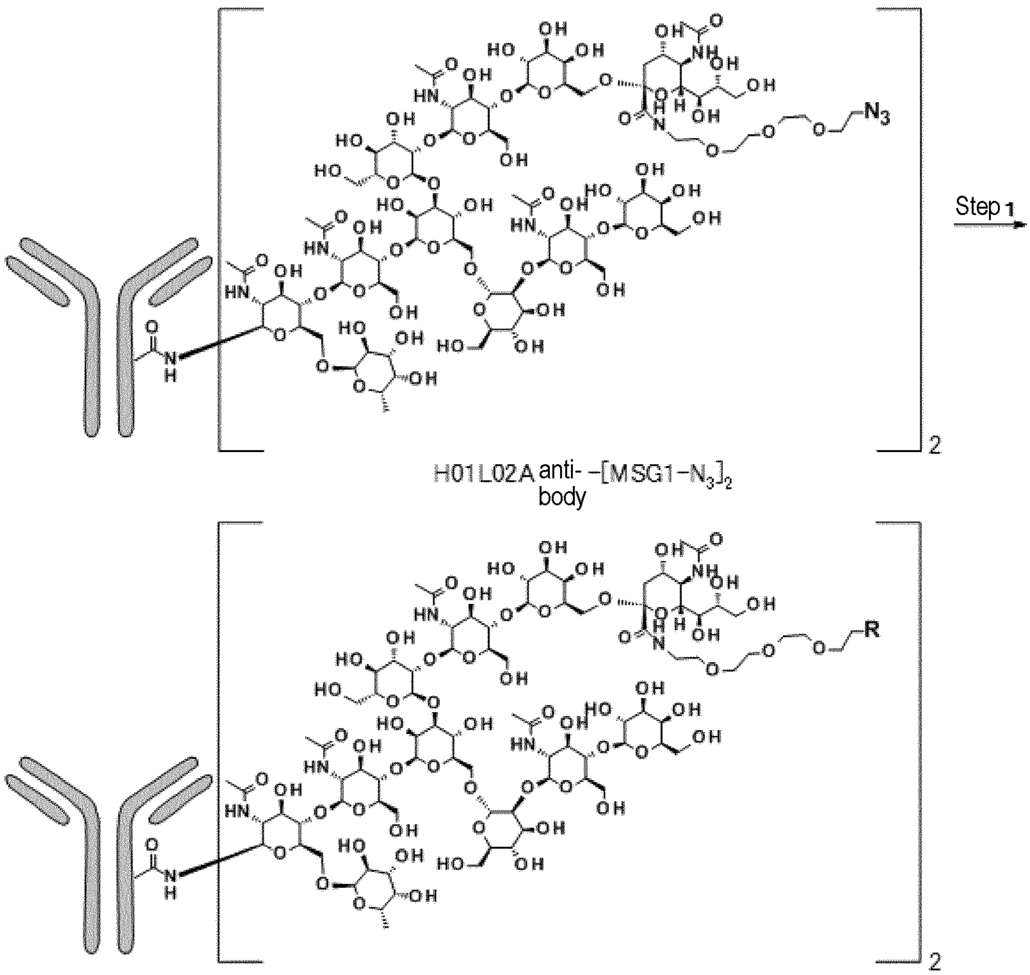

[Formula 72]

R = 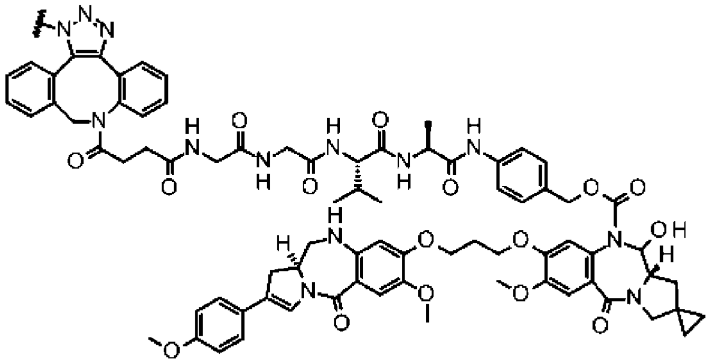

or

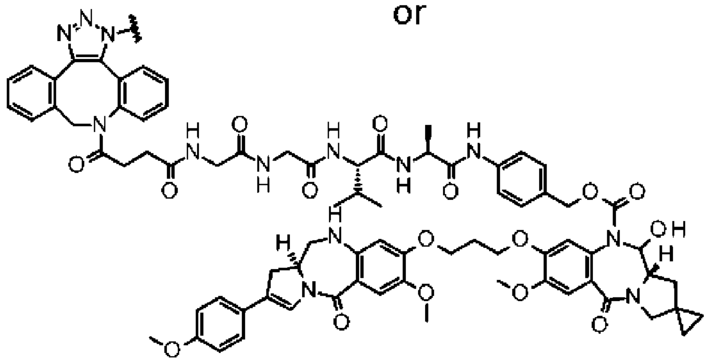

The triazole ring to be formed in step 1 has geometric isomers, and the compound obtained in step 1 of Example 18 has a linker as a mixture of the two structures shown above as R.

Step 1: Conjugation of antibody and drug-linker

To a 10mM acetate buffer, 5% sorbitol (pH 5.5) solution of the antibody (10.0 mg/mL, 100 μL) obtained in step 2 of Example 13, 1,2-propanediol (25 μL) and a mixed solution of a 10 mM dimethyl sulfoxide solution of the compound obtained in step 12 of Example 10-4 (4-12) (8 μL; 12 equivalents per antibody molecule) and 1,2-propane diol (67 μL) were added at room temperature, and the resultant was reacted using a tube rotator (MTR-103, AS ONE Corporation) at room temperature for 48 hours.

Purification operation: The solution was purified by using common operation D to afford 1.2 mL of a solution of the desired compound.

Characterization: The following characteristic values were obtained by using common operations E and F.

Antibody concentration: 0.56 mg/mL, antibody yield: 0.67 mg (67%), average number of conjugated drug molecules per antibody molecule (n): 1.9

[Example 19: ADC3]

The ADC described in Example 19 was synthesized by conjugating the antibody obtained in step 2 of Example 13 with the drug-linker obtained in step 10 of Example 10-5 (5-11) .

[Formula 73]

R =

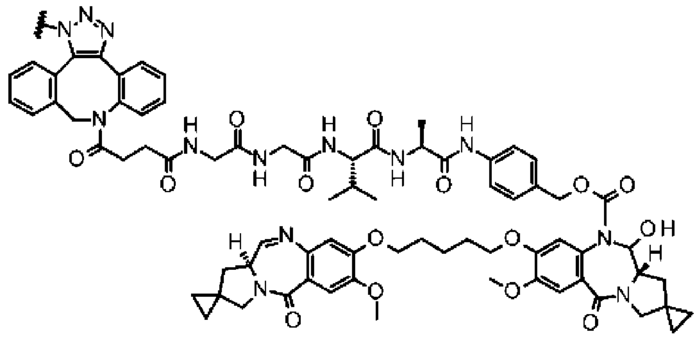

or

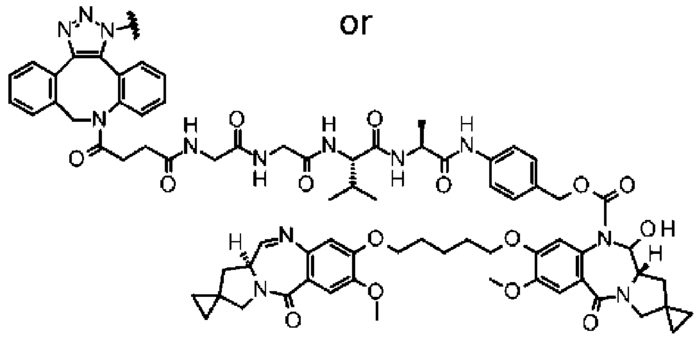

The triazole ring to be formed in step 1 has geometric isomers, and the compound obtained in step 1 of Example 19 has a linker as a mixture of the two structures shown above as R.

Step 1: Conjugation of antibody and drug-linker

To a 10 mM acetate buffer, 5% sorbitol (pH 5.5) solution of the antibody (10.0 mg/mL, 100 μL) obtained in step 2 of Example 13, 1,2-propanediol (25 μL) and a mixed solution of a 10 mM dimethyl sulfoxide solution of the compound obtained in step 10 of Example 10-5 (5-11) (8 μL; 12 equivalents per antibody molecule) and 1,2-propane diol (67 μL) were added at room temperature, and the resultant was reacted using a tube rotator (MTR-103, AS ONE Corporation) at room temperature for 48 hours.

Purification operation: The solution was purified by using common operation D to afford 1.2 mL of a solution of the desired compound.

Characterization: The following characteristic values were obtained by using common operations E and F.

Antibody concentration: 0.53 mg/mL, antibody yield: 0.64 mg (64%), average number of conjugated drug molecules per antibody molecule (n): 1.9

[Example 20: ADC4]

The ADC described in Example 20 was synthesized by conjugating the antibody obtained in step 2 of Example 13 with the drug-linker obtained in step 12 of Example 10-6 (6-13) .

[Formula 74]

R =

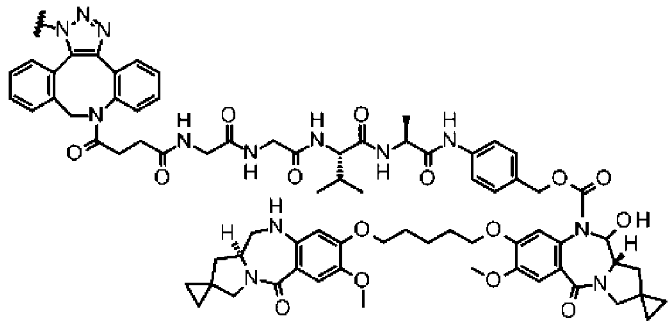

or

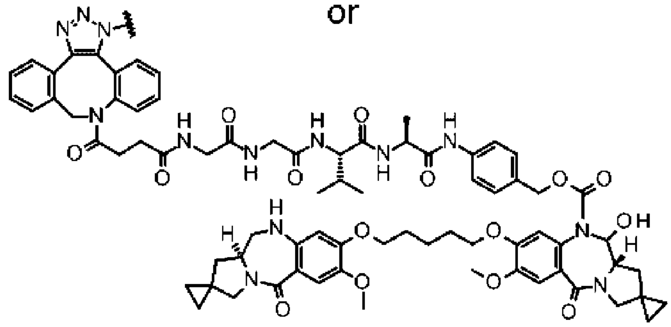

The triazole ring to be formed in step 1 has geometric isomers, and the compound obtained in step 1 of Example 20 has a linker as a mixture of the two structures shown above as R.

Step 1: Conjugation of antibody and drug-linker

To a 10 mM acetate buffer, 5% sorbitol (pH 5.5) solution of the antibody (10.0 mg/mL, 100 μL) obtained in step 2 of Example 13, 1,2-propanediol (25 μL) and a mixed solution of a 10 mM dimethyl sulfoxide solution of the compound obtained in step 12 of Example 10-6 (6-13) (8 μL; 12 equivalents per antibody molecule) and 1,2-propane diol (67 μL) were added at room temperature, and the resultant was reacted using a tube rotator (MTR-103, AS ONE Corporation) at room temperature for 48 hours.

Purification operation: The solution was purified by using common operation D to afford 1.2 mL of a solution of the desired compound.

Characterization: The following characteristic values were obtained by using common operations E and F.

Antibody concentration: 0.56 mg/mL, antibody yield: 0.67 mg (67%), average number of conjugated drug molecules per antibody molecule (n): 1.9

[Example 21: ADC5]

The ADC described in Example 21 was synthesized by conjugating the antibody obtained in step 2 of Example 13 with the drug-linker obtained in step 13 of Example 10-3 (3-14).

[Formula 75]

R =

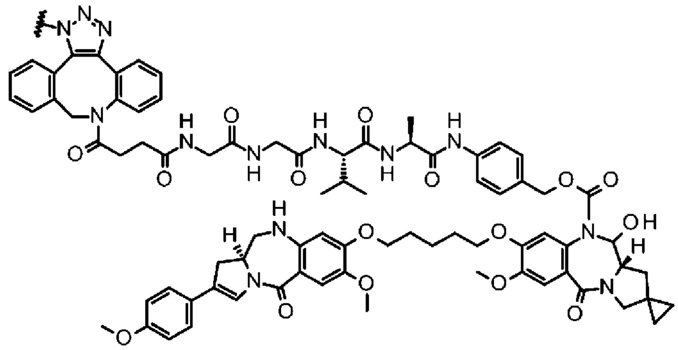

or

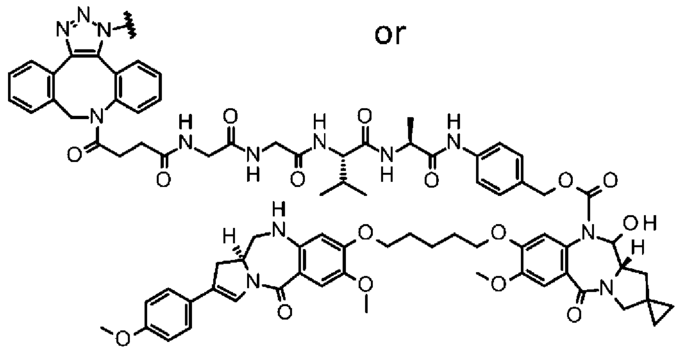

The triazole ring to be formed in step 1 has geometric isomers, and the compound obtained in step 1 of Example 21 has a linker as a mixture of the two structures shown above as R.

Step 1: Conjugation of antibody and drug-linker

To a 10 mM acetate buffer, 5% sorbitol (pH 5.5) solution of the antibody (10.0 mg/mL, 1.70 mL) obtained in step 2 of Example 13, 1,2-propanediol (0.850 mL) and a mixed solution of a 10 mM dimethyl sulfoxide solution of the compound obtained in step 13 of Example 10-3 (3-14) (0.141 mL; 12 equivalents per antibody molecule) and 1,2-propane diol (0.710 mL) were added at room temperature, and the resultant was reacted using a tube rotator (MTR-103, AS ONE Corporation) at room temperature for 48 hours.

Purification operation: The solution was purified by using common operation D to afford 10.5 mL of a solution of the desired compound.

Characterization: The following characteristic values were obtained by using common operations E and F.

Antibody concentration: 1.19 mg/mL, antibody yield: 12.5 mg (73%), average number of conjugated drug molecules per antibody molecule (n): 1.8

[Example 22: ADC6]

The ADC described in Example 22 was synthesized, as illustrated in the following reaction formula, by conjugating the antibody obtained in step 2 of Example 14 with the drug-linker obtained in step 13 of Example 10-3 (3-14). In the formula, R represents the drug-linker used in the Example.

[Formula 76]

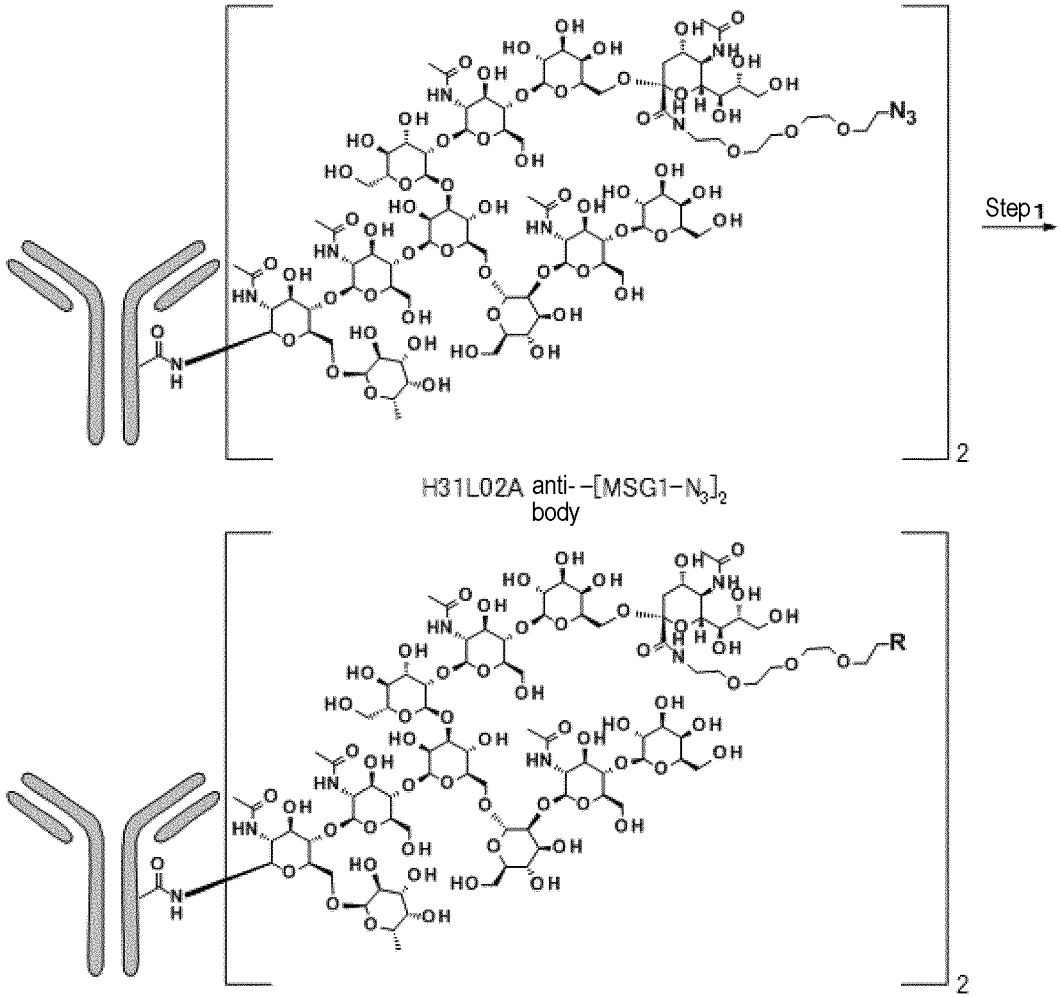


H31L02A antibody-[MSG1-$N_3$]$_2$

[Formula 77]

R =

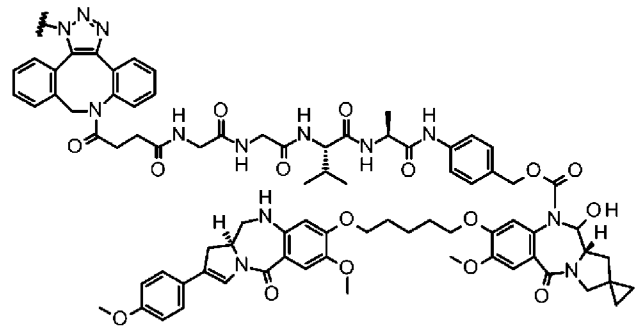

or

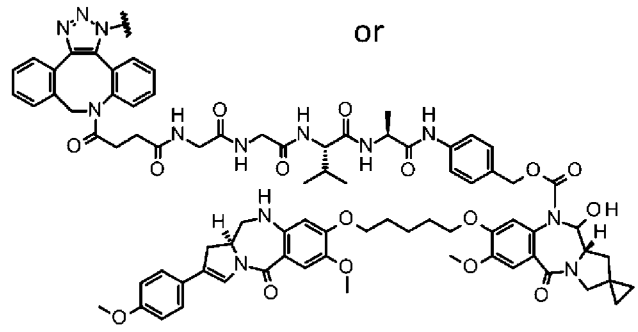

The triazole ring to be formed in step 1 has geometric isomers, and the compound obtained in step 1 of Example 22 has a linker as a mixture of the two structures shown above as R.

Step 1: Conjugation of antibody and drug-linker

To a phosphate buffered saline (pH 6.0) solution of the antibody (10.1 mg/mL, 0.400 mL) obtained in step 2 of Example 14, 1,2-propanediol (0.367 mL) and a 10 mM dimethyl sulfoxide solution of the compound obtained in step 13 of Example 10-3 (3-14) (0.0333 mL; 12 equivalents per antibody molecule) were added at room temperature, and the resultant was reacted using a tube rotator (MTR-103, AS ONE Corporation) at room temperature for 48 hours.

Purification operation: The solution was purified by using common operation D to afford 3.50 mL of a solution of the desired compound.

Characterization: The following characteristic values were obtained by using common operations E and F.

Antibody concentration: 0.820 mg/mL, antibody yield: 2.88 mg (81%), average number of conjugated drug molecules per antibody molecule (n): 1.9

The ADCs described in Examples 23 to 26 were synthesized, as illustrated in the following reaction formula, by conjugating the antibody obtained in step 2 of Example 15 with the drug-linker obtained in Examples 10-3 to 10-6. In the formula, R differs between the drug-linkers used in those Examples.

[Example 23: ADC7]

[Formula 78]

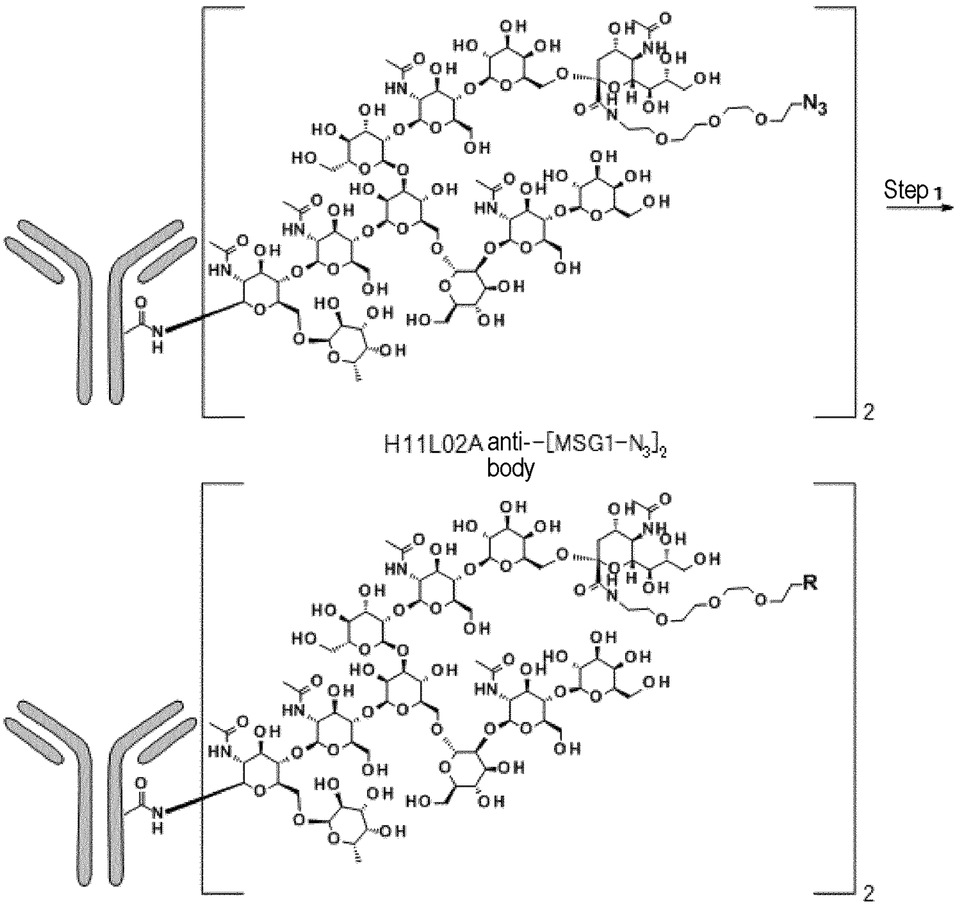


[Formula 79]

R =

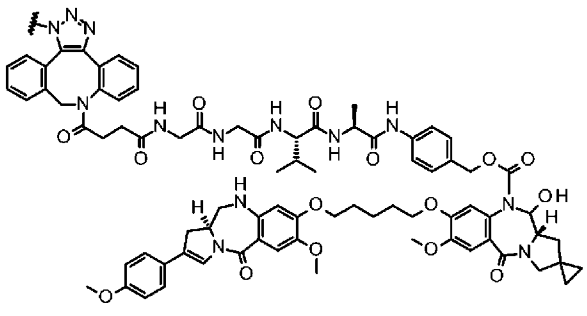

or

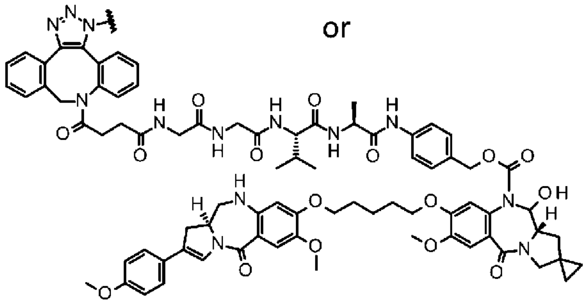

The triazole ring to be formed in step 1 has geometric isomers, and the compound obtained in step 1 of Example 23 has a linker as a mixture of the two structures shown above as R.

Step 1: Conjugation of antibody and drug-linker

To a 10 mM acetate buffer, 5% sorbitol (pH 5.5) solution of the antibody (10.54 mg/mL, 0.4 mL) obtained in step 2 of Example 15, 1,2-propanediol (0.100 mL) and a mixed solution of a 10 mM dimethyl sulfoxide solution of the compound obtained in step 13 of Example 10-3 (3-14) (0.035 mL; 12 equivalents per antibody molecule) and 1,2-propane diol (0.266 mL) were added at room temperature, and the resultant was reacted using a tube rotator (MTR-103, AS ONE Corporation) at room temperature for 48 hours.

Purification operation: The solution was purified by using common operation D to afford 2.5 mL of a solution of the desired compound.

Characterization: The following characteristic values were obtained by using common operations E and F.

Antibody concentration: 1.01 mg/mL, antibody yield: 2.53 mg (73%), average number of conjugated drug molecules per antibody molecule (n): 1.9

[Example 24: ADC8]

[Formula 80]

R =

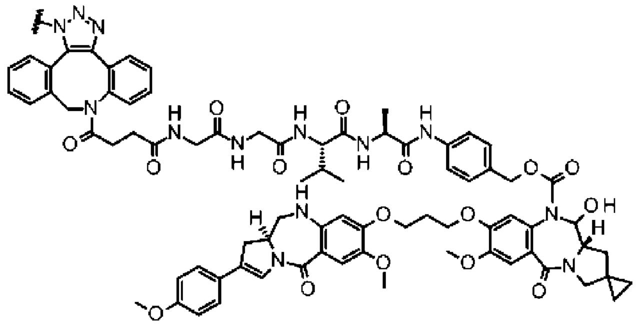

or

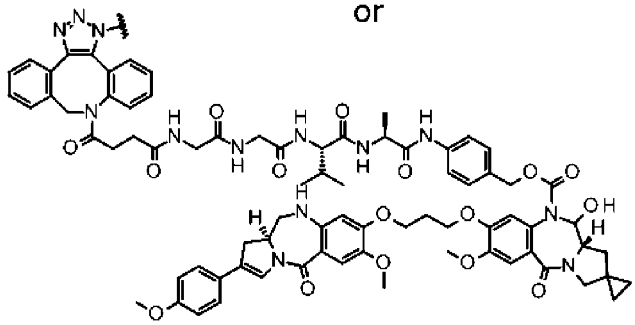

The triazole ring to be formed in step 1 has geometric isomers, and the compound obtained in step 1 of Example 24 has a linker as a mixture of the two structures shown above as R.

Step 1: Conjugation of antibody and drug-linker

To a 10 mM acetate buffer, 5% sorbitol (pH 5.5) solution of the antibody (2.8 mg/mL, 0.3 mL) obtained in step 2 of Example 15, 1,2-propanediol (75 μL) and a mixed solution of a 10 mM dimethyl sulfoxide solution of the compound obtained in step 12 of Example 10-4 (4-12) (7 μL; 12 equivalents per antibody molecule) and 1,2-propane diol (218 μL) were added at room temperature, and the resultant was reacted using a tube rotator (MTR-103, AS ONE Corporation) at room temperature for 48 hours.

Purification operation: The solution was purified by using common operation D to afford 2.5 mL of a solution of the desired compound.

Characterization: The following characteristic values were obtained by using common operations E and F.

Antibody concentration: 0.22 mg/mL, antibody yield: 0.56 mg (67%), Average number of conjugated drug molecules per antibody molecule (n): 1.8

[Example 25: ADC9]

[Formula 81]

R = 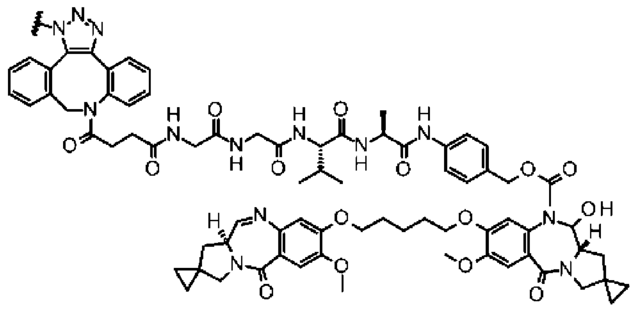

or

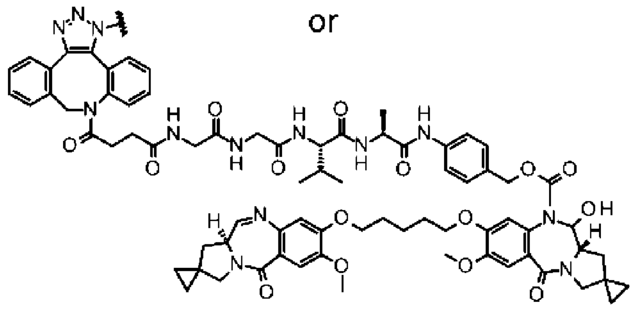

The triazole ring to be formed in step 1 has geometric isomers, and the compound obtained in step 1 of Example 25 has a linker as a mixture of the two structures shown above as R.

Step 1: Conjugation of antibody and drug-linker

To a 10 mM acetate buffer, 5% sorbitol (pH 5.5) solution of the antibody (2.8 mg/mL, 300 μL) obtained in step 2 of Example 15, 1,2-propanediol (75 μL) and a mixed solution of a 10 mM dimethyl sulfoxide solution of the compound obtained in step 10 of Example 10-5 (5-11) (7 μL; 12 equivalents per antibody molecule) and 1,2-propane diol (218 μL) were added at room temperature, and the resultant was reacted using a tube rotator (MTR-103, AS ONE Corporation) at room temperature for 48 hours.

Purification operation: The solution was purified by using common operation D to afford 2.5 mL of a solution of the desired compound.

Characterization: The following characteristic values were obtained by using common operations E and F.

Antibody concentration: 0.25 mg/mL, antibody yield: 0.62 mg (64%), average number of conjugated drug molecules per antibody molecule (n): 1.8

[Example 26: ADC10]

[Formula 82]

R =

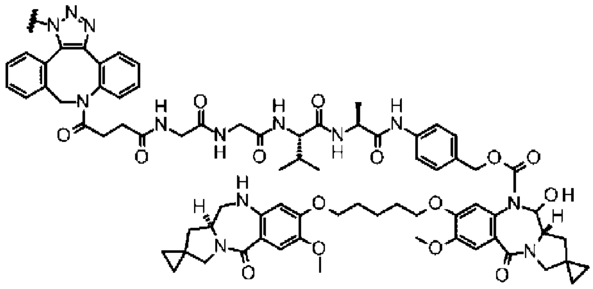

or

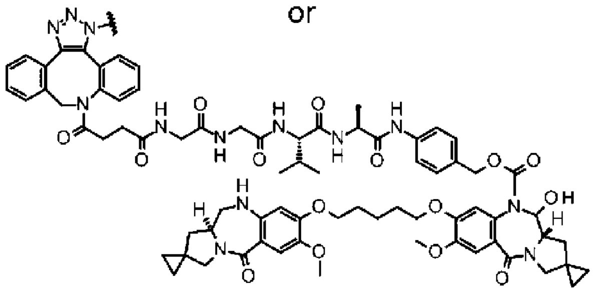

The triazole ring to be formed in step 1 has geometric isomers, and the compound obtained in step 1 of Example 26 has a linker as a mixture of the two structures shown above as R.

Step 1: Conjugation of antibody and drug-linker

To a 10 mM acetate buffer, 5% sorbitol (pH 5.5) solution of the antibody (2.8 mg/mL, 300 μL) obtained in step 2 of Example 15, 1,2-propanediol (75 μL) and a mixed solution of a 10 mM dimethyl sulfoxide solution of the compound obtained in step 12 of Example 10-6 (6-13) (7 μL; 12 equivalents per antibody molecule) and 1,2-propane diol (218 μL) were added at room temperature, and the resultant was reacted using a tube rotator (MTR-103, AS ONE Corporation) at room temperature for 48 hours.

Purification operation: The solution was purified by using common operation D to afford 2.5 mL of a solution of the desired compound.

Characterization: The following characteristic values were obtained by using common operations E and F.

Antibody concentration: 0.25 mg/mL, antibody yield: 0.62 mg (74%), average number of drug molecules conjugated per antibody molecule (n): 1.8

The ADC described in Example 27 was synthesized, as illustrated in the following reaction formula, by conjugating the antibody obtained in step 2 of Example 16 with the drug-linker obtained in step 13 of Example 10-3 (3-14). In the formula, R represents the drug-linker used in the Example.

[Example 27: ADC11]

[Formula 83]

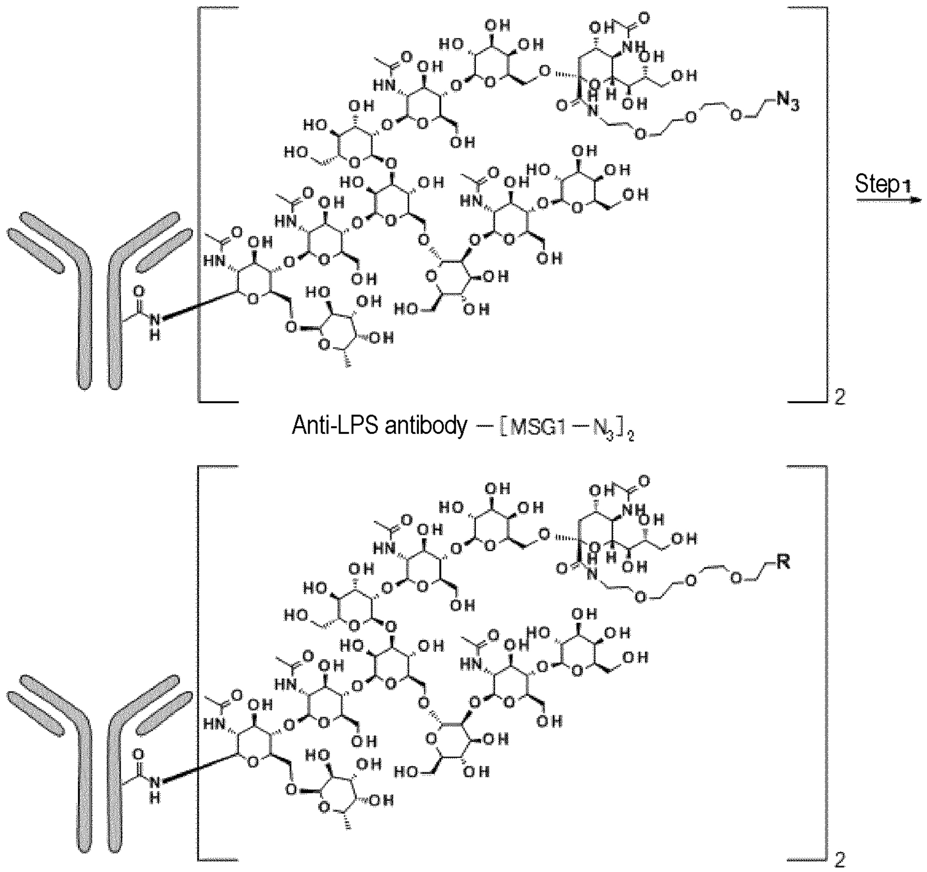


Anti-LPS antibody — [MSG1 — $N_3$]$_2$

[Formula 84]

R =

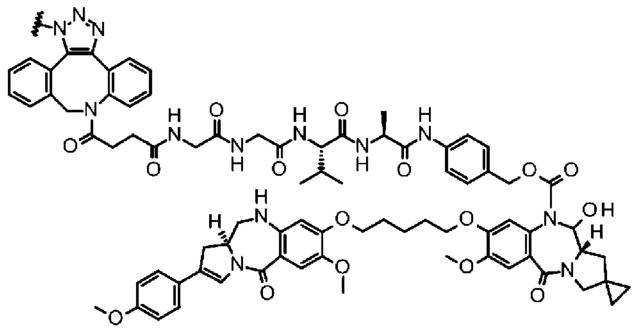

or

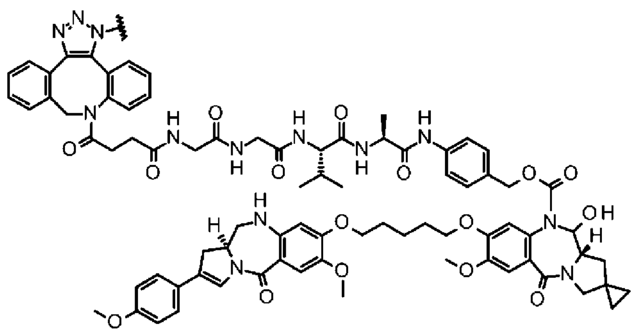

The triazole ring to be formed in step 1 has geometric isomers, and the compound obtained in step 1 of Example

27 has two types of the linkers different in structure.

Step 1: Conjugation of antibody and drug-linker

To a phosphate buffered saline (pH 6.0) solution of the antibody (9.89 mg/mL, 0.40 mL) obtained in step 2 of Example 16, 1,2-propanediol (0.367 mL) and a 10 mM dimethyl sulfoxide solution of the compound obtained in step 13 of Example 10-3 (3-14) (0.0328 mL; 12 equivalents per antibody molecule) were added at room temperature, and the resultant was reacted using a tube rotator (MTR-103, AS ONE Corporation) at room temperature for two days.

Purification operation: The solution was purified by using common operation D to afford 2.50 mL of a solution of the desired compound.

Characterization: The following characteristic values were obtained by using common operations E and F.

Antibody concentration: 1.16 mg/mL, antibody yield: 2.89 mg (72%), average number of conjugated drug molecules per antibody molecule (n): 1.8

[Example 28] Configuration analysis of the compound (1-11) shown in Example 10-1

The compound (1-11) described in [Example 10-1: intermediate 1] was analyzed for absolute steric configuration of the 11'-position based on the correlation (following figure) obtained by Selective 1D ROESY spectrum. Correlation was observed between 1'α-H and 11'-H; between 3'α-H and 11'-H; and between 1'β-H and 3'β-H. From the analysis, it was found that the absolute steric configuration of the 11'-position is S configuration.

[Formula 85]

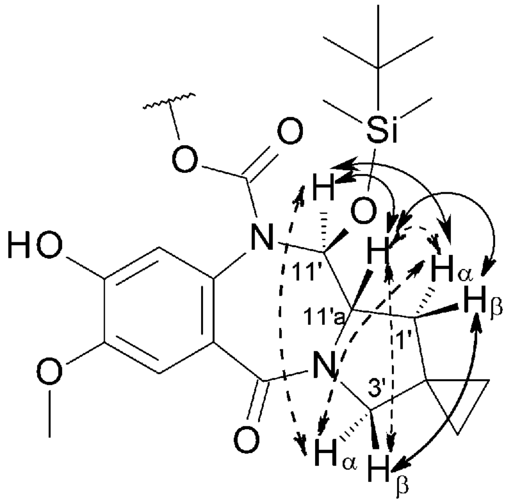


Significant correlation obtained in Selective 1D ROESY spectrum 1H-NMR used in correlation analysis by Selective 1D ROESY spectrum

$^1$HNMR (500 MHz, $CDCl_3$, 27°C) δ: 8.76 (1H, s), 7.43 (2H, brd), 7.20 (1H, s), 7.08 (2H, d, J=8.3 Hz), 7.00 (1H, br), 6.66 (1H, s), 6.44 (1H, s), 6.00 (1H, H11', d, J11', 11'a = 9.2 Hz), 5.89 (1H, m), 5.53 (1H, brd), 5.30 (1H, d, J = 17.2 Hz), 5.20 (1H, d, J=10.3 Hz), 5.15 (1H, d, JABq = 12.5 Hz), 4.85 (1H, d, JABq = 12.5 Hz), 4.66 (1H, m), 4.604.52 (2H, m), 4.07 (1H, m), 3.84 (3H, s), 3.71 (1H, H-3' β, d, Jgem = 11.7 Hz), 3.53 (1H, H-11'a, m), 3.26 (1H, H-3'α, d, Jgem = 11.7 Hz), 2.35 (1H, H-1' β, dd, J1' β, 11'a = 8.30 Hz, Jgem = 13.1 Hz), 2.14 (1H, m), 1.54 (1H, H-1' α, d, Jgem = 13.1 Hz), 1.41 (3H, d, J = 6.90 Hz), 0.95 (3H, d, J = 6.80 Hz), 0.92 (3H, d, J = 6.80 Hz), 0.81 (9H, s), 0.80-0.70 (1H, m), 0.70-0.59 (3H, m), 0.2-0.06 (6H, m)

As a result, it was determined that absolute steric configurations of drug-linkers 1,2 and 4 and the 11'-position of these synthetic intermediates, which were synthesized by using compounds (1-9), (1-10) and (1-11) described in [Example 10-1: Intermediate 1] and compound (1-11) are each S. It was also determined that absolute steric configurations of drug-linker 3 and an intermediate (the 11'-position) are each S.

Accordingly, steps 1 to 10 described in [Example 10-1: intermediate 1] are shown as follows:

[Formula 86]

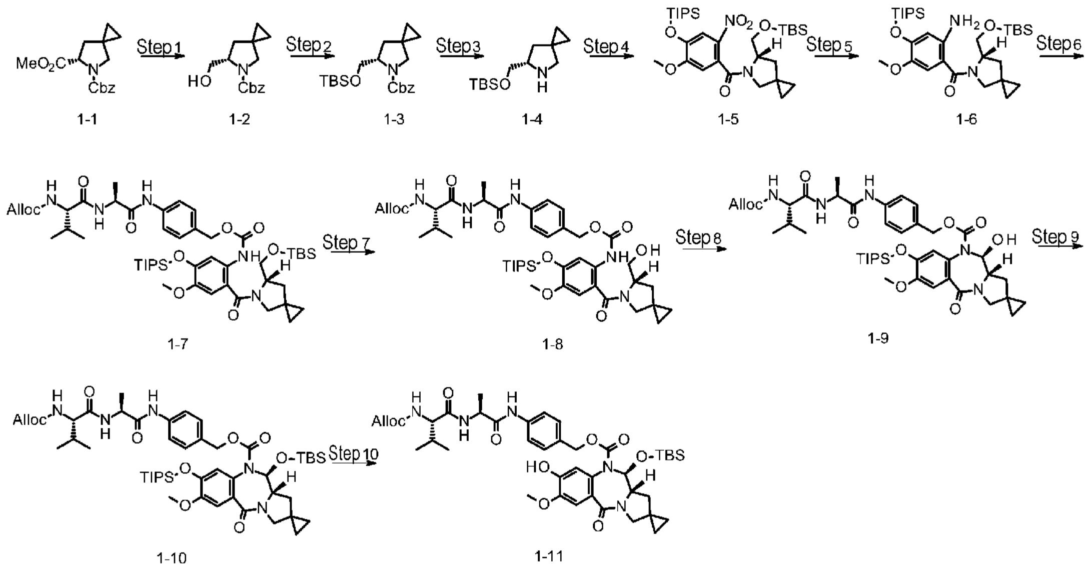


1-1 1-2 1-3 1-4 1-5 1-6 1-7 1-8 1-9 1-10 1-11

(1-9): N-[(prop-2-en-1-yloxy)carbonyl]-L-valyl-N-{4-[({[(11'S,11a'S)-11'-hydroxy-7'-methoxy-5'-oxo-8'-{[tri(propan-2-yl)silyl]oxyl-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

(1-10): N-[(prop-2-en-1-yloxy)carbonyl]-L-valyl-N-{4-[({[(11'S,11a'S)-11'-{[tert-butyl(dimethyl)silyl]oxy}-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

(1-11): N-[(prop-2-en-1-yloxy)carbonyl]-L-valyl-N-{4-[({[(11'S,11a'S)-11'-{[tert-butyl(dimethyl)silyl]oxy}-8'-hydroxy-7'-methoxy-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

Steps 1 to 13 shown in [Example 10-3: Drug-linker 1] are shown as follows:

[Formula 87]

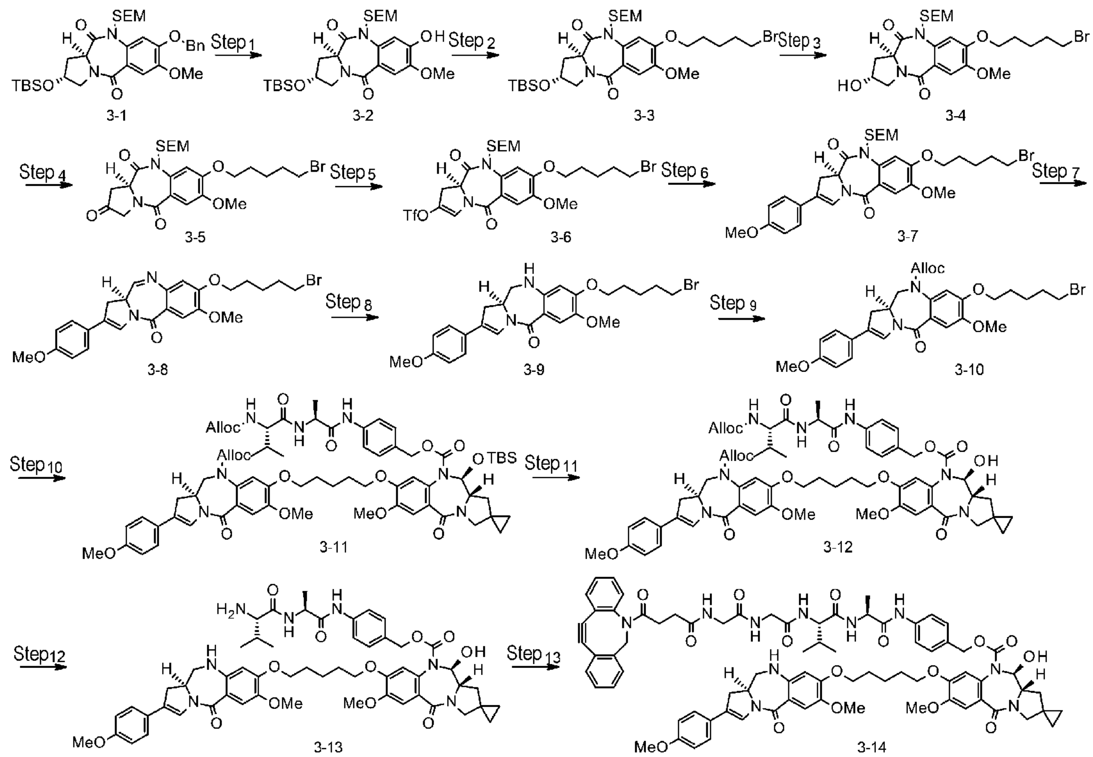

(3-11): N-[(prop-2-en-1-yloxy)carbonyl]-L-valyl-N-{4-[({[(11'S,11a'S)-11'-{[tert-butyl(dimethyl)silyl]oxy}-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c] [1,4]benzodiazepine-8-yl}oxy)pentyl]oxy}-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

(3-12): N-[(prop-2-en-1-yloxy)carbonyl]-L-valyl-N-{4-[({[(11'S,11a'S)-11'-hydroxy-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c][1,4]benzodiazepine-8-yl}oxy)pentyl]oxy}-5'-oxo-11',11a'-dihydro-1'H-spiro[cyclopropane-1,2'-pyrrolo[2,1-c] [1,4]benzodiazepine]-10' (5'H)-yl]carbonyl}oxy)methyl]phenyl}-L-alanine amide

(3-13): L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepine-8-yl]oxy}pentyl)oxy]-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide (3-13)

(3-14): N-[4-(11,12-didehydrodibenzo[b,f]azocine-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepine-8-yl]oxy}pentyl)oxy]-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

Steps 1 to 12 shown in [Example 10-4: Drug-linker 2] are shown as follows.

[Formula 88]

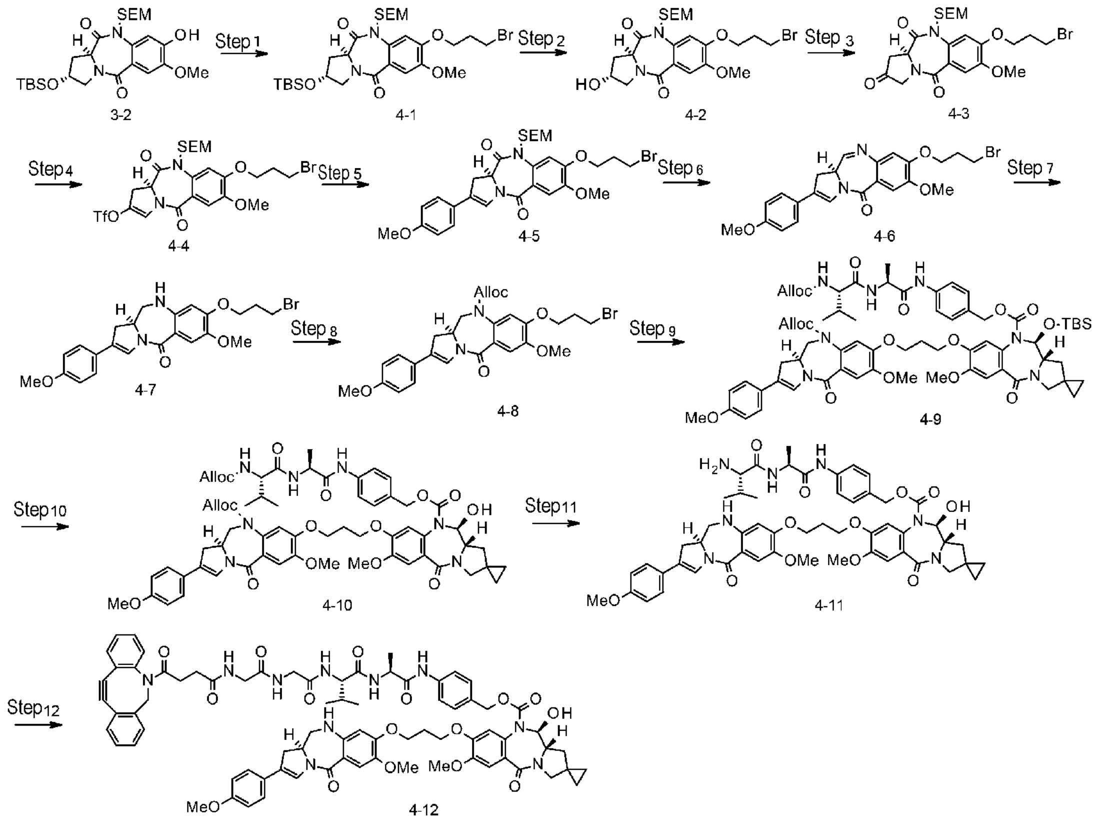


(4-9): N-{[(prop-2-en-1-yl)oxy]carbonyl}-L-valyl-N-[4-({[(11'S,11'aS)-11'-{[tert-butyl(dimethyl)silyl]oxy}-7'-methoxy-8'-(3-{[(11aS)-7-methoxy-2-(4-methoxyphenyl)-5-oxo-10-{[(prop-2-en-1-yl)oxy]carbonyl}-5,10,11,11a-tetrahydro-1H-pyrrolo[2,1-c][1,4]benzodiazepine-8-yl]oxy}propoxy)-5'-oxo-11',11'a-dihydro-1'H, 3'H-spiro[cyclopropane-1,2'-pyrrolo[2,1-c][1,4]benzodiazepine]-10'(5'H)-carbonyl]oxy}methyl) phenyl]-L-alanine amide

(4-10): N-{[(prop-2-en-1-yl)oxy]carbonyl}-L-valyl-N-[4-({[(11'S,11'aS)-11'-hydroxy-7'-methoxy-8'-(3-{[(11aS)-7-methoxy-2-(4-methoxyphenyl)-5-oxo-10-{ [(prop-2-en-1-yl)oxy]carbonyl}-5,10,11,11a-tetrahydro-1H-pyrrolo[2,1-c][1,4]benzodiazepine-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl) phenyl]-L-alanine amide

(4-11): L-valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepine-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl) phenyl]-L-alanine amide

(4-12): N-[4-(11,12-didehydrodibenzo[b,f]azocine-5(6H)-yl)-4-oxobutanoyl]glycylglycyl-L-valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepine-8-yl]oxy}propoxy)-5'-oxo-11',11'a-dihydro-1'H,3'H-spiro[cyclopropane-1,2'-pyrrolo[2,1-c] [1,4]benzodiazepine]-10' (5'H)-carbonyl]oxy}methyl) phenyl]-L-alanine amide

Steps 1 to 10 shown in [Example 10-5: Drug-linker 3] are shown as follows.

[Formula 89]

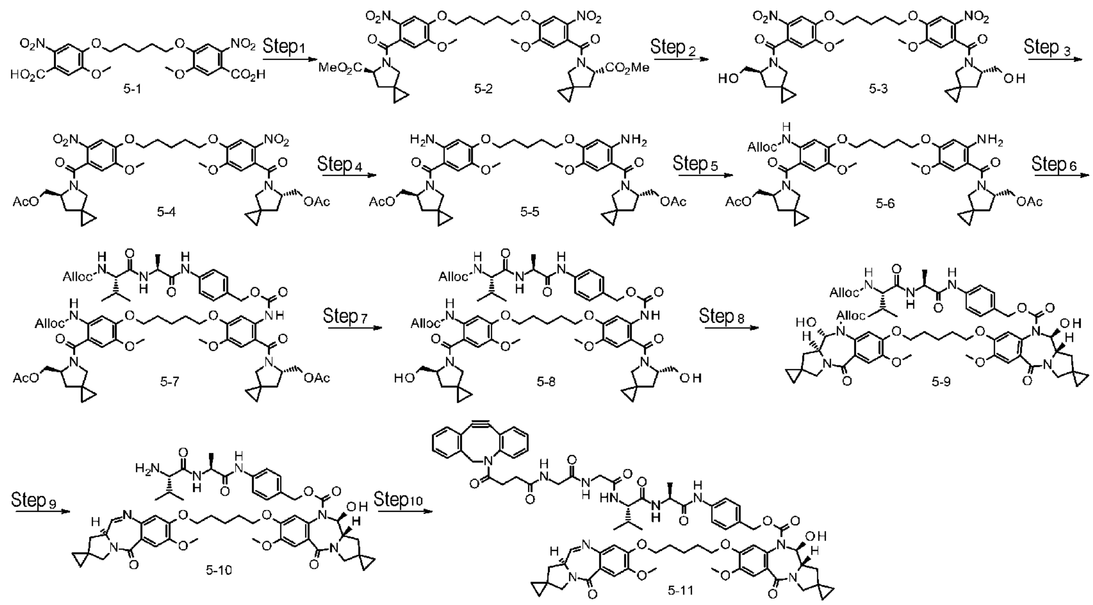


(5-9): N-[(2-propen-1-yloxy)carbonyl]-L-valyl-N-{4-[({[(11'S,11a'S)-11'-hydroxy-8'-{[5-({(11'S,11a'S)-11'-hydroxy-7'-methoxy-5'-oxo-10'-[(2-propen-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-7'-methoxy-5'-oxo-11',11a'-dihydro-1'H-spiro[cyclopropane-1,2'-pyrrolo[2,1-c] [1,4]benzodiazepine]-10' (5'H)-yl]carbonyl}oxy)methyl]phenyl}-L-alanine amide

(5-10): L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

(5-11): N-[4-(11,12-didehydrodibenzo[b,f]azocine-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5', 11a'-dihydro-1'H-spiro[cyclopropa ne-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

Step 1 to 12 shown in [Example 10-6: Drug-linker 4] are shown as follows:

[Formula 90]

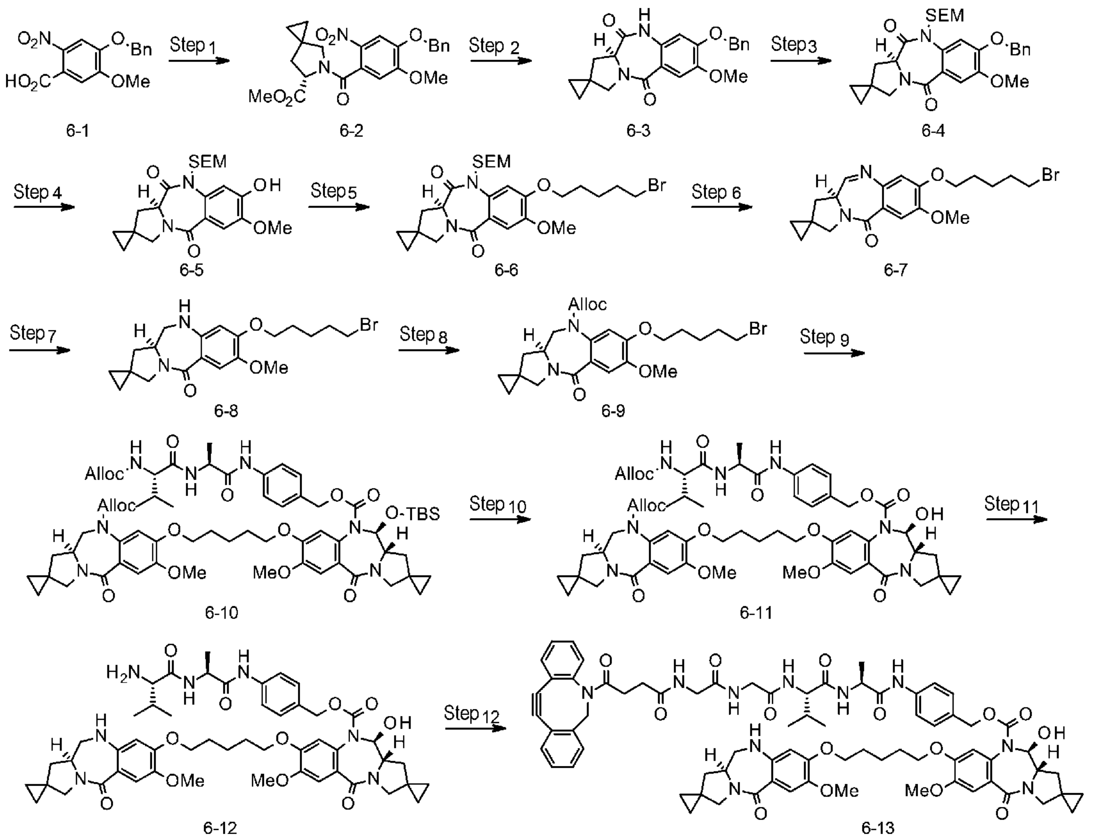


(6-10): N-[(prop-2-en-1-yloxy)carbonyl]-L-valyl-N-{4-[({[(11'S,11a'S)-11'-{[tert-butyl(dimethyl)silyl]oxy}-7'-methoxy-8'-{[5-({(11a'S)-7'-methoxy-5'-oxo-10'-[(prop-2-en-1-yloxy)carbonyl]-5',10',11',11a'-tetrahydro'H-spiro[cyclopropane-1,2'-pyrrolo[2, 1-c][1,4]benzodiazepine]-8'-yl}oxy)pentyl]oxy}-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

(6-11): N-[(prop-2-en-1-yloxy)carbonyl]-L-valyl-N-{4-[({[(11'S,11a'S)-11'-hydroxy-7'-methoxy-8'-{[5-({ (11a'S)-7'-methoxy-5'-oxo-10'-[(prop-2-en-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

(6-12): L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c] [1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

(6-13): N-[4-(11,12-didehydrodibenzo[b,f]azocine-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10' (5'H)-yl]carbonyl}oxy)methyl]phenyl}-L-alanine amide

In the formulas of ADCs obtained in Examples 17 to 27, R represents the following.

R described in Example 17, 21,22, 23 or 27:

[Formula 91]

R =

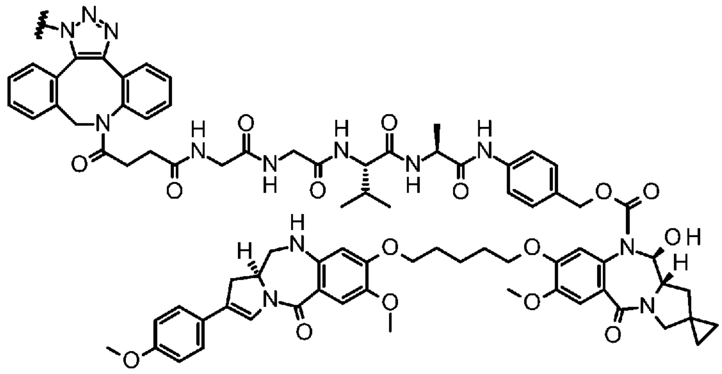

or

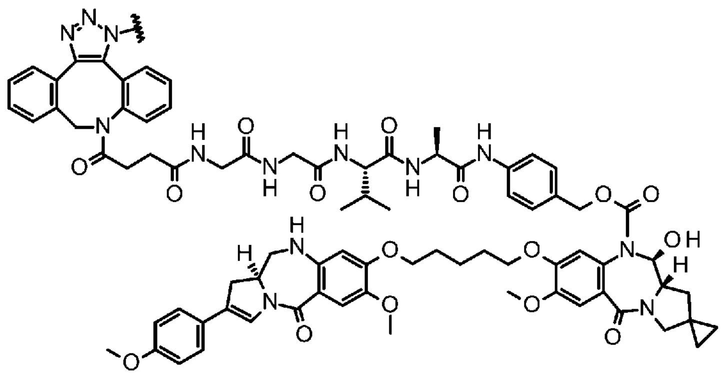

R described in Example 18 or 24:

[Formula 92]

R =

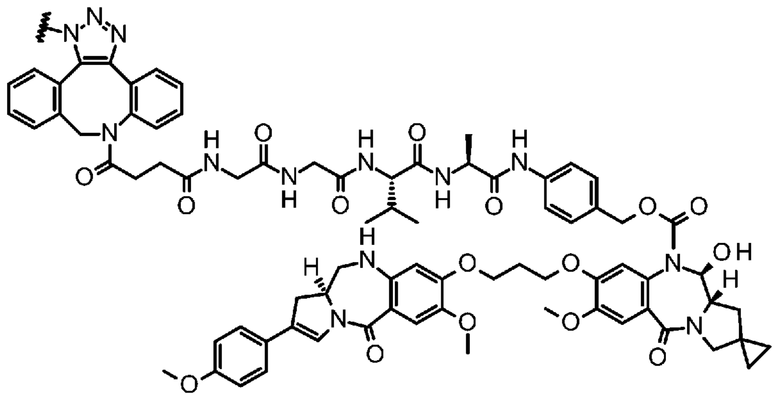

or

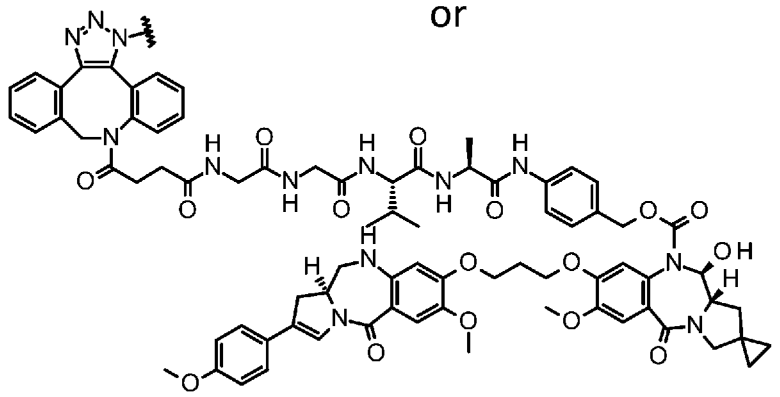

R described in Example 19 or 25:

[Formula 93]

R =

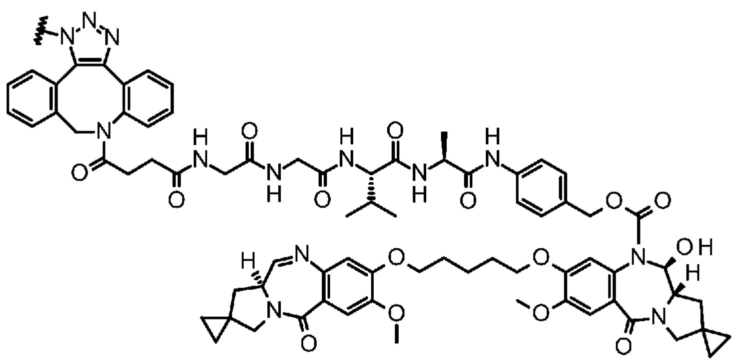

or

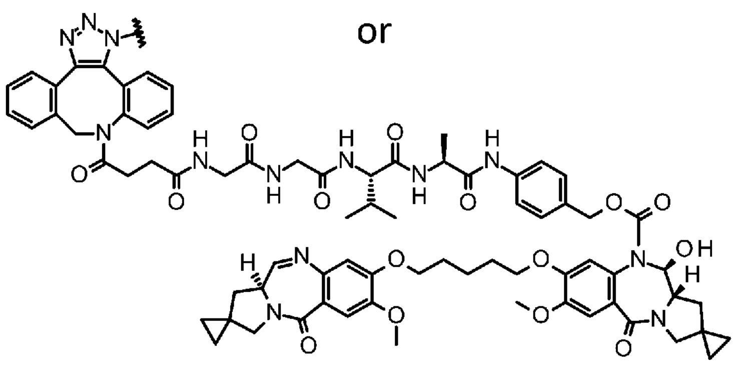

R described in Example 20 or 26:

[Formula 94]

R =

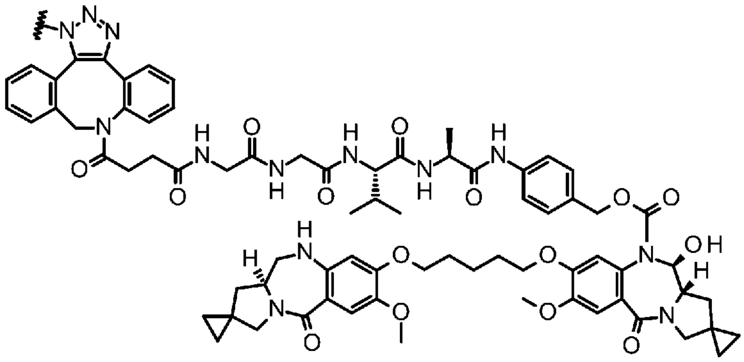

or

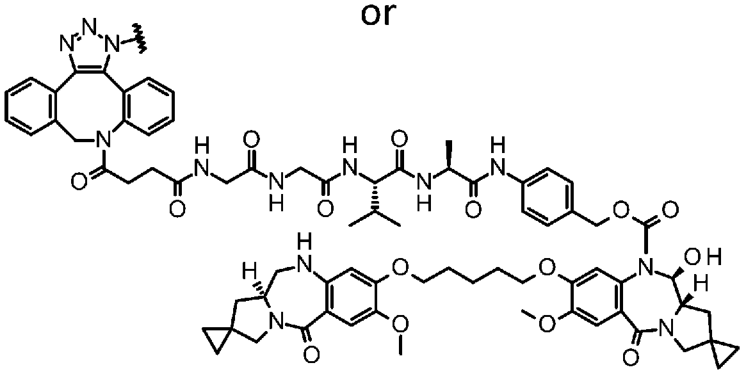

[Reference Example 2: Production of NOV0712-drug conjugate]

Reference Example 2)-1 Production of antibody-drug conjugate NOV0712-DM4

Conjugation of antibody and drug-linker: NOV0712 produced in Reference Example 1 was adjusted to 9.7 mg/mL with 20 mM HEPES8.1 (HEPES, 1 M Buffer Solution (20 mL) manufactured by Life Technologies Corp. was pH-adjusted to 8.1 with 1 M sodium hydroxide, and then brought to 1 L with distilled water) by using common procedures B (using 1.51 $mLmg^{-1}cm^{-1}$ as 280 nm absorption coefficient) and C described in production method 1. The solution was incubated at 20°C for 10 minutes. Subsequently, a 10 mM solution of 1-(2,5-dioxopyrrolidin-1-yloxy)-1-oxo-4-(pyridin-2-yldisulfanyl)butane-2-sulfonic acid described in WO2016/024195 in DMA (0.366 mL; 5.2 equivalents per antibody molecule), a 10 mM solution of N2'-deacetyl-deacetyl-N2'-(4-methyl-4-mercapto-1-oxopentyl)-maytansine (DM4) in DMA (0.366 mL; 6.8 equivalents per antibody molecule), and 0.243 mL of DMA were added thereto, and the obtained mixture was incubated at 20°C for 16 hours to conjugate the drug linker to the antibody. Subsequently, an aqueous solution of 1 M acetic acid was added thereto to adjust the pH to 5.0, and the obtained mixture was further stirred at room temperature for 20 minutes to terminate the reaction of the drug linker.

Purification: The above-described solution was purified by common procedure D described in production method 1 to obtain 28 mL of a solution containing the title antibody-drug conjugate "NOV0712-DM4".

Characterization: Using common procedure E (using $\varepsilon_{A,280}$ = 200500, $\varepsilon_{A,252}$ = 76295, $\varepsilon_{D,280}$ = 43170, and $\varepsilon_{D,252}$ = 23224) described in production method 1, the following characteristic values were obtained.

Antibody concentration: 2.58 mg/mL, antibody yield: 72.2 mg (93%), average number of conjugated drug molecules per antibody molecule (n): 3.0

[Example 29: Evaluation of *in vitro* activity of antibody-drug conjugate]

Evaluation of *in vitro* cell growth inhibition activity of antibody-drug conjugate against CDH6-positive human tumor cell line

Human ovarian tumor cell lines NIH: OVCAR-3, OV-90 and PA-1, and human renal-cell tumor cell line 786-O (all obtained from ATCC), in which expression of CDH6 was confirmed in Example 2)-3, were cultured under conditions

of 37°C and 5% $CO_2$ and seeded over a 96-well plate such that each cell line has a density (per 100 μL/well) described in Figure 9 and cultured under conditions of 37°C and 5% $CO_2$. The following day, each of an antibody-drug conjugates, e.g., ADC11 (prepared in Example 27), ADC1 (prepared in Example 17), ADC5 (prepared in Example 21), ADC7 (prepared in Example 23) and ADC6 (prepared in Example 22) diluted in 5-fold common ratio was added so as to obtain a final concentration 100 (nM) to 0.000256 (nM). After culturing for 6 days, the number of live cells was measured by the quantification of ATP using CellTiter-Glo™ Luminescent Cell viability Assay (Promega). The IC50 value representing cell growth inhibitory activity was calculated in accordance with the following expression and the third place after the decimal point was rounded to the second decimal point.

$$\text{IC50 (nM)} = \text{antilog}\ ((50-d) \times (\text{LOG10}(b)-\text{LOG10}(a)) / (d-c) + \text{LOG10}\ (b))$$

a: test agent concentration a
b: test agent concentration b
c: viable-cell rate at test agent concentration a
d: viable-cell rate at test agent concentration b
a, b satisfy the relationship: a > b at two points sandwiching a viable cell rate of 50%

IC50 values (nM) of cell lines to which individual antibody-drug conjugates are added are shown in Figure 9. In three ovarian tumor cell lines exhibiting a high CDH6 expression level *in vitro,* IC50 values of three types of anti-CDH6 antibody-drug conjugates are extremely low, compared to IC50 value of ADC11, i.e., an antibody-drug conjugate not binding to CDH6. From this, it was demonstrated that these three types of anti-CDH6 antibody-drug conjugates have an extremely strong cell growth inhibitory activity in a CDH6-expression-specific manner, in addition, the binding activity (Table 3) of an antibody to CDH6 has a correlation with the cell growth inhibitory activity of an antibody-drug conjugate. Also in a renal-cell tumor cell line having a low *in-vitro* CDH6 expression level, a similar tendency was confirmed (Figure 9).

[Example 30: *In vivo* antitumor effect 1 of antibody-drug conjugate]

The antitumor effects of the antibody-drug conjugates were evaluated using animal models derived from immunodeficient mice by the inoculation of CDH6-positive human tumor cell line cells. Four- to 5-week-old BALB/c nude mice (CAnN.Cg-Foxnl[nu]/CrlCrlj[Foxnlnu/Foxnlnu], Charles River Laboratories Japan Inc.) and SCID mice (CB17/Icr-Prkdc[scid]/CrlCrlj, Charles River Laboratories Japan Inc.) were acclimatized for 3 days or longer under SPF conditions before use in the experiment. The mice were fed with a sterilized solid diet (FR-2, Funabashi Farms Co., Ltd) and given sterilized tap water (which had been prepared by adding a 5 to 15 ppm sodium hypochlorite solution to tap water). The long diameter and short diameter of the inoculated tumor were measured twice a week using electronic digital calipers (CD-15CX, Mitutoyo Corp.), and the volume of the tumor was then calculated according to the following expression.

$$\text{Tumor volume (mm}^3) = 1/2 \times \text{Long diameter (mm)} \times [\text{Short diameter (mm)}]^2$$

Each antibody-drug conjugate was diluted with ABS buffer (10 mM acetate buffer, 5% sorbitol, pH 5.5) (Nacalai Tesque, Inc.), and the dilution was intravenously administered at a dose shown in each Example to the tail of each mouse. ABS buffer was administered in the same manner as above to a control group (vehicle group). Six mice per group were used in the experiment.

30)-1 Anti-tumor effect (1)

The CDH6-positive human ovarian tumor cell line OV-90 (ATCC), the CDH6 expression of which had been confirmed in Example 2)-3, was suspended in Matrigel (Corning Inc.), and the cell suspension was subcutaneously inoculated at a dose of $2.5 \times 10^6$ cells to the right flank region of each female nude mouse (Day 0). On Day 14, the mice were randomly grouped. On the day of grouping, ADC1 produced in Example 17, or ADC11 produced in Example 27 was intravenously administered at doses of 0.4 mg/kg to the tail of each mouse. Also, NOV0712-DM4 produced in Reference Example 2)-1 was intravenously administered at doses of 10 mg/kg to the tail of each mouse. The results are shown in Figure 10. The abscissa depicts the number of days after administration, and the ordinate depicts tumor

volume. The error range depicts a SE value.

In this tumor model administered with NOV0712-DM4, regrowth of tumor was observed 30 days after administration; however, ADC1 exhibited a strong tumor regression effect at an extremely low dose of 0.4 mg/kg and sustained the strong anti-tumor effect for a long period of 42 days after administration. ADC11 as a control, which binds neither mouse tumor cells nor inoculated human tumor cells used in the anti-tumor test, did not show an anti-tumor effect. It was confirmed that there is no weight loss in all drug administration groups, compared to the control group (the ABS buffer administration group).

30)-2 Anti-tumor effect (2)

The CDH6-positive human renal cell tumor cell line Caki-1 (ATCC), the CDH6 expression of which had been confirmed in Example 2)-3, was suspended in Matrigel (Corning Inc.), and the cell suspension was subcutaneously inoculated at a dose of $2.5 \times 10^6$ cells to the right flank region of each female nude mouse (Day 0). On Day 13, the mice were randomly grouped. On the day of grouping, the antibody-drug conjugates ADC1 produced in Example 17, or ADC11 produced in Example 27 was intravenously administered at a dose of 0.4 mg/kg to the tail of each mouse. Also, NOV0712-DM4 produced in Reference Example 2)-1 was intravenously administered at doses of 10 mg/kg to the tail of each mouse. The results are shown in Figure 11. The abscissa depicts the number of days after administration, and the ordinate depicts tumor volume. The error range depicts a SE value.

In this tumor model, NOV0712-DM4 exhibited no antitumor effect at the dose of 10 mg/kg. On the other hand, ADC1 exerted an antitumor effect at an extremely low dose of 0.4 mg/kg. ADC11 as a control, which binds neither mouse tumor cells nor inoculated human tumor cells used in the antitumor test, did not show an anti-tumor effect at a dose of 0.4 mg/kg (Figure 11). From this, it was shown that the anti-tumor effect exhibited by ADC1 is specifically obtained depending on the expression of CDH6 in tumor cells. It was demonstrated that ADC1 of the present invention is an antibody-drug conjugate having an extremely strong anti-tumor effect compared to conventional conjugate, NOV0712-DM4. It was confirmed that there is no weight loss in all drug administration groups, compared to the control group (the ABS buffer administration group).

30)-3 Anti-tumor effect (3)

The CDH6-positive human ovarian tumor cell line NIH:OVCAR-3 (ATCC), the CDH6 expression of which had been confirmed in Example 2)-3, was suspended in Matrigel (Corning Inc.), and the cell suspension was subcutaneously inoculated at a dose of $1 \times 10^7$ cells to the right flank region of each female nude mouse (Day 0). On Day 28, the mice were randomly grouped. On the day of grouping, ADC1 produced in Example 17, ADC5 produced in Example 21, ADC6 produced in Example 22, or ADC11 produced in Example 27 was intravenously administered at doses of 0.4 mg/kg to the tail of each mouse. The results are shown in Figure 12. The abscissa depicts the number of days after administration, and the ordinate depicts tumor volume. The error range depicts a SE value.

In this tumor model, ADC6 exhibited the strongest anti-tumor effect, ADC1 and ADC5 exhibited almost the same second strongest anti-tumor effect. From this, it was shown that substitution of leucine residues at positions 234 and 235 (specified based on the EU index) of a human-antibody heavy chain with alanine residues hardly at all affects the anti-tumor effect of an antibody-drug conjugate. ADC11 as a control did not exhibit an anti-tumor effect at a dose of 0.4 mg/kg (Figure 12). From this, it was shown that the antitumor effects exhibited by ADC1, ADC5 and ADC5 are specifically obtained depending on the expression of CDH6 in tumor cells. It was confirmed that there is no weight loss in all drug administration groups, compared to the control group (the ABS buffer administration group).

30)-4 Anti-tumor effect (4)

The CDH6-positive human ovarian tumor cell line OV-90 (ATCC), the CDH6 expression of which had been confirmed in Example 2)-3, was suspended in Matrigel (Corning Inc.), and the cell suspension was subcutaneously inoculated at a dose of $2.5 \times 10^6$ cells to the right flank region of each female nude mouse (Day 0). On Day 17, the mice were randomly grouped. On the day of grouping, ADC1 produced in Example 17, ADC5 produced in Example 21, ADC7 produced in Example 23, or ADC6 produced in Example 22 was intravenously administered at doses of 0.2 mg/kg or 0.4 mg/kg to the tail of each mouse. The results are shown in Figure 13. The abscissa depicts the number of days after administration, and the ordinate depicts tumor volume. The error range depicts a SE value.

In this tumor model, ADC5 and ADC6 each exhibited the strongest anti-tumor effect at a dose of 0.4 mg/kg and ADC7 exhibited the second strongest anti-tumor effect at a dose of 0.4 mg/kg (Figure 13). From the results of administration of ADC5 (0.4 mg/kg) and ADC5 (0.2 mg/kg), it was confirmed that the anti-tumor effect changes depending on the dose. At a dose of 0.2 mg/kg, ADC1 and ADC5 exhibited substantially the same anti-tumor effect. From this, it was shown that substitution of leucine residues at positions 234 and 235 (specified based on the EU index) of a human

antibody heavy chain with alanine residues hardly at all affects the anti-tumor effect of an antibody-drug conjugate. It was confirmed that there is no weight loss in all drug administration groups, compared to the control group (the ABS buffer administration group).

30)-5 Anti-tumor effect (5)

The CDH6-positive human ovarian tumor cell line PA-1 (ATCC), the CDH6 expression of which had been confirmed in Example 2)-3, was suspended in Matrigel (Corning Inc.), and the cell suspension was subcutaneously inoculated at a dose of $7.5 \times 10^6$ cells to the right flank region of each female nude mouse (Day 0). On Day 17, the mice were randomly grouped. On the day of grouping, ADC5 produced in Example 21, ADC6 produced in Example 22, or ADC11 produced in Example 27 was intravenously administered at doses of 0.4 mg/kg to the tail of each mouse. The results are shown in Figure 14. The abscissa depicts the number of days after administration, and the ordinate depicts tumor volume. The error range depicts a SE value.

In this tumor model, ADC5 exhibited the strongest anti-tumor effect. It was confirmed that tumors are not observed for a long period of 35 days. ADC6 exhibited the second strongest anti-tumor effect (Figure 14). It was confirmed that there is no weight loss in all drug administration groups, compared to the control group (the ABS buffer administration group).

30)-6 Anti-tumor effect (6)

The CDH6-positive human renal cell tumor cell line 786-O (ATCC), the CDH6 expression of which had been confirmed in Example 2)-3, was suspended in Matrigel (Corning Inc.), and the cell suspension was subcutaneously inoculated at a dose of $5 \times 10^6$ cells to the right flank region of each male SCID mouse (Day 0). On Day 38, the mice were randomly grouped. On the day of grouping, ADC5 produced in Example 21, or ADC6 produced in Example 22 was intravenously administered at a dose of 0.4 mg/kg to the tail of each mouse. Also, ADC11 produced in Example 27 was intravenously administered at a dose of 0.4 mg/kg to the tail of each mouse. The results are shown in Figure 15. The abscissa depicts the number of days after administration, and the ordinate depicts tumor volume. The error range depicts a SE value.

In this tumor model, ADC5 and ADC6 exhibited a strong anti-tumor effect (Figure 15). ADC11 (control) did not exhibit anti-tumor effect (Figure 15). From this, it was shown that anti-tumor effects of ADC5 and ADC6 are specifically obtained depending on the expression of CDH6 in tumor cells. It was confirmed that there is no weight loss in all drug administration groups, compared to the control group (the ABS buffer administration group).

Industrial Applicability

The present invention provided an anti-CDH6 antibody having internalization activity and an antibody-drug conjugate comprising the antibody. The antibody-drug conjugate can be used as a therapeutic drug for cancer, and the like.

Free text of sequence listing

SEQ ID No: 1: Human CDH6 ORF
SEQ ID No: 2: EC1
SEQ ID No: 3: EC2
SEQ ID No: 4: EC3
SEQ ID No: 5: EC4
SEQ ID No: 6: EC5
SEQ ID No: 7: Cynomolgus monkey CDH6 ORF
SEQ ID No: 8: Cynomolgus monkey CDH6 primer 1
SEQ ID No: 9: Cynomolgus monkey CDH6 primer 2
SEQ ID No: 10: rG019 light-chain variable region amino acid sequence
SEQ ID No: 11: rG019 light-chain variable region nucleotide sequence
SEQ ID No: 12: rG019 CDRL1
SEQ ID No: 13: rG019 CDRL2
SEQ ID No: 14: rG019 CDRL3
SEQ ID No: 15: rG019 heavy-chain variable region amino acid sequence
SEQ ID No: 16: rG019 heavy-chain variable region nucleotide sequence

SEQ ID No: 17: rG019 CDRH1
SEQ ID No: 18: rG019 CDRH2
SEQ ID No: 19: rG019 CDRH3
SEQ ID No: 20: DNA fragment comprising DNA sequence encoding human light chain signal sequence and human κ chain constant region
SEQ ID No: 21: DNA fragment comprising DNA sequence encoding human heavy chain signal sequence and human IgG1 constant region
SEQ ID No: 22: DNA fragment comprising a DNA fragment encoding chG019 light chain
SEQ ID No: 23: chG019 light chain full-length amino acid sequence
SEQ ID No: 24: chG019 light chain full-length nucleotide sequence
SEQ ID No: 25: chG019 light-chain variable region nucleotide sequence
SEQ ID No: 26: chG019 heavy chain full-length amino acid sequence
SEQ ID No: 27: chG019 heavy chain full-length nucleotide sequence
SEQ ID No: 28: chG019 heavy-chain variable region amino acid sequence
SEQ ID No: 29: chG019 heavy-chain variable region nucleotide sequence
SEQ ID No: 30: chG019 CDRH2
SEQ ID No: 31: hL02 light chain full-length amino acid sequence
SEQ ID No: 32: hL02 light chain full-length nucleotide sequence
SEQ ID No: 33: hL02 light-chain variable region amino acid sequence
SEQ ID No: 34: hL02 light-chain variable region nucleotide sequence
SEQ ID No: 35: hL03 light chain full-length amino acid sequence
SEQ ID No: 36: hL03 light chain full-length nucleotide sequence
SEQ ID No: 37: hL03 light-chain variable region amino acid sequence
SEQ ID No: 38: hL03 light-chain variable region nucleotide sequence
SEQ ID No: 39: hH01heavy chain full-length amino acid sequence
SEQ ID No: 40: hH01 heavy chain full-length nucleotide sequence
SEQ ID No: 41: hH01 heavy-chain variable region amino acid sequence
SEQ ID No: 42: hH01 heavy-chain variable region nucleotide sequence
SEQ ID No: 43: hH02 heavy chain full-length amino acid sequence
SEQ ID No: 44: hH02 heavy chain full-length nucleotide sequence
SEQ ID No: 45: hH02 heavy-chain variable region amino acid sequence
SEQ ID No: 46: hH02 heavy-chain variable region nucleotide sequence
SEQ ID No: 47: hH04 heavy chain full-length amino acid sequence
SEQ ID No: 48: hH04 heavy chain full-length nucleotide sequence
SEQ ID No: 49: hH04 heavy-chain variable region amino acid sequence
SEQ ID No: 50: hH04 heavy-chain variable region nucleotide sequence
SEQ ID No: 51: NOV0712 light chain full-length amino acid sequence
SEQ ID No: 52: Nucleotide sequence encoding amino acid sequence shown in SEQ ID No. 51
SEQ ID No: 53: NOV0712 heavy chain full-length amino acid sequence
SEQ ID No: 54: Nucleotide sequence encoding amino acid sequence shown in SEQ ID No. 53
SEQ ID No: 55: hH11 heavy-chain variable region amino acid sequence
SEQ ID No: 56: hH11 heavy-chain variable region nucleotide sequence of
SEQ ID No: 57: hH11 CDRH1
SEQ ID No: 58: hH11 CDRH2
SEQ ID No: 59: hH11 CDRH3
SEQ ID No: 60: hH31 heavy-chain variable region amino acid sequence
SEQ ID No: 61: hH31 heavy-chain variable region nucleotide sequence
SEQ ID No: 62: hH31 CDRH1
SEQ ID No: 63: hH31 CDRH2
SEQ ID No: 64: hH31 CDRH3
SEQ ID No: 65: hH01A heavy chain full-length amino acid sequence
SEQ ID No: 66: hH01A heavy chain full-length nucleotide sequence
SEQ ID No: 67: hH11A heavy chain full-length amino acid sequence
SEQ ID No: 68: hH11A heavy chain full-length nucleotide sequence
SEQ ID No: 69: hH31A heavy chain full-length amino acid sequence
SEQ ID No: 70: hH31A heavy chain full-length nucleotide sequence
SEQ ID No: 71: DNA fragment comprising DNA sequence encoding amino acid of human heavy chain signal sequence and human IgG1 LALA constant region

SEQ ID No: 72: anti-LPS antibody light chain full-length amino acid sequence
SEQ ID No: 73: anti-LPS antibody heavy chain full-length amino acid sequence

## Claims

**1.** An antibody-drug conjugate represented by the following formula (X):

[Formula 1]

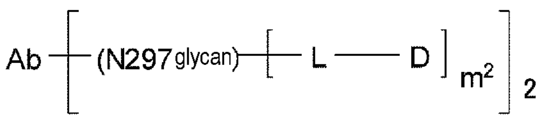

(X)

wherein $m^2$ represents an integer of 1 or 2,

Ab represents an IgG antibody specifically binding to an amino acid sequence comprising the amino acid sequence shown in SEQ ID NO: 4 and having internalization ability that permits cellular uptake, or a functional fragment thereof,
L represents a linker linking a glycan bonding to N297 of Ab (N297 glycan) and D,
N297 glycan may be a remodeled glycan, and
D is any one of the following formulas:

[Formula 2]

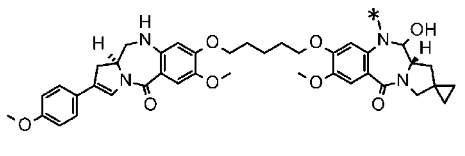
,
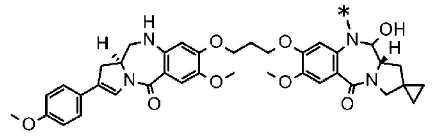
,
(XI)

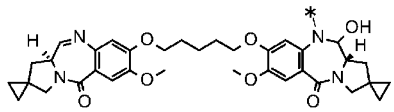
or
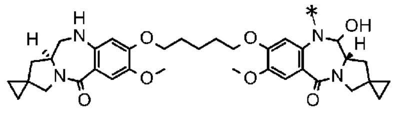

wherein the asterisk (*) represents bonding to L.

2. The antibody-drug conjugate according to claim 1, wherein the antibody or a functional fragment thereof exhibits competitive inhibitory activity, for binding to the amino acid sequence comprising the amino acid sequence shown in SEQ ID NO: 4, against at least any one antibody selected from the group consisting of the following antibodies (1) to (8):

(1) an antibody having a light chain consisting of the amino acid sequence at positions 21 to 233 in SEQ ID NO: 23 and a heavy chain consisting of the amino acid sequence at positions 20 to 471 in SEQ ID NO: 26,
(2) an antibody having a light chain consisting of the amino acid sequence at positions 21 to 233 in SEQ ID NO: 31 and a heavy chain consisting of the amino acid sequence at positions 20 to 471 in SEQ ID NO: 39,
(3) an antibody having a light chain consisting of the amino acid sequence at positions 21 to 233 in SEQ ID NO: 31 and a heavy chain consisting of the amino acid sequence at positions 20 to 471 in SEQ ID NO: 43,
(4) an antibody having a light chain consisting of the amino acid sequence at positions 21 to 233 in SEQ ID NO: 35 and a heavy chain consisting of the amino acid sequence at positions 20 to 471 in SEQ ID NO: 43,
(5) an antibody having a light chain consisting of the amino acid sequence at positions 21 to 233 in SEQ ID NO: 31 and a heavy chain consisting of the amino acid sequence at positions 20 to 471 in SEQ ID NO: 47,
(6) an antibody having a light chain consisting of the amino acid sequence at positions 21 to 233 in SEQ ID NO: 31 and a heavy chain consisting of the amino acid sequence at positions 20 to 471 in SEQ ID NO: 65,
(7) an antibody having a light chain consisting of the amino acid sequence at positions 21 to 233 in SEQ ID NO: 31 and a heavy chain consisting of the amino acid sequence at positions 20 to 471 in SEQ ID NO: 67, and
(8) an antibody having a light chain consisting of the amino acid sequence at positions 21 to 233 in SEQ ID NO: 31 and a heavy chain consisting of the amino acid sequence at positions 20 to 471 in SEQ ID NO: 69.

3. The antibody-drug conjugate according to claim 1 or 2, wherein the antibody or a functional fragment thereof comprises CDRL1, CDRL2 and CDRL3; and CDRH1, CDRH2 and CDRH3 selected from the group consisting of the following (1) to (4) :

(1) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and CDRH1 consisting of the amino acid sequence shown in SEQ ID NO: 17, CDRH2 consisting of the amino acid sequence shown in SEQ ID NO: 18 and CDRH3 consisting of the amino acid sequence shown in SEQ ID NO: 19,
(2) CDRL1 consisting of the amino acid sequence shown in SEQ ID NO: 12, CDRL2 consisting of the amino acid sequence shown in SEQ ID No: 13 and CDRL3 consisting of the amino acid sequence shown in SEQ ID NO: 14; and CDRH1 consisting of the amino acid sequence shown in SEQ ID NO: 17, CDRH2 consisting of the amino acid sequence shown in SEQ ID NO: 30 and CDRH3 consisting of the amino acid sequence shown in SEQ ID NO: 19,
(3) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and CDRH1 consisting of the amino acid sequence shown in SEQ ID NO: 57, CDRH2 consisting of the amino acid sequence shown in SEQ ID NO: 58 and CDRH3 consisting of the amino acid sequence shown in SEQ ID NO: 59, and
(4) CDRL1 consisting of the amino acid sequence shown in SEQ ID NO: 12, CDRL2 consisting of the amino

acid sequence shown in SEQ ID NO: 13 and CDRL3 consisting of the amino acid sequence shown in SEQ ID NO: 14 and CDRH1 consisting of the amino acid sequence shown in SEQ ID NO: 62, CDRH2 consisting of the amino acid sequence shown in SEQ ID NO: 63 and CDRH3 consisting of the amino acid sequence shown in SEQ ID NO: 64.

**4.** The antibody-drug conjugate according to any one of claims 1 to 3, wherein the antibody or a functional fragment thereof comprises any one light chain variable region selected from the group consisting of the following variable regions (1) to (4):

(1) a light chain variable region consisting of the amino acid sequence shown in SEQ ID NO: 33,
(2) a light chain variable region consisting of the amino acid sequence shown in SEQ ID NO: 37,
(3) an amino acid sequence having a sequence identity of at least 95% to the sequence of a framework region except CDR sequences in the amino acid sequence of (1) or (2) and
(4) an amino acid sequence having a deletion, substitution or addition of one or several amino acids in the sequence of a framework region except CDR sequences in the amino acid sequences of (1) to (3); and

any one heavy chain variable region selected from the group consisting of the following variable regions (5) to (11) :

(5) a heavy chain variable region consisting of the amino acid sequence shown in SEQ ID NO: 41,
(6) a heavy chain variable region consisting of the amino acid sequence shown in SEQ ID NO: 45,
(7) a heavy chain variable region consisting of the amino acid sequence shown in SEQ ID NO: 49,
(8) a heavy chain variable region consisting of the amino acid sequence shown in SEQ ID NO: 55,
(9) a heavy chain variable region consisting of the amino acid sequence shown in SEQ ID NO: 60,
(10) an amino acid sequence having a sequence identity of at least 95% to the sequence of a framework region other than at each CDR sequence in the amino acid sequences of (5) to (9), and
(11) an amino acid sequence having a deletion, substitution or addition of one or several amino acids in the sequence of a framework region other than at each CDR sequence in the amino acid sequences of (5) to (10).

**5.** The antibody-drug conjugate according to any one of claims 1 to 4, wherein the antibody or a functional fragment thereof comprises any one of the following (1) to (6) :

(1) a light chain variable region consisting of the amino acid sequence shown in SEQ ID NO: 33 and a heavy chain variable region consisting of the amino acid sequence shown in SEQ ID NO: 41,
(2) a light chain variable region consisting of the amino acid sequence shown in SEQ ID NO: 33 and a heavy chain variable region consisting of the amino acid sequence shown in SEQ ID NO: 45,
(3) a light chain variable region consisting of the amino acid sequence shown in SEQ ID NO: 37 and a heavy chain variable region consisting of the amino acid sequence shown in SEQ ID NO: 45,
(4) a light chain variable region consisting of the amino acid sequence shown in SEQ ID NO: 33 and a heavy chain variable region consisting of the amino acid sequence shown in SEQ ID NO: 49,
(5) a light chain variable region consisting of the amino acid sequence shown in SEQ ID NO: 33 and a heavy chain variable region consisting of the amino acid sequence shown in SEQ ID NO: 55, and
(6) a light chain variable region consisting of the amino acid sequence shown in SEQ ID NO: 33 and a heavy chain variable region consisting of the amino acid sequence shown in SEQ ID NO: 60.

**6.** The antibody-drug conjugate according to any one of claims 1 to 5, wherein the antibody or a functional fragment thereof has any one of the following (1) to (7):

(1) a light chain consisting of the amino acid sequence at positions 21 to 233 in SEQ ID NO: 31 and a heavy chain consisting of the amino acid sequence at positions 20 to 471 in SEQ ID NO: 39,
(2) a light chain consisting of the amino acid sequence at positions 21 to 233 in SEQ ID NO: 31 and a heavy chain consisting of the amino acid sequence at positions 20 to 471 in SEQ ID NO: 43,
(3) a light chain consisting of the amino acid sequence at positions 21 to 233 in SEQ ID NO: 35 and a heavy chain consisting of the amino acid sequence at positions 20 to 471 in SEQ ID NO: 43,
(4) a light chain consisting of the amino acid sequence at positions 21 to 233 in SEQ ID NO: 31 and a heavy chain consisting of the amino acid sequence at positions 20 to 471 in SEQ ID NO: 47,
(5) a light chain consisting of the amino acid sequence at positions 21 to 233 in SEQ ID NO: 31 and a heavy chain consisting of the amino acid sequence at positions 20 to 471 in SEQ ID NO: 65,
(6) a light chain consisting of the amino acid sequence at positions 21 to 233 in SEQ ID NO: 31 and a heavy

chain consisting of the amino acid sequence at positions 20 to 471 in SEQ ID NO: 67, and
(7) a light chain consisting of the amino acid sequence at positions 21 to 233 in SEQ ID NO: 31 and a heavy chain consisting of the amino acid sequence at positions 20 to 471 in SEQ ID NO: 69.

**7.** The antibody-drug conjugate according to claim 6, wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 39.

**8.** The antibody-drug conjugate according to claim 6, wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 43.

**9.** The antibody-drug conjugate according to claim 6, wherein the antibody or a functional fragment thereof has a light chain consisting of the amino acid sequence at positions 21 to 233 in SEQ ID NO: 35 and a heavy chain consisting of the amino acid sequence at positions 20 to 471 in SEQ ID NO: 43.

**10.** The antibody-drug conjugate according to claim 6, wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 47.

**11.** The antibody-drug conjugate according to claim 6, wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 65.

**12.** The antibody-drug conjugate according to claim 6, wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 67.

**13.** The antibody-drug conjugate according to claim 6, wherein the antibody or a functional fragment thereof has a light chain consisting of the amino acid sequence at positions 21 to 233 in SEQ ID NO: 31 and a heavy chain consisting of the amino acid sequence at positions 20 to 471 in SEQ ID NO: 69.

**14.** The antibody-drug conjugate according to any one of claims 1 to 13, wherein

L is represented by $-Lb-La-Lp-NH-B-CH_2-O(C=O)-*$, wherein the asterisk * represents bonding to the nitrogen atom at the N10'-position of D,
B represents a 1,4-phenyl group, a 2,5-pyridyl group, a 3,6-pyridyl group, a 2,5-pyrimidyl group or a 2,5-thienyl group,
Lp represents any one selected from the group consisting of:
-GGVA-, -GG-(D-)VA-, -VA-, -GGFG-, -GGPI-, -GGVCit-, - GGVK- and -GGPL-,
La represents any one selected from the group consisting of:

$- C(=O)-CH_2CH_2-C(=O)$
$- C(=O)-(CH_2CH_2)_2-C(=O)-$,
$- C(=O)-CH_2CH_2-C(=O)-NH-(CH_2CH_2)_2-C(=O)-$,
$- C(=O)-CH_2CH_2-C(=O)-NH-(CH_2CH_2O)_2-CH_2-C(=O)-$,
$- C(=O)-CH_2CH_2-NH-C(=O)-(CH_2CH_2O)_4-CH_2CH_2-C(=O)-$,
$- CH_2-OC(=O)-$ and
$- OC(=O)-$,

and
Lb represents the following formula:

[Formula 3]

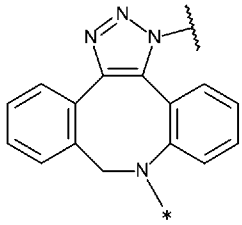

or

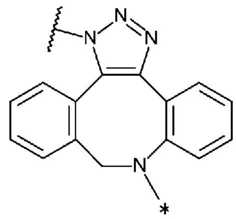

[Formula 4]

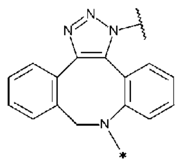

or

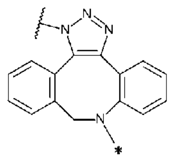

or

[Formula 5]

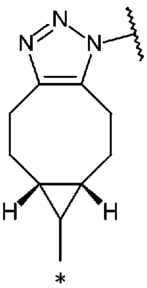

or

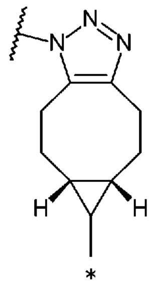

wherein, in each structural formula of Lb shown above, each asterisk * represents bonding to La, and each wavy line represents bonding to the glycan presented by Ab or a remodeled glycan.

**15.** The antibody-drug conjugate according to any one of claims 1 to 14, wherein

L represents any one selected from the group consisting of the following structural formulas:

- $Z^1$-C(=O)-$CH_2CH_2$-C(=O)-GGVA-NH-B-$CH_2$-OC(=O)-,
- $Z^1$-C(=O)-$CH_2CH_2$-C(=O)-GG-(D-)VA-NH-B-$CH_2$-OC(=O)-,
- $Z^1$-C(=O)-$CH_2CH_2$-C(=O)-VA-NH-B-$CH_2$-OC(=O)-,
- $Z^1$-C(=O)-$(CH_2CH_2)_2$-C(=O)-VA-NH-B-$CH_2$-OC(=O)-,
- $Z^1$-C(=O)-$CH_2CH_2$-C(=O)-GGPI-NH-B-$CH_2$-OC(=O)-,
- $Z^1$-C(=O)-$CH_2CH_2$-C(=O)-GGFG-NH-B-$CH_2$-OC(=O)-,
- $Z^1$-C(=O)-$CH_2CH_2$-C(=O)-GGVCit-NH-B-$CH_2$-OC(=O)-,
- $Z^1$-C(=O)-$CH_2CH_2$-C(=O)-GGVK-NH-B-$CH_2$-OC(=O)-,
- $Z^1$-C(=O)-$CH_2CH_2$-C(=O)-GGPL-NH-B-$CH_2$-OC(=O)-,
- $Z^1$-C(=O)-$CH_2CH_2$-C(=O)-NH- $(CH_2CH_2)_2$-C(=O)-VA-NH-B-$CH_2$-OC(=O)-,
- $Z^1$-C(=O)-$CH_2CH_2$-C(=O)-NH-$(CH_2CH_2O)_2$-$CH_2$-C(=O)-VA-NH-B-$CH_2$-OC(=O)-,
- $Z^1$-C(=O)-$CH_2CH_2$-NH-C(=O)-$(CH_2CH_2O)_4$-$CH_2CH_2$-C(=O)-VA-NH-B-$CH_2$-OC(=O)-,
- $Z^2$-OC(=O)-GGVA-NH-B-$CH_2$-OC(=O)- and
- $Z^3$-$CH_2$-OC(=O)-GGVA-NH-B-$CH_2$-OC(=O)-

wherein $Z^1$ represents the following structural formula:

[Formula 6]

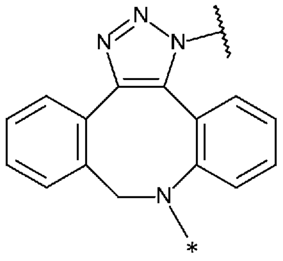

or

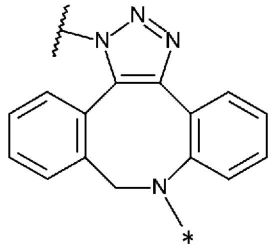

$Z^2$ represents the following structural formula:

[Formula 7]

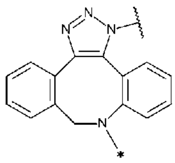

or

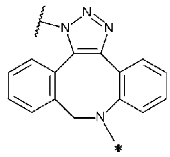

$Z^3$ represents the following structural formula:

[Formula 8]

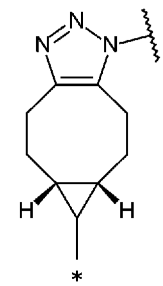

or

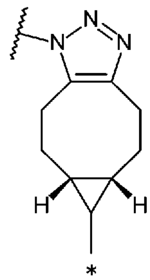

wherein, in each structural formula represented for $Z^1$, $Z^2$ and $Z^3$, each asterisk * represents bonding to the C(=O), O or $CH_2$ neighboring $Z^1$, $Z^2$ or $Z^3$; each wavy line represents bonding to the glycan presented by Ab or a remodeled glycan, and
B represents a 1,4-phenyl group.

**16.** The antibody-drug conjugate according to any one of claims 1 to 15, wherein

L represents any one selected from the group consisting of:

- $Z^1$-C(=O)-$CH_2CH_2$-C(=O)-GGVA-NH-B-$CH_2$-OC(=O)-,
- $Z^1$-C(=O)-$CH_2CH_2$-C(=O)-VA-NH-B-$CH_2$-OC(=O)-,
- $Z^1$-C(=O)-$(CH_2CH_2)_2$-C(=O)-VA-NH-B-$CH_2$-OC(=O)-,
- $Z^1$-C(=O)-$CH_2CH_2$-C(=O)-GGVCit-NH-B-$CH_2$-OC(=O)-,
- $Z^1$-C(=O)-$CH_2CH_2$-C(=O)-NH-$(CH_2CH_2)_2$-C(=O)-VA-NH-B-$CH_2$-OC(=O)-,
- $Z^1$-C(=O)-$CH_2CH_2$-C(=O)-NH-$(CH_2CH_2O)_2$-$CH_2$-C(=O)-VA-NH-B-$CH_2$-OC(=O)- and
- $Z^1$-C(=O)-$CH_2CH_2$-NH-C(=O)-$(CH_2CH_2O)_4$-$CH_2CH_2$-C(=O)-VA-NH-B-$CH_2$-OC(=O)-,

wherein B represents a 1,4-phenyl group, and $Z^1$ represents the following structural formula:

[Formula 9]

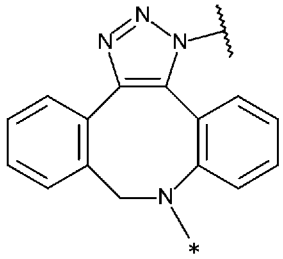

or

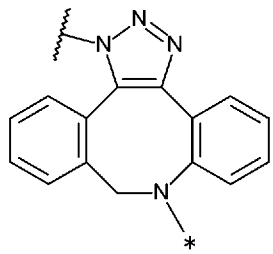

wherein in the structural formula for $Z^1$, each asterisk * represents bonding to the C(=O) neighboring $Z^1$ and each wavy line represents bonding to the glycan bonding to N297 of Ab (N297 glycan) or bonding to a remodeled glycan.

**17.** The antibody-drug conjugate according to any one of claims 1 to 16, wherein the antibody is IgG1, IgG2 or IgG4.

**18.** The antibody-drug conjugate according to any one of claims 1 to 17, wherein the N297 glycan is a remodeled glycan.

**19.** The antibody-drug conjugate according to any one of claims 1 to 18, wherein the N297 glycan is N297-(Fuc)MSG1, N297-(Fuc)MSG2, or a mixture thereof, or N297-(Fuc)SG, with N297-(Fuc)MSG1, N297-(Fuc)MSG2, and N297-(Fuc)SG having structures represented by the following formulas:

[Formula 10]

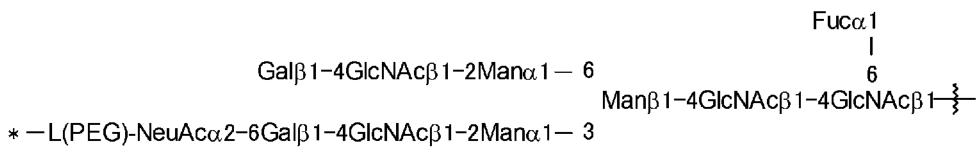

[N297-(Fuc)MSG1]

[Formula 11]

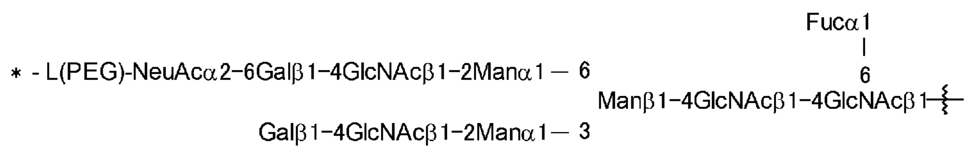

[N297-(Fuc)MSG2]

[Formula 12]

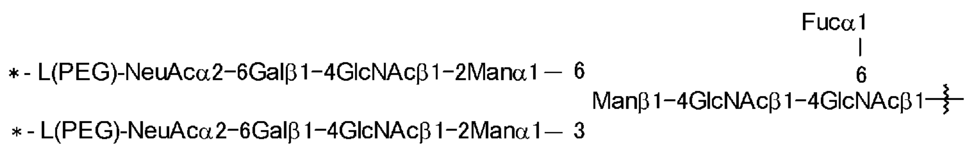

[N297-(Fuc)SG]

wherein the wavy line represents bonding to Asn297 of the antibody,

*-L(PEG)- in the N297 glycan represents *-$(CH_2CH_2\text{-}O)n^5\text{-}CH_2CH_2$-NH-, wherein $n^5$ represents an integer of 2 to 5, the amino group at the right end is bound via an amide bond to the carboxylic acid at the 2-position of the

sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the individual structural formulas.

**20.** The antibody-drug conjugate according to claim 19, wherein $n^5$ represents 3.

**21.** An antibody-drug conjugate represented by the following formula (XII):

[Formula 13]

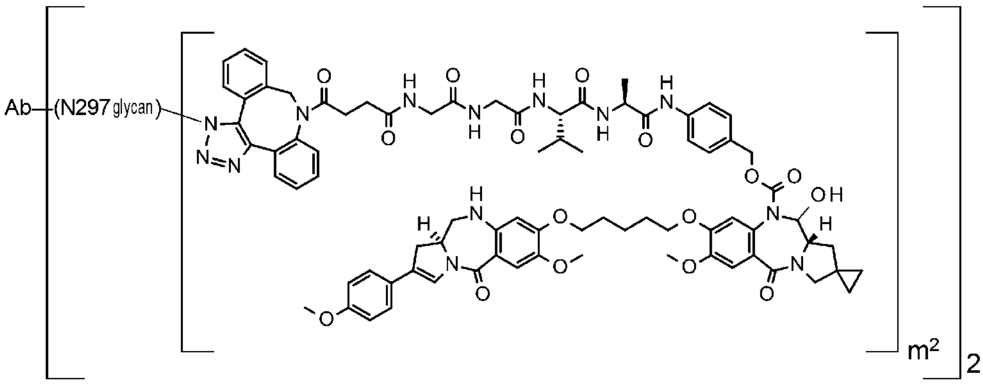

or

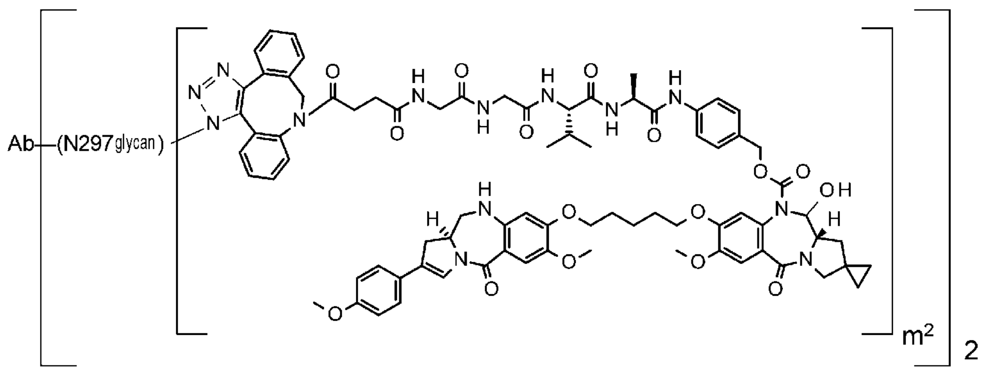

(XII)

wherein, in each structural formula shown above,

$m^2$ represents an integer of 1 or 2,
Ab represents an IgG antibody specifically binding to an amino acid sequence comprising the amino acid sequence shown in SEQ ID NO: 4 and having internalization ability that permits cellular uptake, or a functional fragment thereof,
the N297 glycan of Ab represents any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas,

[Formula 14]

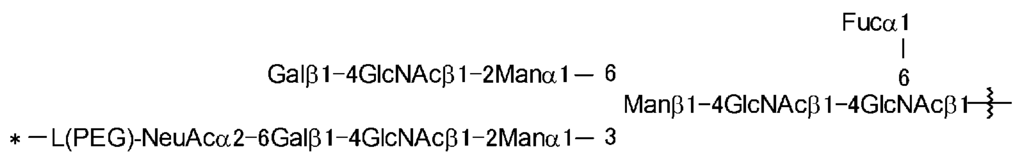

[N297-(Fuc)MSG1]

[Formula 15]

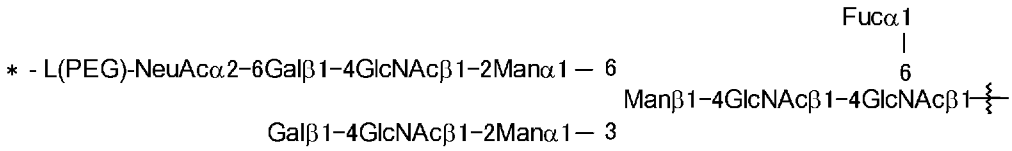

[N297-(Fuc)MSG2]

[Formula 16]

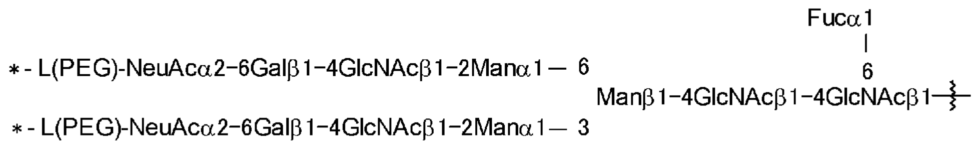

[N297-(Fuc)SG]

wherein each wavy line represents bonding to Asn297 (N297) of the antibody,
*-L(PEG)- in the N297 glycan represents *-$(CH_2CH_2\text{-}O)_3\text{-}CH_2CH_2$-NH-,
wherein
the amino group at the right end is bound via an amide bond to the carboxylic acid at the 2-position of the sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the corresponding structural formula.

**22.** The antibody-drug conjugate according to claim 21, wherein the compound represented by formula (XII) is represented by the following formula (XII'):

[Formula 17]

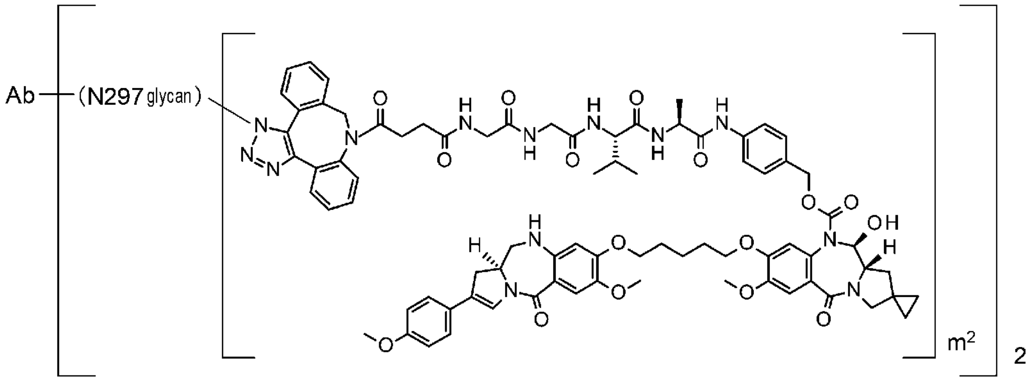

or (XII')

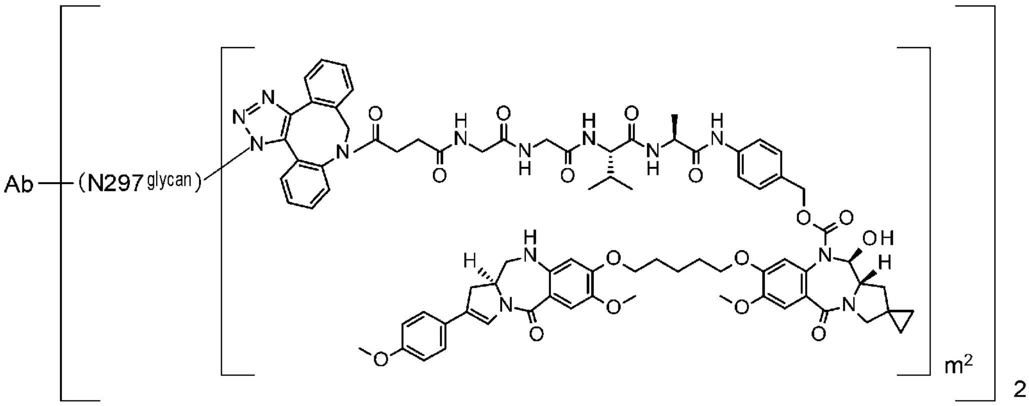

**23.** An antibody-drug conjugate represented by the following formula (XIII):

[Formula 18]

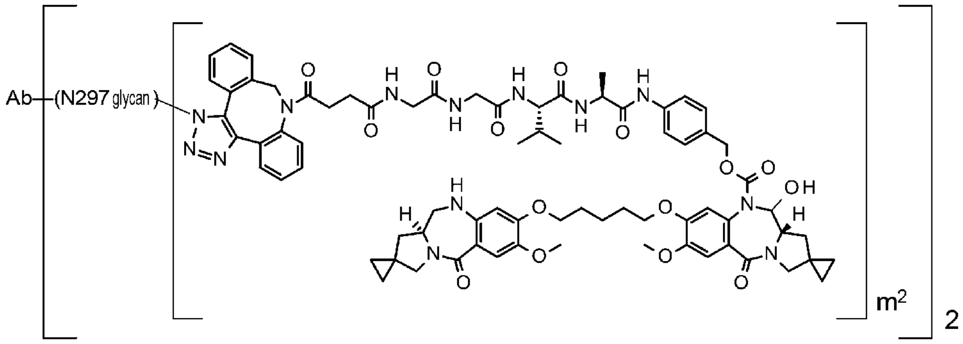

or

(XIII)

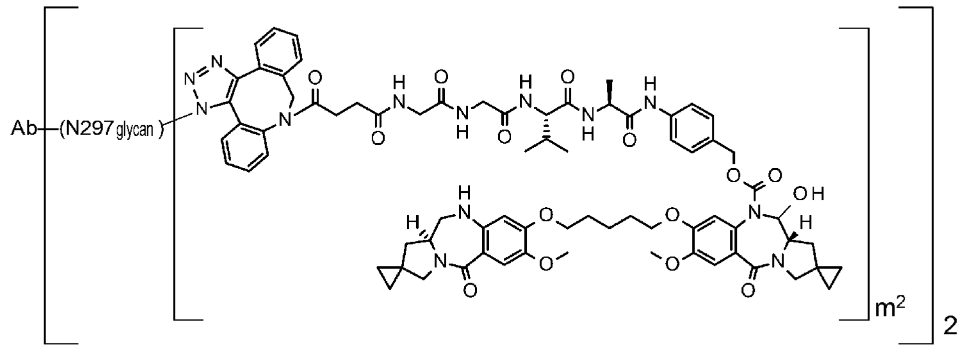

wherein, in each structural formula shown above,

$m^2$ represents an integer of 1 or 2,
Ab represents an IgG antibody specifically binding to an amino acid sequence comprising the amino acid sequence shown in SEQ ID NO: 4 and having internalization ability that permits cellular uptake, or a functional fragment thereof, and
the N297 glycan of Ab represents any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas,

[Formula 19]

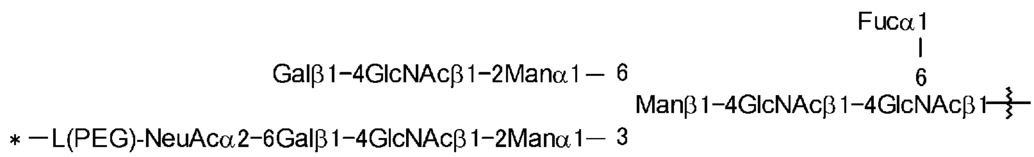

[N297-(Fuc)MSG1]

[Formula 20]

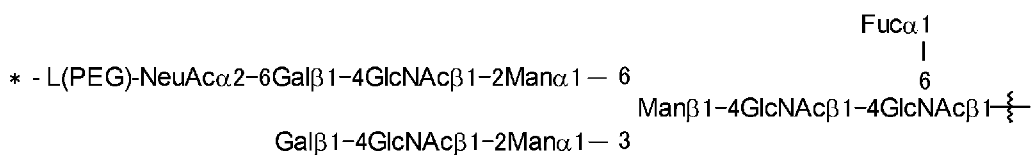

[N297-(Fuc)MSG2]

[Formula 21]

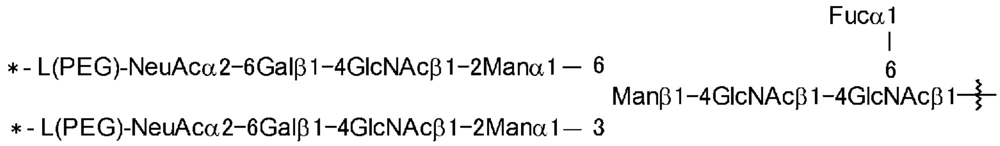

[N297-(Fuc)SG]

wherein each wavy line represents bonding to Asn297 (N297) of the antibody,
*-L(PEG)- in the N297 glycan represents *-$(CH_2CH_2\text{-}O)_3\text{-}CH_2CH_2$-NH-, wherein the amino group at the right end is bound via an amide bond to the carboxylic acid at the 2-position of the sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the individual structural formulas.

**24.** The antibody-drug conjugate according to claim 23, wherein the compound represented by formula (XIII) is represented by the following formula (XIII'):

[Formula 22]

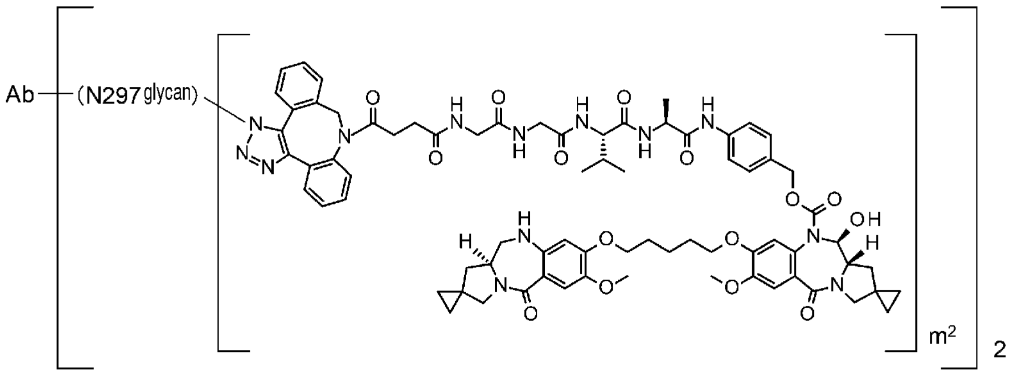

or

(XIII')

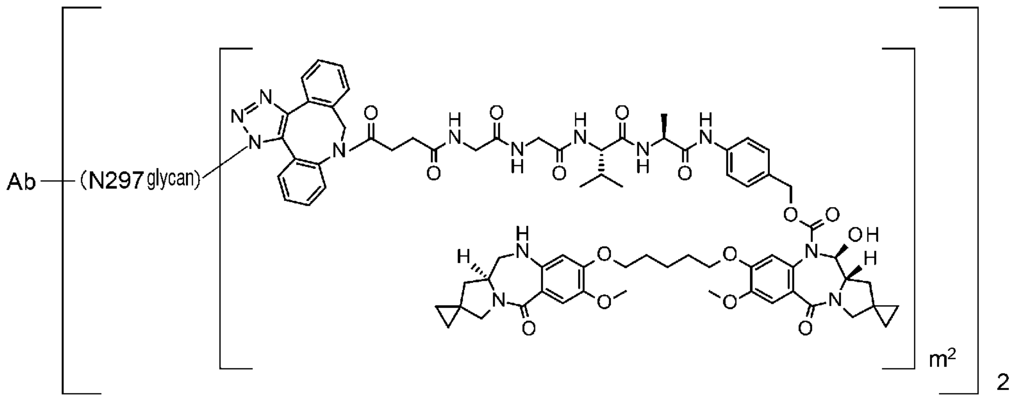

**25.** An antibody-drug conjugate represented by the following formula (XIV):

[Formula 23]

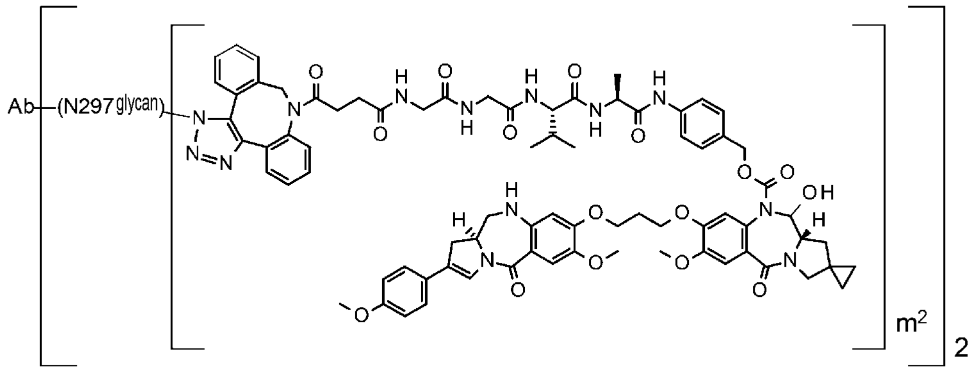

or (XIV)

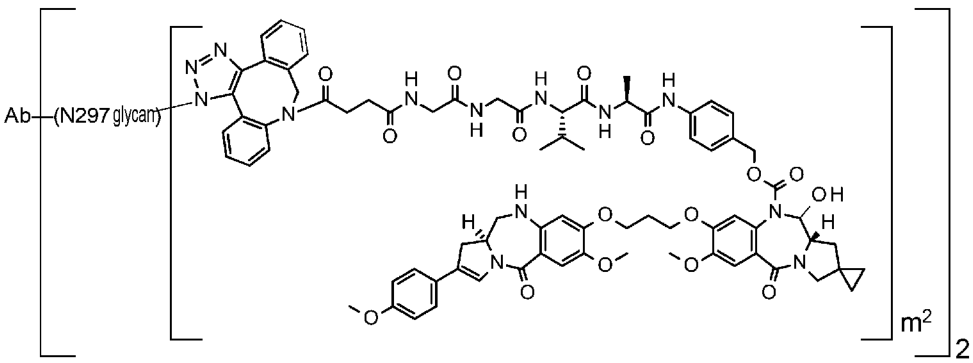

wherein, in each structural formula shown above,

$m^2$ represents an integer of 1 or 2,

Ab represents an IgG antibody specifically binding to an amino acid sequence comprising the amino acid sequence shown in SEQ ID NO: 4 and having internalization ability that permits cellular uptake, or a functional fragment thereof, and

the N297 glycan of Ab represents any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 24]

Fucα1
|
6
Galβ1−4GlcNAcβ1−2Manα1— 6
Manβ1−4GlcNAcβ1−4GlcNAcβ1—
∗ —L(PEG)-NeuAcα2−6Galβ1−4GlcNAcβ1−2Manα1— 3

[N297-(Fuc)MSG1]

[Formula 25]

Fucα1
|
6
∗ - L(PEG)-NeuAcα2−6Galβ1−4GlcNAcβ1−2Manα1 — 6
Manβ1−4GlcNAcβ1−4GlcNAcβ1—
Galβ1−4GlcNAcβ1−2Manα1— 3

[N297-(Fuc)MSG2]

[Formula 26]

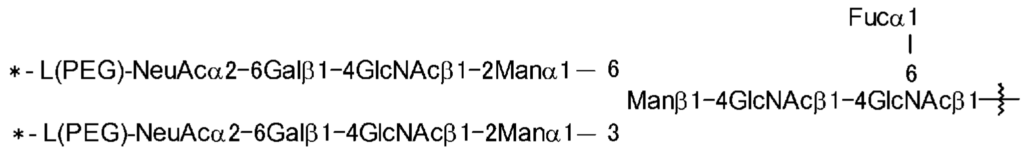

[N297-(Fuc)SG]

wherein each wavy line represents bonding to Asn297 (N297) of the antibody, *-L(PEG)- in the N297 glycan represents *-$(CH_2CH_2\text{-}O)_3\text{-}CH_2CH_2\text{-}NH$-, wherein the amino group at the right end is bound via an amide bond to the carboxylic acid at the 2-position of the sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the individual structural formulas.

**26.** The antibody-drug conjugate according to claim 25, wherein the compound represented by formula (XIV) is represented by the following formula (XIV'):

[Formula 27]

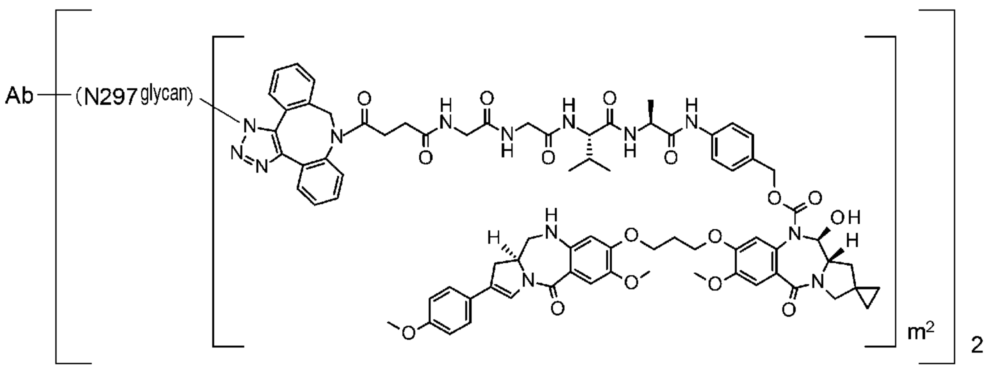

or

(XIV')

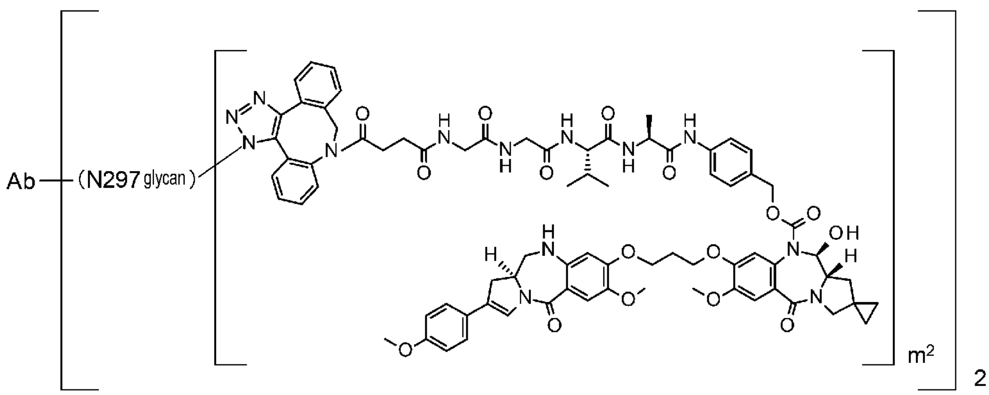

**27.** An antibody-drug conjugate represented by the following formula (XV):

[Formula 28]

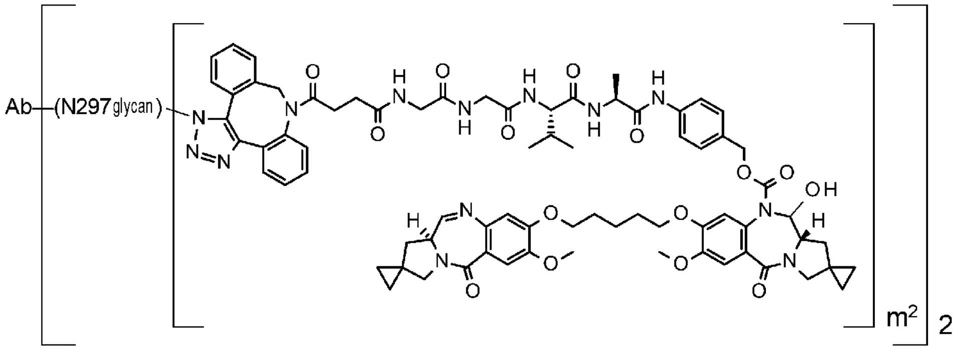

or

(XV)

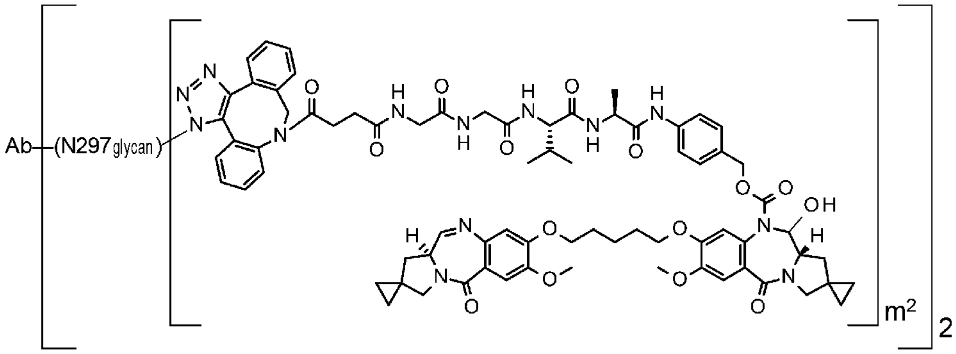

wherein, in each structural formula shown above,

$m^2$ represents an integer of 1 or 2,
Ab represents an IgG antibody specifically binding to an amino acid sequence comprising the amino acid sequence shown in SEQ ID NO: 4 and having internalization ability that permits cellular uptake, or a functional fragment thereof, and
the N297 glycan of Ab represents any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 29]

Fucα1
|
6
Galβ1-4GlcNAcβ1-2Manα1— 6
Manβ1-4GlcNAcβ1-4GlcNAcβ1—
∗ —L(PEG)-NeuAcα2-6Galβ1-4GlcNAcβ1-2Manα1— 3

[N297-(Fuc)MSG1]

[Formula 30]

Fucα1
|
6
∗ - L(PEG)-NeuAcα2-6Galβ1-4GlcNAcβ1-2Manα1— 6
Manβ1-4GlcNAcβ1-4GlcNAcβ1—
Galβ1-4GlcNAcβ1-2Manα1— 3

[N297-(Fuc)MSG2]

[Formula 31]

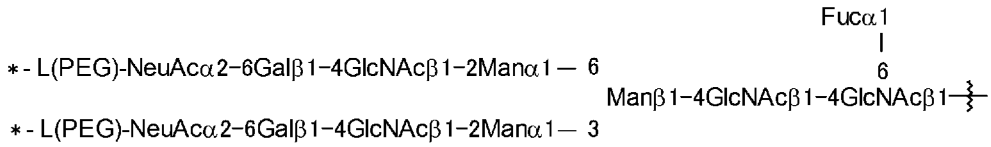

[N297-(Fuc)SG]

wherein each wavy line represents bonding to Asn297 (N297) of the antibody, *-L(PEG)- in the N297 glycan represents *-$(CH_2CH_2\text{-}O)_3\text{-}CH_2CH_2\text{-}NH\text{-}$, wherein the amino group at the right end is bound via an amide bond to the carboxylic acid at the 2-position of the sialic acid at the non-reducing terminal in each or either one of the 1-3 and 1-6 branched chains of the β-Man in the N297 glycan, and each asterisk * represents bonding to the nitrogen atom at the 1- or 3-position of the triazole ring in the individual structural formulas.

**28.** The antibody-drug conjugate according to claim 27, wherein the compound represented by formula (XV) is represented by the following formula (XV'):

[Formula 32]

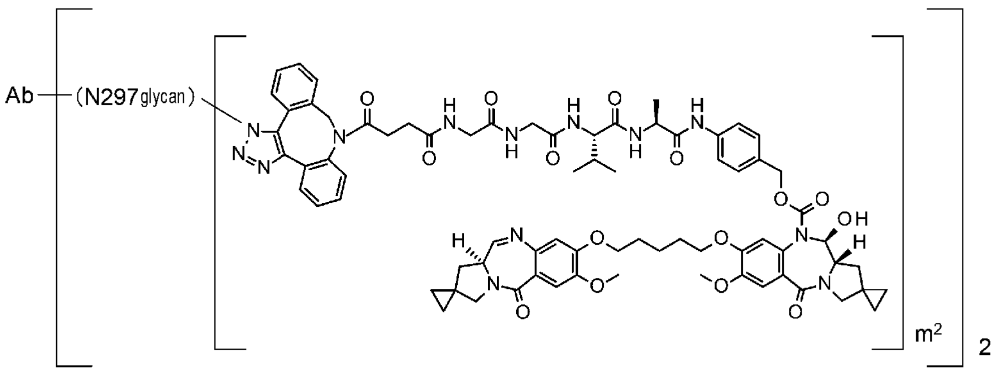

or (XV')

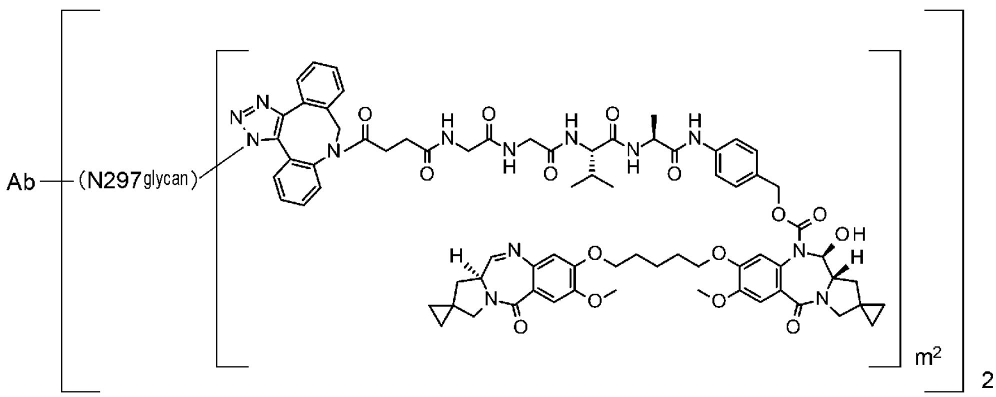

**29.** The antibody-drug conjugate according to any one of claims 21 to 28, wherein the antibody or a functional fragment thereof comprises CDRL1, CDRL2 and CDRL3 and CDRH1, CDRH2 and CDRH3 selected from the group consisting of the following (1) to (4):

(1) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14 and CDRH1 consisting of the amino acid sequence shown in SEQ ID NO: 17, CDRH2 consisting of the amino acid sequence shown in SEQ ID NO: 18 and CDRH3 consisting of the amino acid sequence shown in

SEQ ID NO: 19,
(2) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14 and CDRH1 consisting of the amino acid sequence shown in SEQ ID NO: 17, CDRH2 consisting of the amino acid sequence shown in SEQ ID NO: 30 and CDRH3 consisting of the amino acid sequence shown in SEQ ID NO: 19,
(3) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14 and CDRH1 consisting of the amino acid sequence shown in SEQ ID NO: 57, CDRH2 consisting of the amino acid sequence shown in SEQ ID NO: 58 and CDRH3 consisting of the amino acid sequence shown in SEQ ID NO: 59, and
(4) CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14 and CDRH1 consisting of the amino acid sequence shown in SEQ ID NO: 62, CDRH2 consisting of the amino acid sequence shown in SEQ ID NO: 63 and CDRH3 consisting of the amino acid sequence shown in SEQ ID NO: 64.

**30.** The antibody-drug conjugate according to any one of claims 21 to 29, wherein the antibody or a functional fragment thereof comprises any one of the following (1) to (6) :

(1) a light chain variable region consisting of the amino acid sequence shown in SEQ ID NO: 33 and a heavy chain variable region consisting of the amino acid sequence shown in SEQ ID NO: 41,
(2) a light chain variable region consisting of the amino acid sequence shown in SEQ ID NO: 33 and a heavy chain variable region consisting of the amino acid sequence shown in SEQ ID NO: 45,
(3) a light chain variable region consisting of the amino acid sequence shown in SEQ ID NO: 37 and a heavy chain variable region consisting of the amino acid sequence shown in SEQ ID NO: 45,
(4) a light chain variable region consisting of the amino acid sequence shown in SEQ ID NO: 33 and a heavy chain variable region consisting of the amino acid sequence shown in SEQ ID NO: 49,
(5) a light chain variable region consisting of the amino acid sequence shown in SEQ ID NO: 33 and a heavy chain variable region consisting of the amino acid sequence shown in SEQ ID NO: 55 or
(6) a light chain variable region consisting of the amino acid sequence shown in SEQ ID NO: 33 and a heavy chain variable region consisting of the amino acid sequence shown in SEQ ID NO: 60.

**31.** The antibody-drug conjugate according to any one of claims 21 to 30, wherein the antibody or a functional fragment thereof comprises any one of the following (1) to (7) :

(1) a light chain consisting of the amino acid sequence at positions 21 to 233 in SEQ ID NO: 31 and a heavy chain consisting of the amino acid sequence at positions 20 to 471 in SEQ ID NO: 39,
(2) a light chain consisting of the amino acid sequence at positions 21 to 233 in SEQ ID NO: 31 and a heavy chain consisting of the amino acid sequence at positions 20 to 471 in SEQ ID NO: 43,
(3) a light chain consisting of the amino acid sequence at positions 21 to 233 in SEQ ID NO: 35 and a heavy chain consisting of the amino acid sequence at positions 20 to 471 in SEQ ID NO: 43,
(4) a light chain consisting of the amino acid sequence at positions 21 to 233 in SEQ ID NO: 31 and a heavy chain consisting of the amino acid sequence at positions 20 to 471 in SEQ ID NO: 47,
(5) a light chain consisting of the amino acid sequence at positions 21 to 233 in SEQ ID NO: 31 and a heavy chain consisting of the amino acid sequence at positions 20 to 471 in SEQ ID NO: 65,
(6) a light chain consisting of the amino acid sequence at positions 21 to 233 in SEQ ID NO: 31 and a heavy chain consisting of the amino acid sequence at positions 20 to 471 in SEQ ID NO: 67 and
(7) a light chain consisting of the amino acid sequence at positions 21 to 233 in SEQ ID NO: 31 and a heavy chain consisting of the amino acid sequence at positions 20 to 471 in SEQ ID NO: 69.

**32.** The antibody-drug conjugate according to claim 31, wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 39.

**33.** The antibody-drug conjugate according to claim 31, wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 43.

**34.** The antibody-drug conjugate according to claim 31, wherein the antibody or a functional fragment thereof comprises a light chain consisting of the amino acid sequence at positions 21 to 233 in SEQ ID NO: 35 and a heavy chain consisting of the amino acid sequence at positions 20 to 471 in SEQ ID NO: 43.

**35.** The antibody-drug conjugate according to claim 31, wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 47.

**36.** The antibody-drug conjugate according to claim 31, wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 65.

**37.** The antibody-drug conjugate according to claim 31, wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 67.

**38.** The antibody-drug conjugate according to claim 31, wherein the antibody or a functional fragment thereof comprises a light chain consisting of the amino acid sequence at positions 21 to 233 in SEQ ID NO: 31 and a heavy chain consisting of the amino acid sequence at positions 20 to 471 in SEQ ID NO: 69.

**39.** The antibody-drug conjugate according to any one of claims 1 to 38, wherein the average number of conjugated drug molecules per antibody molecule in the antibody-drug conjugate is 1 to 3.

**40.** The antibody-drug conjugate according to any one of claims 1 to 38, wherein the average number of conjugated drug molecules per antibody molecule in the antibody-drug conjugate is 3 to 5.

**41.** The antibody-drug conjugate according to any one of claims 1 to 40, wherein the antibody contains a heavy chain having one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, and a deletion of one or two amino acids from the carboxyl terminus.

**42.** The antibody-drug conjugate according to any one of claims 1 to 41, wherein the lysine residue at the carboxyl terminus of a heavy chain is deleted.

**43.** A method for producing a glycan-remodeled antibody, the method comprising the steps of:

i) producing an IgG antibody specifically binding to an amino acid sequence comprising the amino acid sequence shown in SEQ ID NO: 4 and having internalization ability that permits cellular uptake, or a functional fragment thereof,
ii) treating the antibody obtained in step i) with hydrolase to produce a (Fuc$\alpha$1,6)GlcNAc-antibody; and
iii)-1 reacting the (Fuc$\alpha$1,6)GlcNAc-antibody and a glycan donor molecule in the presence of a transglycosidase, the glycan donor molecule obtained by introducing a PEG linker having an azide group to the carbonyl group of carboxylic acid at the 2-position of a sialic acid in MSG (9) or SG (10) and oxazolinating the reducing terminal, or
iii)-2 reacting the (Fuc$\alpha$1,6)GlcNAc-antibody and a glycan donor molecule in the presence of a transglycosidase, the glycan donor molecule obtained by introducing a PEG linker having an azide group to the carbonyl group of carboxylic acid at the 2-position of a sialic acid in (MSG-)Asn or (SG-)Asn with an $\alpha$-amino group optionally protected and to the carbonyl group of carboxylic acid in the Asn, causing action of hydrolase, and then oxazolinating the reducing terminal.

**44.** A method for producing an antibody-drug conjugate according to any one of claims 1 to 42, comprising the step of reacting the glycan-remodeled antibody obtained by the method according to claim 43 and a drug linker.

**45.** The antibody-drug conjugate according to any one of claim 1 to 42, produced by the method according to claim 44.

**46.** An antibody or a functional fragment thereof, comprising a light chain variable region, which contains CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14, and a heavy chain

variable region, which contains CDRH1 consisting of the amino acid sequence shown in SEQ ID NO: 57, CDRH2 consisting of the amino acid sequence shown in SEQ ID NO: 58 and CDRH3 consisting of the amino acid sequence shown in SEQ ID NO: 59.

**47.** An antibody or a functional fragment thereof, comprising a light chain variable region, which contains CDRL1 consisting of the amino acid sequence shown in SEQ ID NO: 12, CDRL2 consisting of the amino acid sequence shown in SEQ ID NO: 13 and CDRL3 consisting of the amino acid sequence shown in SEQ ID NO: 14; and a heavy chain variable region, which contains CDRH1 consisting of the amino acid sequence shown in SEQ ID NO: 62, CDRH2 consisting of the amino acid sequence shown in SEQ ID NO: 63 and CDRH3 consisting of the amino acid sequence shown in SEQ ID NO: 64.

**48.** The antibody or a functional fragment thereof according to claim 46, comprising a light chain variable region consisting of the amino acid sequence shown in SEQ ID NO: 33 and a heavy chain variable region consisting of the amino acid sequence shown in SEQ ID NO: 55.

**49.** The antibody or a functional fragment thereof according to claim 47, comprising a light chain variable region consisting of the amino acid sequence shown in SEQ ID NO: 33 and a heavy chain variable region consisting of the amino acid sequence shown in SEQ ID NO: 60.

**50.** The antibody or a functional fragment thereof according to claim 46 or 48, having a light chain consisting of the amino acid sequence at positions 21 to 233 in SEQ ID NO: 31 and a heavy chain consisting of the amino acid sequence at positions 20 to 471 in SEQ ID NO: 67.

**51.** The antibody or a functional fragment thereof according to claim 47 or 49, having a light chain consisting of the amino acid sequence at positions 21 to 233 in SEQ ID NO: 31 and a heavy chain consisting of the amino acid sequence at positions 20 to 471 in SEQ ID NO: 69.

**52.** An antibody or a functional fragment thereof having a light chain consisting of the amino acid sequence at positions 21 to 233 in SEQ ID NO: 31 and a heavy chain consisting of the amino acid sequence at positions 20 to 471 in SEQ ID NO: 65.

**53.** A polynucleotide encoding the antibody according to any one of claims 46 to 52.

**54.** An expression vector containing the polynucleotide according to claim 53.

**55.** A host cell transformed with the expression vector according to claim 54.

**56.** A method for producing the antibody or a functional fragment thereof according to claims 46 to 52, comprising a step of culturing the host cell according to claim 55 and collecting the targeted antibody from the culture obtained from the step of culturing.

**57.** An antibody or a functional fragment thereof, obtained by the method according to claim 56.

**58.** A pharmaceutical composition containing the antibody-drug conjugate according to any one of claims 1 to 42 and 45 or an antibody or a functional fragment thereof according to any one of claims 46 to 52 and 57.

**59.** The pharmaceutical composition according to claim 58, which is an antitumor drug.

**60.** The pharmaceutical composition according to claim 59, wherein the tumor is a tumor expressing CDH6.

**61.** The pharmaceutical composition according to claim 60, wherein the tumor is renal cell carcinoma, renal clear cell carcinoma, papillary renal cell carcinoma, ovarian cancer, ovarian serous adenocarcinoma, thyroid cancer, bile duct cancer, lung cancer, small-cell lung cancer, non-small cell lung cancer, glioblastoma, mesothelioma, uterine cancer, pancreatic cancer, Wilms' tumor or neuroblastoma.

**62.** A method for treating a tumor, which comprises administering the antibody-drug conjugate according to any one of claims 1 to 42 and 45, the antibody or a functional fragment thereof according to any one of claims 46 to 52 and 57 or the pharmaceutical composition according to any one of claims 58 to 61 to an individual.

**63.** The treatment method according to claim 62, wherein the tumor is a tumor having CDH6 expressed therein.

**64.** The treatment method according to claim 62 or 63, wherein the tumor is renal cell carcinoma, renal clear cell carcinoma, papillary renal cell carcinoma, ovarian cancer, ovarian serous adenocarcinoma, thyroid cancer, bile duct cancer, lung cancer, small-cell lung cancer, non-small cell lung cancer, glioblastoma, mesothelioma, uterine cancer, pancreatic cancer, Wilms' tumor or neuroblastoma.

**65.** The treatment method according to any one of claims 62 to 64, which comprises administering the antibody-drug conjugate according to any one of claims 1 to 42 and 45, the antibody or a functional fragment thereof according to any one of claims 46 to 52 and 57, or the pharmaceutical composition according to any one of claims 58 to 61 and at least one antitumor drug to an individual, simultaneously, separately or continuously.

[Figure 1]

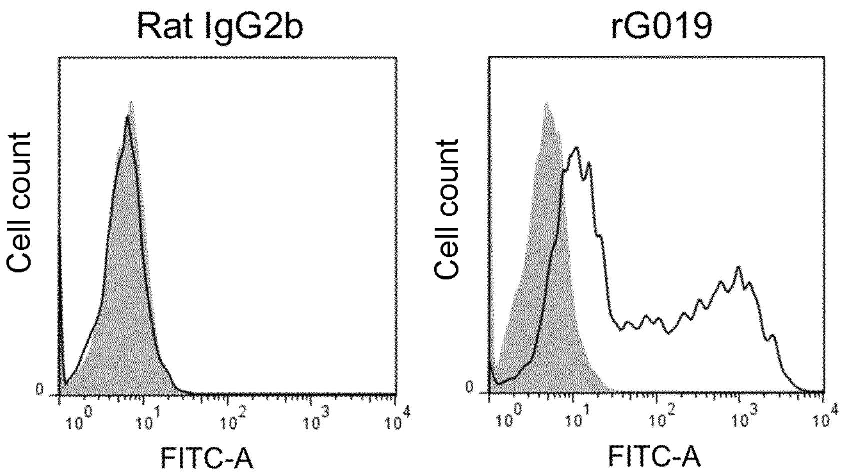

Rat IgG2b
Cell count
0
$10^0$
$10^1$
$10^2$
$10^3$
$10^4$
FITC-A
rG019
Cell count
0
$10^0$
$10^1$
$10^2$
$10^3$
$10^4$
FITC-A

[Figure 2-1]

(1) Control cells or full-length hCDH6-transfected cells

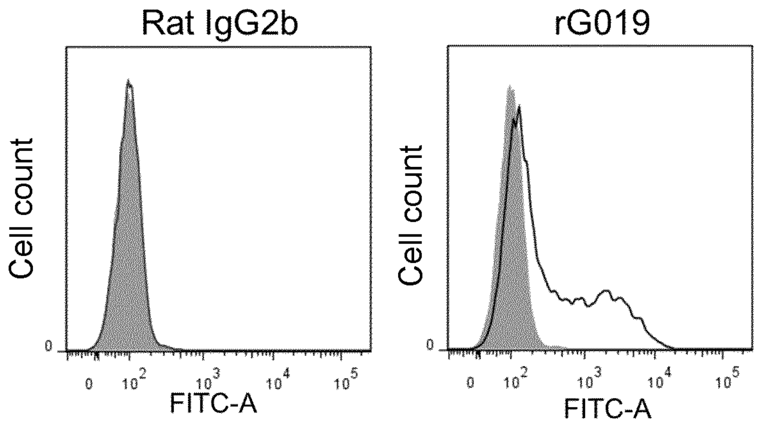


(2) Control cells or EC1-deleted hCDH6-transfected cells

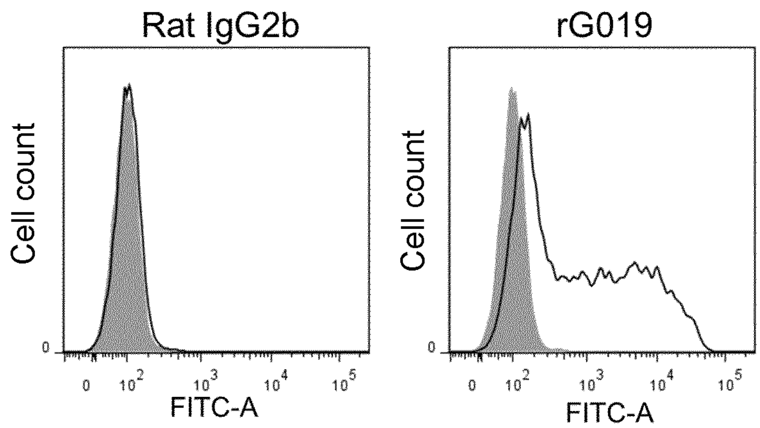


(3) Control cells or EC2-deleted hCDH6-transfected cells

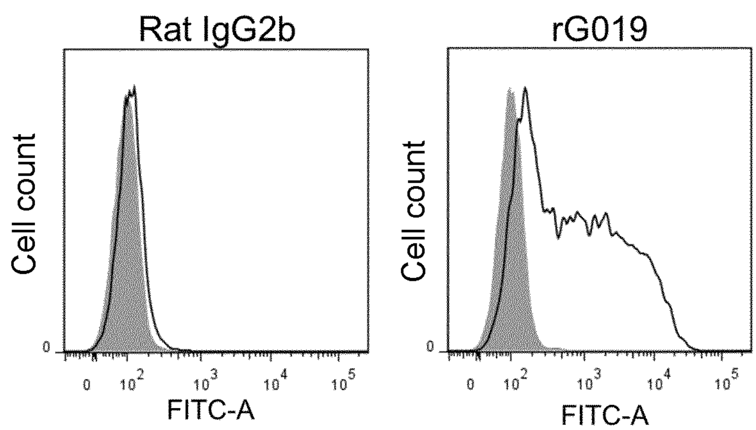

[Figure 2-2]

(4) Control cells or EC3-deleted hCDH6-transfected cells

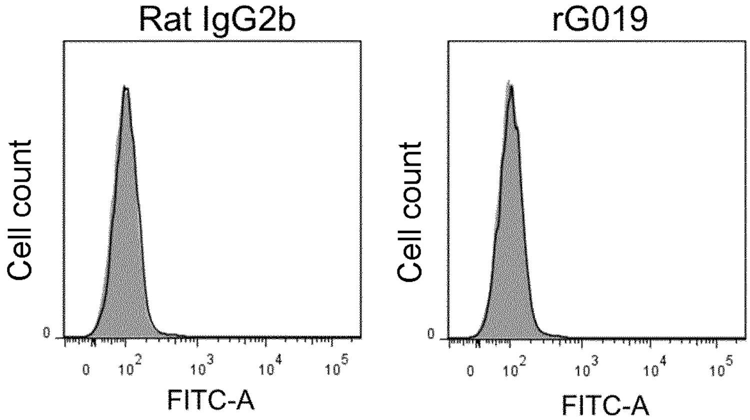


(5) Control cells or EC4-deleted hCDH6-transfected cells

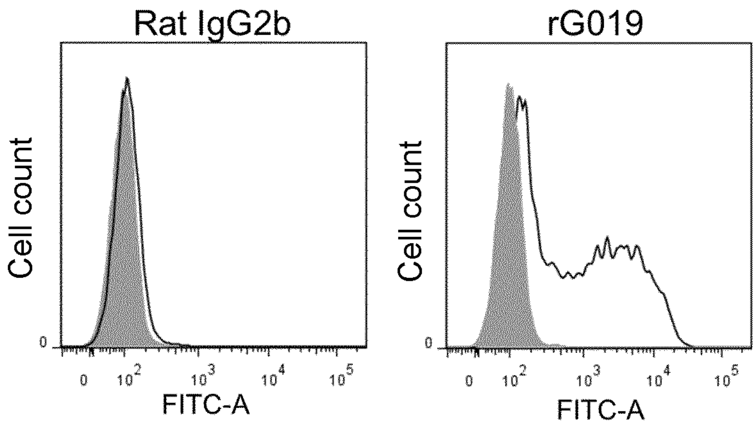


(6) Control cells or EC5-deleted hCDH6-transfected cells

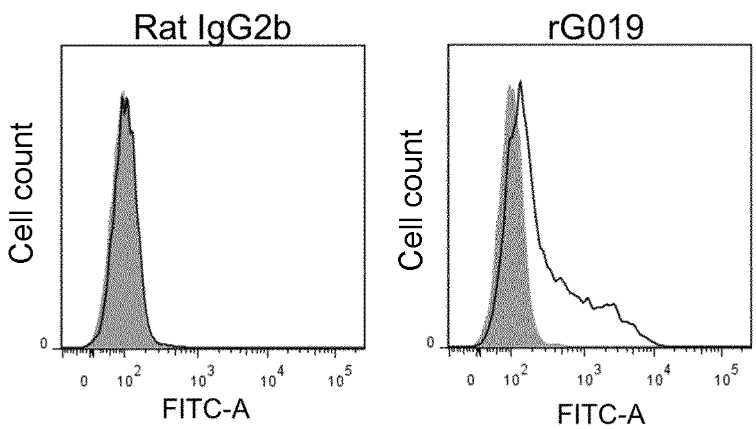

[Figure 3]

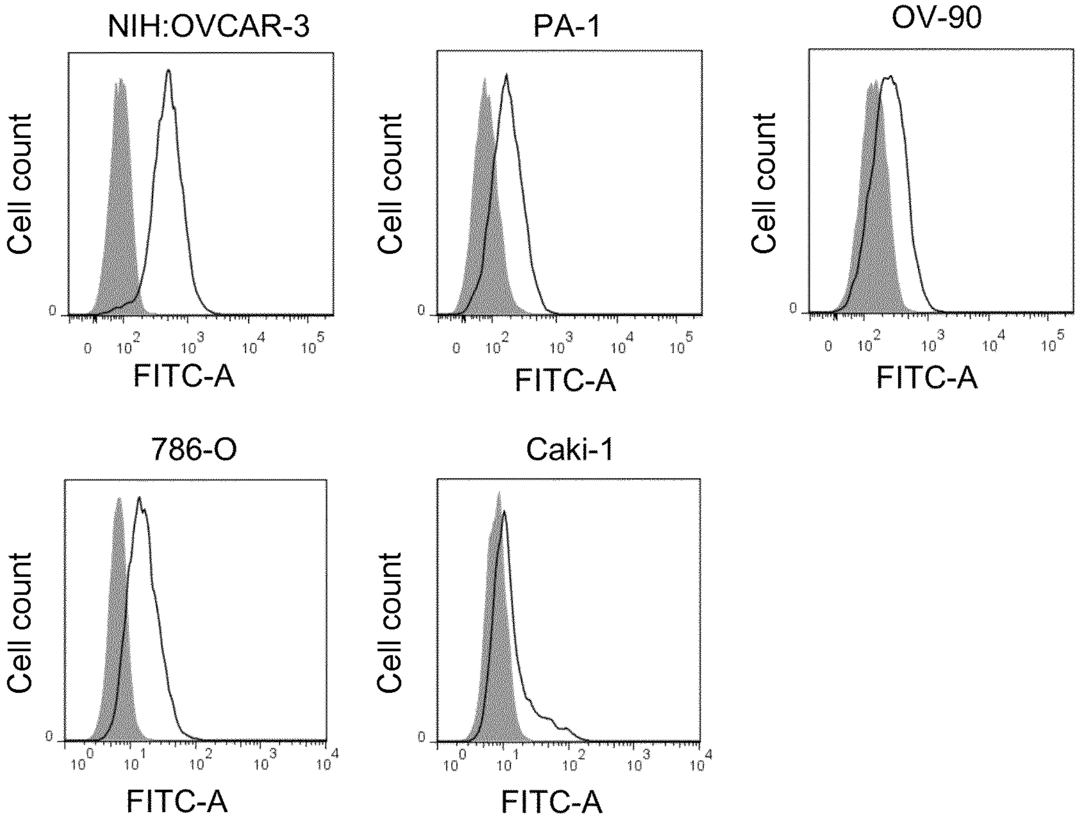

[Figure 4]

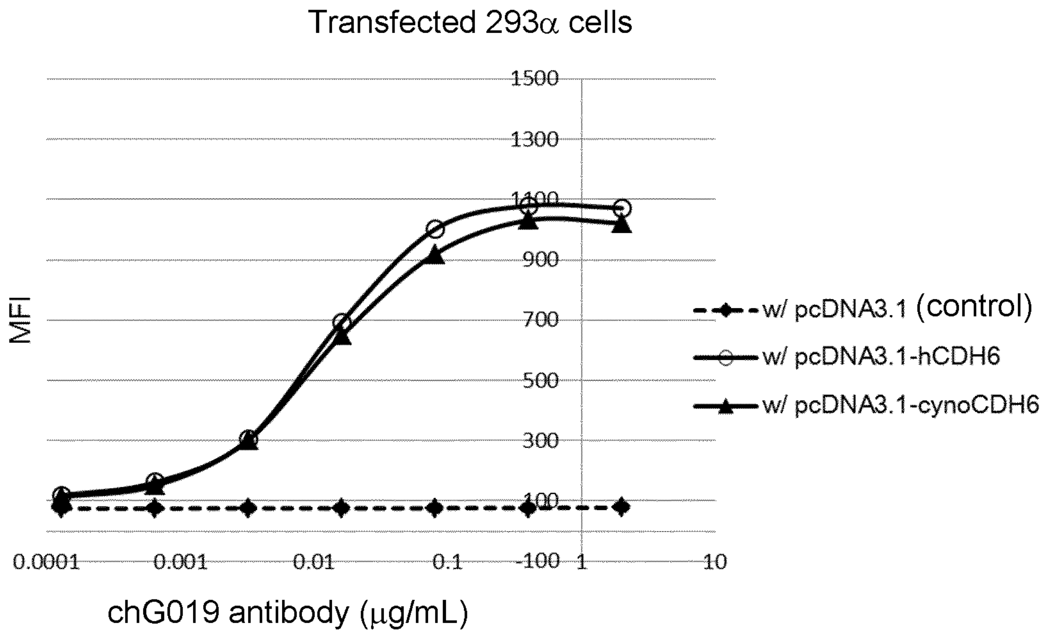
Transfected 293α cells
1500
1300
1100
900
700
500
300
100
-100
MFI
0.0001
0.001
0.01
0.1
1
10
chG019 antibody (μg/mL)
w/ pcDNA3.1 (control)
w/ pcDNA3.1-hCDH6
w/ pcDNA3.1-cynoCDH6

[Figure 5-1]

(1) Control cells or full-length hCDH6-transfected cells

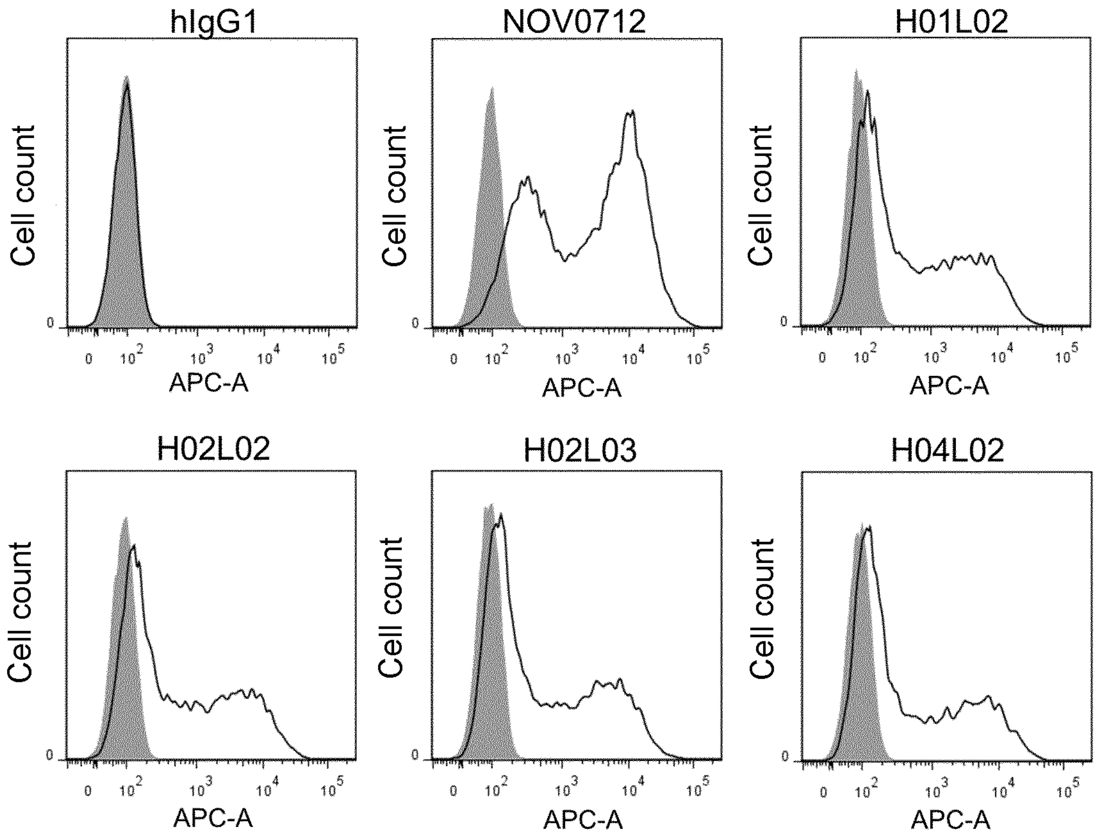

[Figure 5-2]

(2) Control cells or EC1-deleted hCDH6-transfected cells

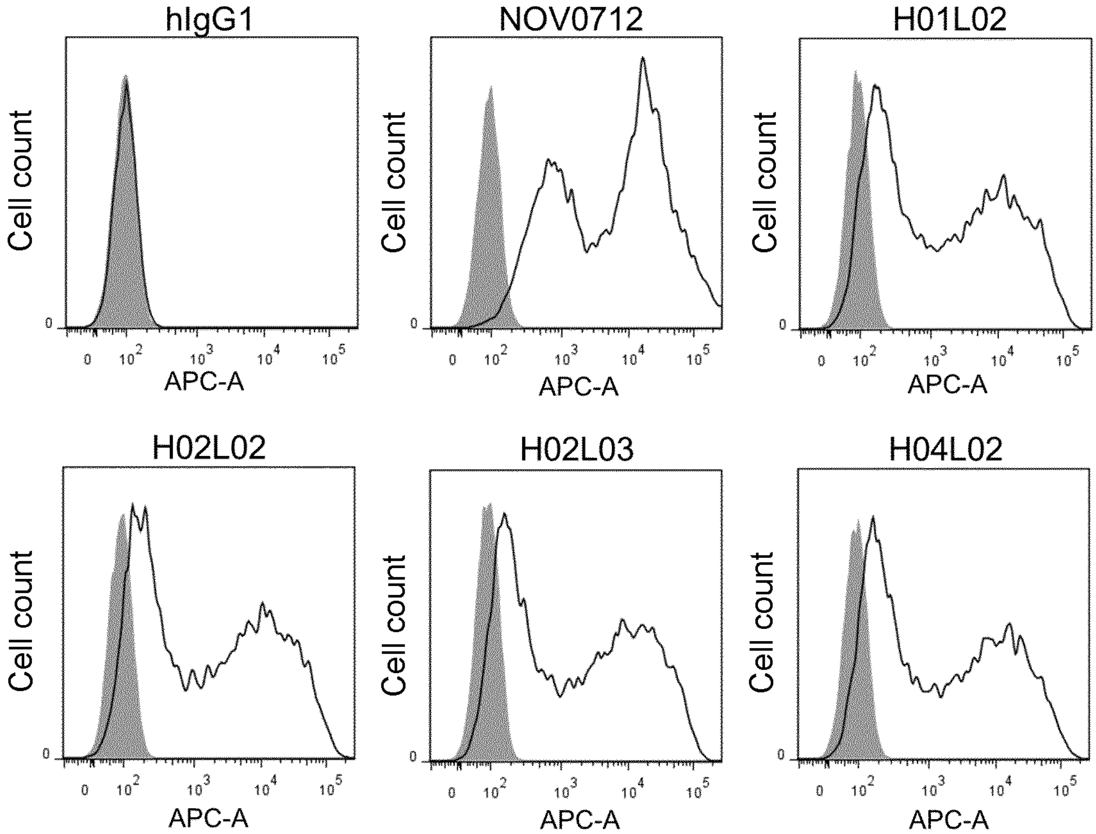

[Figure 5-3]

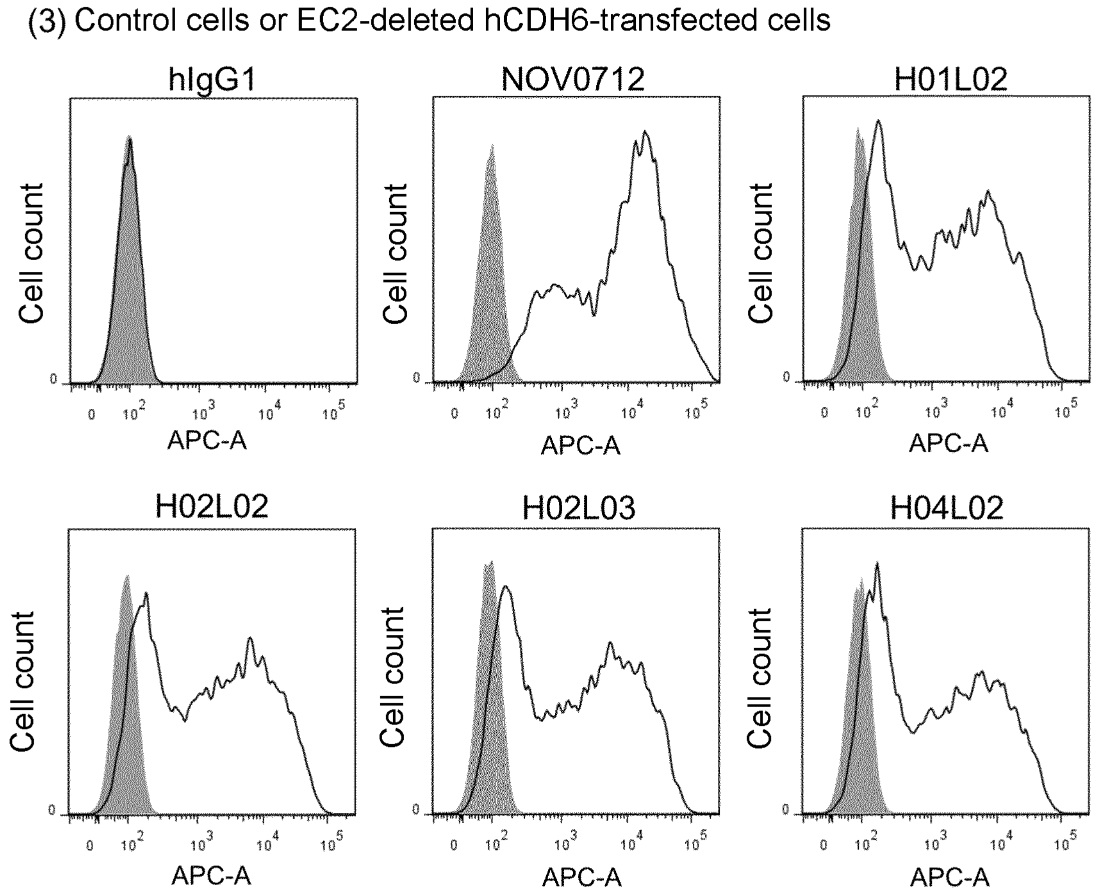
(3) Control cells or EC2-deleted hCDH6-transfected cells
hIgG1
NOV0712
H01L02
H02L02
H02L03
H04L02
Cell count
APC-A
0
$10^2$
$10^3$
$10^4$
$10^5$

[Figure 5-4]

(4) Control cells or EC3-deleted hCDH6-transfected cells

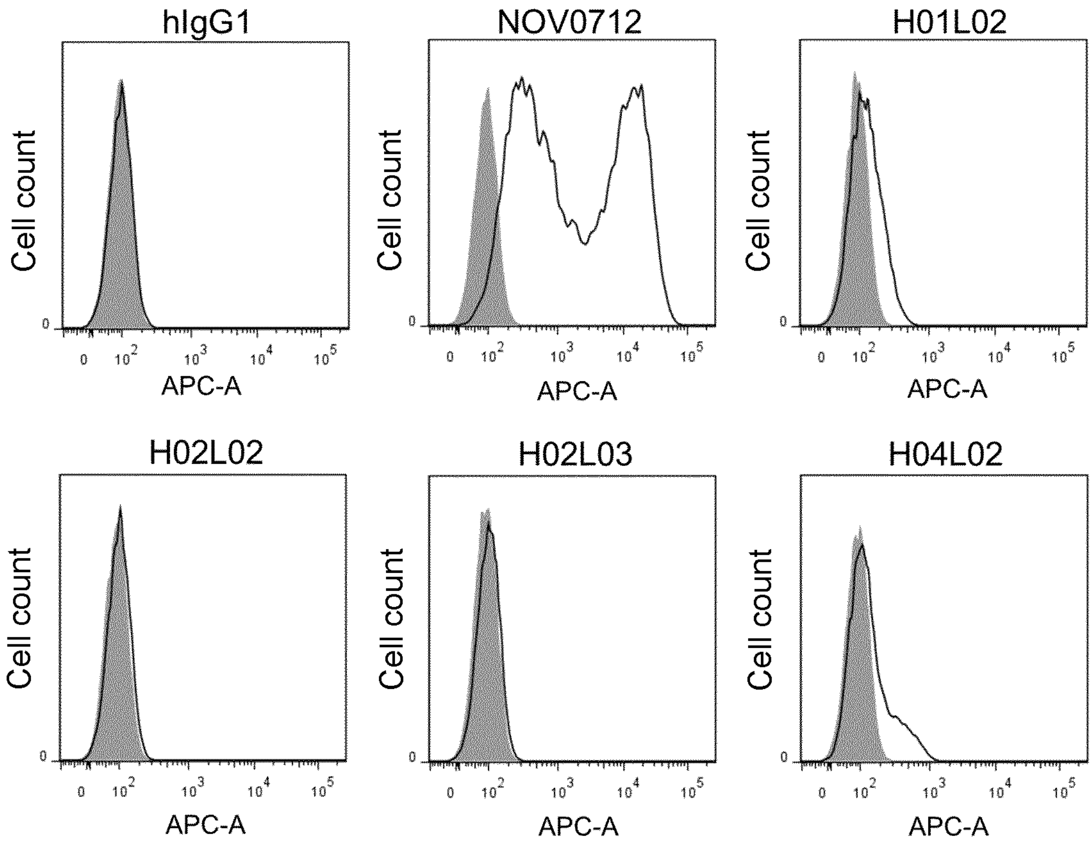

[Figure 5-5]

(5) Control cells or EC4-deleted hCDH6-transfected cells

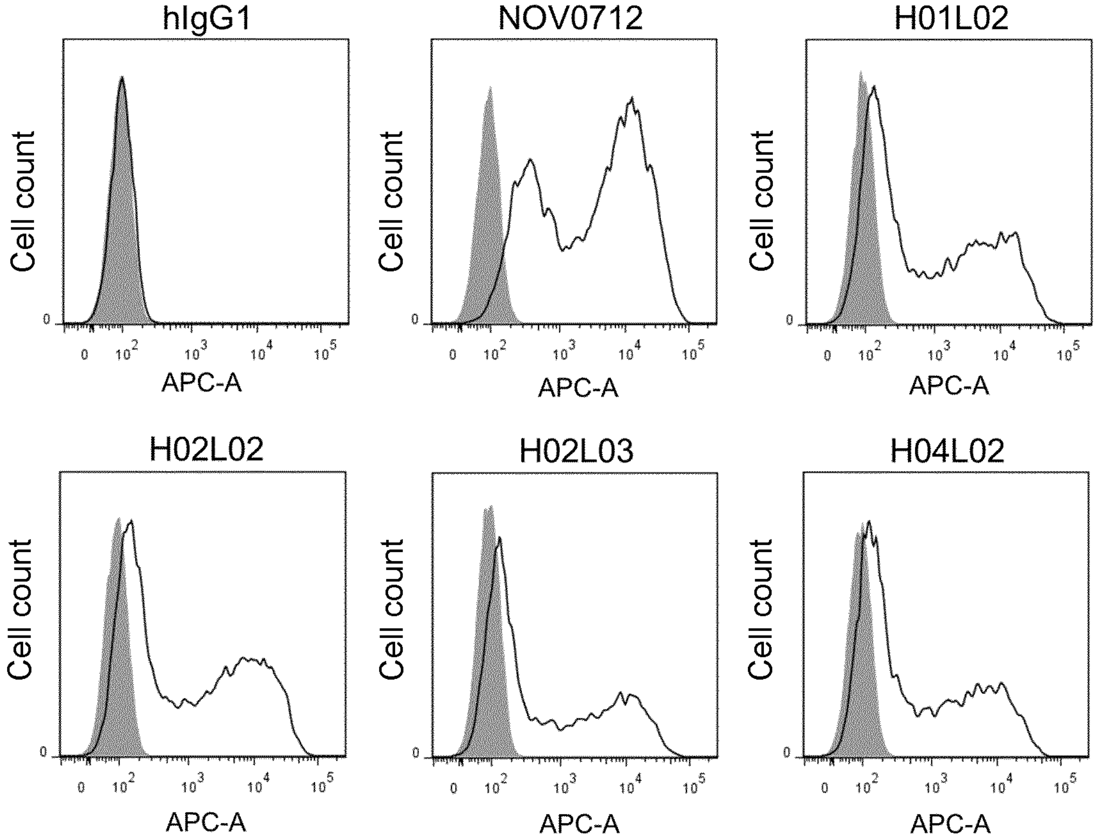

[Figure 5-6]

(6) Control cells or EC5-deleted hCDH6-transfected cells

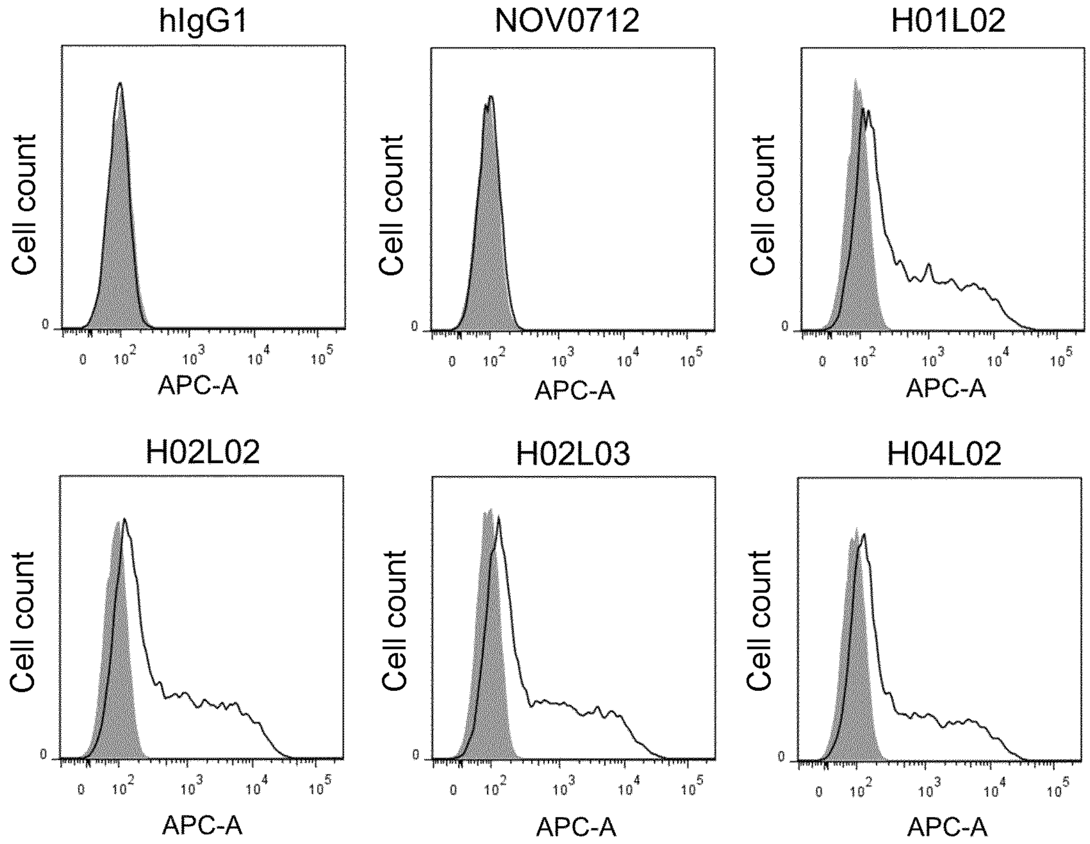

[Figure 6]

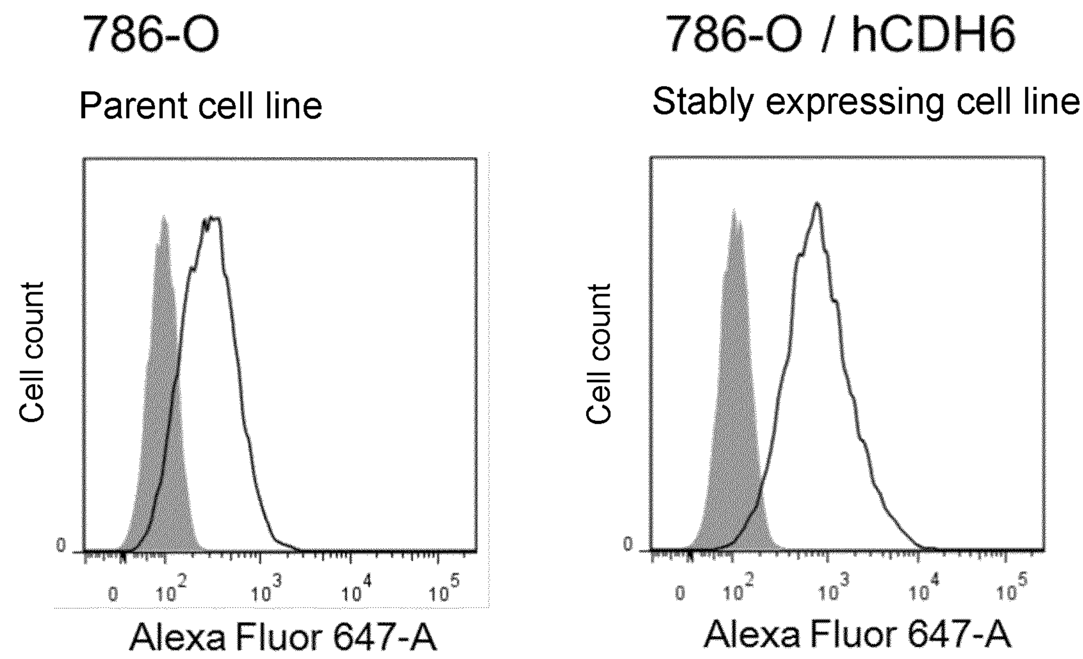
786-O
Parent cell line
Cell count
0
0
10^2
10^3
10^4
10^5
Alexa Fluor 647-A
786-O / hCDH6
Stably expressing cell line
Cell count
0
0
10^2
10^3
10^4
10^5
Alexa Fluor 647-A

[Figure 7]

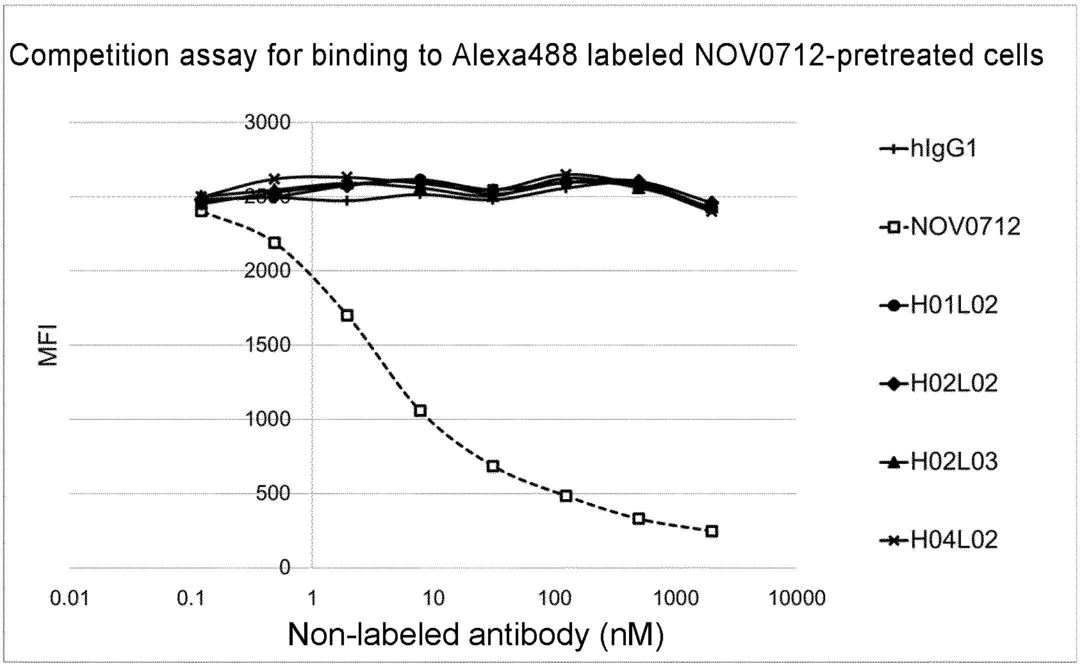
Competition assay for binding to Alexa488 labeled NOV0712-pretreated cells
MFI
3000
2500
2000
1500
1000
500
0
0.01
0.1
1
10
100
1000
10000
Non-labeled antibody (nM)
hIgG1
NOV0712
H01L02
H02L02
H02L03
H04L02

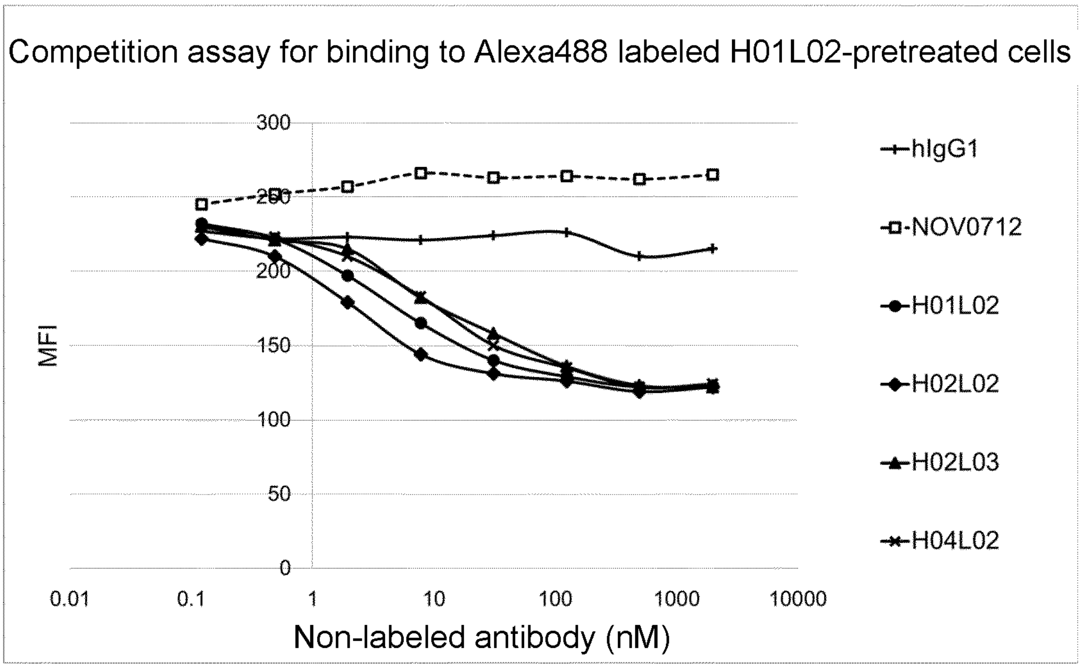
Competition assay for binding to Alexa488 labeled H01L02-pretreated cells
MFI
300
250
200
150
100
50
0
0.01
0.1
1
10
100
1000
10000
Non-labeled antibody (nM)
hIgG1
NOV0712
H01L02
H02L02
H02L03
H04L02

[Figure 8]

NIH:OVCAR-3 cell survival rate % using Hum-ZAP

| Human IgG1 | NOV0712 | H01L02 | H02L02 | H02L03 | H04L02 |
|---|---|---|---|---|---|
| 97.3 | 67.0 | 55.1 | 53.1 | 56.7 | 55.6 |

786-O cell survival rate % using Hum-ZAP

| Human IgG1 | NOV0712 | H01L02 | H02L02 | H02L03 | H04L02 |
|---|---|---|---|---|---|
| 95.9 | 74.8 | 46.0 | 44.1 | 46.5 | 45.9 |

PA-1 cell survival rate % using Hum-ZAP

| Human IgG1 | NOV0712 | H01L02 | H02L02 | H02L03 | H04L02 |
|---|---|---|---|---|---|
| 105.6 | 64.5 | 24.3 | 25.8 | 25.1 | 22.2 |

[Figure 9]

| | | IC50 (nM) | | | | |
|---|---|---|---|---|---|---|
| Cell line | The number of cells | ADC11 | ADC7 | ADC6 | ADC5 | ADC1 |
| NIH:OVCAR-3 | 3000cells/well | 8.4 | 0.11 | 0.056 | 0.0048 | 0.0038 |
| OV-90 | 1500cells/well | 7.8 | 1.4 | 0.71 | 0.026 | 0.02 |
| PA-1 | 500cells/well | 0.47 | 0.063 | 0.048 | 0.0014 | 0.001 |
| 786-0 | 500cells/well | 13 | 11 | 9.6 | 7.2 | 5.4 |

[Figure 10]

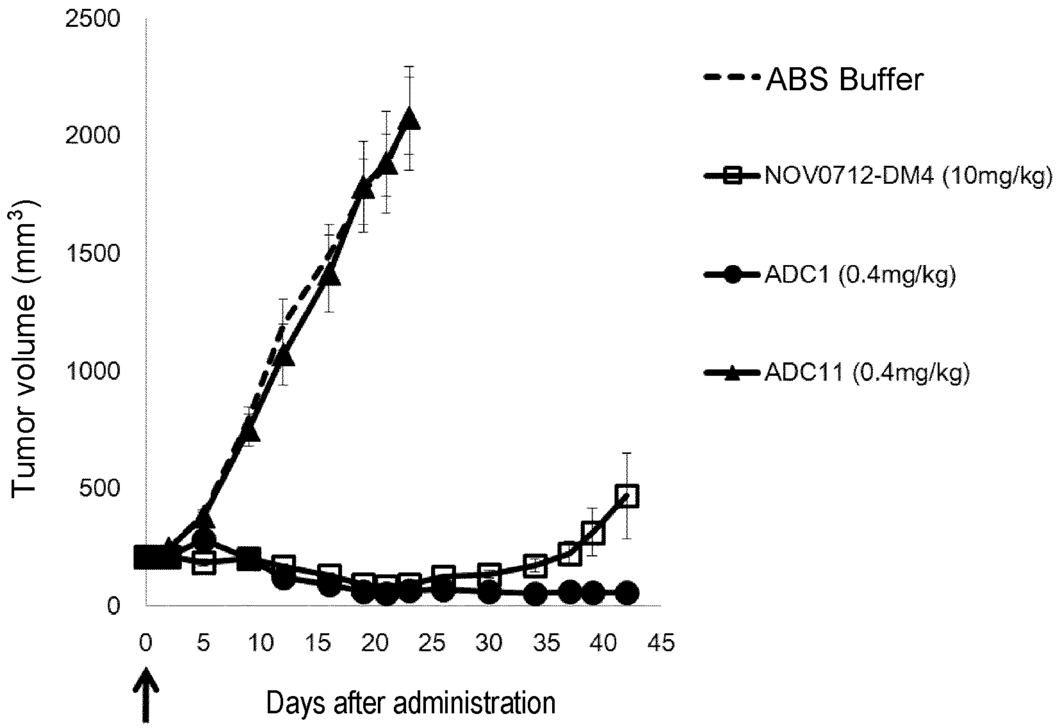


[Figure 11]

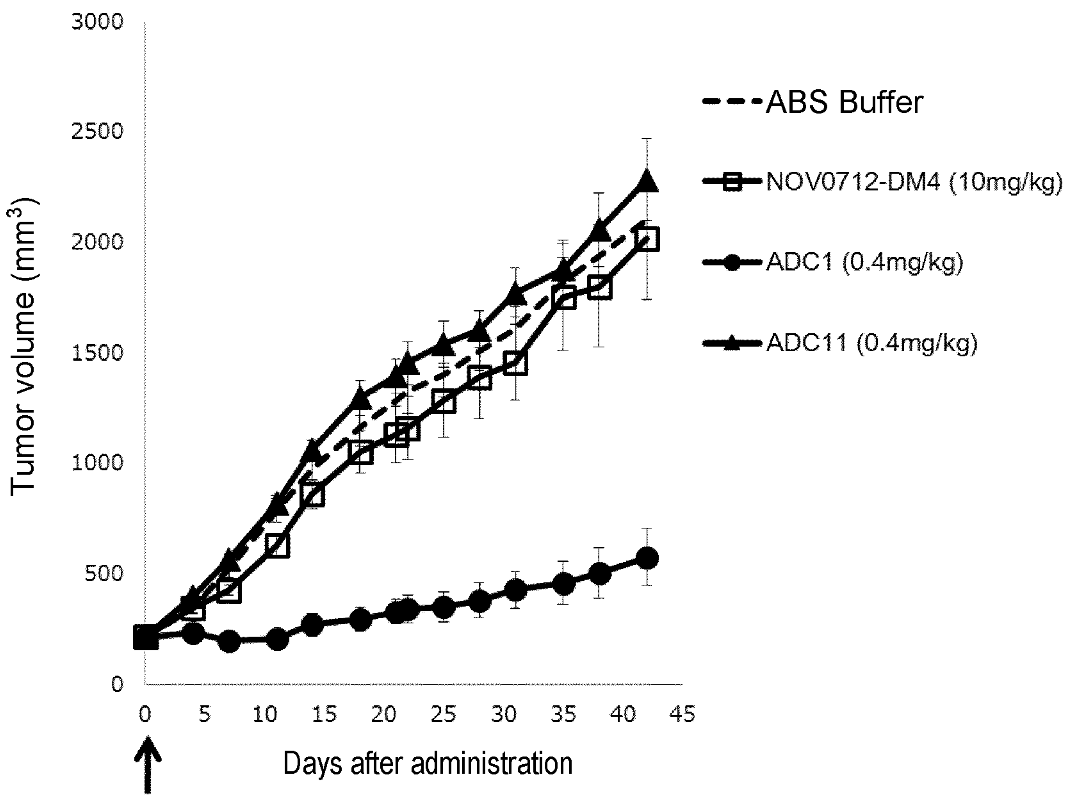

[Figure 12]

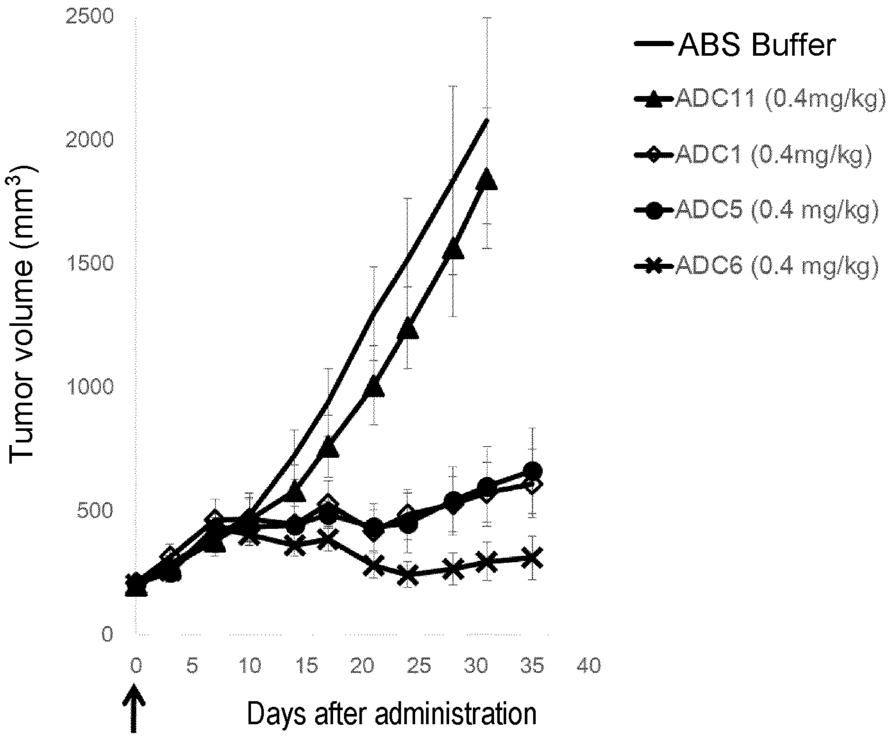


[Figure 13]

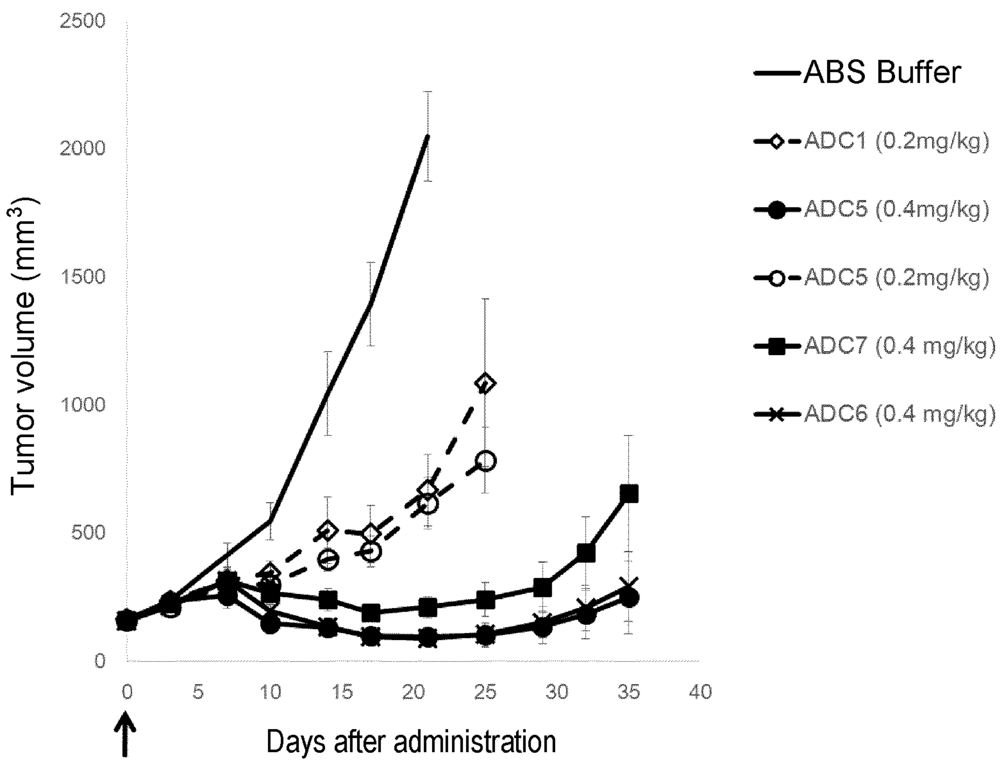

[Figure 14]

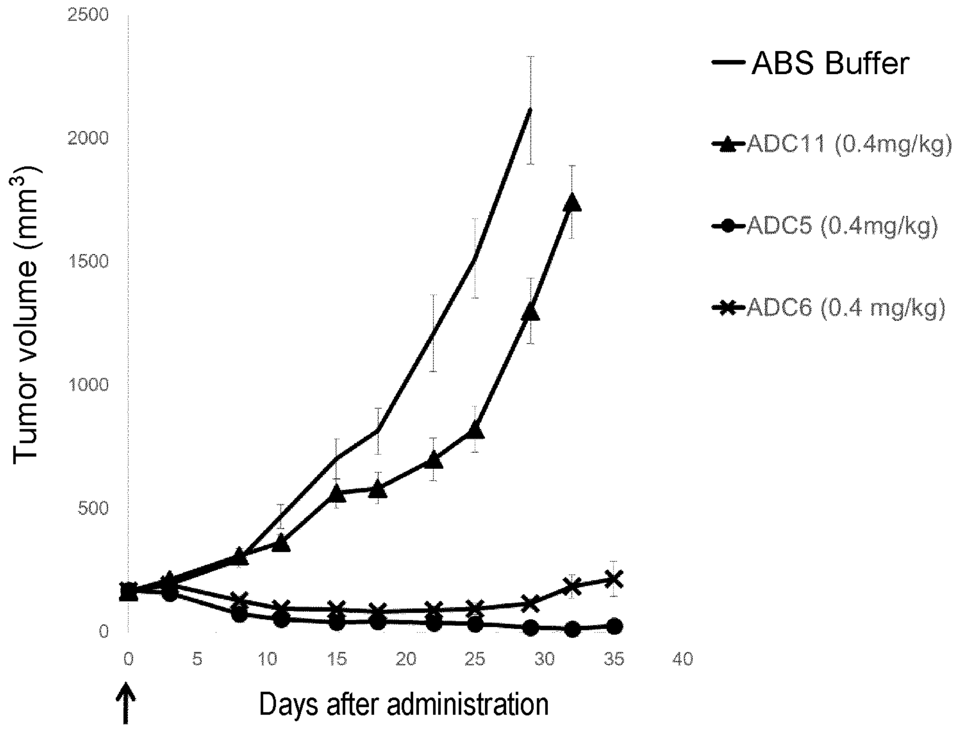


[Figure 15]

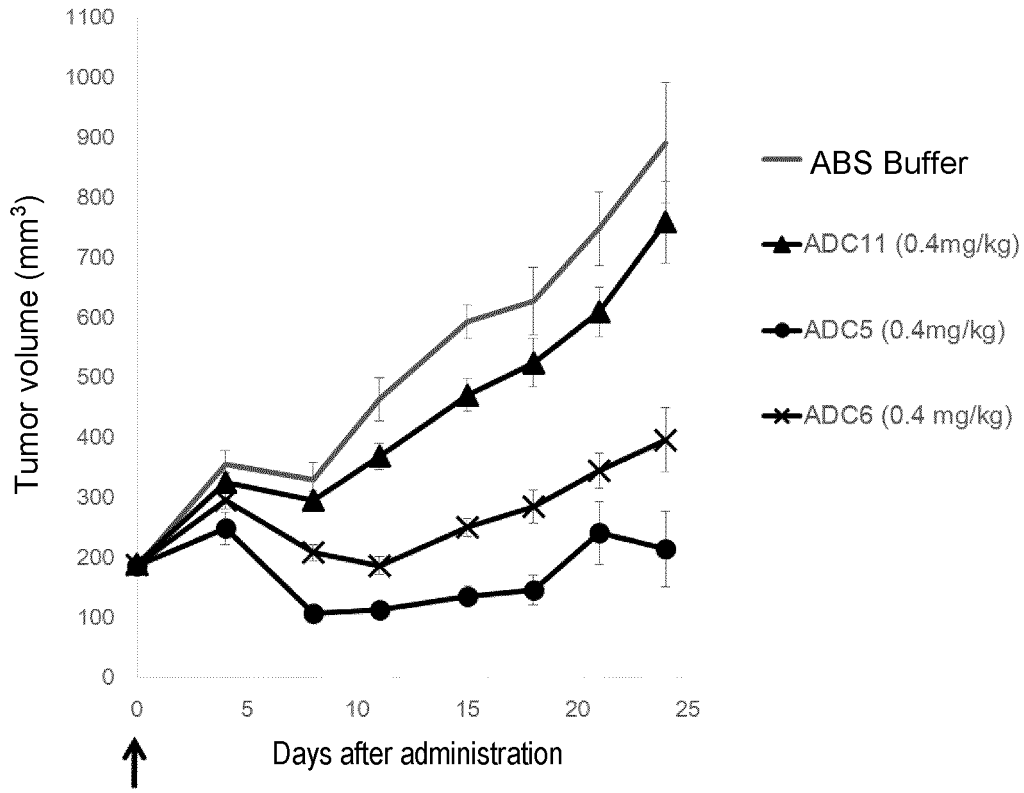

## INTERNATIONAL SEARCH REPORT

International application No.
PCT/JP2019/044588

A. CLASSIFICATION OF SUBJECT MATTER
Int.Cl. C12N15/13(2006.01)i, A61K39/395(2006.01)i, A61K47/68(2017.01)i, A61P35/00(2006.01)i, C07K16/28(2006.01)i, C12N1/15(2006.01)i, C12N1/19(2006.01)i, C12N1/21(2006.01)i, C12N5/10(2006.01)i, C12N15/63(2006.01)i, C12P21/08(2006.01)i
According to International Patent Classification (IPC) or to both national classification and IPC

B. FIELDS SEARCHED
Minimum documentation searched (classification system followed by classification symbols)
Int.Cl. C12N15/00-15/90, A61K, A61P, C07K1/00-19/00, C12N1/00-7/08, C12P1/00-41/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY/MEDLINE/EMBASE/BIOSIS/WPIDS (STN), JSTPlus/JMEDPlus/JST7580 (JDreamIII), UniProt/GeneSeq

C. DOCUMENTS CONSIDERED TO BE RELEVANT

| Category* | Citation of document, with indication, where appropriate, of the relevant passages | Relevant to claim No. |
|---|---|---|
| A | JP 2017-524364 A (NOVARTIS AG.) 31 August 2017, claims, examples & US 2016/0046711 A1, claims, examples & EP 3180360 A & KR 10-2017-0040249 A & CN 106659790 A | 1-65 |
| A | BIALUCHA, C. U. et al, "Discovery and Optimization of HKT288, a Cadherin-6-Targeting ADC for the Treatment of Ovarian and Renal Cancers", CANCER DISCOVERY, 2017, pp. 1030-1045 | 1-65 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 December 2019 (11.12.2019) | 24 December 2019 (24.12.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/044588

C (Continuation). DOCUMENTS CONSIDERED TO BE RELEVANT

| Category* | Citation of document, with indication, where appropriate, of the relevant passages | Relevant to claim No. |
|---|---|---|
| A | WO 2018/003983 A1 (DAIICHI SANKYO COMPANY, LIMITED) 04 January 2018, claims, examples & US 2019/0169293 A1, claims, examples & EP 3480211 A & CN 109462989 A & KR 10-2019-0025576 A | 1-65 |
| A | JP 2018-532695 A (SUZHOU M-CONJ BIOTECH CO., LTD.) 18 November 2018, claims, example 49 & WO 2015/155753 A2, claims, example 49 & CN 108289964 A | 1-65 |
| P, A | WO 2019/065964 A1 (DAIICHI SANKYO COMPANY, LIMITED) 04 April 2019, claims, examples & TW 201922788 A | 1-65 |
| P, A | WO 2018/212136 A1 (DAIICHI SANKYO COMPANY, LIMITED) 22 November 2018, claims, examples & TW 201900683 A | 1-65 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014057687 A
- US 20160297890 A
- WO 2016024195 A
- WO 2013173496 A
- WO 2014130879 A
- WO 2017004330 A
- WO 2017004025 A
- WO 2017020972 A
- WO 2016036804 A
- WO 2015095124 A
- WO 2015052322 A
- WO 2015052534 A
- WO 2016115191 A
- WO 2015052321 A
- WO 2015031693 A
- WO 2011130613 A
- WO 9007861 A
- WO 9201047 A
- WO 9220791 A
- WO 9306213 A
- WO 9311236 A
- WO 9319172 A
- WO 9501438 A
- WO 9515388 A
- WO 199954342 A
- WO 200061739 A
- WO 200231140 A
- WO 2007133855 A
- US 2016361436 A
- WO 20110278681 A
- WO 2013120066 A
- WO 2017010559 A
- WO 2015046505 A
- WO 2012087596 A
- US 20150283262 A
- WO 2011130598 A
- WO 2016149546 A


### Non-patent literature cited in the description


- **SHIMOYAMA Y et al.** *Cancer Research,* 15 May 1995, vol. 55, 2206-2211
- **INOUE T et al.** *Developmental Biology,* 1997, 183-194
- **OSTERHOUT J A et al.** *Neuron,* 25 August 2011, vol. 71, 632-639
- **CHO E A et al.** *Development,* 1998, vol. 125, 803-812
- **MAH S P et al.** *Developmental Biology,* 2000, vol. 223, 38-53
- **PAUL R et al.** *Cancer Research,* 01 July 1997, vol. 57, 2741-2748
- **SHIMAZUI T et al.** *Cancer,* 01 September 2004, vol. 101 (5), 963-968
- **KOEBEL M et al.** *PLoS Medicine,* December 2008, vol. 5 (12), 1749-1760
- **GUGNONI M et al.** *Oncogene,* 2017, vol. 36, 667-677
- **POLAKIS P.** *Pharmacological Reviews,* 2016, vol. 68, 3-19
- **PETERS C et al.** *Bioscience Reports,* 2015, vol. 35, 1-20
- **GOEPPERT B et al.** *Epigenetics,* 2016, vol. 11 (11), 780-790
- **YOKOI S et al.** *American Journal of Pathology,* 2002, vol. 161 (1), 207-216
- **JULIA MANTAJ et al.** *Angewandte Chemie Internationl Edition,* 2016, vol. 55, 2-29
- **DYEISON ANTONOW.** *Chemical Reviews,* 2010, vol. 111, 2815-2864
- In Antibiotics III. Springer Verlag, 3-11
- *Accounts of Chemical Research,* 1986, vol. 19, 230
- *Journal of the American Chemical Society,* 1992, vol. 114, 4939
- *Journal of Organic Chemistry,* 1996, vol. 61, 8141
- *Cell Death and Differentiation,* 2008, vol. 15, 751-761
- *Molecular Biology of the Cell,* December 2004, vol. 15, 5268-5282
- *Bio Techniques,* January 2000, vol. 28, 162-165
- **KOHLER ; MILSTEIN.** *Nature,* 1975, vol. 256, 495-497
- Monoclonal Antibodies. Plenum Press, 1980, 365-367
- **AIHARA H ; MIYAZAKI J.** *Nat Biotechnol.,* September 1998, vol. 16 (9), 867-70

- **MIR LM ; BUREAU MF ; GEHL J ; RANGARA R ; ROUY D ; CAILLAUD JM ; DELAERE P ; BRANELLEC D ; SCHWARTZ B ; SCHERMAN D.** *Proc Natl Acad Sci U S A.,* 13 April 1999, vol. 96 (8), 4262-7
- **MCMAHON JM1 ; SIGNORI E ; WELLS KE ; FAZIO VM ; WELLS DJ.** *Gene Ther.,* August 2001, vol. 8 (16), 1264-70
- *Proc. Natl. Acad. Sci. U.S.A.,* 1984, vol. 81, 6851-6855
- *Nature,* 1986, vol. 321, 522-525
- **LARKIN MA ; BLACKSHIELDS G ; BROWN NP ; CHENNA R ; MCGETTIGAN PA ; MCWILLIAM H ; VALENTIN F ; WALLACE IM ; WILM A ; LOPEZ R.** Clustal W and Clustal X version 2.0. *Bioinformatics,* 2007, vol. 23 (21), 2947-2948
- **TOMIZUKA, K. et al.** *Nature Genetics,* 1997, vol. 16, 133-143
- **KUROIWA, Y. et al.** *Nucl. Acids Res.,* 1998, vol. 26, 3447-3448
- **YOSHIDA, H. et al.** Animal Cell Technology: Basic and Applied Aspects. Kluwer Academic Publishers, 1999, vol. 10, 69-73
- **TOMIZUKA, K. et al.** *Proc. Natl. Acad. Sci. USA,* 2000, vol. 97, 722-727
- **WORMSTONE, I. M. et al.** *Investigative Ophthalmology & Visual Science,* 2002, vol. 43 (7), 2301-2308
- **CARMEN, S. et al.** *Briefings in Functional Genomics and Proteomics,* 2002, vol. 1 (2), 189-203
- **SIRIWARDENA, D. et al.** *Ophthalmology,* 2002, vol. 109 (3), 427-431
- *Nature Biotechnology,* 2005, vol. 23 (9), 1105-1116
- *Annu. Rev. Immunol,* 1994, vol. 12, 433-455
- **LORI BURTON et al.** *Pharmaceutical Development and Technology,* 2007, vol. 12, 265-273
- **GLUZMAN, Y.** *Cell,* 1981, vol. 23, 175-182
- **URLAUB, G. ; CHASIN, L. A.** *Proc. Natl. Acad. Sci. U.S.A.,* 1980, vol. 77, 4126-4220
- *Journal of Chromatography A,* 1995, vol. 705, 129-134
- *Analytical Biochemistry,* 2007, vol. 360, 75-83
- *Journal of Virology,* 15 December 2001, vol. 75 (24), 12161-12168
- *Proc. Natl. Acad. Sci. U.S.A.,* 15 May 1969, vol. 63 (1), 78-85
- Strategies for Protein Purification and Characterization: A Laboratory Course Manual. Cold Spring Harbor Laboratory Press, 1996
- **ED HARLOW ; DAVID LANE.** Antibodies: A Laboratory Manual. Cold Spring Harbor Laboratory, 1988
- *ACS Chemical Biology,* 2012, vol. 7, 110
- *ACS Medicinal Chemistry Letters,* 2016, vol. 7, 1005
- *Bioconjugate Chemistry,* 2015, vol. 26, 2233
- *Angew. Chem. Int. Ed.,* 2016, vol. 55, 2361-2367
- *Biotechnol. Prog.,* 2012, vol. 28, 608-622
- **SANGLIER-CIANFERANI, S.** *Anal. Chem.,* 2013, vol. 85, 715-736
- **EDELMAN et al.** *Proc. Natl. Acad. Sci. U.S.A.,* 15 May 1969, vol. 63, 78-85
- *J. Org Chem.,* 2009, vol. 74 (5), 2210-2212
- *Helv. Chim. Acta,* 2012, vol. 95, 1928-1936
- *J. Am. Chem. Soc.,* 2012, vol. 134, 12308-12318
- *J. AM. CHEM. SOC.,* 2004, vol. 126, 15046-15047
- *Protein Science,* 1995, vol. 4, 2411-2423
- **SAMBROOK, J. ; FRITSCH, E. F. ; MANIATIS, T.** Molecular Cloning. Cold Spring Harbor Laboratory Press, 1989
- *Methods in Enzymology,* 1991, vol. 203, 121-153
- *Nuc. Acid Res.,* 2007, vol. 35, D301-D303
- *Proc. Natl. Acad. Sci. USA,* 1989, vol. 86, 10029-10033
- **KABAT et al.** Sequences of Proteins of Immunological Interest. Public Health Service National Institutes of Health, 1991
- **QUEEN et al.** *Proc. Natl. Acad. Sci. USA,* 1989, vol. 86, 10029-10033
- *Journal of Medicinal Chemistry,* 2004, vol. 47, 1161
- *Tetrahedron Letters,* 2012, vol. 53, 3847
- *Tetrahedron,* 1995, vol. 51, 5617

SEQUENCE LISTING

<110>  DAIICHI SANKYO COMPANY, LIMITED

<120>  ANTI-CDH6 ANTIBODY-PYRROLOBENZODIAZEPINE DERIVATIVES CONJUGATE

<130>  DSPCT-FP1936

<150>  JP2018-214110
<151>  2018-11-14

<150>  JP2019-057327
<151>  2019-03-25

<160>  73

<170>  PatentIn version 3.5

<210>  1
<211>  790
<212>  PRT
<213>  Homo sapiens

<400>  1

```
Met Arg Thr Tyr Arg Tyr Phe Leu Leu Leu Phe Trp Val Gly Gln Pro
1               5                   10                  15

Tyr Pro Thr Leu Ser Thr Pro Leu Ser Lys Arg Thr Ser Gly Phe Pro
            20                  25                  30

Ala Lys Lys Arg Ala Leu Glu Leu Ser Gly Asn Ser Lys Asn Glu Leu
        35                  40                  45

Asn Arg Ser Lys Arg Ser Trp Met Trp Asn Gln Phe Phe Leu Leu Glu
    50                  55                  60

Glu Tyr Thr Gly Ser Asp Tyr Gln Tyr Val Gly Lys Leu His Ser Asp
65                  70                  75                  80

Gln Asp Arg Gly Asp Gly Ser Leu Lys Tyr Ile Leu Ser Gly Asp Gly
                85                  90                  95

Ala Gly Asp Leu Phe Ile Ile Asn Glu Asn Thr Gly Asp Ile Gln Ala
            100                 105                 110

Thr Lys Arg Leu Asp Arg Glu Glu Lys Pro Val Tyr Ile Leu Arg Ala
        115                 120                 125

Gln Ala Ile Asn Arg Arg Thr Gly Arg Pro Val Glu Pro Glu Ser Glu
    130                 135                 140

Phe Ile Ile Lys Ile His Asp Ile Asn Asp Asn Glu Pro Ile Phe Thr
145                 150                 155                 160
```

Lys Glu Val Tyr Thr Ala Thr Val Pro Glu Met Ser Asp Val Gly Thr
165 170 175

Phe Val Val Gln Val Thr Ala Thr Asp Ala Asp Asp Pro Thr Tyr Gly
180 185 190

Asn Ser Ala Lys Val Val Tyr Ser Ile Leu Gln Gly Gln Pro Tyr Phe
195 200 205

Ser Val Glu Ser Glu Thr Gly Ile Ile Lys Thr Ala Leu Leu Asn Met
210 215 220

Asp Arg Glu Asn Arg Glu Gln Tyr Gln Val Val Ile Gln Ala Lys Asp
225 230 235 240

Met Gly Gly Gln Met Gly Gly Leu Ser Gly Thr Thr Thr Val Asn Ile
245 250 255

Thr Leu Thr Asp Val Asn Asp Asn Pro Pro Arg Phe Pro Gln Ser Thr
260 265 270

Tyr Gln Phe Lys Thr Pro Glu Ser Ser Pro Pro Gly Thr Pro Ile Gly
275 280 285

Arg Ile Lys Ala Ser Asp Ala Asp Val Gly Glu Asn Ala Glu Ile Glu
290 295 300

Tyr Ser Ile Thr Asp Gly Glu Gly Leu Asp Met Phe Asp Val Ile Thr
305 310 315 320

Asp Gln Glu Thr Gln Glu Gly Ile Ile Thr Val Lys Lys Leu Leu Asp
325 330 335

Phe Glu Lys Lys Lys Val Tyr Thr Leu Lys Val Glu Ala Ser Asn Pro
340 345 350

Tyr Val Glu Pro Arg Phe Leu Tyr Leu Gly Pro Phe Lys Asp Ser Ala
355 360 365

Thr Val Arg Ile Val Val Glu Asp Val Asp Glu Pro Pro Val Phe Ser
370 375 380

Lys Leu Ala Tyr Ile Leu Gln Ile Arg Glu Asp Ala Gln Ile Asn Thr
385 390 395 400

Thr Ile Gly Ser Val Thr Ala Gln Asp Pro Asp Ala Ala Arg Asn Pro

405 410 415

Val Lys Tyr Ser Val Asp Arg His Thr Asp Met Asp Arg Ile Phe Asn
420 425 430

Ile Asp Ser Gly Asn Gly Ser Ile Phe Thr Ser Lys Leu Leu Asp Arg
435 440 445

Glu Thr Leu Leu Trp His Asn Ile Thr Val Ile Ala Thr Glu Ile Asn
450 455 460

Asn Pro Lys Gln Ser Ser Arg Val Pro Leu Tyr Ile Lys Val Leu Asp
465 470 475 480

Val Asn Asp Asn Ala Pro Glu Phe Ala Glu Phe Tyr Glu Thr Phe Val
485 490 495

Cys Glu Lys Ala Lys Ala Asp Gln Leu Ile Gln Thr Leu His Ala Val
500 505 510

Asp Lys Asp Asp Pro Tyr Ser Gly His Gln Phe Ser Phe Ser Leu Ala
515 520 525

Pro Glu Ala Ala Ser Gly Ser Asn Phe Thr Ile Gln Asp Asn Lys Asp
530 535 540

Asn Thr Ala Gly Ile Leu Thr Arg Lys Asn Gly Tyr Asn Arg His Glu
545 550 555 560

Met Ser Thr Tyr Leu Leu Pro Val Val Ile Ser Asp Asn Asp Tyr Pro
565 570 575

Val Gln Ser Ser Thr Gly Thr Val Thr Val Arg Val Cys Ala Cys Asp
580 585 590

His His Gly Asn Met Gln Ser Cys His Ala Glu Ala Leu Ile His Pro
595 600 605

Thr Gly Leu Ser Thr Gly Ala Leu Val Ala Ile Leu Leu Cys Ile Val
610 615 620

Ile Leu Leu Val Thr Val Val Leu Phe Ala Ala Leu Arg Arg Gln Arg
625 630 635 640

Lys Lys Glu Pro Leu Ile Ile Ser Lys Glu Asp Ile Arg Asp Asn Ile
645 650 655

Val Ser Tyr Asn Asp Glu Gly Gly Gly Glu Glu Asp Thr Gln Ala Phe
660 665 670

Asp Ile Gly Thr Leu Arg Asn Pro Glu Ala Ile Glu Asp Asn Lys Leu
675 680 685

Arg Arg Asp Ile Val Pro Glu Ala Leu Phe Leu Pro Arg Arg Thr Pro
690 695 700

Thr Ala Arg Asp Asn Thr Asp Val Arg Asp Phe Ile Asn Gln Arg Leu
705 710 715 720

Lys Glu Asn Asp Thr Asp Pro Thr Ala Pro Pro Tyr Asp Ser Leu Ala
725 730 735

Thr Tyr Ala Tyr Glu Gly Thr Gly Ser Val Ala Asp Ser Leu Ser Ser
740 745 750

Leu Glu Ser Val Thr Thr Asp Ala Asp Gln Asp Tyr Asp Tyr Leu Ser
755 760 765

Asp Trp Gly Pro Arg Phe Lys Lys Leu Ala Asp Met Tyr Gly Gly Val
770 775 780

Asp Ser Asp Lys Asp Ser
785 790

<210> 2
<211> 106
<212> PRT
<213> Homo sapiens

<400> 2

Ser Trp Met Trp Asn Gln Phe Phe Leu Leu Glu Glu Tyr Thr Gly Ser
1 5 10 15

Asp Tyr Gln Tyr Val Gly Lys Leu His Ser Asp Gln Asp Arg Gly Asp
20 25 30

Gly Ser Leu Lys Tyr Ile Leu Ser Gly Asp Gly Ala Gly Asp Leu Phe
35 40 45

Ile Ile Asn Glu Asn Thr Gly Asp Ile Gln Ala Thr Lys Arg Leu Asp
50 55 60

Arg Glu Glu Lys Pro Val Tyr Ile Leu Arg Ala Gln Ala Ile Asn Arg
65 70 75 80

Arg Thr Gly Arg Pro Val Glu Pro Glu Ser Glu Phe Ile Ile Lys Ile
85 90 95

His Asp Ile Asn Asp Asn Glu Pro Ile Phe
100 105

<210> 3
<211> 109
<212> PRT
<213> Homo sapiens

<400> 3

Thr Lys Glu Val Tyr Thr Ala Thr Val Pro Glu Met Ser Asp Val Gly
1 5 10 15

Thr Phe Val Val Gln Val Thr Ala Thr Asp Ala Asp Asp Pro Thr Tyr
20 25 30

Gly Asn Ser Ala Lys Val Val Tyr Ser Ile Leu Gln Gly Gln Pro Tyr
35 40 45

Phe Ser Val Glu Ser Glu Thr Gly Ile Ile Lys Thr Ala Leu Leu Asn
50 55 60

Met Asp Arg Glu Asn Arg Glu Gln Tyr Gln Val Val Ile Gln Ala Lys
65 70 75 80

Asp Met Gly Gly Gln Met Gly Gly Leu Ser Gly Thr Thr Thr Val Asn
85 90 95

Ile Thr Leu Thr Asp Val Asn Asp Asn Pro Pro Arg Phe
100 105

<210> 4
<211> 115
<212> PRT
<213> Homo sapiens

<400> 4

Pro Gln Ser Thr Tyr Gln Phe Lys Thr Pro Glu Ser Ser Pro Pro Gly
1 5 10 15

Thr Pro Ile Gly Arg Ile Lys Ala Ser Asp Ala Asp Val Gly Glu Asn
20 25 30

Ala Glu Ile Glu Tyr Ser Ile Thr Asp Gly Glu Gly Leu Asp Met Phe
35 40 45

Asp Val Ile Thr Asp Gln Glu Thr Gln Glu Gly Ile Ile Thr Val Lys

50 55 60

Lys Leu Leu Asp Phe Glu Lys Lys Lys Val Tyr Thr Leu Lys Val Glu
65 70 75 80

Ala Ser Asn Pro Tyr Val Glu Pro Arg Phe Leu Tyr Leu Gly Pro Phe
85 90 95

Lys Asp Ser Ala Thr Val Arg Ile Val Val Glu Asp Val Asp Glu Pro
100 105 110

Pro Val Phe
115

<210> 5
<211> 103
<212> PRT
<213> Homo sapiens

<400> 5

Ser Lys Leu Ala Tyr Ile Leu Gln Ile Arg Glu Asp Ala Gln Ile Asn
1 5 10 15

Thr Thr Ile Gly Ser Val Thr Ala Gln Asp Pro Asp Ala Ala Arg Asn
20 25 30

Pro Val Lys Tyr Ser Val Asp Arg His Thr Asp Met Asp Arg Ile Phe
35 40 45

Asn Ile Asp Ser Gly Asn Gly Ser Ile Phe Thr Ser Lys Leu Leu Asp
50 55 60

Arg Glu Thr Leu Leu Trp His Asn Ile Thr Val Ile Ala Thr Glu Ile
65 70 75 80

Asn Asn Pro Lys Gln Ser Ser Arg Val Pro Leu Tyr Ile Lys Val Leu
85 90 95

Asp Val Asn Asp Asn Ala Pro
100

<210> 6
<211> 122
<212> PRT
<213> Homo sapiens

<400> 6

Glu Phe Ala Glu Phe Tyr Glu Thr Phe Val Cys Glu Lys Ala Lys Ala
1 5 10 15

Asp Gln Leu Ile Gln Thr Leu His Ala Val Asp Lys Asp Asp Pro Tyr
20 25 30

Ser Gly His Gln Phe Ser Phe Ser Leu Ala Pro Glu Ala Ala Ser Gly
35 40 45

Ser Asn Phe Thr Ile Gln Asp Asn Lys Asp Asn Thr Ala Gly Ile Leu
50 55 60

Thr Arg Lys Asn Gly Tyr Asn Arg His Glu Met Ser Thr Tyr Leu Leu
65 70 75 80

Pro Val Val Ile Ser Asp Asn Asp Tyr Pro Val Gln Ser Ser Thr Gly
85 90 95

Thr Val Thr Val Arg Val Cys Ala Cys Asp His His Gly Asn Met Gln
100 105 110

Ser Cys His Ala Glu Ala Leu Ile His Pro
115 120

<210> 7
<211> 790
<212> PRT
<213> Macaca fascicularis

<400> 7

Met Arg Thr Tyr Arg Tyr Phe Leu Leu Leu Phe Trp Val Gly Gln Pro
1 5 10 15

Tyr Pro Thr Leu Ser Thr Pro Leu Ser Lys Arg Thr Ser Gly Phe Pro
20 25 30

Ala Lys Lys Arg Ala Leu Glu Leu Ser Gly Asn Ser Lys Asn Glu Leu
35 40 45

Asn Arg Ser Lys Arg Ser Trp Met Trp Asn Gln Phe Phe Leu Leu Glu
50 55 60

Glu Tyr Thr Gly Ser Asp Tyr Gln Tyr Val Gly Lys Leu His Ser Asp
65 70 75 80

Gln Asp Arg Gly Asp Gly Ser Leu Lys Tyr Ile Leu Ser Gly Asp Gly
85 90 95

Ala Gly Asp Leu Phe Ile Ile Asn Glu Asn Thr Gly Asp Ile Gln Ala
100 105 110

Thr Lys Arg Leu Asp Arg Glu Glu Lys Pro Val Tyr Ile Leu Arg Ala
115 120 125

Gln Ala Ile Asn Arg Arg Thr Gly Arg Pro Val Glu Pro Glu Ser Glu
130 135 140

Phe Ile Ile Lys Ile His Asp Ile Asn Asp Asn Glu Pro Ile Phe Thr
145 150 155 160

Lys Glu Val Tyr Thr Ala Thr Val Pro Glu Met Ser Asp Val Gly Thr
165 170 175

Phe Val Val Gln Val Thr Ala Thr Asp Ala Asp Asp Pro Thr Tyr Gly
180 185 190

Asn Ser Ala Lys Val Val Tyr Ser Ile Leu Gln Gly Gln Pro Tyr Phe
195 200 205

Ser Val Glu Ser Glu Thr Gly Ile Ile Lys Thr Ala Leu Leu Asn Met
210 215 220

Asp Arg Glu Asn Arg Glu Gln Tyr Gln Val Val Ile Gln Ala Lys Asp
225 230 235 240

Met Gly Gly Gln Met Gly Gly Leu Ser Gly Thr Thr Thr Val Asn Ile
245 250 255

Thr Leu Thr Asp Val Asn Asp Asn Pro Pro Arg Phe Pro Gln Ser Thr
260 265 270

Tyr Gln Phe Lys Thr Pro Glu Ser Ser Pro Pro Gly Thr Pro Ile Gly
275 280 285

Arg Ile Lys Ala Ser Asp Ala Asp Val Gly Glu Asn Ala Glu Ile Glu
290 295 300

Tyr Ser Ile Thr Asp Gly Glu Gly Leu Asp Met Phe Asp Val Ile Thr
305 310 315 320

Asp Gln Glu Thr Gln Glu Gly Ile Ile Thr Val Lys Lys Leu Leu Asp
325 330 335

Phe Glu Lys Lys Lys Val Tyr Thr Leu Lys Val Glu Ala Ser Asn Pro
340 345 350

His Val Glu Pro Arg Phe Leu Tyr Leu Gly Pro Phe Lys Asp Ser Ala

355 360 365

Thr Val Arg Ile Val Val Glu Asp Val Asp Glu Pro Pro Val Phe Ser
370 375 380

Lys Leu Ala Tyr Ile Leu Gln Ile Arg Glu Asp Ala Gln Ile Asn Thr
385 390 395 400

Thr Ile Gly Ser Val Thr Ala Gln Asp Pro Asp Ala Ala Arg Asn Pro
405 410 415

Val Lys Tyr Ser Val Asp Arg His Thr Asp Met Asp Arg Ile Phe Asn
420 425 430

Ile Asp Ser Gly Asn Gly Ser Ile Phe Thr Ser Lys Leu Leu Asp Arg
435 440 445

Glu Thr Leu Leu Trp His Asn Ile Thr Val Ile Ala Thr Glu Ile Asn
450 455 460

Asn Pro Lys Gln Ser Ser Arg Val Pro Leu Tyr Ile Lys Val Leu Asp
465 470 475 480

Val Asn Asp Asn Ala Pro Glu Phe Ala Glu Phe Tyr Glu Thr Phe Val
485 490 495

Cys Glu Lys Ala Lys Ala Asp Gln Leu Ile Gln Thr Leu Arg Ala Val
500 505 510

Asp Lys Asp Asp Pro Tyr Ser Gly His Gln Phe Ser Phe Ser Leu Ala
515 520 525

Pro Glu Ala Ala Ser Gly Ser Asn Phe Thr Ile Gln Asp Asn Lys Asp
530 535 540

Asn Thr Ala Gly Ile Leu Thr Arg Lys Asn Gly Tyr Asn Arg His Glu
545 550 555 560

Met Ser Thr Tyr Leu Leu Pro Val Val Ile Ser Asp Asn Asp Tyr Pro
565 570 575

Val Gln Ser Ser Thr Gly Thr Val Thr Val Arg Val Cys Ala Cys Asp
580 585 590

His His Gly Asn Met Gln Ser Cys His Ala Glu Ala Leu Ile His Pro
595 600 605

Thr Gly Leu Ser Thr Gly Ala Leu Val Ala Ile Leu Leu Cys Ile Val
610 615 620

Ile Leu Leu Val Thr Val Val Leu Phe Ala Ala Leu Arg Arg Gln Arg
625 630 635 640

Lys Lys Glu Pro Leu Ile Ile Ser Lys Glu Asp Ile Arg Asp Asn Ile
645 650 655

Val Ser Tyr Asn Asp Glu Gly Gly Gly Glu Glu Asp Thr Gln Ala Phe
660 665 670

Asp Ile Gly Thr Leu Arg Asn Pro Glu Ala Ile Glu Asp Asn Lys Leu
675 680 685

Arg Arg Asp Ile Val Pro Glu Ala Leu Phe Leu Pro Arg Arg Thr Pro
690 695 700

Thr Ala Arg Asp Asn Thr Asp Val Arg Asp Phe Ile Asn Gln Arg Leu
705 710 715 720

Lys Glu Asn Asp Thr Asp Pro Thr Ala Pro Pro Tyr Asp Ser Leu Ala
725 730 735

Thr Tyr Ala Tyr Glu Gly Thr Gly Ser Val Ala Asp Ser Leu Ser Ser
740 745 750

Leu Glu Ser Val Thr Thr Asp Gly Asp Gln Asp Tyr Asp Tyr Leu Ser
755 760 765

Asp Trp Gly Pro Arg Phe Lys Lys Leu Ala Asp Met Tyr Gly Gly Val
770 775 780

Asp Ser Asp Lys Asp Ser
785 790

<210> 8
<211> 34
<212> DNA
<213> Artificial Sequence

<220>
<223> primer 1

<400> 8
caccatgaga acttaccgct acttcttgct gctc 34

<210> 9
<211> 30
<212> DNA

<213>  Artificial Sequence

<220>
<223>  primer 2

<400>  9
ttaggagtct ttgtcactgt ccactcctcc    30

<210>  10
<211>  108
<212>  PRT
<213>  Rattus norvegicus

<400>  10

Asp Ile Gln Met Thr Gln Ser Pro Ser Leu Leu Ser Ala Ser Val Gly
1               5                   10                  15

Asp Arg Val Thr Leu Asn Cys Lys Ala Ser Gln Asn Ile Tyr Lys Asn
            20                  25                  30

Leu Ala Trp Tyr Gln Gln Lys Leu Gly Glu Gly Pro Lys Leu Leu Ile
        35                  40                  45

Tyr Asp Ala Asn Thr Leu Gln Thr Gly Ile Pro Ser Arg Phe Ser Gly
    50                  55                  60

Ser Gly Ser Gly Ser Asp Phe Thr Leu Thr Ile Ser Ser Leu Gln Pro
65                  70                  75                  80

Glu Asp Val Ala Thr Tyr Phe Cys Gln Gln Tyr Tyr Ser Gly Trp Ala
                85                  90                  95

Phe Gly Gly Val Thr Asn Leu Glu Leu Lys Arg Ala
            100                 105

<210>  11
<211>  324
<212>  DNA
<213>  Rattus norvegicus

<400>  11
gacatccaga tgacccagtc tccttcactc ctgtctgcat ctgtgggaga cagagtcact    60

ctcaactgca aagcaagtca gaatatttat aagaacttag cctggtatca gcaaaagctt    120

ggagaaggtc ccaaactcct gatttatgat gcaaacactt tgcaaacggg catcccatca    180

aggttcagtg gcagtggatc tggttcagat ttcacactca ccatcagcag cctgcagcct    240

gaagatgttg ccacatattt ctgccagcag tactatagcg ggtgggcgtt cggtggagtc    300

accaacctgg aattgaaacg ggct    324

<210> 12
<211> 11
<212> PRT
<213> Rattus norvegicus

<400> 12

Lys Ala Ser Gln Asn Ile Tyr Lys Asn Leu Ala
1 5 10

<210> 13
<211> 7
<212> PRT
<213> Rattus norvegicus

<400> 13

Asp Ala Asn Thr Leu Gln Thr
1 5

<210> 14
<211> 8
<212> PRT
<213> Rattus norvegicus

<400> 14

Gln Gln Tyr Tyr Ser Gly Trp Ala
1 5

<210> 15
<211> 122
<212> PRT
<213> Rattus norvegicus

<400> 15

Gln Val Gln Leu Gln Gln Ser Gly Ala Glu Leu Val Lys Pro Gly Ser
1 5 10 15

Ser Val Lys Ile Ser Cys Lys Ala Ser Gly Tyr Thr Phe Thr Arg Asn
20 25 30

Phe Met His Trp Ile Lys Gln Gln Pro Gly Asn Gly Leu Glu Trp Ile
35 40 45

Gly Trp Ile Tyr Cys Gly Asp Gly Glu Thr Glu Tyr Asn Gln Lys Phe
50 55 60

Asn Gly Lys Ala Thr Leu Thr Ala Asp Arg Ser Ser Ser Thr Ala Tyr
65 70 75 80

Met Glu Leu Ser Arg Leu Thr Ser Glu Asp Ser Ala Val Tyr Phe Cys
85 90 95

Ala Arg Gly Val Tyr Gly Gly Phe Ala Gly Gly Tyr Phe Asp Phe Trp
100 105 110

Gly Gln Gly Val Met Val Thr Val Ser Ser
115 120

<210> 16
<211> 366
<212> DNA
<213> Rattus norvegicus

<400> 16
caggtacagc tgcagcaatc tggggctgaa ctggtgaagc ctgggtcctc agtgaaaatt 60

tcctgcaagg cttctggcta caccttcacc aggaacttta tgcactggat aaaacagcag 120

cctggaaatg gccttgagtg gattgggtgg atttattgtg gagatggtga gacagagtac 180

aatcaaaagt tcaatgggaa ggcaacactc actgcggaca gatcctccag cacagcctat 240

atggagctca gcagactgac atctgaggac tctgcagtct atttctgtgc aagaggggtt 300

tacggagggt ttgccggggg ctactttgat ttctggggcc aaggagtcat ggtcacagtc 360

tcctca 366

<210> 17
<211> 10
<212> PRT
<213> Rattus norvegicus

<400> 17

Gly Tyr Thr Phe Thr Arg Asn Phe Met His
1 5 10

<210> 18
<211> 10
<212> PRT
<213> Rattus norvegicus

<400> 18

Trp Ile Tyr Cys Gly Asp Gly Glu Thr Glu
1 5 10

<210> 19
<211> 13
<212> PRT
<213> Rattus norvegicus

<400> 19

Gly Val Tyr Gly Gly Phe Ala Gly Gly Tyr Phe Asp Phe
1 5 10

<210> 20
<211> 449
<212> DNA
<213> Artificial Sequence

<220>
<223> DNA comprising DNA sequence coding for human light chain signal sequence and kappa chain constant region

<400> 20
gcctccggac tctagagcca ccatggtgct gcagacccag gtgttcatct ccctgctgct 60

gtggatctcc ggcgcgtacg gcgatatcgt gatgattaaa cgtacggtgg ccgccccctc 120

cgtgttcatc ttcccccct ccgacgagca gctgaagtcc ggcaccgcct ccgtggtgtg 180

cctgctgaat aacttctacc ccagagaggc caaggtgcag tggaaggtgg acaacgccct 240

gcagtccggg aactcccagg agagcgtgac cgagcaggac agcaaggaca gcacctacag 300

cctgagcagc accctgaccc tgagcaaagc cgactacgag aagcacaagg tgtacgcctg 360

cgaggtgacc caccagggcc tgagctcccc cgtcaccaag agcttcaaca gggggagtg 420

ttaggggccc gtttaaacgg gggaggcta 449

<210> 21
<211> 1132
<212> DNA
<213> Artificial Sequence

<220>
<223> DNA comprising DNA sequence coding for human heavy chain signal sequence and IgG1 constant region

<400> 21
gcctccggac tctagagcca ccatgaaaca cctgtggttc ttcctcctgc tggtggcagc 60

tcccagatgg gtgctgagcc aggtgcaatt gtgcaggcgg ttagctcagc ctccaccaag 120

ggcccaagcg tcttcccct ggcaccctcc tccaagagca cctctggcgg cacagccgcc 180

ctgggctgcc tggtcaagga ctacttcccc gaacccgtga ccgtgagctg gaactcaggc 240

gccctgacca gcggcgtgca caccttcccc gctgtcctgc agtcctcagg actctactcc 300

ctcagcagcg tggtgaccgt gccctccagc agcttgggca cccagaccta catctgcaac 360

gtgaatcaca agcccagcaa caccaaggtg gacaagagag ttgagcccaa atcttgtgac 420

aaaactcaca catgcccacc ctgcccagca cctgaactcc tgggggggacc ctcagtcttc 480

ctcttccccc caaaacccaa ggacaccctc atgatctccc ggacccctga ggtcacatgc 540

gtggtggtgg acgtgagcca cgaagaccct gaggtcaagt tcaactggta cgtggacggc 600

gtggaggtgc ataatgccaa gacaaagccc cgggaggagc agtacaacag cacgtaccgg 660

gtggtcagcg tcctcaccgt cctgcaccag gactggctga atggcaagga gtacaagtgc 720

aaggtctcca acaaagccct cccagccccc atcgagaaaa ccatctccaa agccaaaggc 780

cagccccggg aaccacaggt gtacaccctg cccccatccc gggaggagat gaccaagaac 840

caggtcagcc tgacctgcct ggtcaaaggc ttctatccca gcgacatcgc cgtggagtgg 900

gagagcaatg gccagcccga gaacaactac aagaccaccc ctcccgtgct ggactccgac 960

ggctccttct tcctctacag caagctcacc gtggacaaga gcaggtggca gcagggcaac 1020

gtcttctcat gctccgtgat gcatgaggct ctgcacaacc actacaccca gaagagcctc 1080

tccctgtctc ccggcaaatg agatatcggg cccgtttaaa cgggggaggc ta 1132

<210> 22
<211> 749
<212> DNA
<213> Artificial Sequence

<220>
<223> DNA comprising DNA sequence coding for chG019 light chain

<400> 22
ccagcctccg gactctagag ccaccatggt gctgcagacc caggtgttca tcagcctgct 60

gctgtggatc agcggcgcct acggcgacat ccagatgacc cagagcccta gcctgctgag 120

cgccagcgtg ggcgatagag tgaccctgaa ctgcaaggcc agccagaaca tctacaagaa 180

cctggcctgg tatcagcaga agctgggcga gggccccaag ctgctgatct acgacgccaa 240

caccctgcag accggcatcc ccagcagatt ttctggcagc ggcagcggct ccgacttcac 300

cctgacaatc agcagcctgc agcccgagga cgtggccacc tacttttgcc agcagtacta 360

cagcggctgg gccttcggcg gcgtgaccaa cctggaactg aagagagccg tggccgctcc 420

ctccgtgttc atcttcccac ctagcgacga gcagctgaag tccggcacag cctctgtcgt 480

gtgcctgctg aacaacttct acccccgcga ggccaaggtg cagtggaagg tggacaatgc 540

cctgcagtct ggcaacagcc aggaaagcgt gaccgagcag gacagcaagg actccaccta 600

cagcctgagc agcaccctga ccctgagcaa ggccgactac gagaagcaca aggtgtacgc 660

ctgcgaagtg acccaccagg gcctgtctag ccccgtgacc aagagcttca accggggcga 720

gtgttgagtt taaacggggg aggctaact 749

<210> 23
<211> 233
<212> PRT
<213> Artificial Sequence

<220>
<223> amino acid sequence of chG019 light chain full-length

<400> 23

Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Leu Leu Ser
20 25 30

Ala Ser Val Gly Asp Arg Val Thr Leu Asn Cys Lys Ala Ser Gln Asn
35 40 45

Ile Tyr Lys Asn Leu Ala Trp Tyr Gln Gln Lys Leu Gly Glu Gly Pro
50 55 60

Lys Leu Leu Ile Tyr Asp Ala Asn Thr Leu Gln Thr Gly Ile Pro Ser
65 70 75 80

Arg Phe Ser Gly Ser Gly Ser Gly Ser Asp Phe Thr Leu Thr Ile Ser
85 90 95

Ser Leu Gln Pro Glu Asp Val Ala Thr Tyr Phe Cys Gln Gln Tyr Tyr
100 105 110

Ser Gly Trp Ala Phe Gly Gly Val Thr Asn Leu Glu Leu Lys Arg Ala
115 120 125

Val Ala Ala Pro Ser Val Phe Ile Phe Pro Pro Ser Asp Glu Gln Leu
130 135 140

Lys Ser Gly Thr Ala Ser Val Val Cys Leu Leu Asn Asn Phe Tyr Pro
145 150 155 160

Arg Glu Ala Lys Val Gln Trp Lys Val Asp Asn Ala Leu Gln Ser Gly
165 170 175

Asn Ser Gln Glu Ser Val Thr Glu Gln Asp Ser Lys Asp Ser Thr Tyr
180 185 190

Ser Leu Ser Ser Thr Leu Thr Leu Ser Lys Ala Asp Tyr Glu Lys His
195 200 205

Lys Val Tyr Ala Cys Glu Val Thr His Gln Gly Leu Ser Ser Pro Val
210 215 220

Thr Lys Ser Phe Asn Arg Gly Glu Cys
225 230

<210> 24
<211> 699
<212> DNA
<213> Artificial Sequence

<220>
<223> nucleotide sequence of chG019 light chain full-length

<400> 24
atggtgctgc agacccaggt gttcatcagc ctgctgctgt ggatcagcgg cgcctacggc 60

gacatccaga tgacccagag ccctagcctg ctgagcgcca gcgtgggcga tagagtgacc 120

ctgaactgca aggccagcca gaacatctac aagaacctgg cctggtatca gcagaagctg 180

ggcgagggcc ccaagctgct gatctacgac gccaacaccc tgcagaccgg catccccagc 240

agattttctg gcagcggcag cggctccgac ttcaccctga caatcagcag cctgcagccc 300

gaggacgtgg ccacctactt ttgccagcag tactacagcg gctgggcctt cggcggcgtg 360

accaacctgg aactgaagag agccgtggcc gctccctccg tgttcatctt cccacctagc 420

gacgagcagc tgaagtccgg cacagcctct gtcgtgtgcc tgctgaacaa cttctacccc 480

cgcgaggcca aggtgcagtg gaaggtggac aatgccctgc agtctggcaa cagccaggaa 540

agcgtgaccg agcaggacag caaggactcc acctacagcc tgagcagcac cctgaccctg 600

agcaaggccg actacgagaa gcacaaggtg tacgcctgcg aagtgaccca ccagggcctg 660

tctagccccg tgaccaagag cttcaaccgg ggcgagtgt 699

<210> 25
<211> 324
<212> DNA
<213> Artificial Sequence

<220>
<223> nucleotide sequence of chG019 light chain variable region

<400> 25
gacatccaga tgacccagag ccctagcctg ctgagcgcca gcgtgggcga tagagtgacc 60

ctgaactgca aggccagcca gaacatctac aagaacctgg cctggtatca gcagaagctg 120

ggcgagggcc ccaagctgct gatctacgac gccaacaccc tgcagaccgg catccccagc 180

agattttctg gcagcggcag cggctccgac ttcaccctga caatcagcag cctgcagccc 240

gaggacgtgg ccacctactt ttgccagcag tactacagcg gctgggcctt cggcggcgtg 300

accaacctgg aactgaagag agcc 324

<210> 26
<211> 471
<212> PRT
<213> Artificial Sequence

<220>
<223> amino acid sequence of chG019 heavy chain full-length

<400> 26

Met Lys His Leu Trp Phe Phe Leu Leu Leu Val Ala Ala Pro Arg Trp
1 5 10 15

```
Val Leu Ser Gln Val Gln Leu Gln Gln Ser Gly Ala Glu Leu Val Lys
            20                  25                  30

Pro Gly Ser Ser Val Lys Ile Ser Cys Lys Ala Ser Gly Tyr Thr Phe
        35                  40                  45

Thr Arg Asn Phe Met His Trp Ile Lys Gln Gln Pro Gly Asn Gly Leu
    50                  55                  60

Glu Trp Ile Gly Trp Ile Tyr Pro Gly Asp Gly Glu Thr Glu Tyr Asn
65                  70                  75                  80

Gln Lys Phe Asn Gly Lys Ala Thr Leu Thr Ala Asp Arg Ser Ser Ser
                85                  90                  95

Thr Ala Tyr Met Glu Leu Ser Arg Leu Thr Ser Glu Asp Ser Ala Val
            100                 105                 110

Tyr Phe Cys Ala Arg Gly Val Tyr Gly Gly Phe Ala Gly Gly Tyr Phe
        115                 120                 125

Asp Phe Trp Gly Gln Gly Val Met Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270
```

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 27
<211> 1413
<212> DNA
<213> Artificial Sequence

<220>
<223> nucleotide sequence of chG019 heavy chain full-length

<400> 27
atgaaacacc tgtggttctt cctcctgctg gtggcagctc ccagatgggt gctgagccag 60

gtgcagctgc agcagtctgg cgccgagctc gtgaagcctg gcagcagcgt gaagatcagc 120

tgcaaggcca gcggctacac cttcacccgg aacttcatgc actggatcaa gcagcagccc 180

ggcaacggcc tggaatggat cggctggatc tatcccggcg acggcgagac agagtacaac 240

cagaagttca acggcaaggc caccctgacc gccgacagaa gcagctccac cgcctacatg 300

gaactgagcc ggctgaccag cgaggacagc gccgtgtact tttgcgccag aggcgtgtac 360

ggcggcttcg ctggcggcta cttcgatttt tggggccagg gcgtgatggt caccgtcagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga actcctgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 28
<211> 122
<212> PRT
<213> Artificial Sequence

<220>
<223> amino acid sequence of chG019 heavy chain variable region

<400> 28

Gln Val Gln Leu Gln Gln Ser Gly Ala Glu Leu Val Lys Pro Gly Ser
1 5 10 15

Ser Val Lys Ile Ser Cys Lys Ala Ser Gly Tyr Thr Phe Thr Arg Asn

```
            20                  25                  30

Phe Met His Trp Ile Lys Gln Gln Pro Gly Asn Gly Leu Glu Trp Ile
        35                  40                  45

Gly Trp Ile Tyr Pro Gly Asp Gly Glu Thr Glu Tyr Asn Gln Lys Phe
    50                  55                  60

Asn Gly Lys Ala Thr Leu Thr Ala Asp Arg Ser Ser Ser Thr Ala Tyr
65                  70                  75                  80

Met Glu Leu Ser Arg Leu Thr Ser Glu Asp Ser Ala Val Tyr Phe Cys
                85                  90                  95

Ala Arg Gly Val Tyr Gly Gly Phe Ala Gly Gly Tyr Phe Asp Phe Trp
            100                 105                 110

Gly Gln Gly Val Met Val Thr Val Ser Ser
        115                 120
```

<210> 29
<211> 366
<212> DNA
<213> Artificial Sequence

<220>
<223> nucleotide sequence of chG019 heavy chain variable region

<400> 29

```
caggtgcagc tgcagcagtc tggcgccgag ctcgtgaagc ctggcagcag cgtgaagatc     60

agctgcaagg ccagcggcta caccttcacc cggaacttca tgcactggat caagcagcag    120

cccggcaacg gcctggaatg gatcggctgg atctatcccg gcgacggcga gacagagtac    180

aaccagaagt tcaacggcaa ggccaccctg accgccgaca gaagcagctc caccgcctac    240

atggaactga gccggctgac cagcgaggac agcgccgtgt acttttgcgc cagaggcgtg    300

tacggcggct tcgctggcgg ctacttcgat tttggggcc agggcgtgat ggtcaccgtc     360

agctca                                                               366
```

<210> 30
<211> 10
<212> PRT
<213> Artificial Sequence

<220>
<223> chG019 CDRH2

<400> 30

```
Trp Ile Tyr Pro Gly Asp Gly Glu Thr Glu
1               5                   10
```

<210> 31
<211> 233
<212> PRT
<213> Artificial Sequence

<220>
<223> amino acid sequence of hL02 light chain full-length

<400> 31

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

Ala Ser Val Gly Asp Arg Val Thr Ile Thr Cys Lys Ala Ser Gln Asn
35 40 45

Ile Tyr Lys Asn Leu Ala Trp Tyr Gln Gln Lys Pro Gly Lys Ala Pro
50 55 60

Lys Leu Leu Ile Tyr Asp Ala Asn Thr Leu Gln Thr Gly Val Pro Ser
65 70 75 80

Arg Phe Ser Gly Ser Gly Ser Gly Ser Asp Phe Thr Leu Thr Ile Ser
85 90 95

Ser Leu Gln Pro Glu Asp Phe Ala Thr Tyr Phe Cys Gln Gln Tyr Tyr
100 105 110

Ser Gly Trp Ala Phe Gly Gln Gly Thr Lys Val Glu Ile Lys Arg Thr
115 120 125

Val Ala Ala Pro Ser Val Phe Ile Phe Pro Pro Ser Asp Glu Gln Leu
130 135 140

Lys Ser Gly Thr Ala Ser Val Val Cys Leu Leu Asn Asn Phe Tyr Pro
145 150 155 160

Arg Glu Ala Lys Val Gln Trp Lys Val Asp Asn Ala Leu Gln Ser Gly
165 170 175

Asn Ser Gln Glu Ser Val Thr Glu Gln Asp Ser Lys Asp Ser Thr Tyr
180 185 190

Ser Leu Ser Ser Thr Leu Thr Leu Ser Lys Ala Asp Tyr Glu Lys His
195 200 205

Lys Val Tyr Ala Cys Glu Val Thr His Gln Gly Leu Ser Ser Pro Val
210 215 220

Thr Lys Ser Phe Asn Arg Gly Glu Cys
225 230

<210> 32
<211> 699
<212> DNA
<213> Artificial Sequence

<220>
<223> nucleotide sequence of hL02 light chain full-length

<400> 32
atggtgctgc agacccaggt gttcatctcc ctgctgctgt ggatctccgg cgcgtacggc 60

gacatccaga tgacccagag ccctagcagc ctgagcgcca gcgtgggcga cagagtgacc 120

atcacatgca aggccagcca gaacatctac aagaacctgg cctggtatca gcagaagccc 180

ggcaaggccc ccaagctgct gatctacgac gccaacaccc tgcagaccgg cgtgcccagc 240

agattttctg gcagcggcag cggctccgac ttcaccctga caatcagcag cctgcagccc 300

gaggacttcg ccacctactt ttgccagcag tactacagcg gctgggcctt cggccagggc 360

accaaggtgg aaatcaagcg tacggtggcc gccccctccg tgttcatctt cccccccctcc 420

gacgagcagc tgaagtccgg caccgcctcc gtggtgtgcc tgctgaataa cttctacccc 480

agagaggcca aggtgcagtg gaaggtggac aacgccctgc agtccgggaa ctcccaggag 540

agcgtgaccg agcaggacag caaggacagc acctacagcc tgagcagcac cctgaccctg 600

agcaaagccg actacgagaa gcacaaggtg tacgcctgcg aggtgaccca ccagggcctg 660

agctcccccg tcaccaagag cttcaacagg ggggagtgt 699

<210> 33
<211> 108
<212> PRT
<213> Artificial Sequence

<220>
<223> amino acid sequence of hL02 light chain variable region

<400> 33

Asp Ile Gln Met Thr Gln Ser Pro Ser Ser Leu Ser Ala Ser Val Gly
1 5 10 15

Asp Arg Val Thr Ile Thr Cys Lys Ala Ser Gln Asn Ile Tyr Lys Asn
20 25 30

Leu Ala Trp Tyr Gln Gln Lys Pro Gly Lys Ala Pro Lys Leu Leu Ile
35 40 45

Tyr Asp Ala Asn Thr Leu Gln Thr Gly Val Pro Ser Arg Phe Ser Gly
    50                  55                  60

Ser Gly Ser Gly Ser Asp Phe Thr Leu Thr Ile Ser Ser Leu Gln Pro
65                  70                  75                  80

Glu Asp Phe Ala Thr Tyr Phe Cys Gln Gln Tyr Tyr Ser Gly Trp Ala
                85                  90                  95

Phe Gly Gln Gly Thr Lys Val Glu Ile Lys Arg Thr
            100                 105

<210> 34
<211> 324
<212> DNA
<213> Artificial Sequence

<220>
<223> nucleotide sequence of hL02 light chain variable region

<400> 34
gacatccaga tgacccagag ccctagcagc ctgagcgcca gcgtgggcga cagagtgacc 60

atcacatgca aggccagcca gaacatctac aagaacctgg cctggtatca gcagaagccc 120

ggcaaggccc ccaagctgct gatctacgac gccaacaccc tgcagaccgg cgtgcccagc 180

agattttctg gcagcggcag cggctccgac ttcaccctga caatcagcag cctgcagccc 240

gaggacttcg ccacctactt ttgccagcag tactacagcg gctgggcctt cggccagggc 300

accaaggtgg aaatcaagcg tacg 324

<210> 35
<211> 233
<212> PRT
<213> Artificial Sequence

<220>
<223> amino acid sequence of hL03 light chain full-length

<400> 35

Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
            20                  25                  30

Ala Ser Val Gly Asp Arg Val Thr Ile Thr Cys Lys Ala Ser Gln Asn
        35                  40                  45

Ile Tyr Lys Asn Leu Ala Trp Tyr Gln Gln Lys Leu Gly Glu Gly Pro

50 55 60

Lys Leu Leu Ile Tyr Asp Ala Asn Thr Leu Gln Thr Gly Val Pro Ser
65 70 75 80

Arg Phe Ser Gly Ser Gly Ser Gly Thr Asp Phe Thr Leu Thr Ile Ser
85 90 95

Ser Leu Gln Pro Glu Asp Phe Ala Thr Tyr Tyr Cys Gln Gln Tyr Tyr
100 105 110

Ser Gly Trp Ala Phe Gly Gln Gly Thr Lys Val Glu Ile Lys Arg Thr
115 120 125

Val Ala Ala Pro Ser Val Phe Ile Phe Pro Pro Ser Asp Glu Gln Leu
130 135 140

Lys Ser Gly Thr Ala Ser Val Val Cys Leu Leu Asn Asn Phe Tyr Pro
145 150 155 160

Arg Glu Ala Lys Val Gln Trp Lys Val Asp Asn Ala Leu Gln Ser Gly
165 170 175

Asn Ser Gln Glu Ser Val Thr Glu Gln Asp Ser Lys Asp Ser Thr Tyr
180 185 190

Ser Leu Ser Ser Thr Leu Thr Leu Ser Lys Ala Asp Tyr Glu Lys His
195 200 205

Lys Val Tyr Ala Cys Glu Val Thr His Gln Gly Leu Ser Ser Pro Val
210 215 220

Thr Lys Ser Phe Asn Arg Gly Glu Cys
225 230

<210> 36
<211> 699
<212> DNA
<213> Artificial Sequence

<220>
<223> nucleotide sequence of hL03 light chain full-length

<400> 36
atggtgctgc agacccaggt gttcatctcc ctgctgctgt ggatctccgg cgcgtacggc 60

gacatccaga tgacccagag ccctagcagc ctgagcgcca gcgtgggcga cagagtgacc 120

atcacatgca aggccagcca gaacatctac aagaacctgg cctggtatca gcagaagctg 180

ggcgagggcc ccaagctgct gatctacgac gccaacaccc tgcagaccgg cgtgcccagc 240

agattttctg gcagcggctc cggcaccgac ttcaccctga caatcagcag cctgcagccc 300

gaggacttcg ccacctacta ctgccagcag tactacagcg gctgggcctt tggccagggc 360

accaaggtgg aaatcaagcg tacggtggcc gccccctccg tgttcatctt cccccctcc 420

gacgagcagc tgaagtccgg caccgcctcc gtggtgtgcc tgctgaataa cttctacccc 480

agagaggcca aggtgcagtg gaaggtggac aacgccctgc agtccgggaa ctcccaggag 540

agcgtgaccg agcaggacag caaggacagc acctacagcc tgagcagcac cctgaccctg 600

agcaaagccg actacgagaa gcacaaggtg tacgcctgcg aggtgaccca ccagggcctg 660

agctcccccg tcaccaagag cttcaacagg ggggagtgt 699

<210> 37
<211> 108
<212> PRT
<213> Artificial Sequence

<220>
<223> amino acid sequence of hL03 light chain variable region

<400> 37

Asp Ile Gln Met Thr Gln Ser Pro Ser Ser Leu Ser Ala Ser Val Gly
1 5 10 15

Asp Arg Val Thr Ile Thr Cys Lys Ala Ser Gln Asn Ile Tyr Lys Asn
20 25 30

Leu Ala Trp Tyr Gln Gln Lys Leu Gly Glu Gly Pro Lys Leu Leu Ile
35 40 45

Tyr Asp Ala Asn Thr Leu Gln Thr Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Tyr Ser Gly Trp Ala
85 90 95

Phe Gly Gln Gly Thr Lys Val Glu Ile Lys Arg Thr
100 105

<210> 38
<211> 324
<212> DNA
<213> Artificial Sequence

<220>
<223> nucleotide sequence of hL03 light chain variable region

<400> 38
gacatccaga tgacccagag ccctagcagc ctgagcgcca gcgtgggcga cagagtgacc 60

atcacatgca aggccagcca gaacatctac aagaacctgg cctggtatca gcagaagctg 120

ggcgagggcc ccaagctgct gatctacgac gccaacaccc tgcagaccgg cgtgcccagc 180

agattttctg gcagcggctc cggcaccgac ttcaccctga caatcagcag cctgcagccc 240

gaggacttcg ccacctacta ctgccagcag tactacagcg gctgggcctt tggccagggc 300

accaaggtgg aaatcaagcg tacg 324

<210> 39
<211> 471
<212> PRT
<213> Artificial Sequence

<220>
<223> amino acid sequence of hH01 heavy chain full-length

<400> 39

Met Lys His Leu Trp Phe Phe Leu Leu Leu Val Ala Ala Pro Arg Trp
1 5 10 15

Val Leu Ser Glu Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Arg Asn Phe Met His Trp Val Arg Gln Ala Pro Gly Gln Gly Leu
50 55 60

Glu Trp Met Gly Trp Ile Tyr Pro Gly Asp Gly Glu Thr Glu Tyr Ala
65 70 75 80

Gln Lys Phe Gln Gly Arg Val Thr Ile Thr Ala Asp Thr Ser Thr Ser
85 90 95

Thr Ala Tyr Met Glu Leu Ser Ser Leu Arg Ser Glu Asp Thr Ala Val
100 105 110

Tyr Tyr Cys Ala Arg Gly Val Tyr Gly Gly Phe Ala Gly Gly Tyr Phe
115 120 125

Asp Phe Trp Gly Gln Gly Thr Leu Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Arg Val Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys

405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 40
<211> 1413
<212> DNA
<213> Artificial Sequence

<220>
<223> nucleotide sequence of hH01 heavy chain full-length

<400> 40
atgaaacacc tgtggttctt cctcctgctg gtggcagctc ccagatgggt gctgagcgaa 60

gtgcagctgg tgcagtctgg cgccgaagtg aagaaaccag gcgccagcgt gaaggtgtcc 120

tgcaaggcca gcggctacac ctttacccgg aacttcatgc actgggtgcg ccaggctcca 180

ggccagggac tggaatggat gggctggatc tatcccggcg acggcgagac agagtacgcc 240

cagaaattcc agggcagagt gaccatcacc gccgacacca gcacctccac cgcctacatg 300

gaactgagca gcctgcggag cgaggacacc gccgtgtact attgtgccag aggcgtgtac 360

ggcggcttcg ctggcggcta cttcgatttt tggggccagg gcaccctcgt gaccgtcagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga actcctgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 41
<211> 122
<212> PRT
<213> Artificial Sequence

<220>
<223> amino acid sequence of hH01 heavy chain variable region

<400> 41

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Asn
            20                  25                  30

Phe Met His Trp Val Arg Gln Ala Pro Gly Gln Gly Leu Glu Trp Met
        35                  40                  45

Gly Trp Ile Tyr Pro Gly Asp Gly Glu Thr Glu Tyr Ala Gln Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Val Tyr Gly Gly Phe Ala Gly Gly Tyr Phe Asp Phe Trp
            100                 105                 110

Gly Gln Gly Thr Leu Val Thr Val Ser Ser
        115                 120

<210> 42
<211> 366
<212> DNA
<213> Artificial Sequence

<220>
<223> nucleotide sequence of hH01 heavy chain variable region

<400> 42
gaagtgcagc tggtgcagtc tggcgccgaa gtgaagaaac caggcgccag cgtgaaggtg 60

tcctgcaagg ccagcggcta cacctttacc cggaacttca tgcactgggt gcgccaggct 120

ccaggccagg gactggaatg gatgggctgg atctatcccg gcgacggcga gacagagtac 180

gcccagaaat tccagggcag agtgaccatc accgccgaca ccagcacctc caccgcctac 240

atggaactga gcagcctgcg gagcgaggac accgccgtgt actattgtgc cagaggcgtg 300

tacggcggct tcgctggcgg ctacttcgat ttttggggcc agggcaccct cgtgaccgtc 360

agctca 366

<210> 43
<211> 471
<212> PRT
<213> Artificial Sequence

<220>
<223> amino acid sequence of hH02 heavy chain full-length

<400> 43

Met Lys His Leu Trp Phe Phe Leu Leu Leu Val Ala Ala Pro Arg Trp
1 5 10 15

Val Leu Ser Glu Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Arg Asn Phe Met His Trp Val Arg Gln Ala Pro Gly Gln Gly Leu
50 55 60

Glu Trp Met Gly Trp Ile Tyr Pro Gly Asp Gly Glu Thr Glu Tyr Asn
65 70 75 80

Gln Lys Phe Gln Gly Arg Val Thr Ile Thr Ala Asp Arg Ser Thr Ser
85 90 95

Thr Ala Tyr Met Glu Leu Ser Ser Leu Arg Ser Glu Asp Thr Ala Val
100 105 110

Tyr Phe Cys Ala Arg Gly Val Tyr Gly Gly Phe Ala Gly Gly Tyr Phe
115 120 125

Asp Phe Trp Gly Gln Gly Thr Leu Val Thr Val Ser Ser Ala Ser Thr
130 135 140

```
Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Glu Glu Met Thr Lys
    370                 375                 380

Asn Gln Val Ser Leu Thr Cys Leu Val Lys Gly Phe Tyr Pro Ser Asp
385                 390                 395                 400
```

```
Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470
```

<210> 44
<211> 1413
<212> DNA
<213> Artificial Sequence

<220>
<223> nucleotide sequence of hH02 heavy chain full-length

<400> 44

```
atgaaacacc tgtggttctt cctcctgctg gtggcagctc ccagatgggt gctgagcgaa      60
gtgcagctgg tgcagtctgg cgccgaagtg aagaaaccag gcgccagcgt gaaggtgtcc     120
tgcaaggcca gcggctacac ctttacccgg aacttcatgc actgggtgcg ccaggctcca     180
ggccagggac tggaatggat gggctggatc tatcccggcg acggcgagac agagtacaac     240
cagaaattcc agggcagagt gaccatcacc gccgacagaa gcaccagcac cgcctacatg     300
gaactgagca gcctgcggag cgaggatacc gccgtgtact tctgtgccag aggcgtgtac     360
ggcggcttcg ctggcggcta cttcgatttt tggggccagg gcaccctcgt gaccgtcagc     420
tcagcctcca ccaagggccc aagcgtcttc cccctggcac cctcctccaa gagcacctct     480
ggcggcacag ccgccctggg ctgcctggtc aaggactact tccccgaacc cgtgaccgtg     540
agctggaact caggcgccct gaccagcggc gtgcacacct tccccgctgt cctgcagtcc     600
tcaggactct actccctcag cagcgtggtg accgtgccct ccagcagctt gggcacccag     660
acctacatct gcaacgtgaa tcacaagccc agcaacacca aggtggacaa gagagttgag     720
cccaaatctt gtgacaaaac tcacacatgc ccaccctgcc cagcacctga actcctgggg     780
ggaccctcag tcttcctctt ccccccaaaa cccaaggaca ccctcatgat ctcccggacc     840
cctgaggtca catgcgtggt ggtggacgtg agccacgaag accctgaggt caagttcaac     900
tggtacgtgg acggcgtgga ggtgcataat gccaagacaa agccccggga ggagcagtac     960
aacagcacgt accgggtggt cagcgtcctc accgtcctgc accaggactg gctgaatggc    1020
```

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 45
<211> 122
<212> PRT
<213> Artificial Sequence

<220>
<223> amino acid sequence of hH02 heavy chain variable region

<400> 45

Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Arg Asn
20 25 30

Phe Met His Trp Val Arg Gln Ala Pro Gly Gln Gly Leu Glu Trp Met
35 40 45

Gly Trp Ile Tyr Pro Gly Asp Gly Glu Thr Glu Tyr Asn Gln Lys Phe
50 55 60

Gln Gly Arg Val Thr Ile Thr Ala Asp Arg Ser Thr Ser Thr Ala Tyr
65 70 75 80

Met Glu Leu Ser Ser Leu Arg Ser Glu Asp Thr Ala Val Tyr Phe Cys
85 90 95

Ala Arg Gly Val Tyr Gly Gly Phe Ala Gly Gly Tyr Phe Asp Phe Trp
100 105 110

Gly Gln Gly Thr Leu Val Thr Val Ser Ser
115 120

<210> 46
<211> 366
<212> DNA
<213> Artificial Sequence

<220>
<223> nucleotide sequence of hH02 heavy chain variable region

<400> 46
gaagtgcagc tggtgcagtc tggcgccgaa gtgaagaaac caggcgccag cgtgaaggtg 60

tcctgcaagg ccagcggcta cacctttacc cggaacttca tgcactgggt gcgccaggct 120

ccaggccagg gactggaatg gatgggctgg atctatcccg gcgacggcga gacagagtac 180

aaccagaaat tccagggcag agtgaccatc accgccgaca gaagcaccag caccgcctac 240

atggaactga gcagcctgcg gagcgaggat accgccgtgt acttctgtgc cagaggcgtg 300

tacggcggct tcgctggcgg ctacttcgat tttggggcc agggcaccct cgtgaccgtc 360

agctca 366

<210> 47
<211> 471
<212> PRT
<213> Artificial Sequence

<220>
<223> amino acid sequence of hH04 heavy chain full-length

<400> 47

Met Lys His Leu Trp Phe Phe Leu Leu Leu Val Ala Ala Pro Arg Trp
1 5 10 15

Val Leu Ser Gln Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Arg Asn Phe Met His Trp Ile Arg Gln Ala Pro Gly Gln Gly Leu
50 55 60

Glu Trp Met Gly Trp Ile Tyr Pro Gly Asp Gly Glu Thr Glu Tyr Ala
65 70 75 80

Gln Lys Phe Gln Gly Arg Val Thr Leu Thr Ala Asp Arg Ser Thr Ser
85 90 95

Thr Ala Tyr Met Glu Leu Ser Ser Leu Arg Ser Glu Asp Thr Ala Val
100 105 110

Tyr Tyr Cys Ala Arg Gly Val Tyr Gly Gly Phe Ala Gly Gly Tyr Phe
115 120 125

Asp Phe Trp Gly Gln Gly Thr Leu Val Thr Val Ser Ser Ala Ser Thr
130 135 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Glu Glu Met Thr Lys
    370                 375                 380

Asn Gln Val Ser Leu Thr Cys Leu Val Lys Gly Phe Tyr Pro Ser Asp

385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 48
<211> 1413
<212> DNA
<213> Artificial Sequence

<220>
<223> nucleotide sequence of hH04 heavy chain full-length

<400> 48
atgaaacacc tgtggttctt cctcctgctg gtggcagctc ccagatgggt gctgagccag 60

gtgcagctgg tgcagtctgg cgccgaagtg aagaaaccag gcgccagcgt gaaggtgtcc 120

tgcaaggcca gcggctacac ctttacccgg aacttcatgc actggatccg gcaggcccct 180

ggacagggcc tggaatggat gggctggatc tatcccggcg acggcgagac agagtacgcc 240

cagaaattcc agggcagagt gaccctgacc gccgacagaa gcaccagcac cgcctacatg 300

gaactgagca gcctgcggag cgaggacacc gccgtgtact attgtgccag aggcgtgtac 360

ggcggcttcg ctggcggcta cttcgatttt tggggccagg gcaccctcgt gaccgtcagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga actcctgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 49
<211> 122
<212> PRT
<213> Artificial Sequence

<220>
<223> amino acid sequence of hH04 heavy chain variable region

<400> 49

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Arg Asn
20 25 30

Phe Met His Trp Ile Arg Gln Ala Pro Gly Gln Gly Leu Glu Trp Met
35 40 45

Gly Trp Ile Tyr Pro Gly Asp Gly Glu Thr Glu Tyr Ala Gln Lys Phe
50 55 60

Gln Gly Arg Val Thr Leu Thr Ala Asp Arg Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Gly Val Tyr Gly Gly Phe Ala Gly Gly Tyr Phe Asp Phe Trp
100 105 110

Gly Gln Gly Thr Leu Val Thr Val Ser Ser
115 120

<210> 50
<211> 366
<212> DNA

<213>  Artificial Sequence

<220>
<223>  nucleotide sequence of hH04 heavy chain variable region

<400>  50
caggtgcagc tggtgcagtc tggcgccgaa gtgaagaaac caggcgccag cgtgaaggtg 60

tcctgcaagg ccagcggcta cacctttacc cggaacttca tgcactggat ccggcaggcc 120

cctggacagg gcctggaatg gatgggctgg atctatcccg gcgacggcga gacagagtac 180

gcccagaaat tccagggcag agtgaccctg accgccgaca gaagcaccag caccgcctac 240

atggaactga gcagcctgcg gagcgaggac accgccgtgt actattgtgc cagaggcgtg 300

tacggcggct tcgctggcgg ctacttcgat ttttggggcc agggcaccct cgtgaccgtc 360

agctca 366

<210>  51
<211>  235
<212>  PRT
<213>  Artificial Sequence

<220>
<223>  amino acid sequence of NOV0712 light chain full-length

<400>  51

Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
            20                  25                  30

Ala Ser Val Gly Asp Arg Val Thr Ile Thr Cys Arg Ala Ser Gln Ser
        35                  40                  45

Ile Ser Ser Tyr Leu Asn Trp Tyr Gln Gln Lys Pro Gly Lys Ala Pro
    50                  55                  60

Lys Leu Leu Ile Tyr Ala Val Ser Thr Leu Gln Ser Gly Val Pro Ser
65                  70                  75                  80

Arg Phe Ser Gly Ser Gly Ser Gly Thr Asp Phe Thr Leu Thr Ile Ser
                85                  90                  95

Ser Leu Gln Pro Glu Asp Phe Ala Thr Tyr Tyr Cys Gln Gln Ser Gly
            100                 105                 110

Thr Phe Pro Pro Thr Thr Phe Gly Gln Gly Thr Lys Val Glu Ile Lys
        115                 120                 125

```
Arg Thr Val Ala Ala Pro Ser Val Phe Ile Phe Pro Pro Ser Asp Glu
    130                 135                 140

Gln Leu Lys Ser Gly Thr Ala Ser Val Val Cys Leu Leu Asn Asn Phe
145                 150                 155                 160

Tyr Pro Arg Glu Ala Lys Val Gln Trp Lys Val Asp Asn Ala Leu Gln
                165                 170                 175

Ser Gly Asn Ser Gln Glu Ser Val Thr Glu Gln Asp Ser Lys Asp Ser
            180                 185                 190

Thr Tyr Ser Leu Ser Ser Thr Leu Thr Leu Ser Lys Ala Asp Tyr Glu
        195                 200                 205

Lys His Lys Val Tyr Ala Cys Glu Val Thr His Gln Gly Leu Ser Ser
    210                 215                 220

Pro Val Thr Lys Ser Phe Asn Arg Gly Glu Cys
225                 230                 235
```

```
<210>  52
<211>  705
<212>  DNA
<213>  Artificial Sequence

<220>
<223>  nucleotide sequence coding for amino acid sequence of sequence
       number 51

<400>  52
atggtgctgc agacccaggt gttcatctcc ctgctgctgt ggatctccgg cgcgtacggc       60

gacatccaga tgacccagag ccctagcagc ctgagcgcca gcgtgggcga cagagtgacc      120

atcacctgta gagccagcca gagcatcagc agctacctga actggtatca gcagaagccc      180

ggcaaggccc ccaaactgct gatctacgcc gtgtccacac tgcagagcgg cgtgcccagc      240

agattttctg gcagcggctc cggcaccgac ttcaccctga caatcagcag cctgcagccc      300

gaggacttcg ccacctacta ctgtcagcag tccggcacct tcccccccac cacatttggc      360

cagggcacca aggtggaaat caagcgtacg gtggccgccc cctccgtgtt catcttcccc      420

ccctccgacg agcagctgaa gtccggcacc gcctccgtgg tgtgcctgct gaataacttc      480

taccccagag aggccaaggt gcagtggaag gtggacaacg ccctgcagtc cgggaactcc      540

caggagagcg tgaccgagca ggacagcaag gacagcacct acagcctgag cagcaccctg      600

accctgagca aagccgacta cgagaagcac aaggtgtacg cctgcgaggt gacccaccag      660

ggcctgagct cccccgtcac caagagcttc aacagggggg agtgt                      705
```

<210> 53
<211> 467
<212> PRT
<213> Artificial Sequence

<220>
<223> amino acid sequence of NOV0712 heavy chain full-length

<400> 53

Met Lys His Leu Trp Phe Phe Leu Leu Leu Val Ala Ala Pro Arg Trp
1 5 10 15

Val Leu Ser Gln Val Gln Leu Leu Glu Ser Gly Gly Gly Leu Val Gln
20 25 30

Pro Gly Gly Ser Leu Arg Leu Ser Cys Ala Ala Ser Gly Phe Thr Phe
35 40 45

Ser Ser His Gly Met His Trp Val Arg Gln Ala Pro Gly Lys Gly Leu
50 55 60

Glu Trp Val Ser Val Ile Ser Gly Ser Gly Ser Asn Thr Gly Tyr Ala
65 70 75 80

Asp Ser Val Lys Gly Arg Phe Thr Ile Ser Arg Asp Asn Ser Lys Asn
85 90 95

Thr Leu Tyr Leu Gln Met Asn Ser Leu Arg Ala Glu Asp Thr Ala Val
100 105 110

Tyr Tyr Cys Ala Arg Gln Trp Gly Ser Tyr Ala Phe Asp Ser Trp Gly
115 120 125

Gln Gly Thr Leu Val Thr Val Ser Ser Ala Ser Thr Lys Gly Pro Ser
130 135 140

Val Phe Pro Leu Ala Pro Ser Ser Lys Ser Thr Ser Gly Gly Thr Ala
145 150 155 160

Ala Leu Gly Cys Leu Val Lys Asp Tyr Phe Pro Glu Pro Val Thr Val
165 170 175

Ser Trp Asn Ser Gly Ala Leu Thr Ser Gly Val His Thr Phe Pro Ala
180 185 190

Val Leu Gln Ser Ser Gly Leu Tyr Ser Leu Ser Ser Val Val Thr Val
195 200 205

Pro Ser Ser Ser Leu Gly Thr Gln Thr Tyr Ile Cys Asn Val Asn His

210 215 220

Lys Pro Ser Asn Thr Lys Val Asp Lys Arg Val Glu Pro Lys Ser Cys
225 230 235 240

Asp Lys Thr His Thr Cys Pro Pro Cys Pro Ala Pro Glu Leu Leu Gly
245 250 255

Gly Pro Ser Val Phe Leu Phe Pro Pro Lys Pro Lys Asp Thr Leu Met
260 265 270

Ile Ser Arg Thr Pro Glu Val Thr Cys Val Val Val Asp Val Ser His
275 280 285

Glu Asp Pro Glu Val Lys Phe Asn Trp Tyr Val Asp Gly Val Glu Val
290 295 300

His Asn Ala Lys Thr Lys Pro Arg Glu Glu Gln Tyr Asn Ser Thr Tyr
305 310 315 320

Arg Val Val Ser Val Leu Thr Val Leu His Gln Asp Trp Leu Asn Gly
325 330 335

Lys Glu Tyr Lys Cys Lys Val Ser Asn Lys Ala Leu Pro Ala Pro Ile
340 345 350

Glu Lys Thr Ile Ser Lys Ala Lys Gly Gln Pro Arg Glu Pro Gln Val
355 360 365

Tyr Thr Leu Pro Pro Ser Arg Glu Glu Met Thr Lys Asn Gln Val Ser
370 375 380

Leu Thr Cys Leu Val Lys Gly Phe Tyr Pro Ser Asp Ile Ala Val Glu
385 390 395 400

Trp Glu Ser Asn Gly Gln Pro Glu Asn Asn Tyr Lys Thr Thr Pro Pro
405 410 415

Val Leu Asp Ser Asp Gly Ser Phe Phe Leu Tyr Ser Lys Leu Thr Val
420 425 430

Asp Lys Ser Arg Trp Gln Gln Gly Asn Val Phe Ser Cys Ser Val Met
435 440 445

His Glu Ala Leu His Asn His Tyr Thr Gln Lys Ser Leu Ser Leu Ser
450 455 460

Pro Gly Lys
465

<210> 54
<211> 1401
<212> DNA
<213> Artificial Sequence

<220>
<223> nucleotide sequence coding for amino acid sequence of sequence number 53

<400> 54
atgaaacacc tgtggttctt cctcctgctg gtggcagctc ccagatgggt gctgagccag 60

gtgcagctgc tggaatctgg cggaggactg gtgcagcctg gcggctctct gagactgtct 120

tgtgccgcca gcggcttcac cttcagcagc cacggaatgc actgggtgcg ccaggcccct 180

ggaaagggac tggaatgggt gtccgtgatc agcggcagcg gctccaatac cggctacgcc 240

gatagcgtga agggccggtt caccatcagc cgggacaaca gcaagaacac cctgtacctg 300

cagatgaaca gcctgcgggc cgaggacacc gccgtgtact attgtgccag acagtggggc 360

agctacgcct tcgattcttg gggccagggc accctcgtga ccgtcagctc agcctccacc 420

aagggcccaa gcgtcttccc cctggcaccc tcctccaaga gcacctctgg cggcacagcc 480

gccctgggct gcctggtcaa ggactacttc cccgaacccg tgaccgtgag ctggaactca 540

ggcgccctga ccagcggcgt gcacaccttc cccgctgtcc tgcagtcctc aggactctac 600

tccctcagca gcgtggtgac cgtgccctcc agcagcttgg gcacccagac ctacatctgc 660

aacgtgaatc acaagcccag caacaccaag gtggacaaga gagttgagcc caaatcttgt 720

gacaaaactc acacatgccc accctgccca gcacctgaac tcctgggggg accctcagtc 780

ttcctcttcc ccccaaaacc caaggacacc ctcatgatct cccggacccc tgaggtcaca 840

tgcgtggtgg tggacgtgag ccacgaagac cctgaggtca agttcaactg gtacgtggac 900

ggcgtggagg tgcataatgc caagacaaag ccccgggagg agcagtacaa cagcacgtac 960

cgggtggtca gcgtcctcac cgtcctgcac caggactggc tgaatggcaa ggagtacaag 1020

tgcaaggtct ccaacaaagc cctcccagcc cccatcgaga aaaccatctc caaagccaaa 1080

ggccagcccc gggaaccaca ggtgtacacc ctgcccccat cccgggagga gatgaccaag 1140

aaccaggtca gcctgacctg cctggtcaaa ggcttctatc ccagcgacat cgccgtggag 1200

tgggagagca atggccagcc cgagaacaac tacaagacca cccctcccgt gctggactcc 1260

gacggctcct tcttcctcta cagcaagctc accgtggaca agagcaggtg gcagcagggc 1320

aacgtcttct catgctccgt gatgcatgag gctctgcaca accactacac ccagaagagc 1380

ctctccctgt ctcccggcaa a 1401

<210> 55
<211> 122
<212> PRT
<213> Artificial Sequence

<220>
<223> amino acid sequence of hH11 heavy chain variable region

<400> 55

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Asn
            20                  25                  30

Phe Met His Trp Val Arg Gln Ala Pro Gly Gln Gly Leu Glu Trp Met
        35                  40                  45

Gly Trp Ile Ala Pro Gly Asp Gly Glu Thr Glu Tyr Ala Gln Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Val Tyr Gly Gly Phe Ala Gly Gly Tyr Phe Asp Phe Trp
            100                 105                 110

Gly Gln Gly Thr Leu Val Thr Val Ser Ser
        115                 120

<210> 56
<211> 366
<212> DNA
<213> Artificial Sequence

<220>
<223> nucleotide sequence of hH11 heavy chain variable region

<400> 56
gaagtgcagc tggttcagtc tggcgccgaa gtgaaaaagc ctggcgcctc tgtgaaggtg 60

tcctgcaagg cctctggcta cacattcacc cggaacttca tgcactgggt ccgacaggct 120

ccaggacagg gacttgaatg gatgggatgg attgctcccg gcgacggcga gacagagtac 180

gcccagaaat tccagggcag agtgaccatc accgccgaca cctctacaag caccgcctac 240

atggaactga gcagcctgag aagcgaggac accgccgtgt actattgtgc cagaggcgtg 300

tacggcggat tcgctggcgg ctactttgat ttttggggcc agggcaccct ggtcaccgtg 360

agctca 366

<210> 57
<211> 10
<212> PRT
<213> Artificial Sequence

<220>
<223> hH11 CDRH1

<400> 57

Gly Tyr Thr Phe Thr Arg Asn Phe Met His
1               5                   10

<210> 58
<211> 10
<212> PRT
<213> Artificial Sequence

<220>
<223> hH11 CDRH2

<400> 58

Trp Ile Ala Pro Gly Asp Gly Glu Thr Glu
1               5                   10

<210> 59
<211> 13
<212> PRT
<213> Artificial Sequence

<220>
<223> hH11 CDRH3

<400> 59

Gly Val Tyr Gly Gly Phe Ala Gly Gly Tyr Phe Asp Phe
1               5                   10

<210> 60
<211> 122
<212> PRT
<213> Artificial Sequence

<220>
<223> amino acid sequence of hH31 heavy chain variable region

<400> 60

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Asn
            20                  25                  30

Phe Met His Trp Val Arg Gln Ala Pro Gly Gln Gly Leu Glu Trp Met
35 40 45

Gly Trp Ile Tyr Pro Gly Asp Gly Glu Thr Glu Tyr Ala Ser Lys Phe
50 55 60

Gln Gly Arg Val Thr Ile Thr Ala Asp Thr Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Gly Val Tyr Gly Gly Ala Ala Gly Gly Tyr Phe Asp Phe Trp
100 105 110

Gly Gln Gly Thr Leu Val Thr Val Ser Ser
115 120

<210> 61
<211> 366
<212> DNA
<213> Artificial Sequence

<220>
<223> nucleotide sequence of hH31 heavy chain variable region

<400> 61
gaagtgcagc tggttcagtc tggcgccgaa gtgaaaaagc ctggcgcctc tgtgaaggtg 60

tcctgcaagg cctctggcta cacattcacc cggaacttca tgcactgggt ccgacaggct 120

ccaggacagg gacttgaatg gatgggctgg atctatcccg gcgacggcga gacagagtac 180

gccagcaaat ttcagggcag agtgaccatc accgccgaca cctctacaag caccgcctac 240

atggaactga gcagcctgag aagcgaggac accgccgtgt actattgtgc cagaggcgtt 300

tacggcggag ccgctggcgg ctactttgat ttttggggcc agggcaccct ggtcaccgtg 360

agctca 366

<210> 62
<211> 10
<212> PRT
<213> Artificial Sequence

<220>
<223> hH31 CDRH1

<400> 62

Gly Tyr Thr Phe Thr Arg Asn Phe Met His
1 5 10

<210> 63

<211> 10
<212> PRT
<213> Artificial Sequence

<220>
<223> hH31 CDRH2

<400> 63

Trp Ile Tyr Pro Gly Asp Gly Glu Thr Glu
1 5 10

<210> 64
<211> 13
<212> PRT
<213> Artificial Sequence

<220>
<223> hH31 CDRH3

<400> 64

Gly Val Tyr Gly Gly Ala Ala Gly Gly Tyr Phe Asp Phe
1 5 10

<210> 65
<211> 471
<212> PRT
<213> Artificial Sequence

<220>
<223> amino acid sequence of hH01A heavy chain full-length

<400> 65

Met Lys His Leu Trp Phe Phe Leu Leu Leu Val Ala Ala Pro Arg Trp
1 5 10 15

Val Leu Ser Glu Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Arg Asn Phe Met His Trp Val Arg Gln Ala Pro Gly Gln Gly Leu
50 55 60

Glu Trp Met Gly Trp Ile Tyr Pro Gly Asp Gly Glu Thr Glu Tyr Ala
65 70 75 80

Gln Lys Phe Gln Gly Arg Val Thr Ile Thr Ala Asp Thr Ser Thr Ser
85 90 95

Thr Ala Tyr Met Glu Leu Ser Ser Leu Arg Ser Glu Asp Thr Ala Val
100 105 110

Tyr Tyr Cys Ala Arg Gly Val Tyr Gly Gly Phe Ala Gly Gly Tyr Phe
115 120 125

Asp Phe Trp Gly Gln Gly Thr Leu Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Arg Val Glu
225 230 235 240

Pro Lys Ser Cys Asp Lys Thr His Thr Cys Pro Pro Cys Pro Ala Pro
245 250 255

Glu Ala Ala Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg

355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 66
<211> 1413
<212> DNA
<213> Artificial Sequence

<220>
<223> nucleotide sequence of hH01A heavy chain full-length

<400> 66
atgaaacacc tgtggttctt cctcctgctg gtggcagctc ccagatgggt gctgagcgaa 60

gtgcagctgg ttcagtctgg cgccgaagtg aaaaagcctg gcgcctctgt gaaggtgtcc 120

tgcaaggcct ctggctacac attcacccgg aacttcatgc actgggtccg acaggctcca 180

ggacagggac ttgaatggat gggctggatc tatcccggcg acggcgagac agagtacgcc 240

cagaaattcc agggcagagt gaccatcacc gccgacacct ctacaagcac cgcctacatg 300

gaactgagca gcctgagaag cgaggacacc gccgtgtact attgtgccag aggcgtgtac 360

ggcggattcg ctggcggcta ctttgatttt tggggccagg gcaccctggt caccgtgagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

```
cccaaatctt gtgacaaaac tcacacatgc ccaccctgcc cagcacctga agccgcgggg      780

ggaccctcag tcttcctctt ccccccaaaa cccaaggaca ccctcatgat ctcccggacc      840

cctgaggtca catgcgtggt ggtggacgtg agccacgaag accctgaggt caagttcaac      900

tggtacgtgg acggcgtgga ggtgcataat gccaagacaa agccccggga ggagcagtac      960

aacagcacgt accgggtggt cagcgtcctc accgtcctgc accaggactg gctgaatggc     1020

aaggagtaca agtgcaaggt ctccaacaaa gccctcccag cccccatcga gaaaaccatc     1080

tccaaagcca aaggccagcc ccgggaacca caggtgtaca ccctgccccc atcccgggag     1140

gagatgacca agaaccaggt cagcctgacc tgcctggtca aaggcttcta tcccagcgac     1200

atcgccgtgg agtgggagag caatggccag cccgagaaca actacaagac cacccctccc     1260

gtgctggact ccgacggctc cttcttcctc tacagcaagc tcaccgtgga caagagcagg     1320

tggcagcagg gcaacgtctt ctcatgctcc gtgatgcatg aggctctgca caaccactac     1380

acccagaaga gcctctccct gtctcccggc aaa                                  1413
```

<210> 67
<211> 471
<212> PRT
<213> Artificial Sequence

<220>
<223> amino acid sequence of hH11A heavy chain full-length

<400> 67

```
Met Lys His Leu Trp Phe Phe Leu Leu Leu Val Ala Ala Pro Arg Trp
1               5                   10                  15

Val Leu Ser Glu Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Arg Asn Phe Met His Trp Val Arg Gln Ala Pro Gly Gln Gly Leu
    50                  55                  60

Glu Trp Met Gly Trp Ile Ala Pro Gly Asp Gly Glu Thr Glu Tyr Ala
65                  70                  75                  80

Gln Lys Phe Gln Gly Arg Val Thr Ile Thr Ala Asp Thr Ser Thr Ser
                85                  90                  95

Thr Ala Tyr Met Glu Leu Ser Ser Leu Arg Ser Glu Asp Thr Ala Val
            100                 105                 110
```

Tyr Tyr Cys Ala Arg Gly Val Tyr Gly Gly Phe Ala Gly Gly Tyr Phe
115 120 125

Asp Phe Trp Gly Gln Gly Thr Leu Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Arg Val Glu
225 230 235 240

Pro Lys Ser Cys Asp Lys Thr His Thr Cys Pro Pro Cys Pro Ala Pro
245 250 255

Glu Ala Ala Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 68
<211> 1413
<212> DNA
<213> Artificial Sequence

<220>
<223> nucleotide sequence of hH11A heavy chain full-length

<400> 68
atgaaacacc tgtggttctt cctcctgctg gtggcagctc ccagatgggt gctgagcgaa 60

gtgcagctgg ttcagtctgg cgccgaagtg aaaaagcctg gcgcctctgt gaaggtgtcc 120

tgcaaggcct ctggctacac attcacccgg aacttcatgc actgggtccg acaggctcca 180

ggacagggac ttgaatggat gggatggatt gctcccggcg acggcgagac agagtacgcc 240

cagaaattcc agggcagagt gaccatcacc gccgacacct ctacaagcac cgcctacatg 300

gaactgagca gcctgagaag cgaggacacc gccgtgtact attgtgccag aggcgtgtac 360

ggcggattcg ctggcggcta ctttgatttt tggggccagg gcaccctggt caccgtgagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga agccgcgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 69
<211> 471
<212> PRT
<213> Artificial Sequence

<220>
<223> amino acid sequence of hH31A heavy chain full-length

<400> 69

Met Lys His Leu Trp Phe Phe Leu Leu Leu Val Ala Ala Pro Arg Trp
1 5 10 15

Val Leu Ser Glu Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Arg Asn Phe Met His Trp Val Arg Gln Ala Pro Gly Gln Gly Leu
50 55 60

Glu Trp Met Gly Trp Ile Tyr Pro Gly Asp Gly Glu Thr Glu Tyr Ala
65 70 75 80

Ser Lys Phe Gln Gly Arg Val Thr Ile Thr Ala Asp Thr Ser Thr Ser
85 90 95

Thr Ala Tyr Met Glu Leu Ser Ser Leu Arg Ser Glu Asp Thr Ala Val
100 105 110

Tyr Tyr Cys Ala Arg Gly Val Tyr Gly Gly Ala Ala Gly Gly Tyr Phe
        115                 120                 125

Asp Phe Trp Gly Gln Gly Thr Leu Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Ala Ala Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 70
<211> 1413
<212> DNA
<213> Artificial Sequence

<220>
<223> nucleotide sequence of hH31A heavy chain full-length

<400> 70
atgaaacacc tgtggttctt cctcctgctg gtggcagctc ccagatgggt gctgagcgaa 60

gtgcagctgg ttcagtctgg cgccgaagtg aaaaagcctg gcgcctctgt gaaggtgtcc 120

tgcaaggcct ctggctacac attcacccgg aacttcatgc actgggtccg acaggctcca 180

ggacagggac ttgaatggat gggctggatc tatcccggcg acggcgagac agagtacgcc 240

agcaaatttc agggcagagt gaccatcacc gccgacacct ctacaagcac cgcctacatg 300

gaactgagca gcctgagaag cgaggacacc gccgtgtact attgtgccag aggcgtttac 360

ggcggagccg ctggcggcta ctttgatttt tggggccagg gcaccctggt caccgtgagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga agccgcgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 71
<211> 1137
<212> DNA
<213> Artificial Sequence

<220>
<223> DNA comprising DNA sequence coding for amino acid for human heavy chain signal sequence and human IgG1 LALA constant region

<400> 71
ccagcctccg gactctagag ccaccatgaa acacctgtgg ttcttcctcc tgctggtggc 60

agctcccaga tgggtgctga gccaggtgca attgtgcagg cggttagctc agcctccacc 120

aagggcccaa gcgtcttccc cctggcaccc tcctccaaga gcacctctgg cggcacagcc 180

gccctgggct gcctggtcaa ggactacttc cccgaacccg tgaccgtgag ctggaactca 240

ggcgccctga ccagcggcgt gcacaccttc cccgctgtcc tgcagtcctc aggactctac 300

tccctcagca gcgtggtgac cgtgccctcc agcagcttgg gcacccagac ctacatctgc 360

aacgtgaatc acaagcccag caacaccaag gtggacaaga gagttgagcc caaatcttgt 420

gacaaaactc acacatgccc accctgccca gcacctgaag ccgcgggggg accctcagtc 480

ttcctcttcc ccccaaaacc caaggacacc ctcatgatct cccggacccc tgaggtcaca 540

tgcgtggtgg tggacgtgag ccacgaagac cctgaggtca agttcaactg gtacgtggac 600

ggcgtggagg tgcataatgc caagacaaag ccccgggagg agcagtacaa cagcacgtac 660

cgggtggtca gcgtcctcac cgtcctgcac caggactggc tgaatggcaa ggagtacaag 720

tgcaaggtct ccaacaaagc cctcccagcc cccatcgaga aaaccatctc caaagccaaa 780

ggccagcccc gggaaccaca ggtgtacacc ctgcccccat cccgggagga gatgaccaag 840

aaccaggtca gcctgacctg cctggtcaaa ggcttctatc ccagcgacat cgccgtggag 900

tgggagagca atggccagcc cgagaacaac tacaagacca cccctcccgt gctggactcc 960

gacggctcct tcttcctcta cagcaagctc accgtggaca agagcaggtg gcagcagggc 1020

aacgtcttct catgctccgt gatgcatgag gctctgcaca accactacac ccagaagagc 1080

ctctccctgt ctcccggcaa atgagatatc gggcccgttt aaacgggga ggctaac 1137

<210> 72
<211> 234
<212> PRT
<213> Artificial Sequence

<220>
<223> humanized antibody

<220>
<221> MISC_FEATURE
<223> amino acid sequence of anti-LPS antibody (h#1G5-H1L1) light chain

<400> 72

Met Val Leu Gln Thr Gln Val Phe Ile Ser Leu Leu Leu Trp Ile Ser
1 5 10 15

Gly Ala Tyr Gly Asp Ile Val Met Thr Gln Ser Pro Asp Ser Leu Ala
20 25 30

Val Ser Leu Gly Glu Arg Ala Thr Ile Asn Cys Lys Ala Ser Glu Asn
35 40 45

Val Gly Asn Ser Val Ser Trp Tyr Gln Gln Lys Pro Gly Gln Pro Pro
50 55 60

Lys Leu Leu Ile Tyr Gly Ala Ser Asn Arg Tyr Thr Gly Val Pro Asp
65 70 75 80

Arg Phe Ser Gly Ser Gly Ser Gly Thr Asp Phe Thr Leu Thr Ile Ser
85 90 95

Ser Leu Gln Ala Glu Asp Val Ala Val Tyr Tyr Cys Gly Gln Ser Tyr
100 105 110

Ser Tyr Pro Tyr Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

```
Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```

<210> 73
<211> 474
<212> PRT
<213> Artificial Sequence

<220>
<223> humanized antibody

<220>
<221> MISC_FEATURE
<223> amino acid sequence of anti-LPS antibody (h#1G5-H1L1) heavy chain

<400> 73

```
Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr Trp Ile Asn Trp Val Arg Gln Ala Pro Gly Gln Gly Leu
    50                  55                  60

Glu Trp Met Gly Asn Ile Tyr Pro Gly Ser Ser Ser Ile Asn Tyr Asn
65                  70                  75                  80

Glu Lys Phe Lys Ser Arg Val Thr Ile Thr Ala Asp Thr Ser Thr Ser
                85                  90                  95

Thr Ala Tyr Met Glu Leu Ser Ser Leu Arg Ser Glu Asp Thr Ala Val
            100                 105                 110
```

Tyr Tyr Cys Ala Arg Thr Ile Tyr Asn Tyr Gly Ser Ser Gly Tyr Asn
115 120 125

Tyr Ala Met Asp Tyr Trp Gly Gln Gly Thr Leu Val Thr Val Ser Ser
130 135 140

Ala Ser Thr Lys Gly Pro Ser Val Phe Pro Leu Ala Pro Ser Ser Lys
145 150 155 160

Ser Thr Ser Gly Gly Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Gln Thr
210 215 220

Tyr Ile Cys Asn Val Asn His Lys Pro Ser Asn Thr Lys Val Asp Lys
225 230 235 240

Arg Val Glu Pro Lys Ser Cys Asp Lys Thr His Thr Cys Pro Pro Cys
245 250 255

Pro Ala Pro Glu Leu Leu Gly Gly Pro Ser Val Phe Leu Phe Pro Pro
260 265 270

Lys Pro Lys Asp Thr Leu Met Ile Ser Arg Thr Pro Glu Val Thr Cys
275 280 285

Val Val Val Asp Val Ser His Glu Asp Pro Glu Val Lys Phe Asn Trp
290 295 300

Tyr Val Asp Gly Val Glu Val His Asn Ala Lys Thr Lys Pro Arg Glu
305 310 315 320

Glu Gln Tyr Asn Ser Thr Tyr Arg Val Val Ser Val Leu Thr Val Leu
325 330 335

His Gln Asp Trp Leu Asn Gly Lys Glu Tyr Lys Cys Lys Val Ser Asn
340 345 350

Lys Ala Leu Pro Ala Pro Ile Glu Lys Thr Ile Ser Lys Ala Lys Gly

355 360 365

Gln Pro Arg Glu Pro Gln Val Tyr Thr Leu Pro Pro Ser Arg Glu Glu
370 375 380

Met Thr Lys Asn Gln Val Ser Leu Thr Cys Leu Val Lys Gly Phe Tyr
385 390 395 400

Pro Ser Asp Ile Ala Val Glu Trp Glu Ser Asn Gly Gln Pro Glu Asn
405 410 415

Asn Tyr Lys Thr Thr Pro Pro Val Leu Asp Ser Asp Gly Ser Phe Phe
420 425 430

Leu Tyr Ser Lys Leu Thr Val Asp Lys Ser Arg Trp Gln Gln Gly Asn
435 440 445

Val Phe Ser Cys Ser Val Met His Glu Ala Leu His Asn His Tyr Thr
450 455 460

Gln Lys Ser Leu Ser Leu Ser Pro Gly Lys
465 470